# EUROPEAN PATENT SPECIFICATION

(11) **EP 4 056 232 B1**
(45) Date of publication and mention of the grant of the patent: **06.05.2026**
(21) Application number: 20864177.9
(22) Date of filing: 11.09.2020
(51) Int. Cl.: A61P 29/00, A61P 37/06, A61P 37/08, C07K 14/715, C07K 16/00, C07K 19/00, C12N 5/10, C12N 15/12, C12N 15/62, C12N 15/63, A61K 38/16, C12P 21/02

(54) **DCR3 VARIANT**
DCR3-VARIANTE
VARIANT DE DCR3

(30) Priority: 13.09.2019 JP 2019167728
(43) Date of publication of application: 14.09.2022
(73) Proprietor: Kyowa Kirin Co., Ltd., Tokyo 100-0004 (JP)
(72) Inventor: KANAI Akiko, Tokyo 100-0004 (JP); HOSOMI Hitomi, Tokyo 100-0004 (JP); YONEZAWA Sakiko, Tokyo 100-0004 (JP)
(74) Representative: Hoffmann Eitle
(86) International application number: PCT/JP2020/034444
(87) International publication number: WO 2021/049606

(56) References cited:
- JP-A- 2001 516 581
- US-A1- 2014 286 897
- US-B2- 10 407 725
- CHENYANG ZHAN ET AL: "Decoy Strategies: The Structure of TL1A:DcR3 Complex", STRUCTURE, ELSEVIER, AMSTERDAM, NL, vol. 19, no. 2, 6 December 2010 (2010-12-06), pages 162 - 171, XP028357694, ISSN: 0969-2126, [retrieved on 20101221], DOI: 10.1016/J.STR.2010.12.004
- CHENYANG ZHAN ET AL: "Supplemental Information Decoy Strategies: The Structure of TL1A:DcR3 Complex", STRUCTURE, ELSEVIER, AMSTERDAM, NL, 9 February 2011 (2011-02-09), XP055174792
- CARDINALE C J ET AL: "Targeted resequencing identifies defective variants of decoy receptor 3 in pediatric-onset inflammatory bowel disease", GENES AND IMMUNITY, NATURE PUBLISHING GROUP, GB, vol. 14, no. 7, 22 August 2013 (2013-08-22), pages 447 - 452, XP037768627, ISSN: 1466-4879, [retrieved on 20130822], DOI: 10.1038/GENE.2013.43
- S. REBELO ET AL.: "Abstracts", CANCER RESEARCH, WILEY-BLACKWELL PUBLISHING LTD, GB, vol. 116, 9 October 2008 (2008-10-09), pages 2 - 67, XP071275174, ISSN: 0019-2805, DOI: 10.1111/J.1365-2567.2005.02287.X
- LIU WEIFENG ET AL: "Crystal Structure of the Complex of Human FasL and Its Decoy Receptor DcR3", STRUCTURE, vol. 24, no. 11, 1 November 2016 (2016-11-01), pages 2016 - 2023, XP029794121, ISSN: 0969-2126, DOI: 10.1016/J.STR.2016.09.009
- Y.-C. CHANG ET AL: "The Glycosaminoglycan-Binding Domain of Decoy Receptor 3 Is Essential for Induction of Monocyte Adhesion", THE JOURNAL OF IMMUNOLOGY, vol. 176, no. 1, 19 December 2005 (2005-12-19), US, pages 173 - 180, XP055481192, ISSN: 0022-1767, DOI: 10.4049/jimmunol.176.1.173

## Description

### BACKGROUND OF THE INVENTION

### Field of the Invention

The present invention relates to a DcR3 variant as claimed, which is a variant of wild-type DcR3. More particularly, the present invention relates to a DcR3 variant as claimed that has binding activity (preferably neutralizing activity) to a ligand of DcR3, and that results in a decreased amount of aggregates as compared to wild-type DcR3 when produced using a cell derived from a mammal as a host, and/or that exhibits improved *in vivo* kinetics.

### Background Art

A tumor necrosis factor (TNF) superfamily (TNFSF) and a TNF receptor superfamily (TNFRSF) each form 18 ligands and 29 receptor families having similar structures. Antibodies and Fc fusion proteins against many molecules included in these families have been developed and launched, and have exhibited therapeutic effects in treatment of various autoimmune diseases (Non-Patent Document 1).

Although many TNFRSFs are expressed on a cell membrane and transmit a signal downstream by ligand binding, some molecules are decoy receptors (DcRs) not involved in signaling. As the decoy receptors, four types of DcR1, DcR2, DcR3 and osteoprotegerin (OPG) have been identified.

OPG is a soluble decoy receptor for RANKL and TRAIL, and inhibits signaling by competing with the binding to ligands of a RANKL receptor and a TRAIL receptor. Meanwhile, DcR1 and DcR2 are decoy receptors for TRAIL, and DcR3 is a decoy receptor for three molecules of LIGHT, TL1A and FasL, and all of the decoy receptors neutralize ligands by competitively inhibiting the binding of the ligands to a signaling receptor (Non-Patent Document 2).

DcR3 is a soluble molecule consisting of 300 amino acid residues. The N-terminal side has a signal peptide, followed by four cysteine-rich domains (CRDs) (CRD1, CRD2, CRD3 and CRD4), which are characteristic of TNFRSF, and the C-terminal side has a heparan sulfate-binding domain (HBD), which includes a heparan sulfate-binding motif and is rich in basic amino acids. All of LIGHT, TL1A and FasL bind via CRD2 and CRD3 of DcR3 (Non-Patent Documents 3, 4 and 5).

DcR3 has, in addition to a function as a decoy receptor by ligand neutralization, a function as an immunomodulatory molecule based on the activity of HBD. For example, it has been reported that DcR3 directly binds via HBD to glycosaminoglycan (GAG) including heparan sulfate on the cell membrane of monocytes, macrophages or dendritic cells, thus initiating various immunosuppressive and immunostimulatory effects such as Th2 induction by differentiation of dendritic cells, induction of M2 macrophages, enhancement of adhesion of monocytes, osteoclast differentiation, or decreased expression of MHC class II molecules (Non-Patent Documents 6 and 12).

DcR3 ligands have been reported to be involved in autoimmune diseases, inflammatory diseases, allergy, cancer, infection or other various inflammation reactions. For example, all of LIGHT, TL1A and FasL are included in the susceptibility locus in inflammatory bowel disease (IBD), and particularly, regarding TL1A, existence of a plurality of gene polymorphisms associated with pathological conditions has been known. There have also been reports of increased expression of DcR3 ligands in the blood or tissue of IBD patients, and improvement in pathological conditions by inhibition of DcR3 ligands in a mouse enteritis model (Non-Patent Documents 6 to 9).

Although expression of DcR3 in human normal tissues is at an extremely low level, the expression is induced by infection or tissue damage. Furthermore, it has been known that the level of DcR3 in blood is increased in various autoimmune diseases or inflammatory diseases such as IBD, systemic lupus erythematosus (SLE), atopic dermatitis (AD) or rheumatoid arthritis (RA). Although no DcR3 homolog has been identified in mice, improvement in pathological conditions in human DcR3 transgenic mice and drug efficacy by administration of plasmids or recombinant DcR3 have been confirmed in mouse disease models such as a type I diabetes mellitus model, a multiple sclerosis model or a nephritis model (Non-Patent Documents 6 and 10).

Genentech Inc. cloned the human DcR3 gene, and showed that a fusion of DcR3 and the Fc region of human IgG1 binds to soluble human FasL and inhibits human FasL-dependent apoptosis in vitro (Patent Document 1).

Eli Lilly and Company obtained FLINT corresponding to a protease-resistant DcR3 mutant obtained by introducing one-amino-acid mutation (R218Q) into wild-type DcR3, and reported that the *in vivo* kinetics thereof is more improved in mice and monkeys than that of wild-type DcR3. However, the half-lives in blood when wild-type DcR3 and FLINT were administered at 0.5 mg/kg by intravenous injection to cynomolgus monkeys were extremely short, with values of 9 hours or 12.3 hours, respectively (Patent Documents 2 and 3, Non-Patent Document 11).

### Prior Art Document

### [Patent Document]

Patent Document 1: JP 4303883 B2
Patent Document 2: US 6,835,814 B1
Patent document 3: US 6,965,012 B1
Patent document 4: US 10 407 725 relates to methods of treating autoimmune conditions in patients with genetic variations in DcR3 or in a DcR3 network gene
Patent document 5: US 2014/286897 relates to compositions and methods for the Regulation of T Regulatory Cells Using TL1A-Ig Fusion Protein

### [Non-Patent Document]

Non-Patent Document 1: Nature Reviews Drug Discovery, 2013, 12: p.147-168
Non-Patent Document 2: Nature Reviews Cancer, 2002.2: p.420-430
Non-Patent Document 3: Structure, 2011, 19: p.162-171
Non-Patent Document 4: Structure, 2014, 22: p.1252-1262
Non-Patent Document 5: Structure, 2016, 24: p.2016-2023
Non-Patent Document 6: Biochemical Pharmacology, 2011, 81: p.838-847
Non-Patent Document 7: Immunology, 2009, 128: p.451-458
Non-Patent Document 8: P.N.A.S., 2006, 103: p.8441-8446
Non-Patent Document 9: Am. J. Physiol. Gastrointest. Liver Physiol., 2003, 285: p.G754-G760
Non-Patent Document 10: Journal of Biomedical Science, 2017, 24:39
Non-Patent Document 11: Drug Metabolism and Disposition, 2003, 31: p.502-507
Non-Patent Document 12: J. Immunol., 2006, 176: p.173-180
Non-patent document 13, Cardinale et al., Genes and Immunity, vol. 14, no. 7, 2013, p 447-452 relates to targeted resequencing identifies defective variants of decoy receptor 3 in pediatric-onset inflammatory bowel disease
Non-patent document 14, Immunology 2005, 116, Supplement 1, abstract 16.2,p 2-67 relates to conformational defects in mutant forms of tumour necrosis factor receptor I that occur in TNF-receptor-associated periodic syndrome

### SUMMARY OF THE INVENTION

FLINT, which is an amino acid variant of wild-type DcR3, has markedly poor *in vivo* kinetics, and requires frequent administration as a recombinant formulation whose mechanism of action is ligand neutralization, and this is undesirable as a pharmaceutical product. Therefore, a DcR3 variant in which a certain administration interval can be ensured by improvement in the *in vivo* kinetics is expected to be useful as a pharmaceutical product.

As far as is known to date, there has not been developed a functional DcR3 variant that results in a decreased amount of aggregates when expressed, isolated and purified using a cell derived from a mammal as a host, and that has neutralizing activity to a DcR3 ligand.

An object of the present invention is to provide a DcR3 variant as claimed that has binding activity (preferably neutralizing activity) to a ligand of DcR3, and that results in a decreased amount of aggregates as compared to wild-type DcR3 when a DcR3 protein is produced using a cell derived from a mammal as a host, and/or that exhibits improved *in vivo* kinetics; a DNA encoding the DcR3 variant; a vector including the DNA; a transformant obtained by introducing the vector; a method for producing the variant using the transformant; and a pharmaceutical composition and a prophylactic or therapeutic agent for an autoimmune disease, an inflammatory disease or allergy including the variant as an active ingredient.

The abovementioned object is provided by the invention, which is set out in the claims.

According to the present invention, there is provided a DcR3 variant as claimed that has binding activity (preferably neutralizing activity) to a ligand of DcR3, and that results in a decreased amount of aggregates as compared to wild-type DcR3 when a DcR3 protein is produced using a cell derived from a mammal as a host, and/or that exhibits improved *in vivo* kinetics; a DNA encoding the DcR3 variant; a vector including the DNA; a transformant obtained by introducing the vector; a method for producing the variant using the transformant; and a pharmaceutical composition and a prophylactic or therapeutic agent for an autoimmune disease, an inflammatory disease or allergy including the variant as an active ingredient.

### BRIEF DESCRIPTION OF THE DRAWINGS

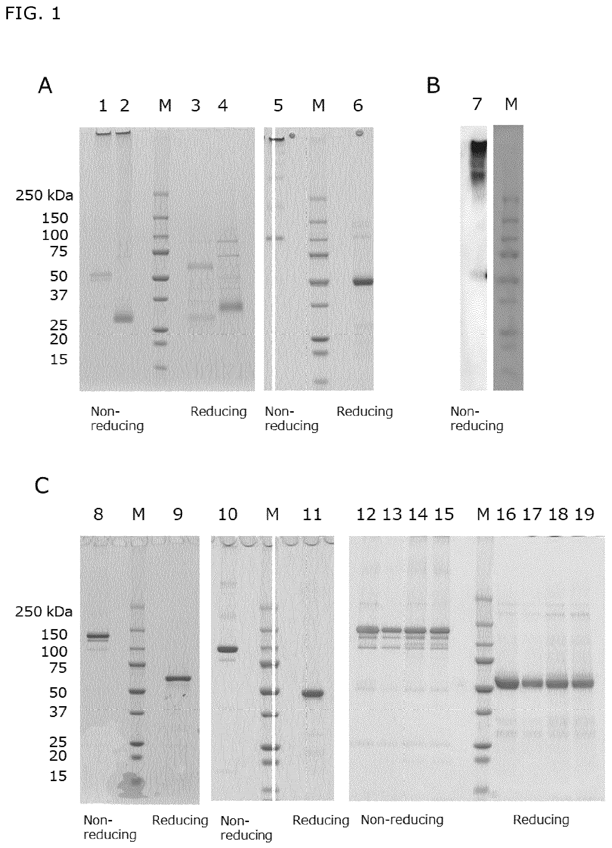

FIG. 1A shows results of SDS-PAGE of various wild-type DcR3 controls produced using mammalian cells. DcR3 FL-Fc in lanes 1 and 3, DcR3 FL-FLAG in lanes 2 and 4, and S195-Fc (g1S) in lanes 5 and 6 each were electrophoresed under non-reducing or reducing conditions. FIG. 1B shows that commercially available human DcR3-Fc produced using an HEK293 cell (lane 7) was electrophoresed under non-reducing conditions and detected by immunoblotting using an anti-human IgG antibody. FIG. 1C shows that commercially available DcR3 FL-Fc (lanes 8 and 9), S195-Fc (g1S) (lanes 10 and 11), DcR3 FL-Fc (g1S) (lanes 14, 15, 18 and 19) and R218Q-Fc(g1S) (lanes 12, 13, 16 and 17) produced using insect cells each were electrophoresed under non-reducing or reducing conditions. M represents a molecular weight marker (Bio-Rad Laboratories, Inc.).

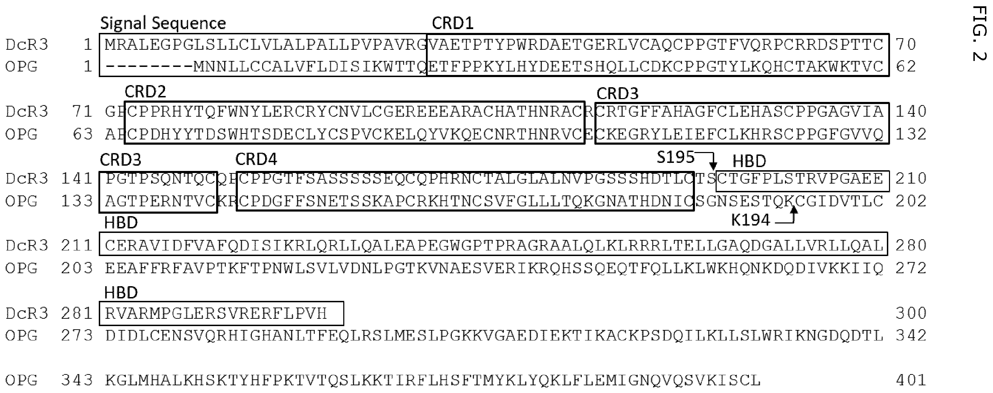

FIG. 2 shows cysteine-rich domains each represented by CRD1, CRD2, CRD3 and CRD4 in the alignment of premature amino acid sequences of human DcR3 and human OPG.

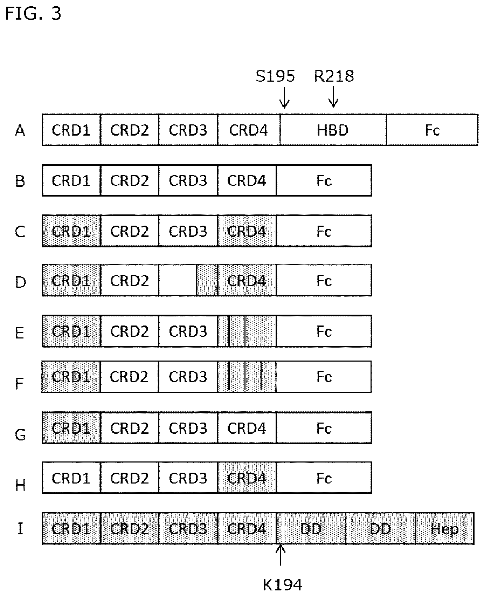

FIG. 3 schematically shows respective domain structures of various wild-type DcR3 controls and various DcR3 variants produced and human OPG. A: DcR3 FL-Fc, B: S195-Fc, C: chimera A-Fc, D: 103-123OPG-Fc, E: N-linked glycan disubstitute-Fc, F: N-linked glycan trisubstitute-Fc, G: chimera B-Fc, H: chimera C-Fc, I: OPG. The vertical lines of CRD4 in E and F represent existence of two or three substitutions of N-linked glycosylated residues, respectively, and DD in I represents a death domain.

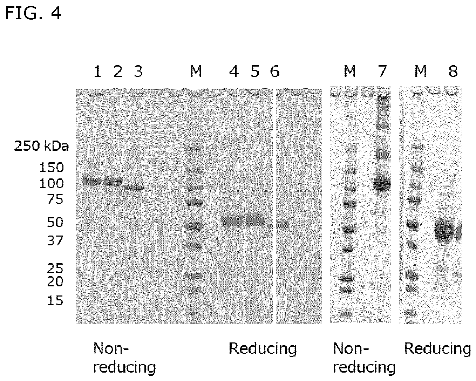

FIG. 4 shows results of SDS-PAGE of various DcR3 variants produced using mammalian cells. Chimera A-Fc (IEGRMD g1S) produced using an Expi293 cell in lanes 1 and 4, chimera A-Fc (IEGRMD g1S) produced using a CHO-S cell in lanes 2 and 5, chimera B-Fc (g1S) produced using an Expi293 cell in lanes 3 and 6, and chimera C-Fc (IEGRMD g15) produced using an Expi293 cell in lanes 7 and 8 were electrophoresed under non-reducing or reducing conditions. M represents a molecular weight marker (Bio-Rad Laboratories, Inc.).

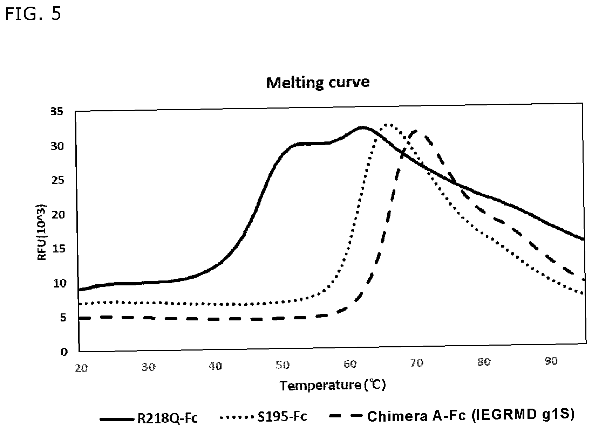

FIG. 5 shows melting curves by the DSF method for R218Q-Fc, S195-Fc and chimera A-Fc (IEGRMD g1S). The vertical axis represents fluorescence intensity RFU (10³), and the horizontal axis represents temperature (°C).

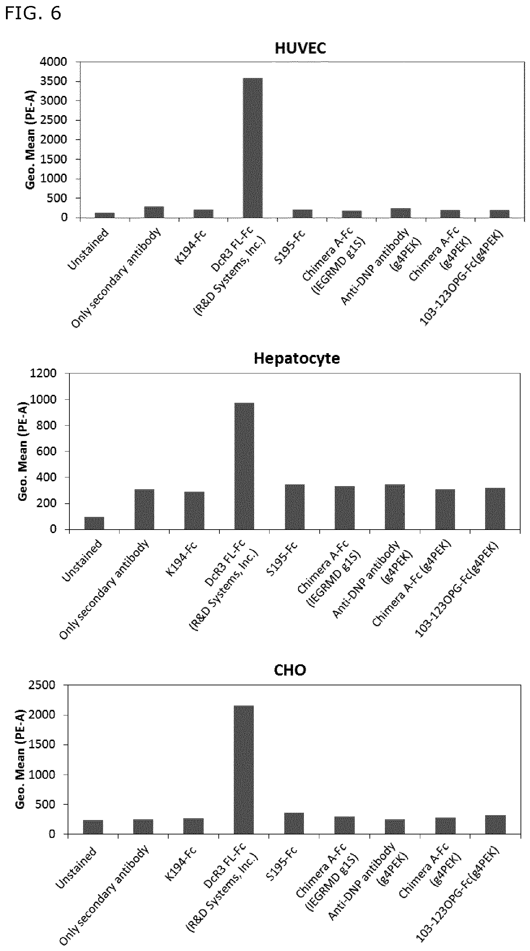

FIG. 6 shows the bindings of various wild-type DcR3 controls and various DcR3 variants to human primary cells and CHO cells. To each cell, 10 µg/mL of each DcR3 variant was reacted, followed by staining with 0.1 µg/mL of a PE-labeled anti-human antibody, and the fluorescence intensity of PE was measured by flow cytometry. The vertical axis of the graph represents the geometric mean (Geo. Mean) of PE. The upper graph represents results of staining of HUVECs, the middle graph represents results of staining of hepatocytes, and the lower graph represents results of staining of CHO cells.

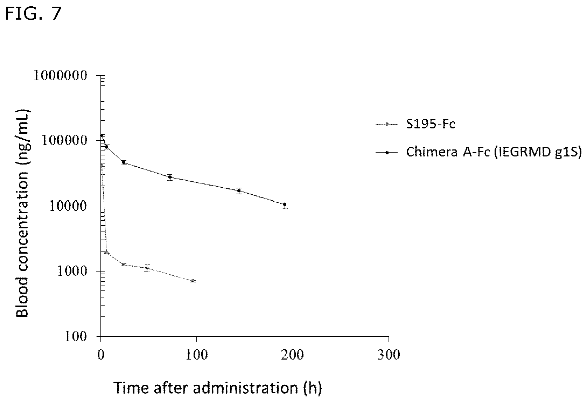

FIG. 7 shows the time course of blood concentration after i.v. administration of 10 mg/kg of S195-Fc and chimera A-Fc (IEGRMD g1S) in BALB/c mice. The vertical axis represents blood concentration (ng/mL), and the horizontal axis represents time after administration (h).

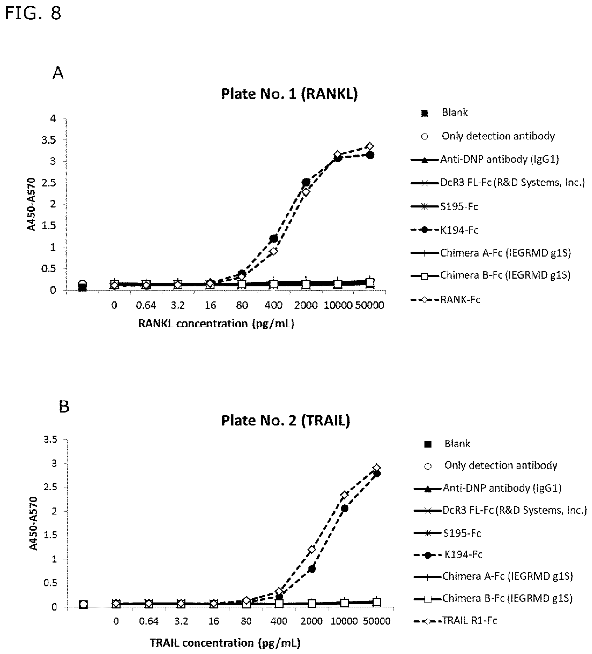

FIG. 8A and FIG. 8B show the bindings of various wild-type DcR3 controls and various DcR3 variants to RANKL (OPG ligand) and TRAIL (OPG ligand), respectively. After various wild-type DcR3 controls and various DcR3 variants were captured on plates to which anti-human antibodies were immobilized, RANKL or TRAIL diluted to each concentration was added, and the bindings were evaluated. For detection, a biotinylated anti-RANKL antibody or a biotinylated anti-TRAIL antibody and streptavidin-HRP were used. The horizontal axis represents the concentration (pg/mL) of RANKL or TRAIL, and the vertical axis represents absorbance (value obtained by subtracting the absorbance at 570 nm from the absorbance at 450 nm).

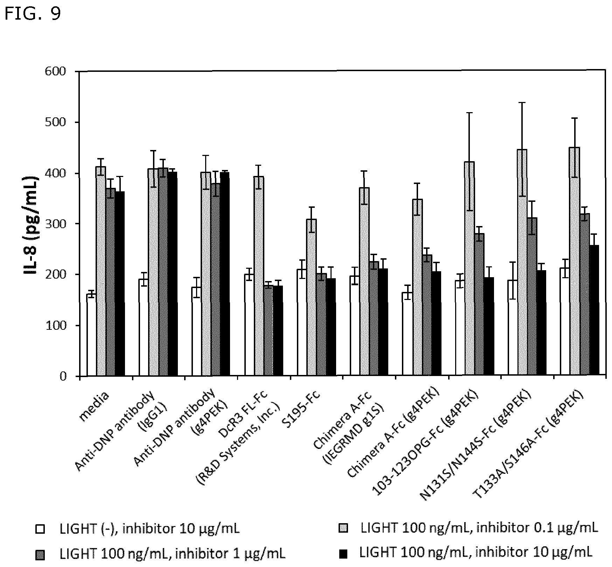

FIG. 9 shows the neutralizing activities of various wild-type DcR3 controls and various DcR3 variants to LIGHT. Production of IL-8 from HT-29 cells when 100 ng/mL of LIGHT and 0.1, 1 or 10 µg/mL of various wild-type DcR3 controls and various DcR3 variants were added is shown. The vertical axis represents IL-8 concentration (pg/mL), and the horizontal axis represents various DcR3 variants added as inhibitors.

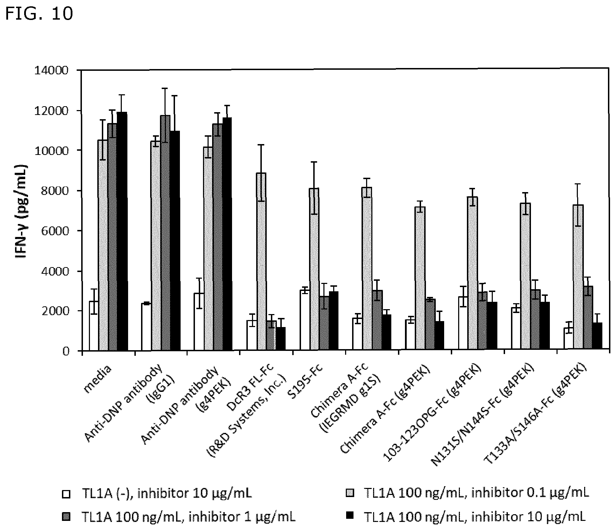

FIG. 10 shows the neutralizing activities of various wild-type DcR3 controls and various DcR3 variants to TL1A. Production of IFN-γ from human T cells when 100 ng/mL of TL1A and 0.1, 1 or 10 µg/mL of various wild-type DcR3 controls and various DcR3 variants were added is shown. The vertical axis represents IFN-γ concentration (pg/mL), and the horizontal axis represents various DcR3 variants added as inhibitors.

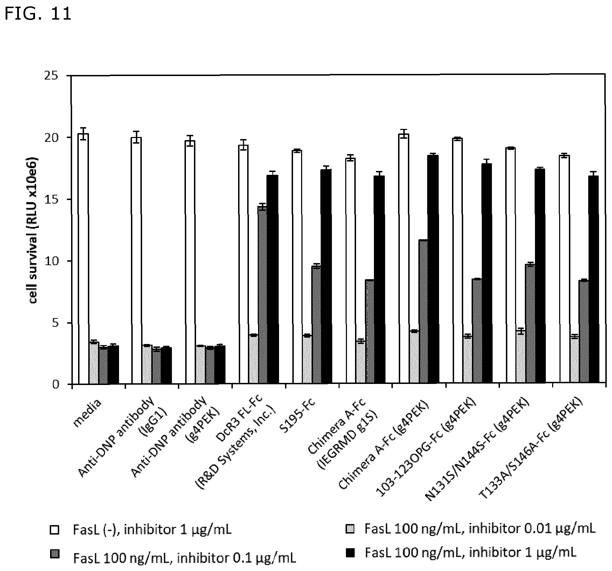

FIG. 11 shows the neutralizing activities of various wild-type DcR3 controls and various DcR3 variants to FasL. The production amount of ATP derived from Jurkat viable cells when 100 ng/mL of FasL and 0.01, 0.1 or 1 µg/mL of various wild-type DcR3 controls and various DcR3 variants were added is represented by RLU. The vertical axis represents cell survival (RLU × 10⁶) using ATP-dependent chemiluminescence as an index, and the horizontal axis represents various DcR3 variants added as inhibitors.

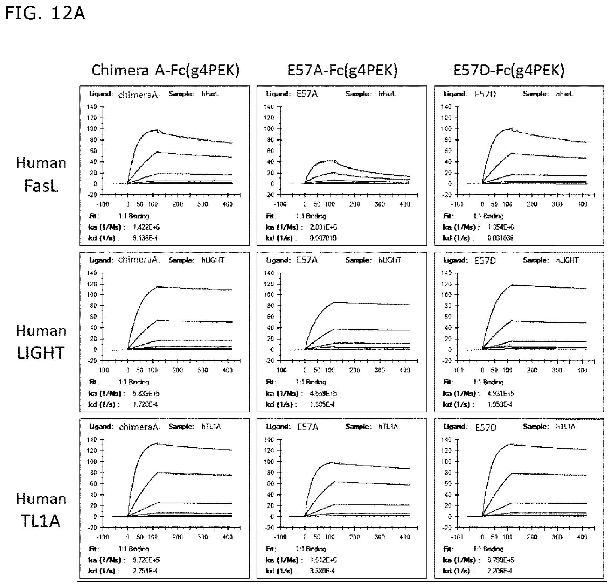

FIG. 12A shows the bindings of chimera A-Fc (g4PEK) and the variants (g4PEK) with low FasL binding activities to each DcR3 ligand. Sensorgrams when chimera A-Fc (g4PEK) or each of the variants with low FasL binding activities was captured on a sensor chip to which anti-human antibodies were immobilized, and a DcR3 ligand (human FasL, human LIGHT or human TL1A) as an analyte was injected are shown. The vertical axis represents resonance unit (RU), and the horizontal axis represents time (sec).

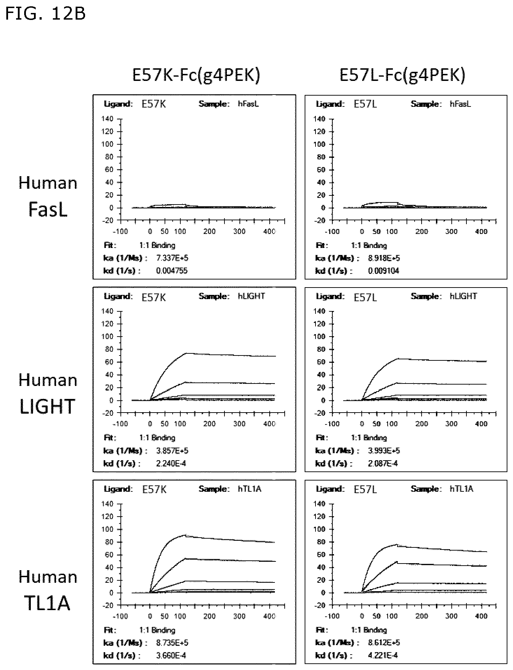

FIG. 12B shows the bindings of the variants (g4PEK) with low FasL binding activities to each DcR3 ligand. Sensorgrams when each of the variants with low FasL binding activities was captured on a sensor chip to which anti-human antibodies were immobilized, and a DcR3 ligand (human FasL, human LIGHT or human TL1A) as an analyte was injected are shown. The vertical axis represents resonance unit (RU), and the horizontal axis represents time (sec).

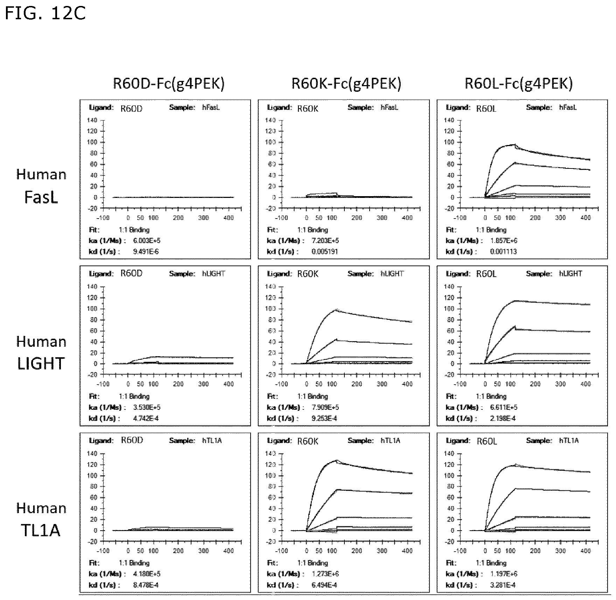

FIG. 12C shows the bindings of the variants (g4PEK) with low FasL binding activities to each DcR3 ligand. Sensorgrams when each of the variants with low FasL binding activities was captured on a sensor chip to which anti-human antibodies were immobilized, and a DcR3 ligand (human FasL, human LIGHT or human TL1A) as an analyte was injected are shown. The vertical axis represents resonance unit (RU), and the horizontal axis represents time (sec).

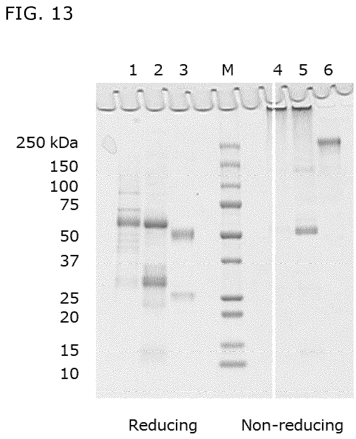

FIG. 13 shows results of SDS-PAGE of wild-type DcR3 controls produced using various mammalian cells. SDS-PAGE was performed by electrophoresing commercially available human DcR3-Fc produced using various mammalian cells under reducing or non-reducing conditions. As the mammalian cells, HEK293 cells (Abcam plc.) were used in lanes 1 and 4, CHO cells (AdipoGen Life Sciences, Inc.) were used in lanes 2 and 5, and HEK293 cells (Enzo Life Sciences, Inc.) were used in lanes 3 and 6. M represents a molecular weight marker (Bio-Rad Laboratories, Inc.).

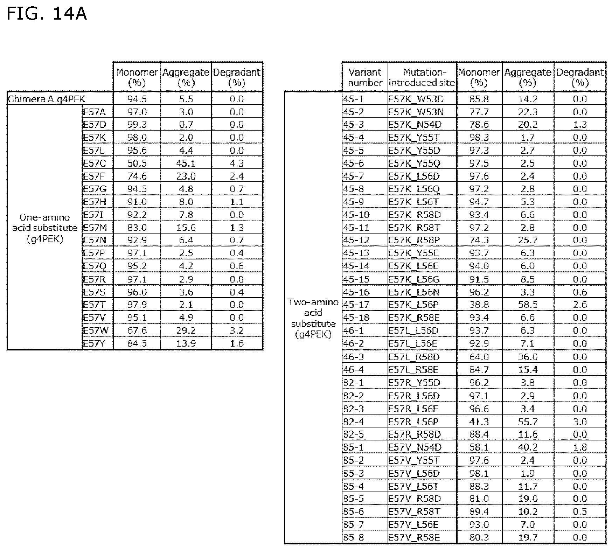

FIG. 14A shows the proportions (%) of the contents of monomers, aggregates and degradants calculated from the peak areas in SEC-HPLC or SEC-UPLC of the Protein A-purified variants with low FasL binding activities. FIG. 14A shows results of all the produced variants (g4PEK) with low FasL binding activities.

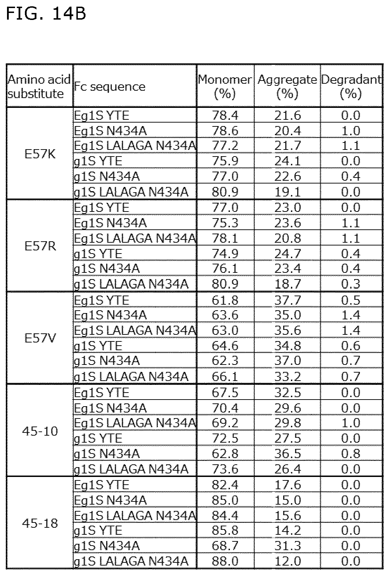

FIG. 14B shows the proportions (%) of the contents of monomers, aggregates and degradants calculated from the peak areas in SEC-HPLC or SEC-UPLC of the Protein A-purified variants with low FasL binding activities. FIG. 14B shows results of various mutated Fc fusions of the selected variants with low FasL binding activities.

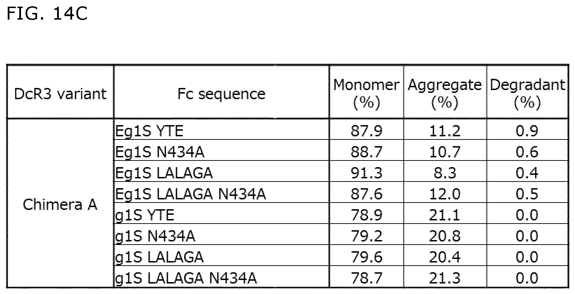

FIG. 14C shows the proportions (%) of the contents of monomers, aggregates and degradants calculated from the peak areas in SEC-HPLC or SEC-UPLC of various Protein A-purified chimera A-mutated Fc fusions.

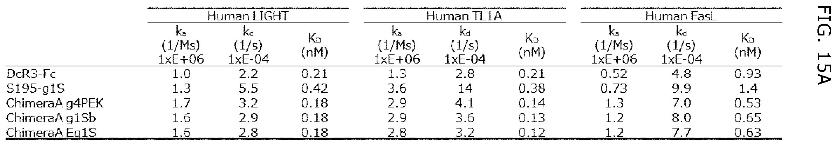

FIG. 15A shows measurement results of the binding activities of various DcR3 variants to each DcR3 ligand by BIAcore. Various kinetic constants (ka, kd and K_{D}) when various DcR3 variants were captured on a sensor chip to which anti-human antibodies were immobilized, and a trimeric DcR3 ligand (human FasL, human LIGHT or human TL1A) as an analyte was injected are shown. FIG. 15A shows results of various chimera A-Fc variants including different Fc sequences.

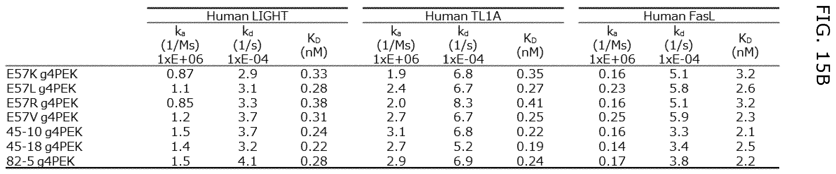

FIG. 15B shows measurement results of the binding activities of various DcR3 variants to each DcR3 ligand by BIAcore. Various kinetic constants (ka, kd and KD) when various DcR3 variants were captured on a sensor chip to which anti-human antibodies were immobilized, and a trimeric DcR3 ligand (human FasL, human LIGHT or human TL1A) as an analyte was injected are shown. FIG. 15B shows results of the variants with low FasL binding activities.

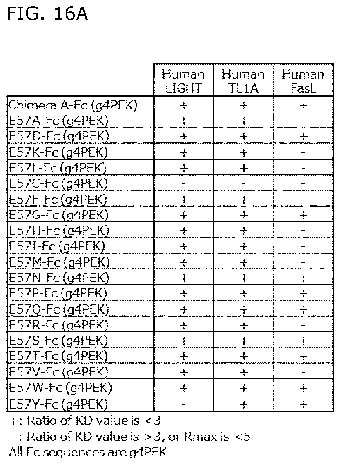

FIG. 16A shows results of the kinetic constant (KD value) to each DcR3 ligand calculated by BIAcore at selection of the variants with low FasL binding activities, compared to that of chimera A-Fc (g4PEK). FIG. 16A shows results of one-amino-acid substitutes.

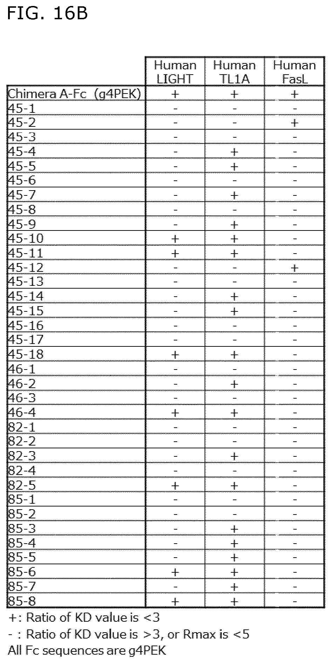

FIG. 16B shows results of the kinetic constant (KD value) to each DcR3 ligand calculated by BIAcore at selection of the variants with low FasL binding activities, compared to that of chimera A-Fc (g4PEK). FIG. 16B shows results of two-amino-acid substitutes.

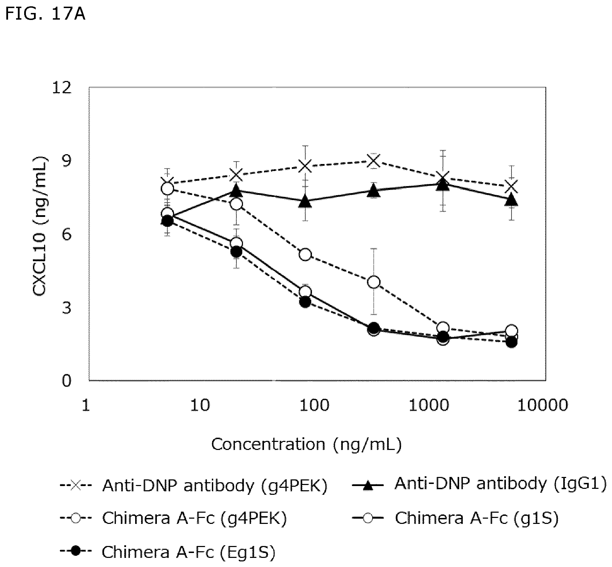

FIG. 17A shows evaluation results of the neutralizing activities of various DcR3 variants to soluble LIGHT. FIG. 17A shows the inhibitory activities of various chimera A-Fc variants including different Fc sequences to LIGHT-dependent-CXCL10 production from IFN-γ-stimulated intestinal myofibroblasts. The vertical axis represents CXCL10 concentration (ng/mL), and the horizontal axis represents the concentration (ng/mL) of DcR3 variants added.

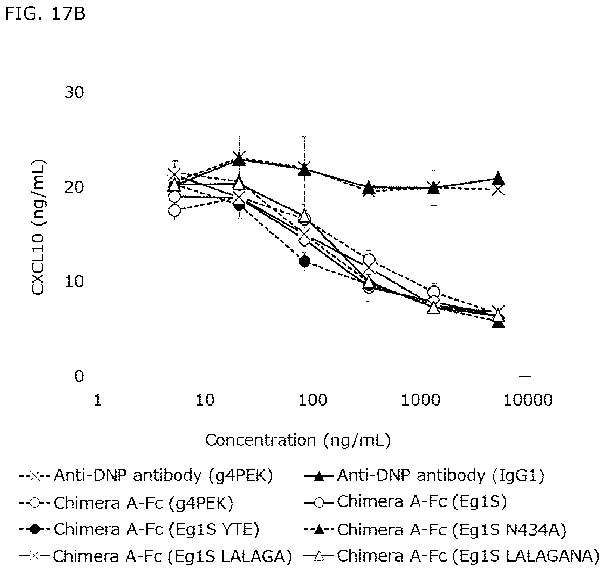

FIG. 17B shows evaluation results of the neutralizing activities of various DcR3 variants to soluble LIGHT. FIG. 17B shows the inhibitory activities of chimera A-Fc variants including different mutation-introduced Fc sequences to LIGHT-dependent CXCL10 production from IFN-γ-stimulated intestinal myofibroblasts. The vertical axis represents CXCL10 concentration (ng/mL), and the horizontal axis represents the concentration (ng/mL) of DcR3 variants added.

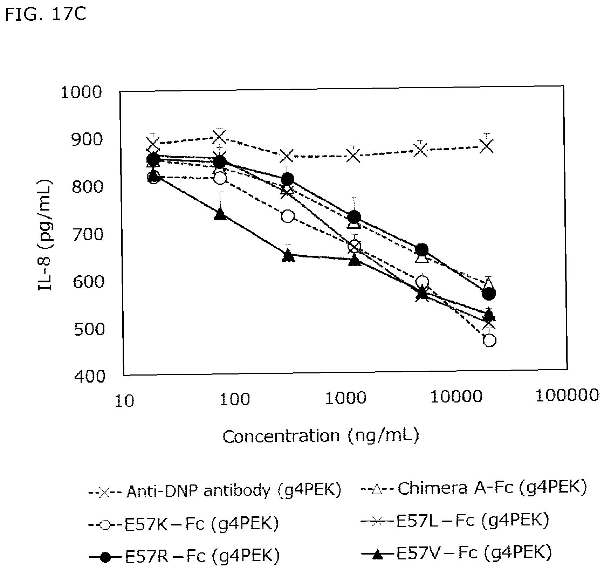

FIG. 17C shows evaluation results of the neutralizing activities of various DcR3 variants to soluble LIGHT. FIG. 17C shows the inhibitory activities of one-amino-acid substituted variants with low FasL binding activites to LIGHT-dependent IL-8 production from HT-29 cells. The vertical axis represents IL-8 concentration (ng/mL), and the horizontal axis represents the concentration (ng/mL) of DcR3 variants added.

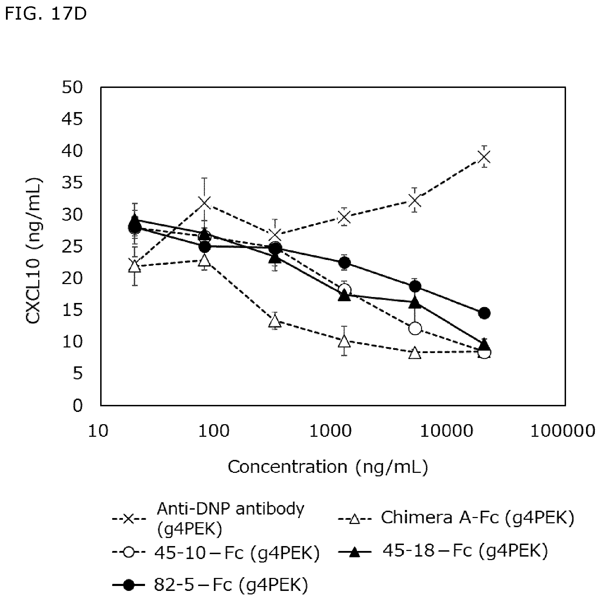

FIG. 17D shows evaluation results of the neutralizing activities of various DcR3 variants to soluble LIGHT. FIG. 17D shows the inhibitory activities of two-amino-acid substituted variants with low FasL binding activities to LIGHT-dependent CXCL10 production from IFN-γ-stimulated intestinal myofibroblasts. The vertical axis represents CXCL10 concentration (ng/mL), and the horizontal axis represents the concentration (ng/mL) of DcR3 variants added.

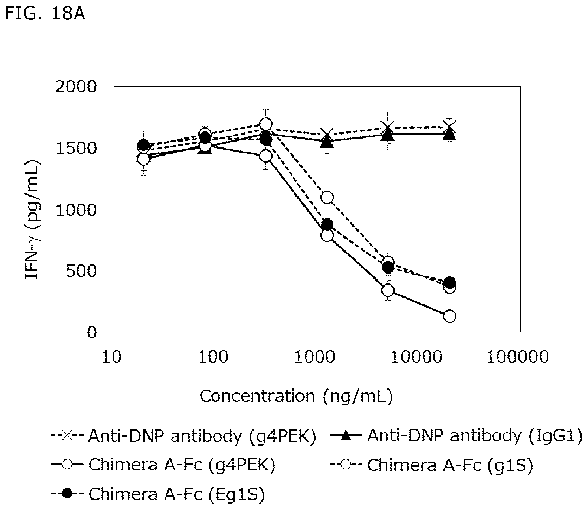

FIG. 18A shows evaluation results of the neutralizing activities of various DcR3 variants to soluble TL1A. FIG. 18A shows the inhibitory activities of various chimera A-Fc variants including different Fc sequences to TL1A-dependent IFN-γ production from IL-12- and IL-18-stimulated human T cells. The vertical axis represents IFN-γ concentration (pg/mL), and the horizontal axis represents the concentration (ng/mL) of DcR3 variants added.

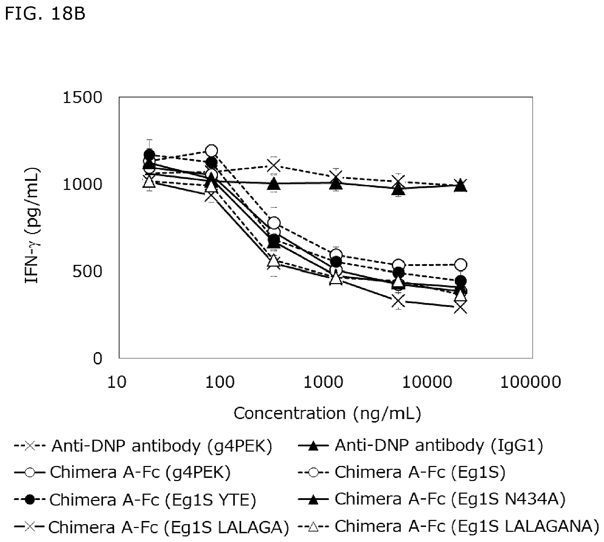

FIG. 18B shows evaluation results of the neutralizing activities of various DcR3 variants to soluble TL1A. FIG. 18B shows the inhibitory activities of chimera A-Fc variants including different mutation-introduced Fc sequences to TL1A-dependent IFN-γ production from IL-12- and IL-18-stimulated human T cells. The vertical axis represents IFN-γ concentration (pg/mL), and the horizontal axis represents the concentration (ng/mL) of DcR3 variants added.

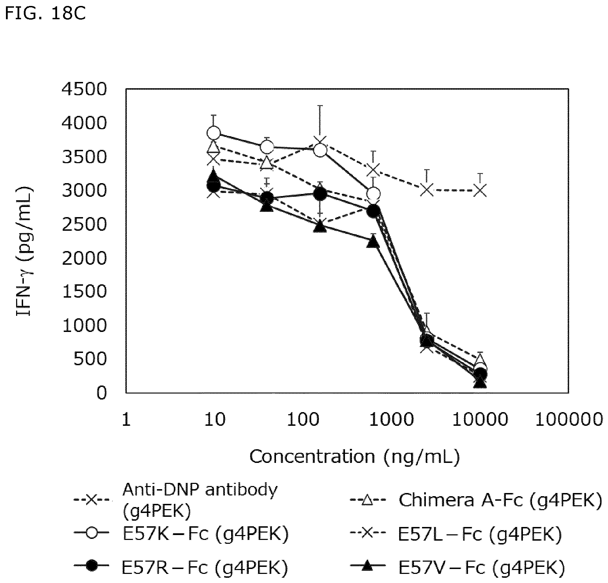

FIG. 18C shows evaluation results of the neutralizing activities of various DcR3 variants to soluble TL1A. FIG. 18C shows the inhibitory activities of one-amino-acid substituted variants with low FasL binding activities to TL1A-dependent IFN-γ production from IL-12- and IL-18-stimulated human T cells. The vertical axis represents IFN-γ concentration (pg/mL), and the horizontal axis represents the concentration (ng/mL) of DcR3 variants added.

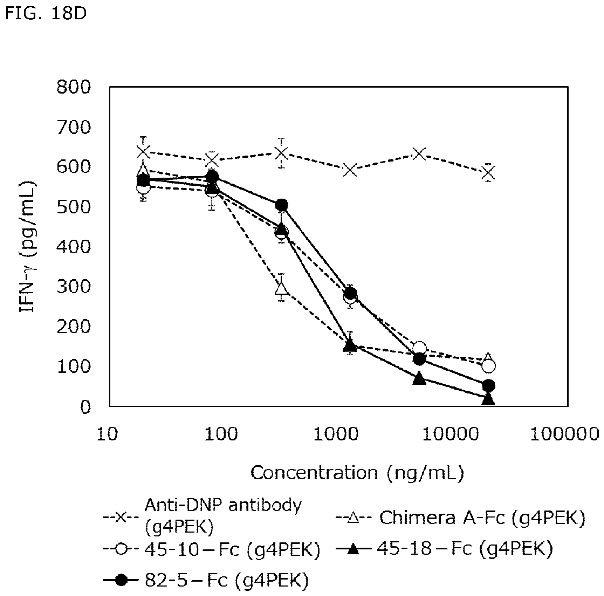

FIG. 18D shows evaluation results of the neutralizing activities of various DcR3 variants to soluble TL1A. FIG. 18D shows the inhibitory activities of two-amino-acid substituted variants with low FasL binding activities to TL1A-dependent IFN-γ production from IL-12- and IL-18-stimulated human T cells. The vertical axis represents IFN-γ concentration (pg/mL), and the horizontal axis represents the concentration (ng/mL) of DcR3 variants added.

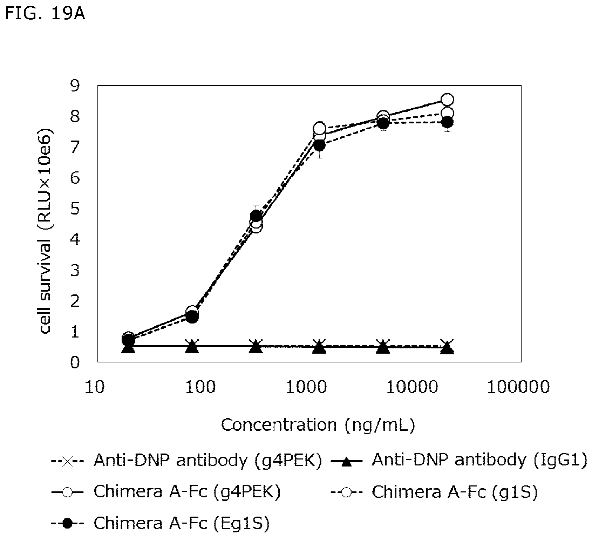

FIG. 19A shows evaluation results of the neutralizing activities of various DcR3 variants to soluble FasL. FIG. 19A shows the inhibitory activities of various chimera A-Fc variants including different Fc sequences to cell death of A3 cells. The vertical axis represents cell survival (RLU × 10⁶) using ATP-dependent chemiluminescence as an index, and the horizontal axis represents the concentration (ng/mL) of various DcR3 variants added.

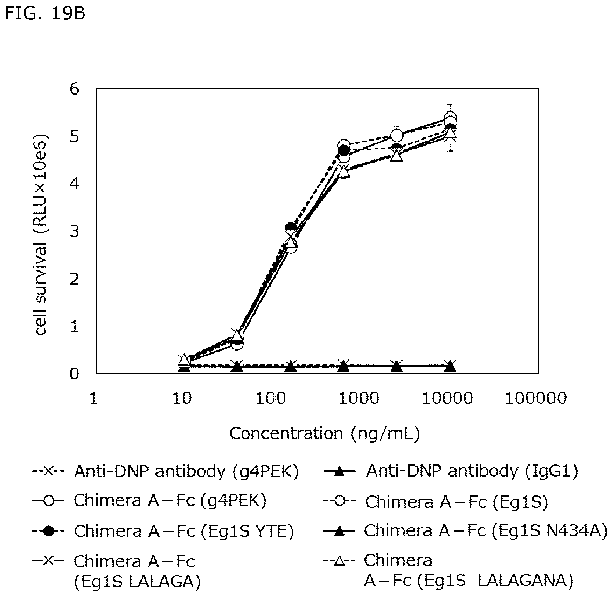

FIG. 19B shows evaluation results of the neutralizing activities of various DcR3 variants to soluble FasL. FIG. 19B shows the inhibitory activities of chimera A-Fc variants including different mutation-introduced Fc sequences to cell death of Jurkat cells. The vertical axis represents cell survival (RLU × 10⁶) using the production amount of ATP as an index, and the horizontal axis represents the concentration (ng/mL) of various DcR3 variants added.

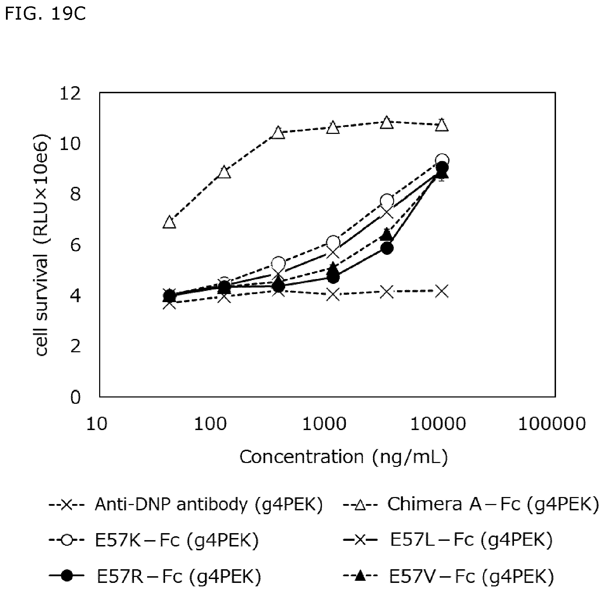

FIG. 19C shows evaluation results of the neutralizing activities of various DcR3 variants to soluble FasL. FIG. 19C shows the inhibitory activities of one-amino-acid substituted variants with low FasL binding activities to cell death of Jurkat cells. The vertical axis represents cell survival (RLU × 10⁶) using the production amount of ATP as an index, and the horizontal axis represents the concentration (ng/mL) of various DcR3 variants added.

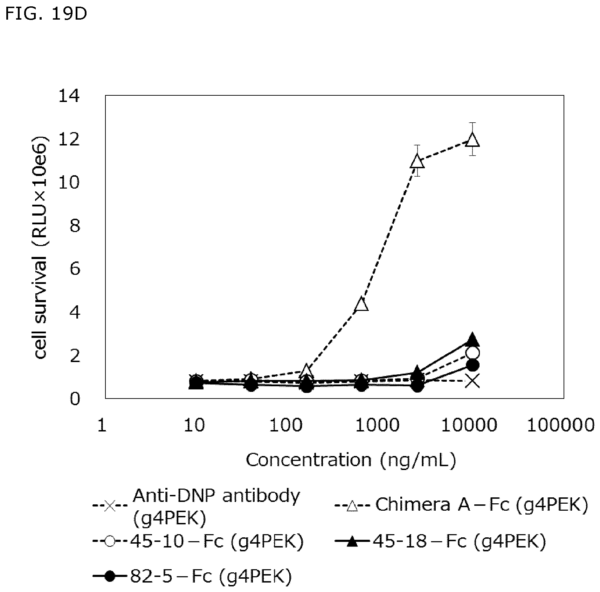

FIG. 19D shows evaluation results of the neutralizing activities of various DcR3 variants to soluble FasL. FIG. 19D shows the inhibitory activities of two-amino-acid substituted variants with low FasL binding activities to cell death of Jurkat cells. The vertical axis represents cell survival (RLU × 10⁶) using the production amount of ATP as an index, and the horizontal axis represents the concentration (ng/mL) of various DcR3 variants added.

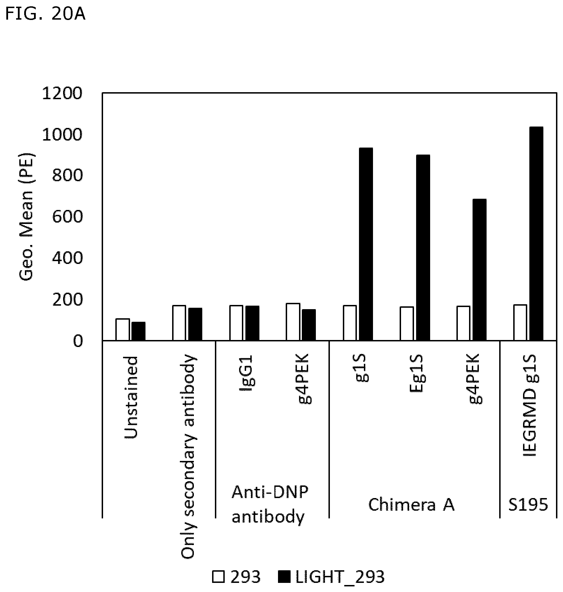

FIG. 20A shows evaluation results of the binding activities of S195-Fc and various chimera A-Fc variants including different Fc sequences to cells of a membrane-bound LIGHT forced expression cell line. To each cell, various DcR3 variants were reacted, followed by staining with a PE-labeled anti-human antibody, and the fluorescence intensity of PE was measured by flow cytometry. The vertical axis of the graph represents the geometric mean (Geo. Mean) of PE.

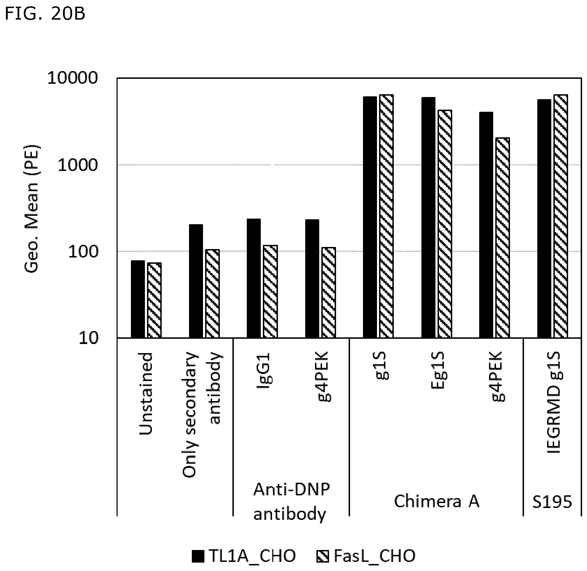

FIG. 20B shows evaluation results of the binding activities of S195-Fc and various chimera A-Fc variants including different Fc sequences to cells of membrane-bound TL1A and membrane-bound FasL forced expression cell lines. To each cell, various DcR3 variants were reacted, followed by staining with a PE-labeled anti-human antibody, and the fluorescence intensity of PE was measured by flow cytometry. The vertical axis of the graph represents the geometric mean (Geo. Mean) of PE.

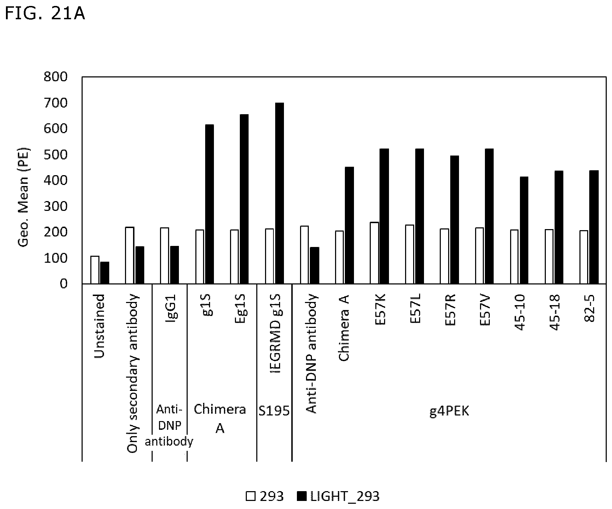

FIG. 21A shows evaluation results of the binding activities of S195-Fc, various chimera A-Fc variants and the variants with low FasL binding activities to cells of a membrane-bound LIGHT forced expression cell line. To each cell, various DcR3 variants were reacted, followed by staining with a PE-labeled anti-human antibody, and the fluorescence intensity of PE was measured by flow cytometry. The vertical axis of the graph represents the geometric mean (Geo. Mean) of PE.

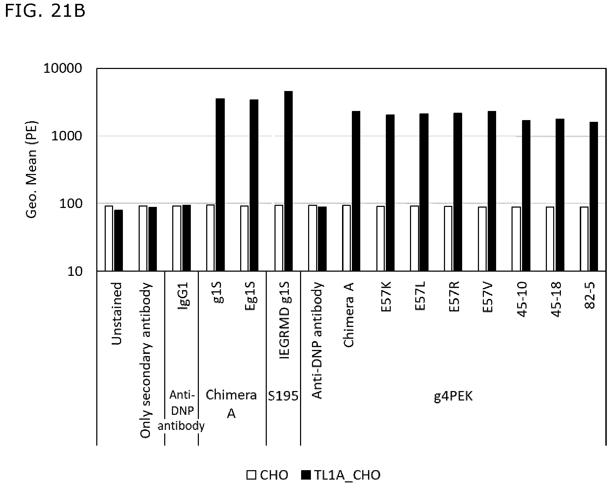

FIG. 21B shows evaluation results of the binding activities of S195-Fc, various chimera A-Fc variants and the variants with low FasL binding activities to cells of a membrane-bound TL1A forced expression cell line. To each cell, various DcR3 variants were reacted, followed by staining with a PE-labeled anti-human antibody, and the fluorescence intensity of PE was measured by flow cytometry. The vertical axis of the graph represents the geometric mean (Geo. Mean) of PE.

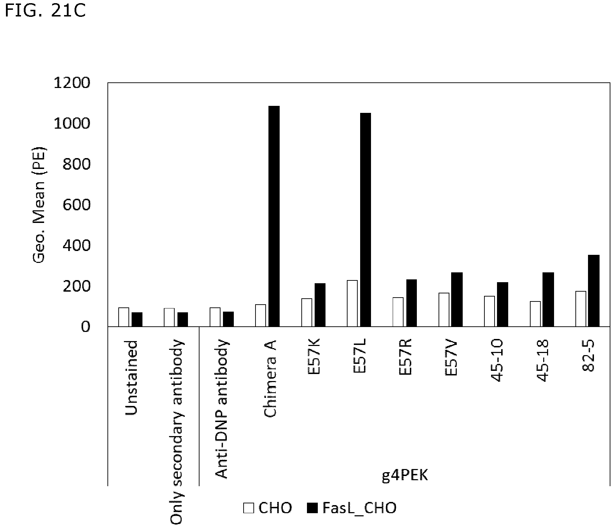

FIG. 21C shows evaluation results of the binding activities of chimera A-Fc and the variants (g4PEK) with low FasL binding activities to cells of a membrane-bound FasL forced expression cell line. To each cell, various DcR3 variants were reacted, followed by staining with a PE-labeled anti-human antibody, and the fluorescence intensity of PE was measured by flow cytometry. The vertical axis of the graph represents the geometric mean (Geo. Mean) of PE.

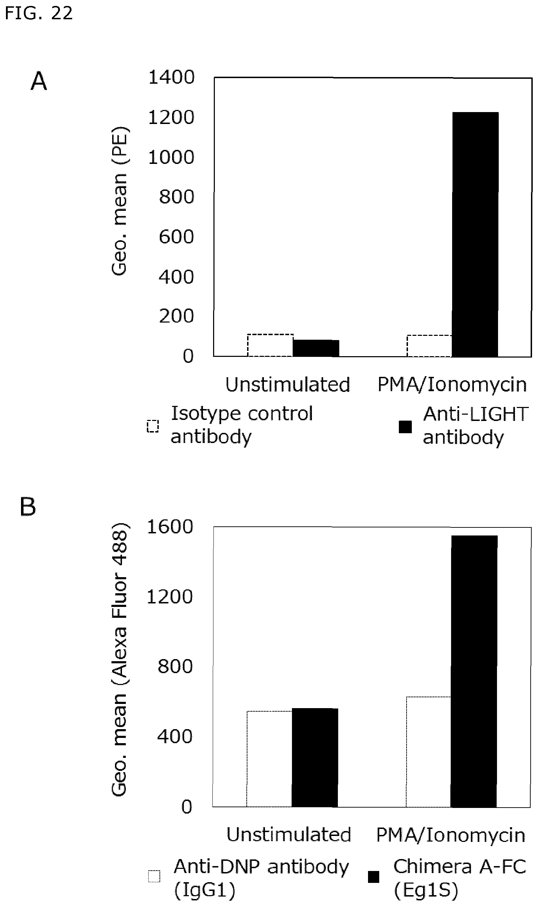

FIG. 22 shows evaluation results of the binding activity of chimera A-Fc to membrane-bound LIGHT of a primary cell. FIG. 22A shows measurement results of the expression level of membrane-bound LIGHT on activated human T cells by a PE-labeled anti-LIGHT antibody. FIG. 22B shows the geometric mean (Geo. Mean) calculated by measuring the binding of Alexa Fluor 488-labeled chimera A-Fc to membrane-bound LIGHT on activated human T cells by flow cytometry. In both figures, the vertical axis represents the geometric mean (Geo. Mean) of fluorescence intensity.

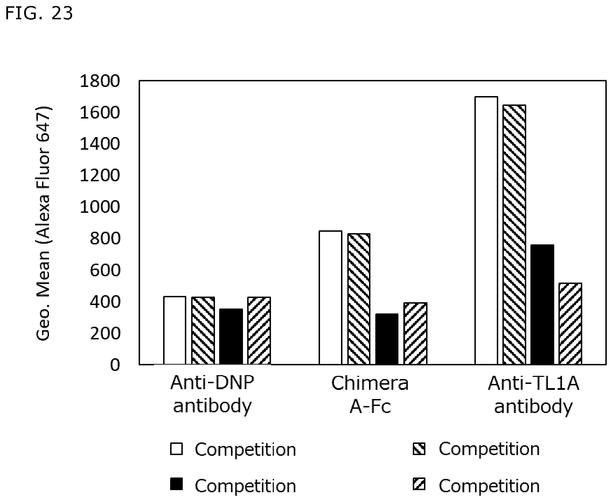

FIG. 23 shows evaluation results of the binding activity of chimera A-Fc to membrane-bound TL1A of primary cells. FIG. 23 shows the geometric mean (Geo. Mean) calculated by measuring the binding of Alexa Fluor 647-labeled chimera A-Fc in the presence or absence of competitive proteins to membrane-bound TL1A on HUVECs by flow cytometry.

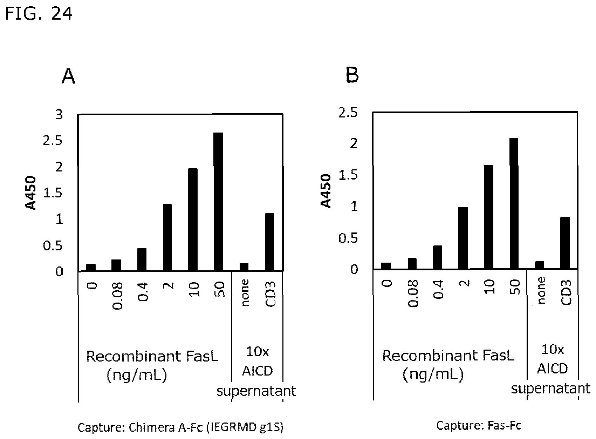

FIG. 24 shows measurement results of the binding activity of chimera A-Fc to FasL derived from primary cells by sandwich ELISA to soluble FasL in culture supernatants of human T cells stimulated for AICD. The figure shows the value of absorbance at 450 nm when a culture supernatant was added to a plate on which chimera A-Fc (FIG. 24A) or Fas-Fc (FIG. 24B) was captured, followed by detection with an anti-FasL antibody.

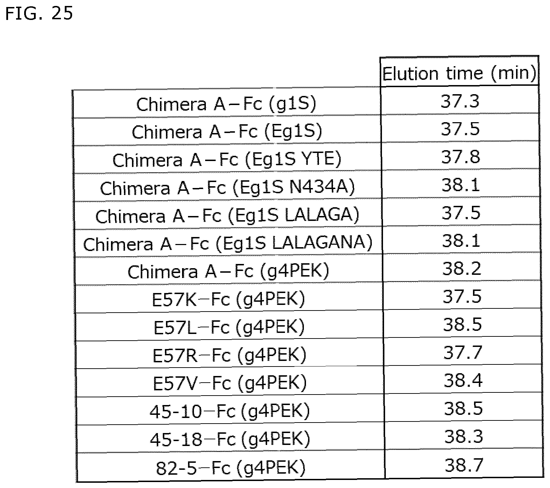

FIG. 25 shows results of the elution times (minute) of various chimera A-Fc variants including different Fc sequences and the variants with low FasL binding activities in hydrophobic interaction chromatography (HIC).

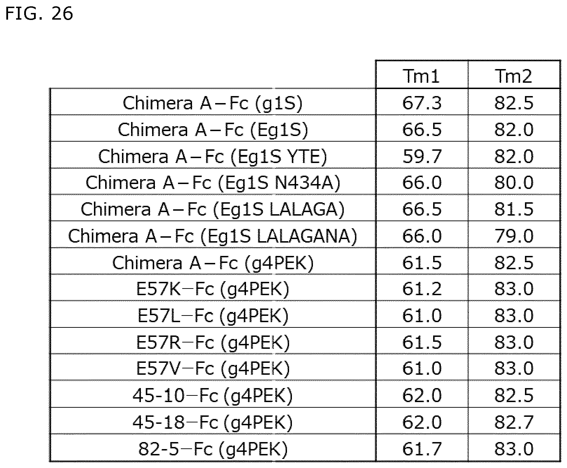

FIG. 26 shows results of the Tm values (°C) calculated by the differential scanning fluorimetry (DSF) method of various chimera A-Fc variants including different Fc sequences and the variants with low FasL binding activities.

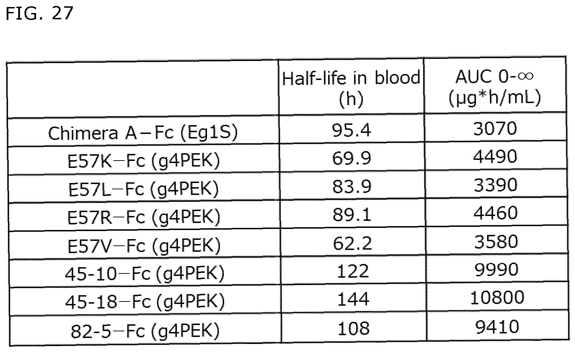

FIG. 27 shows the values of the half-life in blood (h) during the elimination phase and the area under the concentration-time curve to infinity AUC0-∞ (µg*h/mL) after i.v. administration of 10 mg/kg of chimera A-Fc (Eg1S) including a different Fc sequence and the variants with low FasL binding activities in BALB/c mice.

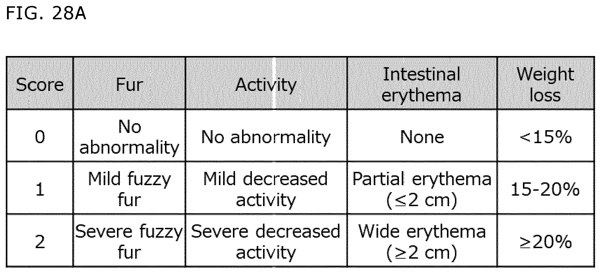

FIG. 28A shows indices of gross pathological scores in a drug efficacy study of chimera A-Fc using a mouse acute xenogeneic GVHD model.

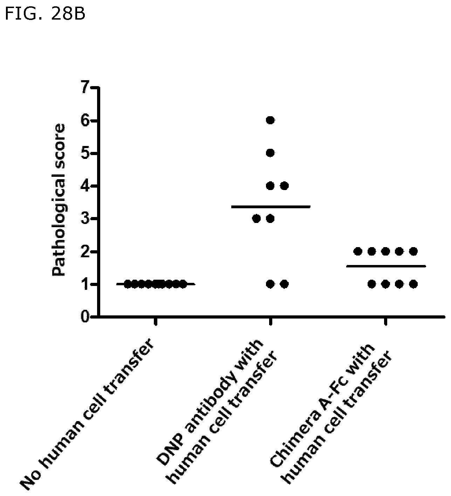

FIG. 28B shows pathological scores for each individual of each group and as a mean of each group. The vertical axis represents the pathological score, and the horizontal axis represents each treatment group.

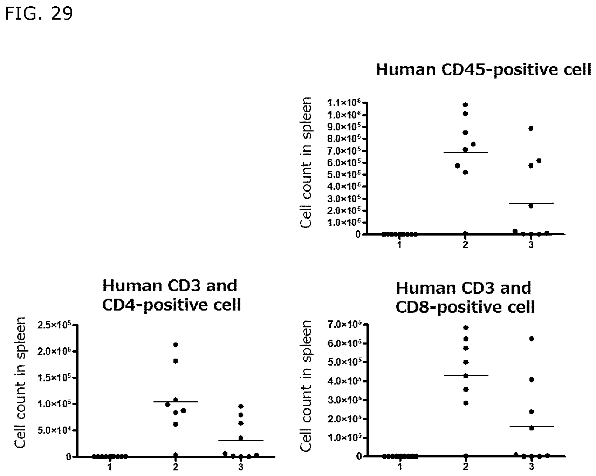

FIG. 29 shows the cell count per spleen for each individual of each group and as a mean value of each group regarding human CD45-positive cells, human CD3 and CD4-positive cells, and human CD3 and CD8-positive cells in a drug efficacy study of chimera A-Fc using a mouse acute xenogeneic GVHD model. Group 1 represents no human cell transfer, group 2 represents a group of administration of anti-DNP antibody with human cell transfer, and group 3 represents a group of administration of chimera A-Fc with human cell transfer. The vertical axis represents each surface marker-positive cell count, and the horizontal axis represents each treatment group.

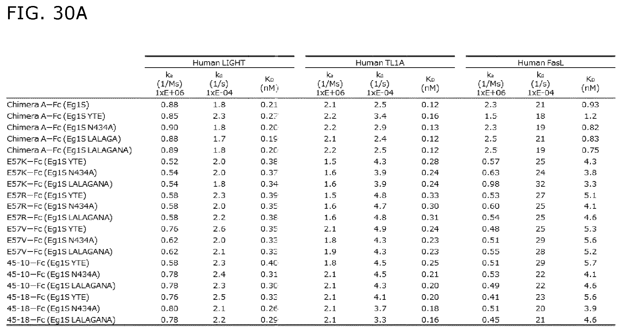

FIG. 30A shows measurement results of the binding activities of various DcR3 variants to human DcR3 ligands by BIAcore. Various kinetic constants (ka, kd and K_{D}) when a trimer of human FasL, human LIGHT or human TL1A was used as a human DcR3 ligand are shown.

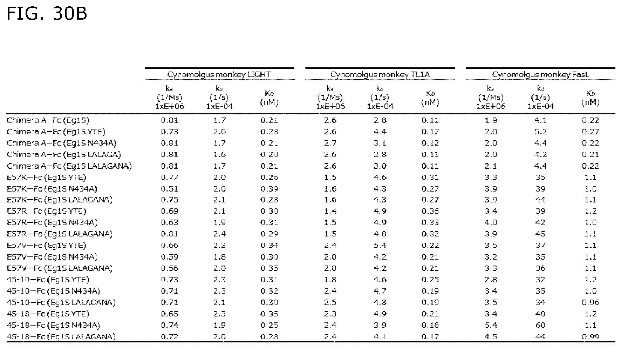

FIG. 30B shows measurement results of the binding activities of various DcR3 variants to cynomolgus monkey DcR3 ligands by BIAcore. Various kinetic constants (ka, kd and K_{D}) when a trimer of cynomolgus monkey FasL, cynomolgus monkey LIGHT or cynomolgus monkey TL1A was used as a cynomolgus monkey DcR3 ligand are shown.

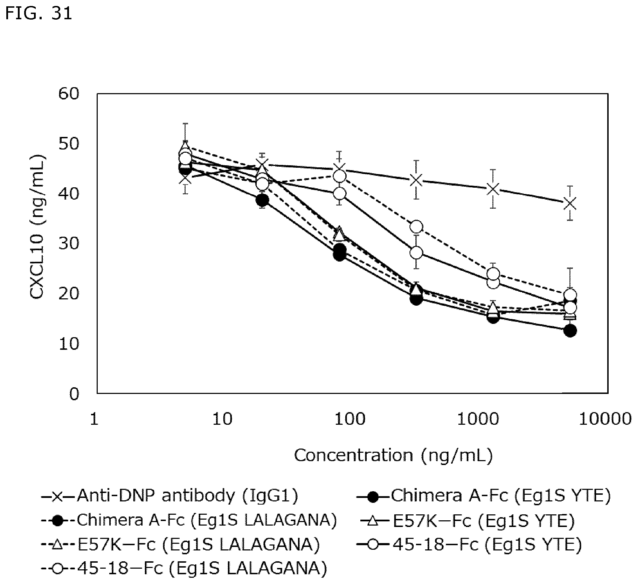

FIG. 31 shows evaluation results of the neutralizing activities of various DcR3 variants to soluble LIGHT. FIG. 31 shows the inhibitory activities of chimera A-Fc variants and the variants with low FasL binding activities including various mutated Fc sequences to LIGHT-dependent CXCL10 production from intestinal myofibroblasts. The vertical axis represents CXCL10 concentration (ng/mL), and the horizontal axis represents the concentration (ng/mL) of DcR3 variants added.

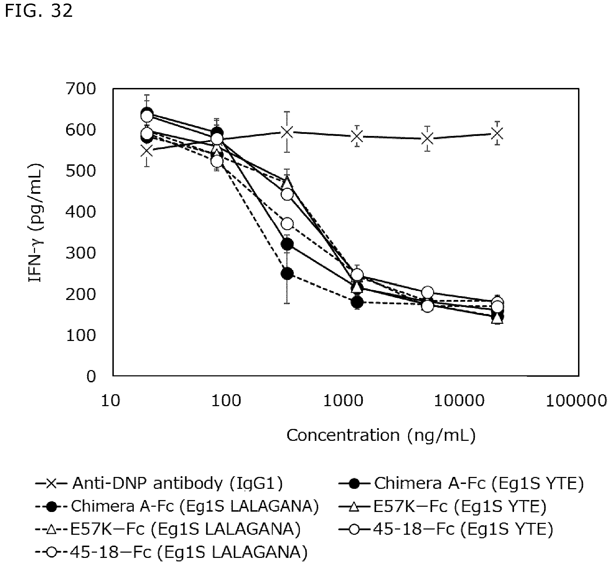

FIG. 32 shows evaluation results of the neutralizing activities of various DcR3 variants to soluble TL1A. FIG. 32 shows the inhibitory activities of chimera A-Fc variants and the variants with low FasL binding activities including various mutated Fc sequences to TL1A-dependent IFN-γ production from human T cells. The vertical axis represents IFN-γ concentration (pg/mL), and the horizontal axis represents the concentration (ng/mL) of DcR3 variants added.

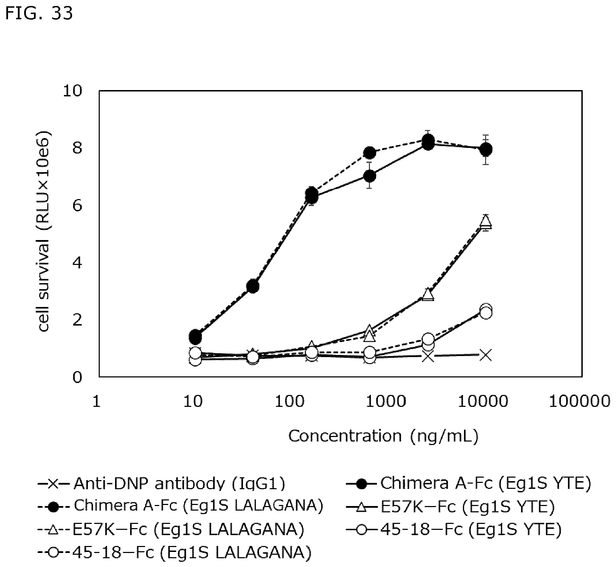

FIG. 33 shows evaluation results of the neutralizing activities of various DcR3 variants to soluble FasL. FIG. 33 shows the inhibitory activities of chimera A-Fc variants and the variants with low FasL binding activities including various mutated Fc sequences to cell death of Jurkat cells. The vertical axis represents cell survival (RLU × 10⁶) using ATP-dependent chemiluminescence as an index, and the horizontal axis represents the concentration (ng/mL) of various DcR3 variants added.

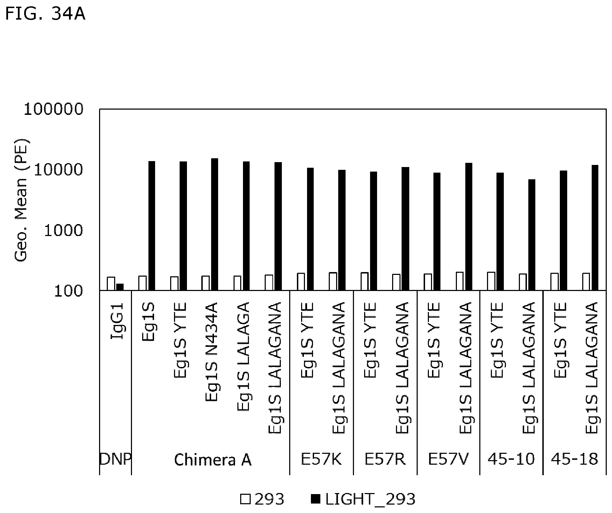

FIG. 34A shows evaluation results of the binding activities of various DcR3 variants to cells of a membrane-bound LIGHT forced expression cell line by flow cytometry. The vertical axis of the graph represents the geometric mean (Geo. Mean) of PE.

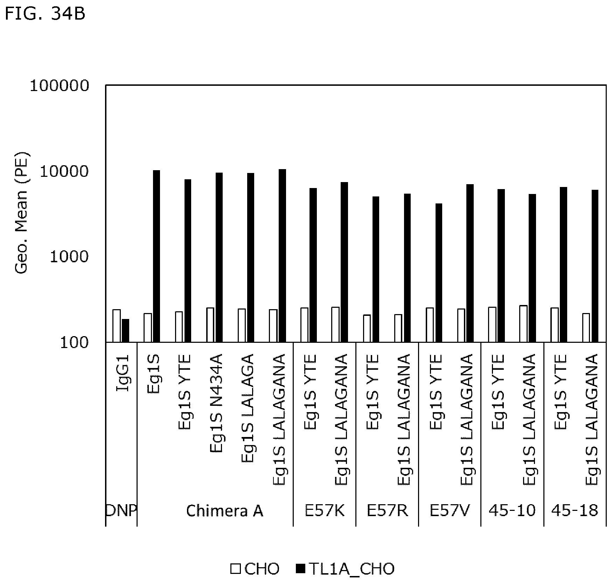

FIG. 34B shows evaluation results of the binding activities of various DcR3 variants to cells of a membrane-bound TL1A forced expression cell line by flow cytometry. The vertical axis of the graph represents the geometric mean (Geo. Mean) of PE.

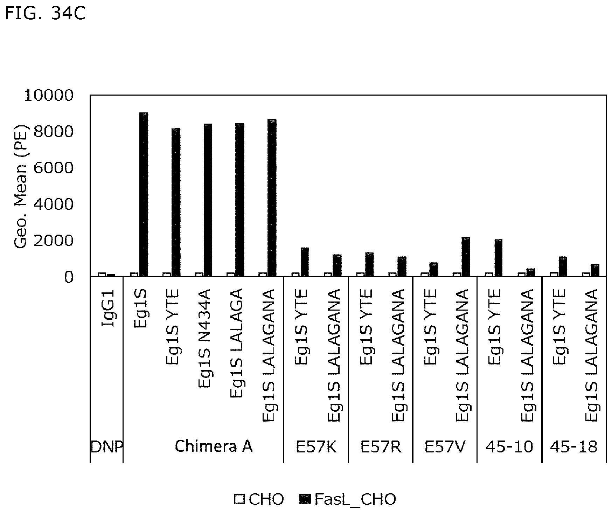

FIG. 34C shows evaluation results of the binding activities of various DcR3 variants to cells of a membrane-bound FasL forced expression cell line by flow cytometry. The vertical axis of the graph represents the geometric mean (Geo. Mean) of PE.

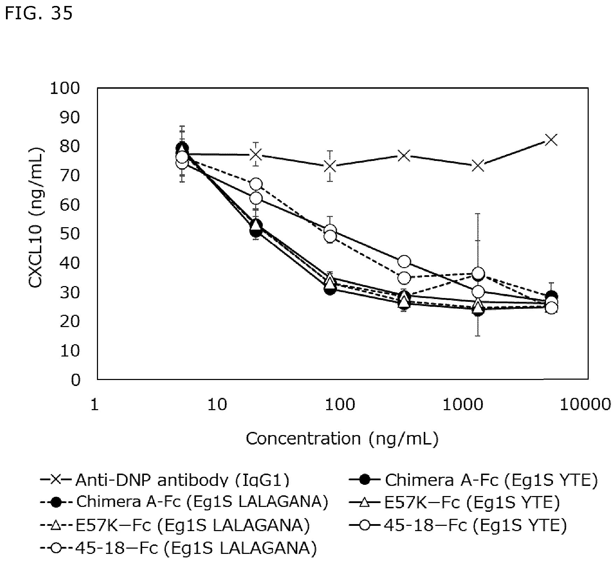

FIG. 35 shows evaluation results of the neutralizing activities of various DcR3 variants to membrane-bound LIGHT. FIG. 35 shows the inhibitory activities of chimera A-Fc variants and the variants with low FasL binding activities including various mutated Fc sequences to membrane-bound LIGHT-dependent CXCL10 production from intestinal myofibroblasts. The vertical axis represents CXCL10 concentration (ng/mL), and the horizontal axis represents the concentration (ng/mL) of DcR3 variants added.

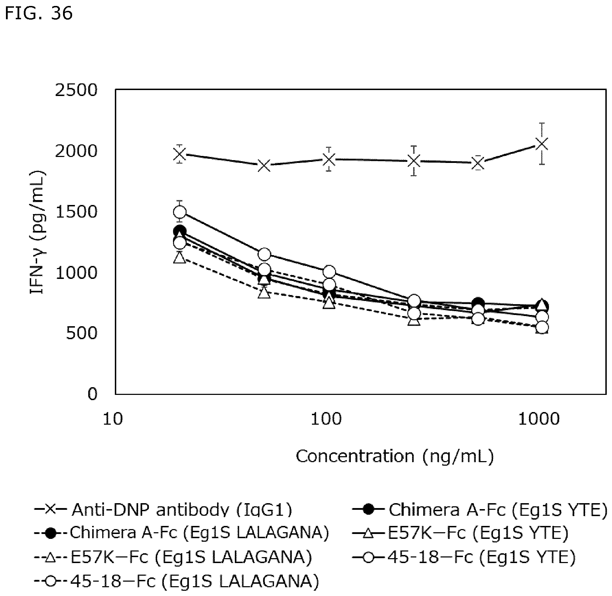

FIG. 36 shows evaluation results of the neutralizing activities of various DcR3 variants to membrane-bound TL1A. FIG. 36 shows the inhibitory activities of chimera A-Fc variants and the variants with low FasL binding activities including various mutated Fc sequences to membrane-bound TL1A-dependent IFN-γ production from human CD4-positive T cells. The vertical axis represents IFN-γ concentration (pg/mL), and the horizontal axis represents the concentration (ng/mL) of DcR3 variants added.

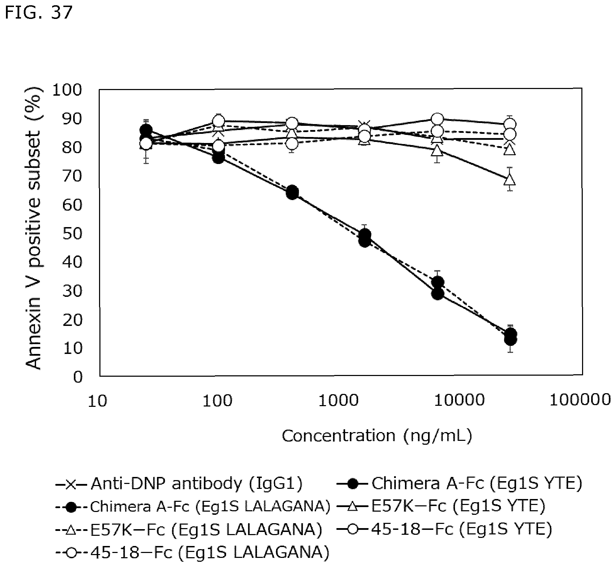

FIG. 37 shows evaluation results of the neutralizing activities of various DcR3 variants to membrane-bound FasL. FIG. 37 shows the inhibitory activities of chimera A-Fc variants and the variants with low FasL binding activities including various mutated Fc sequences to membrane-bound FasL-dependent cell death of Jurkat cells. The vertical axis of the graph represents the proportion (%) of an Annexin V-positive dead cell subset, and the horizontal axis represents the concentration (ng/mL) of various DcR3 variants added.

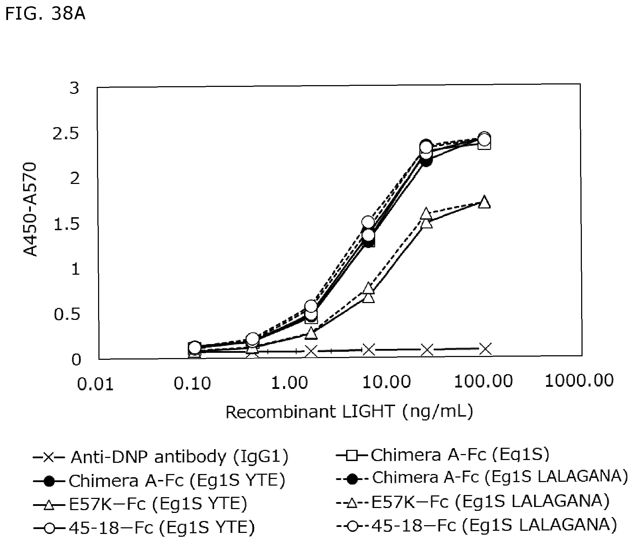

FIG. 38A shows measurement results of the binding activities of various DcR3 variants to recombinant human LIGHT by sandwich ELISA. The vertical axis represents the value obtained by subtracting the absorbance at 570 nm from the absorbance at 450 nm, and the horizontal axis represents the concentration (ng/mL) of recombinant human LIGHT added.

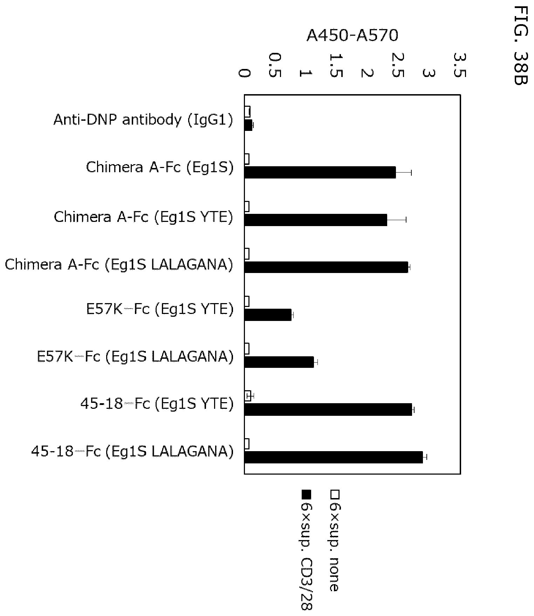

FIG. 38B shows results of the binding activities of various DcR3 variants to LIGHT derived from human primary cells, measured by sandwich ELISA to soluble LIGHT in culture supernatants of human T cells. The open bar represents results using culture supernatants when human T cells were cultured without stimulation, and the solid bar represents results using culture supernatants of human T cells stimulated with an anti-CD3 antibody and an anti-CD28 antibody. The vertical axis represents the value obtained by subtracting the absorbance at 570 nm from the absorbance at 450 nm.

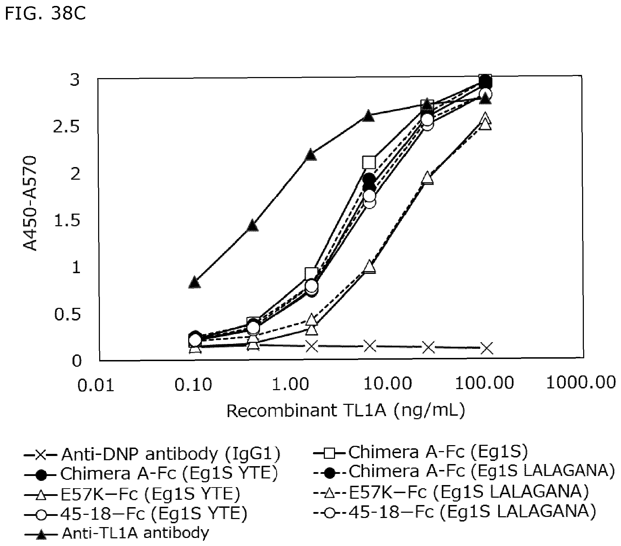

FIG. 38C shows results of the binding activities of various DcR3 variants to recombinant human TL1A, measured by sandwich ELISA. The vertical axis represents the value obtained by subtracting the absorbance at 570 nm from the absorbance at 450 nm, and the horizontal axis represents the concentration (ng/mL) of recombinant TL1A.

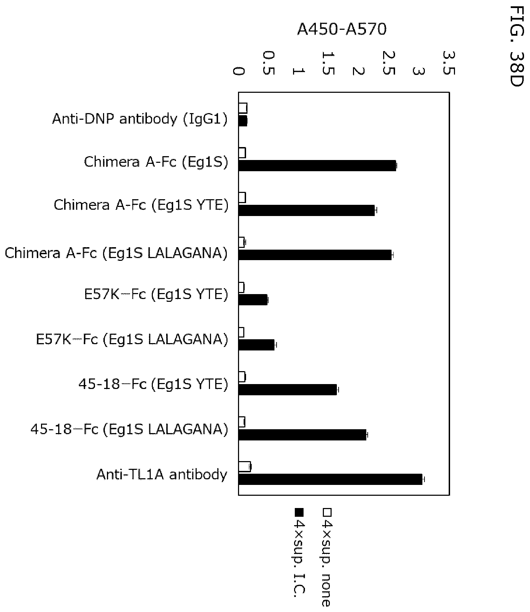

FIG. 38D shows results of the binding activities of various DcR3 variants to TL1A derived from human primary cells, measured by sandwich ELISA to soluble TL1A in culture supernatants of human PBMCs. The open bar represents results using culture supernatants when human PBMCs were cultured without stimulation, and the solid bar represents results using culture supernatants of human PBMCs stimulated with an immune complex. The vertical axis represents the value obtained by subtracting the absorbance at 570 nm from the absorbance at 450 nm.

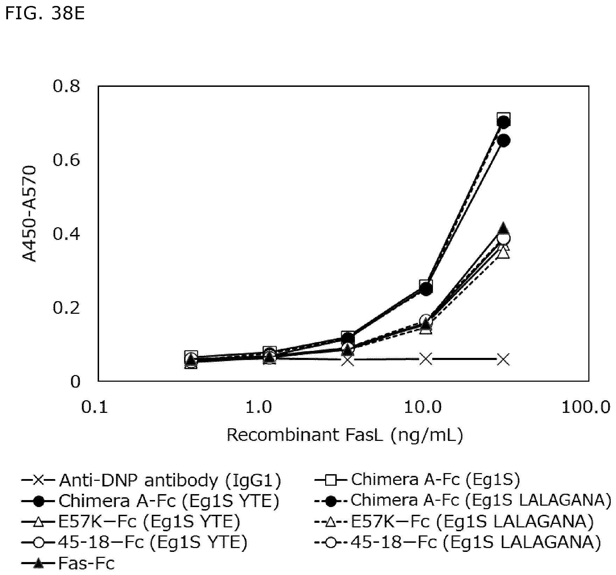

FIG. 38E shows results of the binding activities of various DcR3 variants to recombinant human FasL, measured by sandwich ELISA. The vertical axis represents the value obtained by subtracting the absorbance at 570 nm from the absorbance at 450 nm, and the horizontal axis represents the concentration (ng/mL) of human FasL.

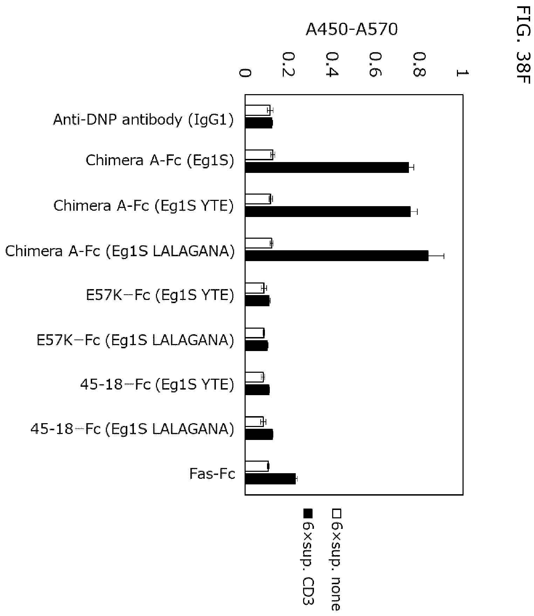

FIG. 38F shows results of the binding activities of various DcR3 variants to FasL derived from human primary cells, measured by sandwich ELISA to soluble FasL in culture supernatants of human T cells stimulated for AICD. The open bar represents results using culture supernatants of human T cells not stimulated for AICD, and the solid bar represents results using culture supernatants of human T cells stimulated for AICD. The vertical axis represents the value obtained by subtracting the absorbance at 570 nm from the absorbance at 450 nm.

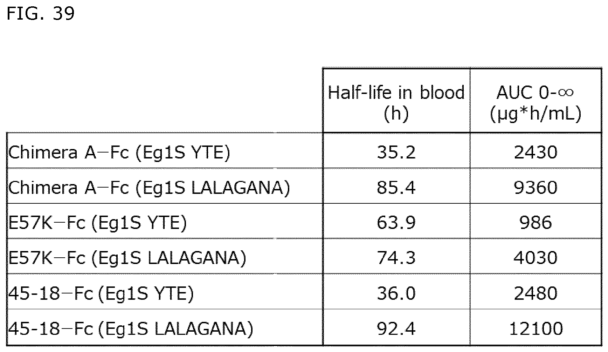

FIG. 39 shows the values of the half-life in blood (h) during the elimination phase and the area under the concentration-time curve to infinity AUC0-∞ (µg*h/mL) after i.v. administration of 10 mg/kg of various DcR3 variants to BALB/c mice.

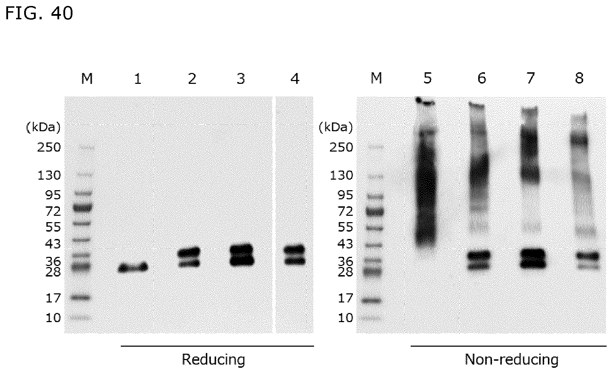

FIG. 40 shows results of immunoblotting detection of various DcR3 variants without Fc fusion that were transiently expressed on mammalian cells. A 30-fold concentrated solution of a culture supernatant of the cells expressing S195-His6 in lanes 1 and 5, a 6-fold diluted solution of a culture supernatant of the cells expressing chimera A-His6 in lanes 2 and 6, a 6-fold diluted solution of a culture supernatant of the cells expressing E57K-His6 in lanes 3 and 7, and a 6-fold diluted solution of a culture supernatant of the cells expessing 45-18-His6 in lanes 4 and 8 each were electrophoresed under non-reducing or reducing conditions, followed by detection by immunoblotting using an anti-6-His tag antibody.

### DETAILED DESCRIPTION OF THE INVENTION

Preferred embodiments for carrying out the present invention will be described below.

It is possible to combine two or more embodiments of the embodiments described below, and such combinations are also included in the present invention.

The present invention relates to a DcR3 variant as claimed, which is a variant of wild-type DcR3. Specifically, the present invention relates to a DcR3 variant as claimed that has binding activity (or neutralizing activity) to a ligand of DcR3, and that exhibits improved *in vivo* kinetics as compared to wild-type DcR3 and/or lower aggregability as compared to wild-type DcR3 when produced using a mammalian cell, and to a DcR3 variant including a cysteine-rich domain obtained by introducing a mutation(s) into one or two or more amino acids in a cysteine-rich domain of wild-type DcR3.

### 1. Wild-Type DcR3

DcR3 is generally also referred to as decoy receptor 3, DCR3, tumor necrosis factor receptor superfamily member 6B (TNFRSF6B), TR6 or M68. DcR3 is a soluble decoy receptor that belongs to a TNF receptor superfamily and that has no transmembrane domain, and by binding to each of three ligands, i.e., LIGHT, TL1A or FasL, DcR3 competitively inhibits the binding of each ligand to a receptor, thus neutralizing the ligand. As a different function from neutralization of a ligand, it has been reported that DcR3 directly binds via a heparan sulfate-binding domain (HBD) to glycosaminoglycan (GAG) including heparan sulfate on the cell membrane of monocytes, macrophages or dendritic cells, thus initiating various immunosuppressive and immunostimulatory effects [Biochemical. Pharmacology, 2011, 81: p.838-847, J. Immunol., 2006, 176: p.173-180].

Naturally occurring DcR3 includes CRD1, CRD2, CRD3, CRD4 and HBD in this order from the N-terminal side. Naturally occurring DcR3 further includes a region existing between CRD1 and CRD2, a region existing between CRD2 and CRD3, a region existing between CRD3 and CRD4, and a region existing between CRD4 and HBD. A cysteine-rich region of naturally occurring DcR3 is a region from the N-terminus of CRD1 to the C-terminus of CRD4, and includes CRD1, CRD2, CRD3 and CRD4, and further includes a region existing between CRD1 and CRD2, a region existing between CRD2 and CRD3, and a region existing between CRD3 and CRD4. "Wild-type DcR3" means a molecule including a cysteine-rich region and HBD, wherein the cysteine-rich region and the HBD of the molecule are wild-type (in other word, the cysteine-rich region and the HBD of the molecule are the same as those of naturally occurring DcR3). Therefore, in addition to naturally occurring DcR3, mutants of naturally occurring DcR3, such as gene polymorphisms and isoforms, are also included in "wild-type DcR3" as long as they include the cysteine-rich region and the HBD of naturally occurring DcR3. Premature DcR3 and mature DcR3 are also included in "wild-type DcR3". "Premature DcR3" means DcR3 including a signal peptide, and "mature DcR3" means DcR3 from which a signal peptide has been cleaved. The signal peptide may be any of a sequence derived from naturally occurring DcR3, an artificial sequence, a sequence derived from an expression vector, and a sequence derived from another protein suitable for a host cell in which naturally occurring DcR3 is expressed. When a cleavage site is different according to a signal peptide to be used, a mature sequence having a different amino acid(s) at the N-terminus is also included in "wild-type DcR3". The CRD of naturally occurring DcR3 and the CRD of wild-type DcR3 are also referred to as "wild-type CRD", and the cysteine-rich region of naturally occurring DcR3 and the cysteine-rich region of wild-type DcR3 are also referred to as "wild-type cysteine-rich region".

In the present invention, the origin of wild-type DcR3 is human

The cDNA sequence of DcR3 derived from a human is represented by SEQ ID NO: 1, and the corresponding mRNA sequence has been registered as Accession Number: NM_003823.3 in GenBank (US NCBI). The amino acid sequence of DcR3 derived from a human is represented by SEQ ID NO: 2, and has been registered as Accession Number: NP_003814.1 in GenBank (US NCBI). Premature human DcR3 includes a signal peptide at the N-terminus, followed by four CRDs (CRD1, CRD2, CRD3, CRD4), which are characteristic of a TNF receptor superfamily, and includes HBD, which is rich in basic amino acids, at the C-terminus. Mature human DcR3 is obtained by cleaving a signal peptide from premature one, and the amino acid sequence of the mature human DcR3 is represented by, for example, SEQ ID NO: 4, and the nucleotide sequence of a DNA encoding the amino acid sequence of the mature human DcR3 is represented by, for example, SEQ ID NO: 3. In the present invention, of the amino acid sequence (SEQ ID NO: 2) of human DcR3, a region at positions 30 to 70 from the N-terminus is defined as CRD1 (SEQ ID NO: 6), a region at positions 73 to 113 is defined as CRD2 (SEQ ID NO: 8), a region at positions 115 to 150 is defined as CRD3 (SEQ ID NO: 10), a region at positions 153 to 193 is defined as CRD4 (SEQ ID NO: 12), and a region at positions 196 to 300 is defined as HBD (SEQ ID NO: 48) (FIG. 2). The nucleotide sequences of DNAs encoding the amino acid sequences of CRD1, CRD2, CRD3, CRD4 and HBD of human DcR3 are represented by, for example, SEQ ID NOs: 5, 7, 9, 11 and 47, respectively. With respect to an amino acid sequence of CRD, although there are a plurality of definitions other than those mentioned above, any definition of an amino acid sequence of CRD can be used for the DcR3 variant of the present invention by using known information [UniProt O95407, GenBank NP_003814.1, Structure, 2011,19: p.162-171].

As mentioned above, examples of the ligand of DcR3 include LIGHT, TL1A and FasL, all of which belong to TNFSF.

LIGHT (lymphotoxin-like, exhibits inducible expression, and competes with herpes simplex virus (HSV) glycoprotein D (gD) for HVEM, a receptor expressed by T lymphocytes) is generally also referred to as tumor necrosis factor superfamily member 14 (TNFSF14), LTg, HVEM-L or CD258. The mRNA sequence of human LIGHT and the corresponding cDNA sequence have been registered as Accession Number: NM_003807.4, and the amino acid sequence thereof has been registered as Accession Number: NP_003798.2 in GenBank (US NCBI). Soluble LIGHT is generated by being expressed on the cell membrane as membrane-bound LIGHT, followed by shedding of the extracellular region by protease. A cleavage site in membrane-bound LIGHT is between amino acids at positions 82 and 83 of NP_003798.2. Both soluble and membrane-bound LIGHTs are functional.

Tumor necrosis factor (TNF)-like cytokine 1A (TL1A) is generally also referred to as TNF superfamily member 15 (TNFSF15), TL1, VEGI or VEGI-251. The mRNA sequence of human TL1A and the corresponding cDNA sequence have been registered as Accession Number: NM_005118.3, and the amino acid sequence thereof has been registered as Accession Number: NP_005109.2 in GenBank (US NCBI). Soluble TL1A is generated by being expressed on the cell membrane as membrane-bound TL1A, followed by shedding of the extracellular region by protease. A cleavage site in membrane-bound TL1A is between amino acids at positions 71 and 72 of NP_005109.2, and both soluble and membrane-bound TL1As are functional.

Fas ligand (FasL) is generally also referred to as FASLG, tumor necrosis factor superfamily member 6 (TNFSF6), CD178 or APT1LG1. The mRNA sequence of human FasL and the corresponding cDNA sequence have been registered as Accession Number: NM_000639.2, and the amino acid sequence thereof has been registered as Accession Number: NP_000630.1 in GenBank (US NCBI). Soluble FasL is generated by being expressed on the cell membrane as membrane-bound FasL, followed by shedding of the extracellular region by protease. A cleavage site in membrane-bound FasL is between amino acids at positions 81 and 82 or between amino acids at positions 129 and 130 of NP_000630.1. It has been reported that membrane-bound FasL is mainly a functional ligand *in vivo.*

In a gene encoding a protein of a eukaryote, polymorphisms or isoforms of the gene are often observed. With respect to a gene used in the present invention, a gene including a mutation introduced into a nucleotide sequence or amino acid sequence by such polymorphisms is also included in a gene encoding LIGHT, TL1A or FasL used in the present invention.

### 2. DcR3 Variant

The DcR3 variant of the present invention includes a chimeric cysteine-rich region in accordance with the claims.

### 2-1. Chimeric Cysteine-Rich Region

The chimeric cysteine-rich region of the present invention is defined in the claims. "A mutation(s) introduced into an amino acid sequence/nucleotide sequence" means substitution, deletion, insertion or addition of one or two or more amino acids/bases in the sequence. "Substitution, deletion, insertion or addition" also includes a combination of two or more mutations selected from substitution, deletion, insertion and addition. A mutation(s) is/are introduced into at least one or two or more CRDs selected from CRD1, CRD2, CRD3 and CRD4 of the cysteine-rich region of wild-type DcR3. A mutation(s) may be or may not be introduced into a region existing between CRD1 and CRD2, a region existing between CRD2 and CRD3, and a region existing between CRD3 and CRD4 of the cysteine-rich region of wild-type DcR3. When a mutation(s) is/are introduced into one or two or more regions selected from a region existing between CRD1 and CRD2, a region existing between CRD2 and CRD3, and a region existing between CRD3 and CRD4, the number of amino acids constituting each region after introduction of the mutation(s) is usually 1 to 10, preferably 1 to 7, more preferably 1 to 5, still more preferably 1 to 3, and yet more preferably 1 to 2. The amino acid sequence of each region after introduction of the mutation(s) is not particularly limited. The mutation(s) to be introduced into the cysteine-rich region of wild-type DcR3 may be a naturally occurring mutation or an artificial mutation. Examples of the chimeric cysteine-rich region include a chimeric cysteine-rich region consisting of an amino acid sequence obtained by introducing into the amino acid sequence of the cysteine-rich region of wild-type DcR3, substitution, deletion, insertion or addition of one or two or more amino acids, and examples of such a chimeric cysteine-rich region can include first and second chimeric cysteine-rich regions mentioned below.

### 2-1-1. First Chimeric Cysteine-Rich Region

The first chimeric cysteine-rich region consists of an amino acid sequence according to the claims.

Twenty-nine (29) receptors belong to human TNFRSF, and all receptors have CRDs in the extracellular domain of the N-terminus, and it is typical that six Cys residues form three disulfide bonds per CRD, and each receptor has one to four CRDs [Trends Biochem Sci, 2002. 27: p-19-26.].

Examples of the human TNFRSF include DcR1 (TNFRSF10C, TRAIL-R3, LIT, TRID, CD263), DcR2 (TNFRSF10D, TRAIL-R4, TRUNDD, CD264), TNFR type I (TNFRSF1A, TNF-R, CD120a, TNFAR, TNF-R55, TNFR60), TNFR type II (TNFRSF1B, TNFBR, CD120b, TNFR80, p75, TNF-R75), LTBR (lymphotoxin beta receptor, TNFRSF3, TNFR III, TNFCR, TNFR-RP, TNFR2-RP), OX-40 (TNFRSF4, ACT35, TXGP1L, CD134), CD40 (TNFRSF5, Bp50, p50), Fas (Fas cell surface death receptor, TNFRSF6, CD95, APO-1, APT1, FAS1), CD27 (TNFRSF7, S152, Tp55), CD30 (TNFRSF8, Ki-1), 4-1BB (TNFRSF9, CD137, ILA), DR4 (TNFRSF10A, Apo2, TRAILR-1, CD261), DR5 (TNFRSF10B, TRAIL-R2, KILLER, TRICK2A, TRICKB, CD262), RANK (TNFRSF11A, CD265, FEO), FN14 (TNFRSF12A, TweakR, CD266), TACI (TNFRSF13B, CD267, IGAD2), BAFFR (TNFRSF13C, CD268), HVEM (TNFRSF14, ATAR, TR2, LIGHTR, HVEA, CD270), NGFR (nerve growth factor receptor, TNFRSF16, p75NTR, CD271), BCMA (TNFRSF17, BCM, CD269, TNFRSF13A), GITR (TNFRSF18, AITR, CD357), TROY (TNFRSF19, TAJ-alpha, TAJ, TRADE), RELT (TNFRSF19L), DR6 (death receptor 6, TNFRSF21, CD358), DR3 (death receptor 3, TNFRSF25, TRAMP, WSL-1, LARD, WSL-LR, DDR3, TR3, APO-3), EDAR (ectodysplasin A receptor, ED3, DL, ED5, EDA3, Edar, ED1R, EDA1R), EDAR2R (ectodysplasin A2 receptor, XEDAR, EDAA2R, EDA-A2R, TNFRSF27) or osteoprotegerin (OPG, TNFRSF11B, TR1, OCIF) or the like.

The origin of OPG is not limited, and examples of OPG include OPG derived from various eukaryotes. Examples thereof include OPG derived from amphibians such as frogs, birds such as chickens, or mammals, for example, primates including humans, artiodactyls such as pigs and cattle, or rodents including mice, or the like.

The cDNA sequence of OPG derived from a human is represented by SEQ ID NO: 13, and the corresponding mRNA sequence has been registered as Accession Number: NM_002546.3 in GenBank (US NCBI). The amino acid sequence of OPG derived from a human is represented by SEQ ID NO: 14, and has been registered as Accession Number: NP_002537.3 in GenBank (US NCBI).

Premature human OPG (SEQ ID NO: 14) has a signal peptide at the N-terminus. Mature human OPG is obtained by cleaving a signal peptide from premature one. The amino acid sequence of the mature human OPG is represented by, for example, SEQ ID NO: 16, and the nucleotide sequence of a DNA encoding the amino acid sequence of the mature human OPG is represented by, for example, SEQ ID NO: 15. In the present invention, of the amino acid sequence (SEQ ID NO: 14) of premature human OPG, a region at positions 22 to 62 from the N-terminus is defined as CRD1 (SEQ ID NO: 18), a region at positions 65 to 105 is defined as CRD2 (SEQ ID NO: 20), a region at positions 107 to 142 is defined as CRD3 (SEQ ID NO: 22), and a region at positions 145 to 185 is defined as CRD4 (SEQ ID NO: 24). The nucleotide sequences of DNAs encoding the amino acid sequences of CRD1, CRD2, CRD3 and CRD4 of human OPG are represented by, for example, SEQ ID NOs: 17, 19, 21 and 23, respectively. With respect to an amino acid sequence of CRD, although there are a plurality of definitions other than those mentioned above, any definition of an amino acid sequence of CRD can be used for the DcR3 variant of the present invention by using known information [UniProt O00300, GenBank NP_002537.3].

In a gene encoding a protein of a eukaryote, polymorphisms or isoforms of the gene are often observed. With respect to a gene used in the present invention, a gene including a mutation introduced into a nucleotide sequence or amino acid sequence by such polymorphisms is also included in a gene encoding OPG used in the present invention.

OPG binds to RANKL, and neutralizes the activity thereof, thus suppressing bone destruction by osteoclasts [J. Immunol., 2012, 189: p.245-252]. OPG also binds to TRAIL, and neutralizes the activity thereof, thus inhibiting apoptosis mediated by TRAIL [Am. J. Cancer. Res., 2012, 2: p.45-64]. Since neutralization of any ligand may cause undesired activity or the like, it is desirable that the DcR3 variant of the present invention has no neutralizing activity to any of RANKL and TRAIL.

The first chimeric cysteine-rich region consists of
(a) an amino acid sequence consisting of amino acids at positions 1 to 164 from the N-terminus of the amino acid sequence set forth in SEQ ID NO: 26 or 50;
(b) an amino acid sequence consisting of amino acids at positions 1 to 164 from the N-terminus of the amino acid sequence set forth in SEQ ID NO: 28 or 52;
(c) an amino acid sequence consisting of amino acids at positions 1 to 164 from the N-terminus of the amino acid sequence set forth in SEQ ID NO: 30 or 54; and
(d) an amino acid sequence consisting of amino acids at positions 1 to 164 from the N-terminus of the amino acid sequence set forth in SEQ ID NO: 32 or 56.

SEQ ID NO: 26 represents an amino acid sequence of chimera B-HBD (a DcR3 variant obtained by introducing into wild-type DcR3 (SEQ ID NO: 4), substitution of CRD1 with CRD1 of OPG); SEQ ID NO: 28 represents an amino acid sequence of chimera C-HBD (a DcR3 variant obtained by introducing into wild-type DcR3 (SEQ ID NO: 4), substitution of CRD4 with CRD4 of OPG); SEQ ID NO: 30 represents an amino acid sequence of chimera A-HBD (a DcR3 variant obtained by introducing into wild-type DcR3 (SEQ ID NO: 4), substitution of CDR1 and CDR4 with CDR1 and CDR4 of OPG, respectively); SEQ ID NO: 32 represents an amino acid sequence of 103-123OPG-HBD (a DcR3 variant obtained by introducing into chimera A-HBD, substitution of an amino acid sequence including a part at positions 18 to 36 of CRD3 and two amino acids at the C-terminal side thereof with human OPG); SEQ ID NO: 50 represents an amino acid sequence of chimera B (a DcR3 variant obtained by introducing into wild-type DcR3 (SEQ ID NO: 4), substitution of CRD1 with CRD1 of OPG and deletion of a heparan sulfate-binding domain); SEQ ID NO: 52 represents an amino acid sequence of chimera C (a DcR3 variant obtained by introducing into wild-type DcR3 (SEQ ID NO: 4), substitution of CRD4 with CRD4 of OPG and deletion of a heparan sulfate-binding domain); SEQ ID NO: 54 represents an amino acid sequence of chimera A (a DcR3 variant obtained by introducing into wild-type DcR3 (SEQ ID NO: 4), substitution of CDR1 and CDR4 with CDR1 and CDR4 of OPG, respectively, and deletion of a heparan sulfate-binding domain); and SEQ ID NO: 56 represents an amino acid sequence of 103-123OPG (a DcR3 variant obtained by introducing into chimera A, substitution of an amino acid sequence including a part at positions 18 to 36 of CRD3 and two amino acids at the C-terminal side thereof with human OPG).

In a preferred embodiment, examples of the DcR3 variant including the first chimeric cysteine-rich region include a DcR3 variant having binding activity to at least one or more of LIGHT, TL1A and FasL, a DcR3 variant having binding activity to all of LIGHT, TL1A and FasL, a DcR3 variant having no binding activity to FasL and having binding activity to one of LIGHT and TL1A, or a DcR3 variant having no binding activity to FasL and having binding activity to both of LIGHT and TL1A.

In the present invention, "having binding activity to a ligand" is used as a meaning including the case where the binding activity of the DcR3 variant including the first chimeric cysteine-rich region to the ligand is comparable and is not significantly lowered compared to the binding activity of wild-type DcR3 to the ligand, and the case where the binding activity of the DcR3 variant to the ligand is significantly enhanced compared to the binding activity of wild-type DcR3 to the ligand. For example, in the case of measurement by the surface plasmon resonance method (SPR method), when the dissociation constant (K_{D}) value of a DcR3 variant is less than 3-fold compared to that of wild-type DcR3, it can be judged that the DcR3 variant has binding activity to the ligand.

In the present invention, the expression of the DcR3 variant "having no binding activity to a ligand" is used as a meaning including the case where the binding activity of the DcR3 variant including the first chimeric cysteine-rich region to the ligand is not detected, and the case where the binding activity of the DcR3 variant to the ligand is significantly lowered compared to the binding activity of wild-type DcR3 to the ligand. For example, in the case of measurement by the SPR method, when the KD value of a DcR3 variant is more than 3-fold compared to that of wild-type DcR3, or the Rmax of a DcR3 variant is less than 5, it is judged that the binding activity to the ligand is significantly lowered, and it can be defined that the DcR3 variant has no binding activity to the ligand.

In a particularly preferred embodiment, examples of the DcR3 variant including the first chimeric cysteine-rich region include a DcR3 variant with lowered FasL binding activity. "Variant with lowered FasL binding activity" means a DcR variant including a chimeric cysteine-rich region, having no binding activity to FasL and having binding activity to one or more of LIGHT and TL1A, or a DcR3 variant including a chimeric cysteine-rich region, having no binding activity to FasL and having binding activity to both of LIGHT and TL1A.

In a preferred embodiment, the DcR3 variant of the present invention is a DcR3 variant having neutralizing activity to at least one or more of LIGHT, TL1A and FasL, a DcR3 variant having neutralizing activity to all of LIGHT, TL1A and FasL, a DcR3 variant having no neutralizing activity to FasL and having neutralizing activity to one of LIGHT and TL1A, or a DcR3 variant having no neutralizing activity to FasL and having neutralizing activity to both of LIGHT and TL1A.

In the present invention, the expression of "neutralizing activity to a ligand" includes the case where the binding of the ligand to the DcR3 variant inhibits the binding of the ligand to a receptor on the cell membrane surface, and the case where inhibition of the binding of the ligand to a receptor on the cell membrane surface inhibits a cell function initiated by the binding of the ligand to the receptor on the cell membrane surface, namely, biological activity of the ligand (e.g., biological activity such as cytokine production, proliferation enhancement and apoptosis induction to a cell).

In the present invention, the expression of the DcR3 variant "having neutralizing activity" is used as a meaning including the case where the neutralizing activity of the DcR3 variant to the ligand has no significant difference compared to the neutralizing activity of wild-type DcR3 to the ligand, and the case where the neutralizing activity of the DcR3 variant to the ligand is significantly enhanced compared to the neutralizing activity of wild-type DcR3 to the ligand.

In the present invention, the expression of the DcR3 variant "having no neutralizing activity" is used as a meaning including the case where the neutralizing activity of the DcR3 variant to the ligand is significantly lowered compared to the neutralizing activity of wild-type DcR3 to the ligand.

In a particularly preferred embodiment, the DcR3 variant in the present invention is a DcR3 variant in which the binding activity to FasL is lowered. "Variant with lowered FasL binding activity" means a DcR3 variant having no neutralizing activity to FasL and having neutralizing activity to one or more of LIGHT and TL1A, or a DcR3 variant having no neutralizing activity to FasL and having neutralizing activity to both of LIGHT and TL1A.

### 2-1-2. Second Chimeric Cysteine-Rich Region

The second chimeric cysteine-rich region is a region obtained by introducing into the amino acid sequence of the first chimeric cysteine-rich region, deletion, substitution, insertion or addition of 1 to 5 amino acids.

Examples of a method for obtaining a polypeptide including an amino acid sequence obtained by introducing into the amino acid sequence of the first chimeric cysteine-rich region, deletion, substitution, insertion or addition of one or more amino acids include a site-specific mutation introduce method [Molecular Cloning, A Laboratory Manual, Second Edition, Cold Spring Harbor Laboratory Press (1989), Current Protocols in Molecular Biology, John Wiley & Sons (1987-1997), Nucleic Acids Research, 10, 6487 (1982), Proc. Natl. Acad. Sci. USA., 79, 6409, (1982), Gene, 34, 315 (1985), Proc. Natl. Acad. Sci. USA., 82, 488 (1985)].

A mutation (modification) to be added to the first chimeric cysteine-rich region may be a natural mutation or an artificial substitution, deletion, insertion or addition of an amino acid. Examples of the amino acid sequence of the second chimeric cysteine-rich region include an amino acid sequence obtained by introducing into the amino acid sequence of the first chimeric cysteine-rich region, substitution, deletion, insertion or addition of 1 or 2 or 1 to 5, and more preferably 1 to 3 amino acids, or an amino acid sequence having 97% or more, 98% or more or 99% or more identity to the amino acid sequence of the first chimeric cysteine-rich region. With respect to a method for describing an amino acid substitute, for example, when an amino acid substitution is obtained by introducing substitution of Asn with Ser at position 131 from the N-terminus into an amino acid sequence, the substitute can be expressed as N131S.

In a preferred embodiment, the second chimeric cysteine-rich region includes or consists of the following amino acid sequence (e):
(e) an amino acid sequence obtained by introducing into the amino acid sequence (a), (b), (c) or (d), deletion, substitution, insertion or addition of 1 to 5 amino acids.

Examples of a mutation (modification) to be added to the first chimeric cysteine-rich region include addition or deletion of a glycosylation site. By adding or deleting a glycosylation site to the first chimeric cysteine-rich region, it is possible to control the biological activity of the DcR3 variant of the present invention or properties thereof, *in vivo* kinetics such as half-life in blood, or physical or chemical properties such as stability of a protein.

Glycosylation generally means that a glycan is N-glycoside-linked to an asparagine residue of a peptide or a protein, and/or that a glycan is O-glycoside-linked to a serine or threonine residue. Examples of the O-linked glycan added to a DcR3 variant include core 1, core 2 or the like, or examples of the N-linked glycan include high-mannose-type, hybrid-type or complex glycan, but a complex glycan is preferable.

Examples of a mutation (modification) to be added to the first chimeric cysteine-rich region include, for example, substitution of at least one or more amino acids of the amino acid sequence of the first chimeric cysteine-rich region with an amino acid(s) to which a glycan(s) can be added via an N-glycoside linkage or O-glycoside linkage, and followed by addition of a glycan(s). Particularly, addition of an N-glycoside-linked glycan is preferable.

The present invention also includes, for example, substitution of at least one or more, preferably two or more amino acids associated with an N-glycoside linkage, of the amino acid sequence of the chimeric cysteine-rich region, with (an)other amino acid(s), followed by removal of a glycan(s). Generally, when a peptide or a protein is expressed using a yeast, an insect cell or a mammalian cell, an N-glycoside linkage of a glycan occurs by recognizing the sequence of Asn-X-Thr/Ser (wherein X represents any amino acid residue other than Pro). For example, by substituting Asn, Ser or Thr of the Asn-X-Thr/Ser sequence existing in the DcR3 variant with another amino acid, it is possible to remove an N-glycoside-linked glycan.

As the second chimeric cysteine-rich region, in order to decrease aggregates of the DcR3 variant of the present invention, particularly preferred is a chimeric cysteine-rich region having an N-glycoside-linked glycan to Asn at position 157 from the N-terminus of the amino acid sequence of the first chimeric cysteine-rich region (e.g., an amino acid sequence consisting of amino acids at positions 1 to 164 from the N-terminus of SEQ ID NO: 30).

In a preferred embodiment, the amino acid sequence of the second chimeric cysteine-rich region lacking a glycosylation site (one embodiment of the amino acid sequence (e)) includes substitution selected from:
(f) substitution of Asn at positions 131 and 144 from the N-terminus of the amino acid sequence (b), (c) or (d) (e.g., an amino acid sequence consisting of amino acids at positions 1 to 164 from the N-terminus of the amino acid sequence set forth in SEQ ID NO: 28, 30, 32, 52, 54 or 56) with other amino acids;
(g) substitution of Asn at positions 131, 144 and 157 from the N-terminus of the amino acid sequence (b), (c) or (d) (e.g., an amino acid sequence consisting of amino acids at positions 1 to 164 from the N-terminus of the amino acid sequence set forth in SEQ ID NO: 28, 30, 32, 52, 54 or 56) with other amino acids;
(h) substitution of Thr at position 133 and Ser at position 146 from the N-terminus of the amino acid sequence (b), (c) or (d) (e.g., an amino acid sequence consisting of amino acids at positions 1 to 164 from the N-terminus of the amino acid sequence set forth in SEQ ID NO: 28, 30, 32, 52, 54 or 56) with other amino acids; and
(i) substitution of Thr at position 133, Ser at position 146 and Thr at position 159 from the N-terminus of the amino acid sequence (b), (c) or (d) (e.g., an amino acid sequence consisting of amino acids at positions 1 to 164 from the N-terminus of the amino acid sequence set forth in SEQ ID NO: 28, 30, 32, 52, 54 or 56) with other amino acids.

In a further preferred embodiment, the amino acid sequence lacking a glycosylation site (one embodiment of the amino acid sequence (e)) includes substitution selected from:
(f') substitution of Asn at positions 131 and 144 from the N-terminus of the amino acid sequence (b), (c) or (d) (e.g., an amino acid sequence consisting of amino acids at positions 1 to 164 from the N-terminus of the amino acid sequence set forth in SEQ ID NO: 28, 30, 32, 52, 54 or 56) with Ser;
(g') substitution of Asn at positions 131, 144 and 157 from the N-terminus of the amino acid sequence (b), (c) or (d) (e.g., an amino acid sequence consisting of amino acids at positions 1 to 164 from the N-terminus of the amino acid sequence set forth in SEQ ID NO: 28, 30, 32, 52, 54 or 56) with Ser;
(h') substitution of Thr at position 133 and Ser at position 146 from the N-terminus of the amino acid sequence (b), (c) or (d) (e.g., an amino acid sequence consisting of amino acids at positions 1 to 164 from the N-terminus of the amino acid sequence set forth in SEQ ID NO: 28, 30, 32, 52, 54 or 56) with Ala; and
(i') substitution of Thr at position 133, Ser at position 146 and Thr at position 159 from the N-terminus of the amino acid sequence (b), (c) or (d) (e.g., an amino acid sequence consisting of amino acids at positions 1 to 164 from the N-terminus of the amino acid sequence set forth in SEQ ID NO: 28, 30, 32, 52, 54 or 56) with Ala.

Examples of an amino acid sequence including the substitution (f') include an amino acid sequence obtained by introducing into the amino acid sequence consisting of amino acids at positions 1 to 164 from the N-terminus of the amino acid sequence set forth in SEQ ID NO: 30 or SEQ ID NO: 54, substitution of Asn at positions 131 and 144 from the N-terminus with Ser (N1315/N144S) (an amino acid sequence consisting of amino acids at positions 1 to 164 from the N-terminus of the amino acid sequence set forth in SEQ ID NO: 34 or SEQ ID NO: 58) and the like.

SEQ ID NO: 34 represents an amino acid sequence of N1315/N144S-HBD (a DcR3 variant obtained by introducing into chimera A-HBD, substitution of Asn at positions 131 and 144 from the N-terminus with Ser), and SEQ ID NO: 58 represents an amino acid sequence of N131S/N144S (a DcR3 variant obtained by introducing into chimera A, substitution of Asn at positions 131 and 144 from the N-terminus with Ser).

Examples of an amino acid sequence including the substitution (h') include an amino acid sequence obtained by introducing into the amino acid sequence consisting of amino acids at positions 1 to 164 from the N-terminus of the amino acid sequence set forth in SEQ ID NO: 30 or SEQ ID NO: 54, substitution of Thr at position 133 and Ser at position 146 from the N-terminus with Ala (T133A/S146A) (an amino acid sequence consisting of amino acids at positions 1 to 164 from the N-terminus of the amino acid sequence set forth in SEQ ID NO: 36 or SEQ ID NO: 60) and the like.

SEQ ID NO: 36 represents an amino acid sequence of T133A/S146A-HBD (a DcR3 variant obtained by introducing into chimera A-HBD, substitution of Thr at position 133 and Ser at position 146 from the N-terminus with Ala), and SEQ ID NO: 60 represents an amino acid sequence of T133A/S146A (a DcR3 variant obtained by introducing into chimera A, substitution of Thr at position 133 and Ser at position 146 from the N-terminus with Ala).

Examples of an amino acid sequence including the substitution (g') include an amino acid sequence obtained by introducing into the amino acid sequence consisting of amino acids at positions 1 to 164 from the N-terminus of the amino acid sequence set forth in SEQ ID NO: 30 or SEQ ID NO: 54, substitution of Asn at positions 131, 144 and 157 from the N-terminus with Ser (N131S/N144S/N157S) (an amino acid sequence consisting of amino acids at positions 1 to 164 from the N-terminus of the amino acid sequence set forth in SEQ ID NO: 38 or SEQ ID NO: 62) and the like.

SEQ ID NO: 38 represents an amino acid sequence of N131S/N144S/N157S-HBD (a DcR3 variant obtained by introducing into chimera A-HBD, substitution of Asn at positions 131, 144 and 157 from the N-terminus with Ser), and SEQ ID NO: 62 represents an amino acid sequence of N131S/N144S/N157S (a DcR3 variant obtained by introducing into chimera A, substitution of Asn at positions 131, 144 and 157 from the N-terminus with Ser).

Examples of an amino acid sequence including the substitution (i') include an amino acid sequence obtained by introducing into the amino acid sequence consisting of amino acids at positions 1 to 164 from the N-terminus of the amino acid sequence set forth in SEQ ID NO: 30 or SEQ ID NO: 54, substitution of Thr at position 133, Ser at position 146 and Thr at position 159 from the N-terminus with Ala (T133A/S146A/T159A) (an amino acid sequence consisting of amino acids at positions 1 to 164 from the N-terminus of the amino acid sequence set forth in SEQ ID NO: 40 or SEQ ID NO: 64) and the like.

SEQ ID NO: 40 represents an amino acid sequence of T133A/S146A/T159A-HBD (a DcR3 variant obtained by introducing into chimera A-HBD, substitution of Thr at position 133, Ser at position 146 and Thr at position 159 from the N-terminus with Ala), and SEQ ID NO: 64 represents an amino acid sequence of T133A/S146A/T159A (a DcR3 variant obtained by introducing into chimera A, substitution of Thr at position 133, Ser at position 146 and Thr at position 159 from the N-terminus with Ala).

With respect to a mutation (modification) to be added to the first chimeric cysteine-rich region, for example, by not introducing a mutation into CRD2 and CRD3 of the first chimeric cysteine-rich region, which are associated with the binding to LIGHT, TL1A and FasL, and introducing a mutation into CRD1 and/or CRD4 of the first chimeric cysteine-rich region, it is possible to obtain a DcR3 variant including a chimeric cysteine-rich region in which the binding activities of LIGHT, TL1A and FasL to the chimeric cysteine-rich region are not lowered. Meanwhile, by introducing a mutation into CRD2 or/and CRD3 of the first chimeric cysteine-rich region, which are associated with the binding to LIGHT, TL1A or FasL, it is possible to obtain a DcR3 variant including a chimeric cysteine-rich region in which the binding activity of LIGHT, TL1A or FasL to the chimeric cysteine-rich region is changed. In other words, by introducing the abovementioned mutation, it is possible to obtain a DcR3 variant including a chimeric cysteine-rich region and having a desirable binding property to LIGHT, TL1A or FasL.

In a preferred embodiment, examples of the DcR3 variant including the second chimeric cysteine-rich region include a DcR3 variant including a chimeric cysteine-rich region and having binding activity to at least one or more of LIGHT, TL1A and FasL, a DcR3 variant including a chimeric cysteine-rich region and having binding activity to all of LIGHT, TL1A and FasL, a DcR3 variant including a chimeric cysteine-rich region, having no binding activity to FasL and having binding activity to one of LIGHT and TL1A, or a DcR3 variant including a chimeric cysteine-rich region, having no binding activity to FasL and having binding activity to both of LIGHT and TL1A, or the like. With respect to the DcR3 variant of the present invention, the meanings of the expressions of "having binding activity to a ligand" and "having no binding activity to a ligand" are as mentioned above.

In a particularly preferred embodiment, examples of the DcR3 variant including the second chimeric cysteine-rich region include a DcR3 variant with lowered FasL binding activity. "Variant with lowered FasL binding activity" means a DcR3 variant including a chimeric cysteine-rich region, having no binding activity to FasL and having binding activity to one or more of LIGHT and TL1A, or a DcR3 variant including a chimeric cysteine-rich region, having no binding activity to FasL and having binding activity to both of LIGHT and TL1A.

In a preferred embodiment, examples of the DcR3 variant including the second chimeric cysteine-rich region include a DcR3 variant including a chimeric cysteine-rich region and having neutralizing activity to at least one or more of LIGHT, TL1A and FasL, a DcR3 variant including a chimeric cysteine-rich region and having neutralizing activity to all of LIGHT, TL1A and FasL, a DcR3 variant including a chimeric cysteine-rich region, having no neutralizing activity to FasL and having neutralizing activity to one of LIGHT and TL1A, or a DcR3 variant including a chimeric cysteine-rich region, having no neutralizing activity to FasL and having neutralizing activity to both of LIGHT and TL1A, or the like. With respect to the DcR3 variant of the present invention, the meanings of the expressions of "having neutralizing activity to a ligand" and "having no neutralizing activity to a ligand" are as mentioned above.

In a particularly preferred embodiment, examples of the DcR3 variant including the second chimeric cysteine-rich region include a DcR3 variant with lowered FasL neutralizing activity. "Variant with lowered FasL neutralizing activity" means a DcR3 variant including a chimeric cysteine-rich region, having no neutralizing activity to FasL and having neutralizing activity to one or more of LIGHT and TL1A, or a DcR3 variant including a chimeric cysteine-rich region, having no neutralizing activity to FasL and having neutralizing activity to both of LIGHT and TL1A.

For example, a DcR3 variant that is a variant with lowered FasL binding activity and includes a chimeric cysteine-rich region can be obtained by producing a modified product in which a binding site to each ligand of DcR3 or a DcR3 variant predicted by a crystal structure analysis or the like is substituted with Ala or another amino acid, and measuring the binding activity and the neutralizing activity to a LIGHT, TL1A or FasL ligand. The DcR3 variant can also be obtained by producing a gene library in which regions around the ligandbinding site of DcR3 or a DcR3 variant are randomly converted to other amino acids, displaying it on a phage, a yeast, a mammalian cell or the like, and performing screening using the binding activity and the neutralizing activity to a LIGHT, TL1A or FasL ligand as indices.

Examples of an amino acid sequence of a chimeric cysteine-rich region included in a variant with lowered FasL binding activity (one embodiment of the amino acid sequence (e)) include an amino acid sequence obtained by introducing into the amino acid sequence (a), (b), (c) or (d), substitution of one or two or more amino acids selected from the group consisting of Glu at position 57, Arg at position 58 and Arg at position 60 from the N-terminus with (an)other amino acid(s).

Another amino acid used for substitution of Glu at position 57 is not particularly limited, and can be appropriately selected from 19 types of amino acids excluding Glu, of 20 types of amino acids (Glu, Ala, Asp, Lys, Leu, Cys, Phe, Gly, His, Ile, Met, Asn, Pro, Gln, Arg, Ser, Thr, Val, Trp and Tyr), but it is preferably selected from Lys, Leu, Arg, Val, Ala, Phe, His, Ile and Met, more preferably selected from Lys, Leu, Arg and Val, still more preferably selected from Lys, Arg and Val, and yet more preferably selected from Lys and Arg.

Another amino acid used for substitution of Arg at position 58 is not particularly limited, and can be appropriately selected from 19 types of amino acids excluding Arg, of 20 types of amino acids, but it is preferably selected from Asp, Glu and Thr, and more preferably selected from Asp and Glu.

Another amino acid used for substitution of Arg at position 60 is not particularly limited, and can be appropriately selected from 19 types of amino acids excluding Arg, of 20 types of amino acids, but it is preferably Lys.

In a preferred embodiment, one amino acid consisting of Glu at position 57 is selected as the one or two or more amino acids selected from the group consisting of Glu at position 57, Arg at position 58 and Arg at position 60.

In further another preferred embodiment, two amino acids consisting of Glu at position 57 and Arg at position 58 are selected as the one or two or more amino acids selected from the group consisting of Glu at position 57, Arg at position 58 and Arg at position 60. In this embodiment, it is preferable to combine substitution of Glu at position 57 with Lys, Leu, Arg, Val, Ala, Phe, His, Ile or Met and substitution of Arg at position 58 with Asp, Glu or Thr, it is more preferable to combine substitution of Glu at position 57 with Lys, Leu, Arg or Val and substitution of Arg at position 58 with Asp or Glu, and it is still more preferable to combine substitution of Glu at position 57 with Lys or Arg and substitution of Arg at position 58 with Asp or Glu.

The second chimeric cysteine-rich region may include substitution of one or two or more amino acids other than Glu at position 57, Arg at position 58 and Arg at position 60 with (an)other amino acid(s). Examples of an amino acid other than Glu at position 57, Arg at position 58 and Arg at position 60 include Trp at position 53, Asn at position 54, Tyr at position 55, Leu at position 56 or the like from the N-terminus of the amino acid sequence (a), (b), (c) or (d).

Another amino acid used for substitution of Trp at position 53 is not particularly limited, and can be appropriately selected from 19 types of amino acids excluding Trp, of 20 types of amino acids, but it is preferably selected from Asp and Asn. Substitution of Trp at position 53 with another amino acid can be combined with, for example, substitution of Glu at position 57 with another amino acid.

Another amino acid used for substitution of Asn at position 54 is not particularly limited, and can be appropriately selected from 19 types of amino acids excluding Asn, of 20 types of amino acids, but it is preferably Asp. Substitution of Asn at position 54 with another amino acid can be combined with, for example, substitution of Glu at position 57 with another amino acid.

Another amino acid used for substitution of Tyr at position 55 is not particularly limited, and can be appropriately selected from 19 types of amino acids excluding Tyr, of 20 types of amino acids, but it is preferably selected from Thr, Asp, Gln and Glu. Substitution of Tyr at position 55 with another amino acid can be combined with, for example, substitution of Glu at position 57 with another amino acid.

Another amino acid used for substitution of Leu at position 56 is not particularly limited, and can be appropriately selected from 19 types of amino acids excluding Leu, of 20 types of amino acids, but it is preferably selected from Asp, Gln, Thr, Glu, Gly, Asn and Pro. Substitution of Leu at position 56 with another amino acid can be combined with, for example, substitution of Glu at position 57 with another amino acid.

Specific examples of an amino acid sequence including substitution of one or two or more amino acids selected from the group consisting of Glu at position 57, Arg at position 58 and Arg at position 60 from the N-terminus of the amino acid sequence (a), (b), (c) or (d) with (an)other amino acid(s) include:
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Lys (an amino acid sequence consisting of amino acids at positions 1 to 164 from the N-terminus of the amino acid sequence set forth in SEQ ID NO: 42);
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Lys (an amino acid sequence consisting of amino acids at positions 1 to 164 from the N-terminus of the amino acid sequence set forth in SEQ ID NO: 66);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Leu (an amino acid sequence consisting of amino acids at positions 1 to 164 from the N-terminus of the amino acid sequence set forth in SEQ ID NO: 44);
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Leu (an amino acid sequence consisting of amino acids at positions 1 to 164 from the N-terminus of the amino acid sequence set forth in SEQ ID NO: 68);
an amino acid sequence obtained by introducing into the amino acid sequence consisting of amino acids at positions 1 to 164 from the N-terminus of the amino acid sequence set forth in SEQ ID NO: 30, substitution of Arg at position 60 from the N-terminus with Lys (an amino acid sequence consisting of amino acids at positions 1 to 164 from the N-terminus of the amino acid sequence set forth in SEQ ID NO: 46);
an amino acid sequence obtained by introducing into the amino acid sequence consisting of amino acids at positions 1 to 164 from the N-terminus of the amino acid sequence set forth in SEQ ID NO: 54, substitution of Arg at position 60 from the N-terminus with Lys (an amino acid sequence consisting of amino acids at positions 1 to 164 from the N-terminus of the amino acid sequence set forth in SEQ ID NO: 70);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Arg;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Arg (an amino acid sequence consisting of amino acids at positions 1 to 164 from the N-terminus of the amino acid sequence set forth in SEQ ID NO: 180);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Val;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Val (an amino acid sequence consisting of amino acids at positions 1 to 164 from the N-terminus of the amino acid sequence set forth in SEQ ID NO: 182);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Ala;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Ala (an amino acid sequence consisting of amino acids at positions 1 to 164 from the N-terminus of the amino acid sequence set forth in SEQ ID NO: 270);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Phe;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Phe (an amino acid sequence consisting of amino acids at positions 1 to 164 from the N-terminus of the amino acid sequence set forth in SEQ ID NO: 272);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with His;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with His (an amino acid sequence consisting of amino acids at positions 1 to 164 from the N-terminus of the amino acid sequence set forth in SEQ ID NO: 274);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Ile;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Ile (an amino acid sequence consisting of amino acids at positions 1 to 164 from the N-terminus of the amino acid sequence set forth in SEQ ID NO: 276);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Met;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Met (an amino acid sequence consisting of amino acids at positions 1 to 164 from the N-terminus of the amino acid sequence set forth in SEQ ID NO: 278);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Lys, and substitution of Arg at position 58 from the N-terminus with Asp;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Lys, and substitution of Arg at position 58 from the N-terminus with Asp (an amino acid sequence consisting of amino acids at positions 1 to 164 from the N-terminus of the amino acid sequence set forth in SEQ ID NO: 184);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Lys, and substitution of Arg at position 58 from the N-terminus with Glu;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Lys, and substitution of Arg at position 58 from the N-terminus with Glu (an amino acid sequence consisting of amino acids at positions 1 to 164 from the N-terminus of the amino acid sequence set forth in SEQ ID NO: 186);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Arg, and substitution of Arg at position 58 from the N-terminus with Asp;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Arg, and substitution of Arg at position 58 from the N-terminus with Asp (an amino acid sequence consisting of amino acids at positions 1 to 164 from the N-terminus of the amino acid sequence set forth in SEQ ID NO: 188);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Lys, and substitution of Arg at position 58 from the N-terminus with Thr;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Lys, and substitution of Arg at position 58 from the N-terminus with Thr (an amino acid sequence consisting of amino acids at positions 1 to 164 from the N-terminus of the amino acid sequence set forth in SEQ ID NO: 280);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Leu, and substitution of Arg at position 58 from the N-terminus with Glu;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Leu, and substitution of Arg at position 58 from the N-terminus with Glu (an amino acid sequence consisting of amino acids at positions 1 to 164 from the N-terminus of the amino acid sequence set forth in SEQ ID NO: 282);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Val, and substitution of Arg at position 58 from the N-terminus with Thr;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Val, and substitution of Arg at position 58 from the N-terminus with Thr (an amino acid sequence consisting of amino acids at positions 1 to 164 from the N-terminus of the amino acid sequence set forth in SEQ ID NO: 284);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Val, and substitution of Arg at position 58 from the N-terminus with TGlu; and
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Val, and substitution of Arg at position 58 from the N-terminus with Glu (an amino acid sequence consisting of amino acids at positions 1 to 164 from the N-terminus of the amino acid sequence set forth in SEQ ID NO: 286).

SEQ ID NO: 42 represents an amino acid sequence of chimera A-E57K-HBD (a DcR3 variant obtained by introducing into chimera A-HBD, substitution of Glu at position 57 from the N-terminus with Lys); SEQ ID NO: 44 represents an amino acid sequence of chimera A-E57L-HBD (a DcR3 variant obtained by introducing into chimera A-HBD, substitution of Glu at position 57 from the N-terminus with Leu); SEQ ID NO: 46 represents an amino acid sequence of chimera A-R60K-HBD (a DcR3 variant obtained by introducing into chimera A-HBD, substitution of Arg at position 60 from the N-terminus with Lys); SEQ ID NO: 66 represents an amino acid sequence of chimera A-E57K (a DcR3 variant obtained by introducing into chimera A, substitution of Glu at position 57 from the N-terminus with Lys); SEQ ID NO: 68 represents an amino acid sequence of chimera A-E57L (a DcR3 variant obtained by introducing into chimera A, Glu at position 57 from the N-terminus with Leu); SEQ ID NO: 70 represents an amino acid sequence of chimera A-R60K (a DcR3 variant obtained by introducing into chimera A, substitution of Arg at position 60 from the N-terminus with Lys); SEQ ID NO: 180 represents an amino acid sequence of chimera A-E57R (a DcR3 variant obtained by introducing into chimera A, substitution of Glu at position 57 from the N-terminus with Arg); SEQ ID NO: 182 represents an amino acid sequence of chimera A-E57V (a DcR3 variant obtained by introducing into chimera A, substitution of Glu at position 57 from the N-terminus with Val); SEQ ID NO: 184 represents an amino acid sequence of chimera A-E57K_R58D (a DcR3 variant obtained by introducing into chimera A, substitution of Glu at position 57 from the N-terminus with Lys, and substitution of Arg at position 58 from the N-terminus with Asp); SEQ ID NO: 186 represents an amino acid sequence of chimera A-E57K_R58E (a DcR3 variant obtained by introducing into chimera A, substitution of Glu at position 57 from the N-terminus with Lys, and substitution of Arg at position 58 from the N-terminus with Glu); SEQ ID NO: 188 represents an amino acid sequence of chimera A-E57R_R58D (a DcR3 variant obtained by introducing into chimera A, substitution of Glu at position 57 from the N-terminus with Arg, and substitution of Arg at position 58 from the N-terminus with Asp); SEQ ID NO: 270 represents an amino acid sequence of chimera A-E57A (a DcR3 variant obtained by introducing into chimera A, substitution of Glu at position 57 from the N-terminus with Ala); SEQ ID NO: 272 represents an amino acid sequence of chimera A-E57F (a DcR3 variant obtained by introducing into chimera A, substitution of Glu at position 57 from the N-terminus with Phe); SEQ ID NO: 274 represents an amino acid sequence of chimera A-E57H (a DcR3 variant obtained by introducing into chimera A, substitution of Glu at position 57 from the N-terminus with His); SEQ ID NO: 276 represents an amino acid sequence of chimera A-E571 (a DcR3 variant obtained by introducing into chimera A, substitution of Glu at position 57 from the N-terminus with Ile); SEQ ID NO: 278 represents an amino acid sequence of chimera A-E57M (a DcR3 variant obtained by introducing into chimera A, substitution of Glu at position 57 from the N-terminus with Met); SEQ ID NO: 280 represents an amino acid sequence of chimera A-E57K_R58T (a DcR3 variant obtained by introducing into chimera A, substitution of Glu at position 57 from the N-terminus with Lys, and substitution of Arg at position 58 from the N-terminus with Thr); SEQ ID NO: 282 represents an amino acid sequence of chimera A-E57K_R58T (a DcR3 variant obtained by introducing into chimera A, substitution of Glu at position 57 from the N-terminus with Lys, and substitution of Arg at position 58 from the N-terminus with Thr); SEQ ID NO: 284 represents an amino acid sequence of chimera A-E57V_R58T (a DcR3 variant obtained by introducing into chimera A, substitution of Glu at position 57 from the N-terminus with Val, and substitution of Arg at position 58 from the N-terminus with Thr); and SEQ ID NO: 286 represents an amino acid sequence of chimera A-E57V_R58E (a DcR3 variant obtained by introducing into chimera A, substitution of Glu at position 57 from the N-terminus with Val, and substitution of Arg at position 58 from the N-terminus with Glu).

### 2-2. Other Region

The DcR3 variant of the present invention may or may not include one or two or more other regions bound to the C-terminal side of the first or second chimeric cysteine-rich region. Examples of the other regions include a part or a whole of a region existing between CRD4 and HBD in wild-type DcR3, a part or a whole of a region following the C-terminus of the amino acid sequence of CRD4 in a TNF receptor superfamily (TNFRSF) molecule other than DcR3, a part or a whole of HBD of wild-type DcR3 and the like. The expression of "other region bound to the C-terminal side of the first or second chimeric cysteine-rich region" is used as a meaning including the case where the other region is directly bound to the C-terminus of the first or second chimeric cysteine-rich region, and the case where the other region is bound to the C-terminus of the first or second chimeric cysteine-rich region via another further region.

In one embodiment, the DcR3 variant of the present invention includes a part or a whole of a region existing between CRD4 and HBD in wild-type DcR3 as the other region bound to the C-terminal side of the first or second chimeric cysteine-rich region. In this embodiment, the other region is preferably directly bound to the C-terminus of the first or second chimeric cysteine-rich region.

In another embodiment, the DcR3 variant of the present invention includes a part or a whole of a region extending to the C-terminus of the amino acid sequence of CRD4 in a TNF receptor superfamily (TNFRSF) molecule other than DcR3 as the other region bound to the C-terminal side of the first or second chimeric cysteine-rich region. In this embodiment, the other region is preferably directly bound to the C-terminus of the first or second chimeric cysteine-rich region. The description on TNFRSF is the same as mentioned above. TNFRSF is preferably OPG.

When the first or second chimeric cysteine-rich region (e.g., the first chimeric cysteine-rich region consisting of the amino acid sequence (b), (c) or (d)) includes substitution of CRD4 of wild-type DcR3 with CRD4 of OPG, the DcR3 variant of the present invention preferably includes a plurality of amino acid residues following the C-terminus of the amino acid sequence of the CRD4 of the OPG as the other region bound to the C-terminus of the chimeric cysteine-rich region. In other words, when CR4 of wild-type DcR3 is substituted with CRD4 of OPG, a plurality of amino acid residues following the C-terminus of the amino acid sequence of the CRD4 of the wild-type DcR3 is also preferably substituted with a plurality of amino acid residues following the C-terminus of the amino acid sequence of the CRD4 of the OPG. As a plurality of amino acid residues following the C-terminus of the amino acid sequence of CRD4 of OPG, for example, amino acid residues at positions 186 to 194 of the amino acid sequence (SEQ ID NO: 14) of OPG are preferable, but it is possible to appropriately adjust the number of amino acid residues used for substitution of a plurality of amino acid residues following the C-terminus of CRD4 of wild-type DcR3. The number of a plurality of amino acid residues extending to the C-terminus of the amino acid sequence of CRD4 of OPG is usually 1 to 12, preferably 1 to 10, more preferably 1 to 9, 1 to 6, or 1 to 3. When the first or second chimeric cysteine-rich region (e.g., the first chimeric cysteine-rich region consisting of the amino acid sequence (a) or (d)) includes CRD4 derived from wild-type DcR3, it is preferable that an amino acid sequence following the C-terminus of the amino acid sequence of the CRD4 of the wild-type DcR3 is bound to the C-terminus of the amino acid sequence of the CRD4 of the first chimeric cysteine-rich region. Examples of such amino acid sequence include amino acids at positions 194 to 195 of SEQ ID NO: 2, but it is possible to appropriately adjust the number of amino acid residues constituting the amino acid sequence following the C-terminus of the amino acid sequence of CRD4 of wild-type DcR3. The number of amino acid residues constituting the amino acid sequence extending to the C-terminus of CRD4 of wild-type DcR3 is usually 1 to 10, preferably 1 to 5, more preferably 1 to 3, and still more preferably 1 to 2.

In further another embodiment, the DcR3 variant of the present invention includes a part or a whole of HBD of wild-type DcR3 as the other region bound to the C-terminal side of the first or second chimeric cysteine-rich region. In this embodiment, the other region may be directly bound to the C-terminus of the first or second chimeric cysteine-rich region, or may be bound to the C-terminus of the first or second chimeric cysteine-rich region via a part or a whole of a region existing between CRD4 and HBD in wild-type DcR3 or via a part or a whole of a region extending to the C-terminus of the amino acid sequence of CRD4 in a TNFRSF molecule other than DcR3.

It is preferable that the DcR3 variant of the present invention does not include HBD and includes amino acid residues at positions 186 to 194 of the amino acid sequence (SEQ ID NO: 14) of OPG as the other regions bound to the C-terminal side of the first or second chimeric cysteine-rich region.

### 3. DcR3 Variant Including Glycan

The DcR3 variant of the present invention also includes a DcR3 variant including at least one glycan. If at least one glycan is bound to the cysteine-rich region included in the DcR3 variant mentioned above or an amino acid residue other than it, any DcR3 variant including a glycan is also included in the present invention.

Glycoprotein has one or two or more glycans. When glycoprotein has two or more glycans, one type of glycan may be included or two or more types of glycans may be included in the glycoprotein. Examples of the glycan included in the glycoprotein include a glycan that is N-glycoside-linked to an amino acid residue (e.g., an asparagine residue, etc.) of a peptide or a protein, a glycan that is O-glycoside-linked to an amino acid residue (e.g., a serine residue, a threonine residue, etc.) of a peptide or a protein, or the like. Examples of the O-linked glycan include core 1, core 2 or the like, or examples of the N-linked glycan include high-mannose-type, hybrid-type or complex glycan, but a complex glycan is preferable.

### 4. DcR3 Variant Including Fc Region

The DcR3 variant of the present invention also includes a protein in which a homogeneous or heterogeneous peptide, polypeptide or protein is bound or fused directly or, if necessary, via an appropriate peptide linker to the N-terminal side or the C-terminal side of the chimeric cysteine-rich region (or a chimeric cysteine-rich region including (an)other region(s)). The number of amino acids constituting the peptide linker is not particularly limited, but examples thereof include 4, 5, 6 or 15 or the like.

Examples of the polypeptide or protein to be bound or fused to the chimeric cysteine-rich region include a polypeptide or protein such as a constant region or Fc region of an immunoglobulin, a peptide that binds to a neonatal Fc receptor (FcRn), albumin, Protein A, Protein G, β-galactosidase, glutathione-S-transferase (GST), a maltose-binding protein, polyhistidine and an FLAG peptide, but it is preferably an Fc region of an immunoglobulin or a mutant thereof (mutated Fc region), and more preferably an Fc region of an immunoglobulin derived from a mammal or a mutant thereof (mutated Fc region).

As an Fc region of an immunoglobulin (also referred to as antibody), an Fc region of a human immunoglobulin is preferable when used for a human. Examples of a class and a subclass of the immunoglobulin include, but are not limited to, IgG, IgD, IgE, IgM, IgA, IgG1, IgG2, IgG2a, IgG2b, IgG2c, IgG3, IgG4 or IgA1 or the like, and, if used for a human, it is preferable to use a class and a subclass of a human immunoglobulin. When an Fc region of an immunoglobulin is used as a polypeptide or protein to be bound or fused to the chimeric cysteine-rich region, preferably the Fc region of the immunoglobulin is bound or fused to the C-terminal side of the chimeric cysteine-rich region.

The immunoglobulin is composed of polypeptides of a heavy chain and a light chain, and the constant region of a heavy chain of human IgG is composed of a CH1 domain, a hinge domain, a CH2 domain and a CH3 domain in this order from the N-terminus. The Fc region of IgG in the present invention also includes a region combining the CH2 domain and the CH3 domain, and a region combining a part or a whole of the hinge domain, the CH2 domain and the CH3 domain. Each domain included in the Fc region of IgG in the present invention can be identified by the number of the EU index. Specifically, the hinge domain is identified by EU-index positions 216 to 230, the CH2 domain is identified by EU-index positions 231 to 340, and CH3 is identified by EU-index positions 341 to 447.

The polypeptide or protein to be bound or fused to the chimeric cysteine-rich region also includes a modified polypeptide or protein further including substitution, deletion, insertion or addition of one or more amino acids in order to change the biological activity of the DcR3 variant or properties thereof, *in vivo* kinetics such as half-life in blood, or physical or chemical properties such as stability of a protein. The modification of the polypeptide or protein to be bound or fused to the chimeric cysteine-rich region may be a natural mutation or an artificial substitution, deletion, insertion or addition of an amino acid. Examples of the modified polypeptide or protein include a mutated Fc region consisting of an amino acid sequence obtained by introducing into the amino acid sequence of the Fc region of the immunoglobulin, substitution, deletion, insertion or addition of one or several amino acids. Examples of the amino acid sequence of the mutated Fc region include an amino acid sequence obtained by introducing into the amino acid sequence of the Fc region of the immunoglobulin, substitution, deletion, insertion or addition of one or several, preferably 2 to 30, more preferably 2 to 10, and particularly preferably 2 to 5 amino acids, or an amino acid sequence having 80% or more, preferably 85% or more, and more preferably 90% or more identity to the amino acid sequence of the Fc region of the immunoglobulin.

An addition or deletion of the abovementioned glycosylation site is also included in the modification of a polypeptide or protein to be bound or fused to the chimeric cysteine-rich region. For example, by adding or inserting a polypeptide including an N-linked glycosylation site, it is possible to add the glycosylation site. Specific examples of the sequence of the polypeptide include GGNGT or YGNGT consisting of five amino acids [WO 2014/153111 A].

Examples of substitution introduced into human IgG1 in order to lower or eliminate complement-dependent cytotoxicity (CDC) activity include substitution of one or two or more amino acids selected from Leu at EU-index position 234 (L234), Leu at EU-index position 235 (L235), Asp at EU-index position 265 (D265), Asp at EU-index position 270 (D265), Lys at EU-index position 322 (K322), Pro at EU-index position 329 (P329), Pro at EU-index position 331 (P331) and the like with (an)other amino acid(s) or the like, and specific examples thereof include substitution of one or two or more amino acids selected from L234, L235, D270, K322, P329, P331 and the like indicated by the EU index with Ala, or substitution of P331 with Ser or Gly [J. Immunol., 2000, 164: p.4178-4184, Cell. Immunol., 2000, 200: p.16-26].

Examples of substitution introduced into human IgG1 in order to lower or eliminate effector activity such as antibody-dependent cellular cytotoxicity (ADCC) activity and antibody-dependent cellular phagocytosis (ADCP) include substitution of one or two or more amino acids selected from Asn at EU-index position 297 (N297), Leu at EU-index position 234 (L234), Leu at EU-index position 235 (L235), Gly at EU-index position 237 (G237), Cys at EU-index position 226 (C226), Cys at EU-index position 229 (C229), Pro at EU-index position 238 (P238), Glu at EU-index position 233 (E233), Ser at EU-index position 267 (S267), Leu at EU-index position 328 (L328), Pro at EU-index position 331 (P331) and the like with (an)other amino acid(s), and specific examples thereof include an amino acid substitution such as substitution of N297 indicated by the EU index with Ala (N297A), substitution of N297 indicated by the EU index with Gln (N297Q), substitution of N297 indicated by the EU index with Gly (N297G), or substitution of L234 indicated by the EU index with Ala (L234A)/substitution of L235 indicated by the EU index with Ala (L235A)/substitution of G237 indicated by the EU index with Ala (G237A) or the like. "/" means "and" (the same hereinafter).

In addition, examples of modification that increases the binding activity to FcγRIIb, which is an inhibitory receptor, include substitution of Gly at EU-index position 236 with Asp (G236D), substitution of Leu at EU-index position 328 with Phe (L328F), substitution of Ser at EU-index position 239 with Asp (S239D), substitution of Ser at EU-index position 267 with Glu (S267E) or the like [Curr. Opin. Cell. Biol., 2009, 20: p.685-691]. Examples of modification that enhances the binding to a neonatal Fc receptor (FcRn) in an environment with low pH in an endosome, thus avoiding the elimination of an antibody and prolonging the half-life in blood include substitution of Thr at EU-index position 250 with Gln (T250Q), substitution of Met at EU-index position 428 with Leu (M428L), substitution of Met at EU-index position 252 with Tyr (M252Y), substitution of Ser at EU-index position 254 with Thr (S254T), substitution of Thr at EU-index position 256 with Glu (T256E), substitution of Met at EU-index position 252 with Tyr (M252Y)/substitution of Ser at EU-index position 254 with Thr (S254T)/substitution of Thr at EU-index position 256 with Glu (T256E), substitution of Met at EU-index position 428 with Leu (M428L)/substitution of Asn at EU-index position 434 with Ser (N434S), substitution of Asn at EU-index position 434 with Ala (N434A), substitution of Asn at EU-index position 434 with His (N434H) or the like [J. Immunol., 2009, 182: p.7663-7671, MAbs, 2017, 9: p.844-853].

In a preferred embodiment, the mutated Fc region includes substitution of Cys at EU-index position 220 with Ser, of an amino acid sequence of a heavy chain of an antibody belonging to a human IgG1 subclass. Examples of the mutated Fc region according to this embodiment include an Fc region of an immunoglobulin including an amino acid sequence obtained by introducing into an Fc region (EU-index positions 217 to 447) in which a CH1 domain and Glu at EU-index position 216 of a hinge domain have been removed from a constant region (SEQ ID NO: 153) of a heavy chain of human IgG1, substitution of Cys at EU-index position 220 , which is associated with the binding to a light chain, with Ser (C220S) (hereinafter referred to as g1S, SEQ ID NO: 72), an Fc region of an immunoglobulin including an amino acid sequence obtained by introducing into an Fc region (EU-index positions 216 to 447) in which a CH1 domain has been removed from a constant region (SEQ ID NO: 153) of a heavy chain of human IgG1, substitution of Cys at position 220 with Ser (C220S) (hereinafter referred to as Eg1S, SEQ ID NO: 156) or the like. When the DcR3 variant of the present invention includes the mutated Fc region according to this embodiment, as the first chimeric cysteine-rich region included in the DcR3 variant of the present invention, referred is the first chimeric cysteine-rich region as defined in the claims including substitution of CRD1 of wild-type DcR3 with CRD1 of OPG and substitution of CRD4 of wild-type DcR3 with CRD4 of OPG. When the DcR3 variant of the present invention includes the mutated Fc region according to this embodiment, the second chimeric cysteine-rich region included in the DcR3 variant of the present invention preferably includes substitution of Glu at position 57 with another amino acid, or substitution of Glu at position 57 with another amino acid and substitution of Arg at position 58 with another amino acid. Another amino acid used for substitution of Glu at position 57 is preferably selected from Lys, Leu, Arg, Val, Ala, Phe, His, Ile and Met, and more preferably selected from Lys, Leu, Arg and Val. Another amino acid used for substitution of Arg at position 58 is preferably selected from Asp, Glu and Thr, and more preferably selected from Asp and Glu. When combining substitution of Glu at position 57 with another amino acid and substitution of Arg at position 58 with another amino acid, it is preferable to combine substitution of Glu at position 57 with Lys, Leu, Arg, Val, Ala, Phe, His, Ile or Met and substitution of Arg at position 58 with Asp, Glu or Thr, it is more preferable to combine substitution of Glu at position 57 with Lys, Leu, Arg or Val and substitution of Arg at position 58 with Asp or Glu, and it is still more preferable to combine substitution of Glu at position 57 with Lys or Arg and substitution of Arg at position 58 with Asp or Glu.

In another preferred embodiment, the mutated Fc region includes substitution of Ser at EU-index position 228 with Pro, substitution of Leu at EU-index position 235 with Glu, and substitution of Arg at EU-index position 409 with Lys, of an amino acid sequence of a heavy chain of an antibody belonging to a human IgG4 subclass. Examples of the mutated Fc region according to this embodiment include Fc of an immunoglobulin including an amino acid sequence including, in addition to removal of a CH1 domain from a constant region (SEQ ID NO: 154) of a heavy chain of human IgG4, substitution of Ser at EU-index position 228 with Pro, substitution of Leu at EU-index position 235 with Glu, and substitution of Arg at EU-index position 409 with Lys as described in WO 2006/33386 A (hereinafter referred to as g4PEK, SEQ ID NO: 74). When the DcR3 variant of the present invention includes the mutated Fc region according to this embodiment, as the first chimeric cysteine-rich region included in the DcR3 variant of the present invention, preferred is the first chimeric cysteine-rich region including substitution of CRD1 of wild-type DcR3 with CRD1 of OPG, and substitution of CRD4 of wild-type DcR3 with CRD4 of OPG. When the DcR3 variant of the present invention includes the mutated Fc region according to this embodiment, the second chimeric cysteine-rich region included in the DcR3 variant of the present invention preferably includes substitution of Glu at position 57 with another amino acid, or substitution of Glu at position 57 with another amino acid and substitution of Arg at position 58 with another amino acid. Another amino acid used for substitution of Glu at position 57 is preferably selected from Lys, Leu, Arg, Val, Ala, Phe, His, Ile and Met, and more preferably selected from Lys, Leu, Arg and Val. Another amino acid used for substitution of Arg at position 58 is preferably selected from Asp, Glu and Thr, and more preferably selected from Asp and Glu. When combining substitution of Glu at position 57 with another amino acid and substitution of Arg at position 58 with another amino acid, it is preferable to combine substitution of Glu at position 57 with Lys, Leu, Arg, Val, Ala, Phe, His, Ile or Met and substitution of Arg at position 58 with Asp, Glu or Thr, it is more preferable to combine substitution of Glu at position 57 with Lys, Leu, Arg or Val and substitution of Arg at position 58 with Asp or Glu, and it is still more preferable to combine substitution of Glu at position 57 with Lys or Arg and substitution of Arg at position 58 with Asp or Glu.

In further another preferred embodiment, the mutated Fc region includes substitution of Leu at EU-index position 234 with Ala, substitution of Leu at EU-index position 235 with Ala, and substitution of Gly at EU-index position 237 with Ala, of an amino acid sequence of a heavy chain of an antibody belonging to a human IgG1 subclass. Examples of the mutated Fc region according to this embodiment include an Fc region of an immunoglobulin including an amino acid sequence obtained by introducing into an Fc region of g1S set forth in SEQ ID NO: 72 or Eg1S set forth in SEQ ID NO: 156, substitution of Leu at position 234 with Ala, substitution of Leu at position 235 with Ala, and substitution of Gly at position 237 with Ala (hereinafter referred to as g1S LALAGA or Eg1S LALAGA, SEQ ID NO: 162 or 164) or the like. When the DcR3 variant of the present invention includes the mutated Fc region according to this embodiment, as the first chimeric cysteine-rich region included in the DcR3 variant of the present invention, preferred is the first chimeric cysteine-rich region including substitution of CRD1 of wild-type DcR3 with CRD1 of OPG, and substitution of CRD4 of wild-type DcR3 with CRD4 of OPG. When the DcR3 variant of the present invention includes the mutated Fc region according to this embodiment, the second chimeric cysteine-rich region included in the DcR3 variant of the present invention preferably includes substitution of Glu at position 57 with another amino acid, or substitution of Glu at position 57 with another amino acid and substitution of Arg at position 58 with another amino acid. Another amino acid used for substitution of Glu at position 57 is preferably selected from Lys, Leu, Arg, Val, Ala, Phe, His, Ile and Met, and more preferably selected from Lys, Leu, Arg and Val. Another amino acid used for substitution of Arg at position 58 is preferably selected from Asp, Glu and Thr, and more preferably selected from Asp and Glu. When combining substitution of Glu at position 57 with another amino acid and substitution of Arg at position 58 with another amino acid, it is preferable to combine substitution of Glu at position 57 with Lys, Leu, Arg, Val, Ala, Phe, His, Ile or Met and substitution of Arg at position 58 with Asp, Glu or Thr, it is more preferable to combine substitution of Glu at position 57 with Lys, Leu, Arg or Val and substitution of Arg at position 58 with Asp or Glu, and it is still more preferable to combine substitution of Glu at position 57 with Lys or Arg and substitution of Arg at position 58 with Asp or Glu.

In further another preferred embodiment, the mutated Fc region includes substitution of Asn at EU-index position 434 with Ala, of an amino acid sequence of a heavy chain of an antibody belonging to a human IgG1 subclass. Examples of the mutated Fc region according to this embodiment include an Fc region of an immunoglobulin including an amino acid sequence obtained by introducing into an Fc region of g1S set forth in SEQ ID NO: 72 or Eg1S set forth in SEQ ID NO: 156, substitution of Asn at position 434 with Ala (hereinafter referred to as g1S N434A or Eg1S N434A, SEQ ID NO: 312 or 160), an Fc region of an immunoglobulin including an amino acid sequence obtained by introducing into an Fc region of g1S LALAGA set forth in SEQ ID NO: 162 or Eg1S LALAGA set forth in SEQ ID NO: 164, substitution of Asn at position 434 with Ala (hereinafter referred to as g1S LALAGANA or Eg1S LALAGANA, SEQ ID NO: 313 or 166) or the like. When the DcR3 variant of the present invention includes the mutated Fc region according to this embodiment, as the first chimeric cysteine-rich region included in the DcR3 variant of the present invention, preferred is the first chimeric cysteine-rich region including substitution of CRD1 of wild-type DcR3 with CRD1 of OPG, and substitution of CRD4 of wild-type DcR3 with CRD4 of OPG. When the DcR3 variant of the present invention includes the mutated Fc region according to this embodiment, the second chimeric cysteine-rich region included in the DcR3 variant of the present invention preferably includes substitution of Glu at position 57 with another amino acid, or substitution of Glu at position 57 with another amino acid and substitution of Arg at position 58 with another amino acid. Another amino acid used for substitution of Glu at position 57 is preferably selected from Lys, Leu, Arg, Val, Ala, Phe, His, Ile and Met, and more preferably selected from Lys, Leu, Arg and Val. Another amino acid used for substitution of Arg at position 58 is preferably selected from Asp, Glu and Thr, and more preferably selected from Asp and Glu. When combining substitution of Glu at position 57 with another amino acid and substitution of Arg at position 58 with another amino acid, it is preferable to combine substitution of Glu at position 57 with Lys, Leu, Arg, Val, Ala, Phe, His, Ile or Met and substitution of Arg at position 58 with Asp, Glu or Thr, it is more preferable to combine substitution of Glu at position 57 with Lys, Leu, Arg or Val and substitution of Arg at position 58 with Asp or Glu, and it is still more preferable to combine substitution of Glu at position 57 with Lys or Arg and substitution of Arg at position 58 with Asp or Glu.

In further another preferred embodiment, the mutated Fc region includes substitution of Met at EU-index position 252 with Tyr, substitution of Ser at EU-index position 254 with Thr, and substitution of Thr at EU-index position 256 with Glu, of an amino acid sequence of a heavy chain of an antibody belonging to a human IgG1 subclass. Examples of the mutated Fc region according to this embodiment include an Fc region of an immunoglobulin including an amino acid sequence obtained by introducing into an Fc region of g1S set forth in SEQ ID NO: 72 or Eg1S set forth in SEQ ID NO: 156, substitution of Met at position 252 with Tyr, substitution of Ser at position 254 with Thr, and substitution of Thr at position 256 with Glu (hereinafter referred to as g1S YTE or Eg1S YTE, SEQ ID NO: 311 or 158) or the like. When the DcR3 variant of the present invention includes the mutated Fc region according to this embodiment, as the first chimeric cysteine-rich region included in the DcR3 variant of the present invention, preferred is the first chimeric cysteine-rich region including substitution of CRD1 of wild-type DcR3 with CRD1 of OPG, and substitution of CRD4 of wild-type DcR3 with CRD4 of OPG. When the DcR3 variant of the present invention includes the mutated Fc region according to this embodiment, the second chimeric cysteine-rich region included in the DcR3 variant of the present invention preferably includes substitution of Glu at position 57 with another amino acid, or substitution of Glu at position 57 with another amino acid and substitution of Arg at position 58 with another amino acid. Another amino acid used for substitution of Glu at position 57 is preferably selected from Lys, Leu, Arg, Val, Ala, Phe, His, Ile and Met, and more preferably selected from Lys, Leu, Arg and Val. Another amino acid used for substitution of Arg at position 58 is preferably selected from Asp, Glu and Thr, and more preferably selected from Asp and Glu. When combining substitution of Glu at position 57 with another amino acid and substitution of Arg at position 58 with another amino acid, it is preferable to combine substitution of Glu at position 57 with Lys, Leu, Arg, Val, Ala, Phe, His, Ile or Met and substitution of Arg at position 58 with Asp, Glu or Thr, it is more preferable to combine substitution of Glu at position 57 with Lys, Leu, Arg or Val and substitution of Arg at position 58 with Asp or Glu, and it is still more preferable to combine substitution of Glu at position 57 with Lys or Arg and substitution of Arg at position 58 with Asp or Glu.

Examples of the mutated Fc region include, but are not limited to, a mutated Fc region including or consisting of an amino acid sequence set forth in SEQ ID NO: 72, 74, 156, 158, 160, 162, 164, 166, 311, 312 or 313 the amino acid sequence.

Examples of the peptide linker to be added in order to link further a homogeneous or heterogeneous peptide, polypeptide or protein to the N-terminal or C-terminal side of the chimeric cysteine-rich region include, but are not limited to, a peptide linker such as an IEGRMD linker or a GS linker, chemical linker or the like.

As the DcR3 variant of the present invention, most preferably a DcR3 variant as claimed including the first or second chimeric cysteine-rich region and the Fc region or the mutated Fc region is exemplified.

In one embodiment, examples of an amino acid sequence included in the DcR3 variant of the present invention include an amino acid sequence set forth in SEQ ID NO: 26, 28, 30, 32, 34, 36, 38, 40, 42, 44 or 46, or an amino acid sequence obtained by introducing into the amino acid sequence, deletion, substitution, insertion or addition of 1 to 30 amino acids. In this embodiment, the DcR3 variant of the present invention may consist of the abovementioned amino acid sequence or may include the abovementioned amino acid sequence, but preferably consists of the abovementioned amino acid sequence. The abovementioned amino acid sequence includes HBD.

In another embodiment, examples of an amino acid sequence included in the DcR3 variant of the present invention include an amino acid sequence set forth in SEQ ID NO: 50, 52, 54, 56, 58, 60, 62, 64, 66, 68, 70, 180, 182, 184, 186, 188, 270, 272, 274, 276, 278, 280, 282, 284 or 286, or an amino acid sequence obtained by introducing into the amino acid sequence, deletion, substitution, insertion or addition of 1 to 30 amino acids. In this embodiment, the DcR3 variant of the present invention may consist of the abovementioned amino acid sequence or may include the abovementioned amino acid sequence, but preferably consists of the abovementioned amino acid sequence. The abovementioned amino acid sequence does not include HBD.

In another embodiment, examples of an amino acid sequence included in the DcR3 variant of the present invention include an amino acid sequence including both of an amino acid sequence set forth in SEQ ID NO: 50, 52, 54, 56, 58, 60, 62, 64, 66, 68, 70, 180, 182, 184, 186, 188, 270, 272, 274, 276, 278, 280, 282, 284 or 286, or an amino acid sequence obtained by introducing into the amino acid sequence, deletion, substitution, insertion or addition of 1 to 5 amino acids, and, as a mutated Fc region, an amino acid sequence set forth in SEQ ID NO: 72, 74, 156, 158, 160, 162, 164, 166, 311, 312 or 313.

In further another embodiment, examples of an amino acid sequence included in the DcR3 variant of the present invention include an amino acid sequence set forth in SEQ ID NO: 76, 78, 80, 82, 84, 86, 88, 90, 92, 94, 96, 98, 150, 168, 170, 172, 174, 176, 178, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 288, 290, 292, 294, 296, 298, 300, 302, 304, 314, 315, 316, 317, 318, 319, 320, 321, 322, 323, 324, 325, 326, 327, 328, 329, 330, 331, 332, 333, 334, 335, 336 or 337, or an amino acid sequence or an amino acid sequence having 99% or more identity thereto In this embodiment, the DcR3 variant of the present invention may consist of the abovementioned amino acid sequence or may include the abovementioned amino acid sequence, but preferably consists of the abovementioned amino acid sequence. The abovementioned amino acid sequence includes an Fc region or a mutant thereof (mutated Fc region).

SEQ ID NO: 76 represents an amino acid sequence of chimera B-Fc (IEGRMD g1S) (a DcR3 variant obtained by fusion of chimera B, an IEGRMD linker and Fc (g1S)); SEQ ID NO: 78 represents an amino acid sequence of chimera C-Fc (IEGRMD g1S) (a DcR3 variant obtained by fusion of chimera C, an IEGRMD linker and Fc (g1S)); SEQ ID NO: 80 represents an amino acid sequence of chimera A-Fc (IEGRMD g1S) (a DcR3 variant obtained by fusion of chimera A, an IEGRMD linker and Fc (g1S)); SEQ ID NO: 82 represents an amino acid sequence of chimera A-Fc (g4PEK) (a DcR3 variant obtained by fusion of chimera A and Fc (g4PEK)); SEQ ID NO: 84 represents an amino acid sequence of 103-1230PG-Fc (g4PEK) (a DcR3 variant obtained by fusion of 103-123OPG and Fc (g4PEK)); SEQ ID NO: 86 represents an amino acid sequence of N131S/N144S-Fc (g4PEK) (a DcR3 variant obtained by fusion of N131S/N144S and Fc (g4PEK)); SEQ ID NO: 88 represents an amino acid sequence of T133A/S146A-Fc (g4PEK) (a DcR3 variant obtained by fusion of T133A/S146A and Fc (g4PEK)); SEQ ID NO: 90 represents an amino acid sequence of N131S/N144S/N157S-Fc (g4PEK) (a DcR3 variant obtained by fusion of N131S/N144S/N157S and Fc (g4PEK)); SEQ ID NO: 92 represents an amino acid sequence of T133A/S146A/T159A-Fc (g4PEK) (a DcR3 variant obtained by fusion of T133A/S146A/T159A and Fc (g4PEK)); SEQ ID NO: 94 represents an amino acid sequence of chimera A-E57K-Fc (g4PEK) (a DcR3 variant obtained by fusion of chimera A-E57K and Fc (g4PEK)); SEQ ID NO: 96 represents an amino acid sequence of chimera A-E57L-Fc (g4PEK) (a DcR3 variant obtained by fusion of chimera A-E57L and Fc (g4PEK)); SEQ ID NO: 98 represents an amino acid sequence of chimera A-R60K-Fc (g4PEK) (a DcR3 variant obtained by fusion of chimera A-R60K and Fc (g4PEK)); SEQ ID NO: 150 represents an amino acid sequence of chimera A-Fc (g1S) (a DcR3 variant obtained by fusion of chimera A and Fc (g1S)); SEQ ID NO: 168 represents an amino acid sequence of chimera A-Fc (Eg1S) (a DcR3 variant obtained by fusion of chimera A and Fc (Eg1S)); SEQ ID NO: 170 represents an amino acid sequence of chimera A-Fc (Eg1S-YTE) (a DcR3 variant obtained by fusion of chimera A and Fc (Eg1S YTE)); SEQ ID NO: 172 represents an amino acid sequence of chimera A-Fc (Eg1S-N434A) (a DcR3 variant obtained by fusion of chimera A and Fc (Eg15-N434A)); SEQ ID NO: 174 represents an amino acid sequence of chimera A-Fc (g1S-LALAGA) (a DcR3 variant obtained by fusion of chimera A and Fc (g1S-LALAGA)); SEQ ID NO: 176 represents an amino acid sequence of chimera A-Fc (Eg1S-LALAGA) (a DcR3 variant obtained by fusion of chimera A and Fc (Eg1S-LALAGA)); SEQ ID NO: 178 represents an amino acid sequence of chimera A-Fc (Eg1S-LALAGANA) (a DcR3 variant obtained by fusion of chimera A and Fc (Eg1S-LALAGANA)); SEQ ID NO: 190 represents an amino acid sequence of chimera A-E57R-Fc (g4PEK) (a DcR3 variant obtained by fusion of chimera A-E57R and Fc (g4PEK)); SEQ ID NO: 192 represents an amino acid sequence of chimera A-E57V-Fc (g4PEK) (a DcR3 variant obtained by fusion of chimera A-E57V and Fc (g4PEK)); SEQ ID NO: 194 represents an amino acid sequence of chimera A-E57K_R58D-Fc (g4PEK) (a DcR3 variant obtained by fusion of chimera A-E57K_R58D and Fc (g4PEK)); SEQ ID NO: 196 represents an amino acid sequence of chimera A-E57K_R58E-Fc (g4PEK) (a DcR3 variant obtained by fusion of chimera A-E57K_R58E and Fc (g4PEK)); SEQ ID NO: 198 represents an amino acid sequence of chimera A-E57R_R58D-Fc (g4PEK) (a DcR3 variant obtained by fusion of chimera A-E57R_R58D and Fc (g4PEK)); SEQ ID NO: 200 represents an amino acid sequence of chimera A-E57K-Fc (Eg1S) (a DcR3 variant obtained by fusion of chimera A-E57K and Fc (Eg1S)); SEQ ID NO: 202 represents an amino acid sequence of chimera A-E57L-Fc (Eg1S) (a DcR3 variant obtained by fusion of chimera A-E57L and Fc (Eg1S)); SEQ ID NO: 204 represents an amino acid sequence of chimera A-E57R-Fc (Eg1S) (a DcR3 variant obtained by fusion of chimera A-E57R and Fc (Eg1S)); SEQ ID NO: 206 represents an amino acid sequence of chimera A-E57V-Fc (Eg1S) (a DcR3 variant obtained by fusion of chimera A-E57V and Fc (Eg1S)); SEQ ID NO: 208 represents an amino acid sequence of chimera A-E57K_R58D-Fc (Eg1S) (a DcR3 variant obtained by fusion of chimera A-E57K_R58D and Fc (Eg1S)); SEQ ID NO: 210 represents an amino acid sequence of chimera A-E57K_R58E-Fc (Eg1S) (a DcR3 variant obtained by fusion of chimera A-E57K_R58E and Fc (Eg1S)); SEQ ID NO: 212 represents an amino acid sequence of chimera A-E57R_R58D-Fc (Eg1S) (a DcR3 variant obtained by fusion of chimera A-E57R_R58D and Fc (Eg1S)); SEQ ID NO: 214 represents an amino acid sequence of chimera A-E57K-Fc (Eg1S YTE) (a DcR3 variant obtained by fusion of chimera A-E57K and Fc (Eg1S YTE)); SEQ ID NO: 216 represents an amino acid sequence of chimera A-E57L-Fc (Eg1S YTE) (a DcR3 variant obtained by fusion of chimera A-E57L and Fc (Eg1S YTE)); SEQ ID NO: 218 represents an amino acid sequence of chimera A-E57R-Fc (Eg1S YTE) (a DcR3 variant obtained by fusion of chimera A-E57R and Fc (Eg1S YTE)); SEQ ID NO: 220 represents an amino acid sequence of chimera A-E57V-Fc (Eg1S YTE) (a DcR3 variant obtained by fusion of chimera A-E57V and Fc (Eg1S YTE)); SEQ ID NO: 222 represents an amino acid sequence of chimera A-E57K_R58D-Fc (Eg1S YTE) (a DcR3 variant obtained by fusion of chimera A-E57K_R58D and Fc (Eg1S YTE)); SEQ ID NO: 224 represents an amino acid sequence of chimera A-E57K_R58E-Fc (Eg1S YTE) (a DcR3 variant obtained by fusion of chimera A-E57K_R58E and Fc (Eg1S YTE)); SEQ ID NO: 226 represents an amino acid sequence of chimera A-E57R_R58D-Fc (Eg1S YTE) (a DcR3 variant obtained by fusion of chimera A-E57R_R58D and Fc (Eg1S YTE)); SEQ ID NO: 228 represents an amino acid sequence of chimera A-E57K-Fc (Eg1S N434A) (a DcR3 variant obtained by fusion of chimera A-E57K and Fc (Eg1S N434A)); SEQ ID NO: 230 represents an amino acid sequence of chimera A-E57L-Fc (Eg1S N434A) (a DcR3 variant obtained by fusion of chimera A-E57L and Fc (Eg1S N434A)); SEQ ID NO: 232 represents an amino acid sequence of chimera A-E57R-Fc (Eg1S N434A) (a DcR3 variant obtained by fusion of chimera A-E57R and Fc (Eg1S N434A)); SEQ ID NO: 234 represents an amino acid sequence of chimera A-E57V-Fc (Eg1S N434A) (a DcR3 variant obtained by fusion of chimera A-E57V and Fc (Eg1S N434A)); SEQ ID NO: 236 represents an amino acid sequence of chimera A-E57K_R58D-Fc (Eg1S N434A) (a DcR3 variant obtained by fusion of chimera A-E57K_R58D and Fc (Eg1S N434A)); SEQ ID NO: 238 represents an amino acid sequence of chimera A-E57K_R58E-Fc (Eg1S N434A) (a DcR3 variant obtained by fusion of chimera A-E57K_R58E and Fc (Eg1S N434A)); SEQ ID NO: 240 represents an amino acid sequence of chimera A-E57R_R58D-Fc (Eg1S N434A) (a DcR3 variant obtained by fusion of chimera A-E57R_R58D and Fc (Eg1S N434A)); SEQ ID NO: 242 represents an amino acid sequence of chimera A-E57K-Fc (Eg1S LALAGA) (a DcR3 variant obtained by fusion of chimera A-E57K and Fc (Eg1S LALAGA)); SEQ ID NO: 244 represents an amino acid sequence of chimera A-E57L-Fc (Eg1S LALAGA) (a DcR3 variant obtained by fusion of chimera A-E57L and Fc (Eg1S LALAGA)); SEQ ID NO: 246 represents an amino acid sequence of chimera A-E57R-Fc (Eg1S LALAGA) (a DcR3 variant obtained by fusion of chimera A-E57R and Fc (Eg1S LALAGA)); SEQ ID NO: 248 represents an amino acid sequence of chimera A-E57V-Fc (Eg1S LALAGA) (a DcR3 variant obtained by fusion of chimera A-E57V and Fc (Eg1S LALAGA)); SEQ ID NO: 250 represents an amino acid sequence of chimera A-E57K_R58D-Fc (Eg1S LALAGA) (a DcR3 variant obtained by fusion of chimera A-E57K_R58D and Fc (Eg1S LALAGA)); SEQ ID NO: 252 represents an amino acid sequence of chimera A-E57K_R58E-Fc (Eg1S LALAGA) (a DcR3 variant obtained by fusion of chimera A-E57K_R58E and Fc (Eg1S LALAGA)); SEQ ID NO: 254 represents an amino acid sequence of chimera A-E57R_R58D-Fc (Eg1S LALAGA) (a DcR3 variant obtained by fusion of chimera A-E57R_R58D and Fc (Eg1S LALAGA)); SEQ ID NO: 256 represents an amino acid sequence of chimera A-E57K-Fc (Eg1S LALAGANA) (a DcR3 variant obtained by fusion of chimera A-E57K and Fc (Eg1S LALAGANA)); SEQ ID NO: 258 represents an amino acid sequence of chimera A-E57L-Fc (Eg1S LALAGANA) (a DcR3 variant obtained by fusion of chimera A-E57L and Fc (Eg1S LALAGANA)); SEQ ID NO: 260 represents an amino acid sequence of chimera A-E57R-Fc (Eg1S LALAGANA) (a DcR3 variant obtained by fusion of chimera A-E57R and Fc (Eg1S LALAGANA)); SEQ ID NO: 262 represents an amino acid sequence of chimera A-E57V-Fc (Eg1S LALAGANA) (a DcR3 variant obtained by fusion of chimera A-E57V and Fc (Eg1S LALAGANA)); SEQ ID NO: 264 represents an amino acid sequence of chimera A-E57K_R58D-Fc (Eg1S LALAGANA) (a DcR3 variant obtained by fusion of chimera A-E57K_R58D and Fc (Eg1S LALAGANA)); SEQ ID NO: 266 represents an amino acid sequence of chimera A-E57K_R58E-Fc (Eg1S LALAGANA) (a DcR3 variant obtained by fusion of chimera A-E57K_R58E and Fc (Eg1S LALAGANA)); SEQ ID NO: 268 represents an amino acid sequence of chimera A-E57R_R58D-Fc (Eg1S LALAGANA) (a DcR3 variant obtained by fusion of chimera A-E57R_R58D and Fc (Eg1S LALAGANA)); SEQ ID NO: 288 represents an amino acid sequence of chimera A-E57A-Fc (g4PEK) (a DcR3 variant obtained by fusion of chimera A-E57A and Fc (g4PEK)); SEQ ID NO: 290 represents an amino acid sequence of chimera A-E57F-Fc (g4PEK) (a DcR3 variant obtained by fusion of chimera A-E57F and Fc (g4PEK)); SEQ ID NO: 292 represents an amino acid sequence of chimera A-E57H-Fc (g4PEK) (a DcR3 variant obtained by fusion of chimera A-E57H and Fc (g4PEK)); SEQ ID NO: 294 represents an amino acid sequence of chimera A-E57I-Fc (g4PEK) (a DcR3 variant obtained by fusion of chimera A-E57I and Fc (g4PEK)); SEQ ID NO: 296 represents an amino acid sequence of chimera A-E57M-Fc (g4PEK) (a DcR3 variant obtained by fusion of chimera A-E57M and Fc (g4PEK)); SEQ ID NO: 298 represents an amino acid sequence of chimera A-E57K_R58T-Fc (g4PEK) (a DcR3 variant obtained by fusion of chimera A-E57K_R58T and Fc (g4PEK)); SEQ ID NO: 300 represents an amino acid sequence of chimera A-E57L_R58E-Fc (g4PEK) (a DcR3 variant obtained by fusion of chimera A-E57L_R58E and Fc (g4PEK)); SEQ ID NO: 302 represents an amino acid sequence of chimera A-E57V_R58T-Fc (g4PEK) (a DcR3 variant obtained by fusion of chimera A-E57V_R58T and Fc (g4PEK)); SEQ ID NO: 304 represents an amino acid sequence of chimera A-E57V_R58E-Fc (g4PEK) (a DcR3 variant obtained by fusion of chimera A-E57V_R58E and Fc (g4PEK)); SEQ ID NO: 314 represents an amino acid sequence of chimera A-Fc (g1S YTE) (a DcR3 variant obtained by fusion of chimera A and Fc (g1S YTE)); SEQ ID NO: 315 represents an amino acid sequence of chimera A-Fc (g15 N434A) (a DcR3 variant obtained by fusion of chimera A and Fc (g1S N434A)); SEQ ID NO: 316 represents an amino acid sequence of chimera A-Fc (g15 LALAGANA) (a DcR3 variant obtained by fusion of chimera A and Fc (g1S LALAGANA)); SEQ ID NO: 317 represents an amino acid sequence of chimera A-E57K-Fc (g1S YTE) (a DcR3 variant obtained by fusion of chimera A-E57K and Fc (g1S YTE)); SEQ ID NO: 318 represents an amino acid sequence of chimera A-E57L-Fc (g1S YTE) (a DcR3 variant obtained by fusion of chimera A-E57L and Fc (g1S YTE)); SEQ ID NO: 319 represents an amino acid sequence of chimera A-E57R-Fc (g15 YTE) (a DcR3 variant obtained by fusion of chimera A-E57R and Fc (g1S YTE)); SEQ ID NO: 320 represents an amino acid sequence of chimera A-E57V-Fc (g1S YTE) (a DcR3 variant obtained by fusion of chimera A-E57V and Fc (g1S YTE)); SEQ ID NO: 321 represents an amino acid sequence of chimera A-E57K_R58D-Fc (g1S YTE) (a DcR3 variant obtained by fusion of chimera A-E57K_R58D and Fc (g1S YTE)); SEQ ID NO: 322 represents an amino acid sequence of chimera A-E57K_R58E-Fc (g1S YTE) (a DcR3 variant obtained by fusion of chimera A-E57K_R58E and Fc (g1S YTE)); SEQ ID NO: 323 represents an amino acid sequence of chimera A-E57R_R58D-Fc (g1S YTE) (a DcR3 variant obtained by fusion of chimera A-E57R_R58D and Fc (g1S YTE)); SEQ ID NO: 324 represents an amino acid sequence of chimera A-E57K-Fc (g1S N434A) (a DcR3 variant obtained by fusion of chimera A-E57K and Fc (g1S N434A)); SEQ ID NO: 325 represents an amino acid sequence of chimera A-E57L-Fc (g1S N434A) (a DcR3 variant obtained by fusion of chimera A-E57L and Fc(g1S N434A)); SEQ ID NO: 326 represents an amino acid sequence of chimera A-E57R-Fc (g1S N434A) (a DcR3 variant obtained by fusion of chimera A-E57R and Fc (g1S N434A)); SEQ ID NO: 327 represents an amino acid sequence of chimera A-E57V-Fc (g1S N434A) (a DcR3 variant obtained by fusion of chimera A-E57V and Fc (g1S N434A)); SEQ ID NO: 328 represents an amino acid sequence of chimera A-E57K_R58D-Fc (g1S N434A) (a DcR3 variant obtained by fusion of chimera A-E57K_R58D and Fc (g1S N434A)); SEQ ID NO: 329 represents an amino acid sequence of chimera A-E57K_R58E-Fc (g1S N434A) (a DcR3 variant obtained by fusion of chimera A-E57K_R58E and Fc (g1S N434A)); SEQ ID NO: 330 represents an amino acid sequence of chimera A-E57R_R58D-Fc (g1S N434A) (a DcR3 variant obtained by fusion of chimera A-E57R_R58D and Fc (g1S N434A)); SEQ ID NO: 331 represents an amino acid sequence of chimera A-E57K-Fc (g1S LALAGANA) (a DcR3 variant obtained by fusion of chimera A-E57K and Fc (g1S LALAGANA)); SEQ ID NO: 332 represents an amino acid sequence of chimera A-E57L-Fc (g1S LALAGANA) (a DcR3 variant obtained by fusion of chimera A-E57L and Fc (g1S LALAGANA)); SEQ ID NO: 333 represents an amino acid sequence of chimera A-E57R-Fc (g1S LALAGANA) (a DcR3 variant obtained by fusion of chimera A-E57R and Fc (g1S LALAGANA)); SEQ ID NO: 334 represents an amino acid sequence of chimera A-E57V-Fc (g1S LALAGANA) (a DcR3 variant obtained by fusion of chimera A-E57V and Fc (g1S LALAGANA)); SEQ ID NO: 335 represents an amino acid sequence of chimera A-E57K_R58D-Fc (g1S LALAGANA) (a DcR3 variant obtained by fusion of chimera A-E57K_R58D and Fc (g1S LALAGANA)); SEQ ID NO: 336 represents an amino acid sequence of chimera A-E57K_R58E-Fc (g1S LALAGANA) (a DcR3 variant obtained by fusion of chimera A-E57K_R58E and Fc (g15 LALAGANA)); and SEQ ID NO: 337 represents an amino acid sequence of chimera A-E57R_R58D-Fc (g1S LALAGANA) (a DcR3 variant obtained by fusion of chimera A-E57R_R58D and Fc (g1S LALAGANA)).

In any of the abovementioned embodiments, the mutation (modification) to be added to the abovementioned amino acid sequence may be a natural mutation or an artificial substitution, deletion, insertion or addition of an amino acid. In any of the abovementioned embodiments, examples of an amino acid sequence obtained by introducing into the abovementioned amino acid sequence, deletion, substitution, insertion or addition of 1 to 30 amino acids include an amino acid sequence obtained by introducing into the abovementioned amino acid sequence, deletion, substitution, insertion or addition of 1 or 2 or more, preferably 2 to 30, more preferably 2 to 10, and particularly preferably 2 to 5 amino acids, or an amino acid sequence having 80% or more, preferably 85% or more, more preferably 90% or more, for example, 93% or more, 95% or more, 97% or more, 98% or more or 99% or more identity to the abovementioned amino acid sequence.

The DcR3 variant of the present invention may be further chemically modified in order to change the biological activity or properties thereof, *in vivo* kinetics such as half-life in blood, or physical or chemical properties such as stability of a protein.

Examples of the chemical modification include polyethylene glycolylation (PEGylation), acetylation, amidation or phosphorylation or the like, and PEGylation is particularly preferable. PEGylation means, for example, binding of one or a plurality of PEG molecules to an amino group of the N-terminus of a protein, or an amino acid residue having a functional group, such as an ε-amino group of Lys, a carboxyl group, a thiol group or a hydroxyl group, at the side chain.

The average molecular weight of the PEG molecule can be used in the range of about 3,000 to about 50,000, but it is not limited thereto.

Examples of the method for binding the PEG molecule to the DcR3 variant include a method introducing, for example, an active group such as a carboxyl group, a formyl (aldehyde) group, an N-hydroxysuccinimide ester group, an amino group, a thiol group or a maleimide group into the PEG terminal part, thus reacting with a functional group such as an amino group, a carboxyl group, a thiol group or a hydroxyl group included at the side chain of the DcR3 variant.

Examples of the signal peptide include, but are not limited to, a signal peptide of human DcR3 consisting of an amino acid sequence at positions 1 to 29 of the amino acid sequence set forth in SEQ ID NO: 2 when the CRD domain at the N-terminal side included in the DcR3 variant is derived from human DcR3. When the CRD domain at the N-terminal side included in the DcR3 variant is derived from human OPG, a signal peptide derived from human OPG consisting of an amino acid sequence at positions 1 to 21 of the amino acid sequence set forth in SEQ ID NO: 14 is exemplified. All of an artificial sequence, a sequence derived from an expression vector, and a sequence derived from another protein suitable for a host cell in which a DcR3 variant is expressed are also included in the signal peptide of the present invention. A DcR3 variant including the signal peptide is a premature polypeptide, and in one embodiment, the signal peptide is cleaved in a maturation process. The DcR3 variant of the present invention also includes a DcR3 variant having a different N-terminus resulting from cleavage of the abovementioned signal peptide at a position different from a predicted site.

### 5. Method for Producing DcR3 Variant

The DcR3 variant of the present invention can be produced by using the method mentioned in Molecular Cloning: A Laboratory Manual, 3rd edition, Cold Spring Harbor Laboratory Press (2001) or the like and expressing a DNA encoding the DcR3 variant in a host cell by, for example, the following method.

Examples of the signal peptide include, but are not limited to, a signal peptide of human DcR3 consisting of an amino acid sequence at positions 1 to 29 set forth in SEQ ID NO: 2 when the CRD domain at the N-terminal side included in the DcR3 variant is derived from human DcR3. When the CRD domain at the N-terminal side included in the DcR3 variant is derived from human OPG, a signal peptide derived from human OPG consisting of an amino acid sequence at positions 1 to 21 set forth in SEQ ID NO: 14 is exemplified. All of an artificial sequence, a sequence derived from an expression vector, and a sequence derived from another protein suitable for a host cell in which a DcR3 variant is expressed can also be used for production of the DcR3 variant of the present invention. The DcR3 variant of the present invention also includes a DcR3 variant having a different N-terminus obtained by cleaving the abovementioned signal peptide at a position different from a predicted site.

The DcR3 variant of the present invention can be obtained by artificially designing based on an amino acid sequence of a cysteine-rich region of wild-type DcR3 before amino acid substitution, an amino acid sequence of a cysteine-rich region of a DcR3 variant, or an amino acid sequence of a DcR3 variant, or can be obtained by analyzing a mutant. As the mutation introduction method, a site-specific mutagenesis method utilizing the PCR method using a primer is preferable (Kunkel et al., Proc. Natl. Acad. Sci. USA, 1985, 82: 488-492). Other examples thereof include a method for totally synthesizing a gene including a mutation introduced thereinto, or a method in which, using a primer including a mutation, PCR is performed for a part before a mutation site and a part after the mutation site separately, and the two fragments are linked by an overlap of a region including the mutation, followed by insertion into a vector by the in-fusion cloning method (Clontech Laboratories, Inc.) or the like.

Examples of the method for identifying a mutation having a target binding mode include a method for obtaining a mutant having a target binding mode by producing a library including mutations randomly introduced thereinto, displaying it on a phage, a yeast or the like, and performing screening using the binding activity. Alternatively, exemplified is a method for obtaining a mutant having a target binding mode by expressing in a host cell a vector including a DNA mutation thereinto for substituting a certain amino acid with a different amino acid and producing the mutant. The DNA encoding the DcR3 variant of the present invention can be synthesized with a DNA synthesizer by designing, from the amino acid sequence of the DcR3 variant of the present invention, a nucleotide sequence encoding it. The DNA can also be isolated by PCR using a cDNA of a human or the like as a template.

By inserting the DNA encoding the DcR3 variant of the present invention obtained above into downstream of a promoter of an appropriate expression vector, a recombinant vector is produced, and the recombinant vector is introduced into a host cell compatible with the expression vector.

As the nucleotide sequence of the DNA encoding the DcR3 variant, a nuceleotide can be substituted so that the codon is optimal for expression in a host, thereby it is possible to improve the production rate of a target DcR3 variant. When the DNA encoding the DcR3 variant mentioned above is produced, it is possible to produce the DNA by adding a DNA encoding a signal peptide of a secretory protein at the 5' end thereof, using the DNA to produce a recombinant vector in the same manner as mentioned above, and introducing the recombinant vector into a host cell to allow secretion of the peptide into a medium. Examples of the signal peptide include a sequence derived from DcR3 or OPG, an artificial sequence, a sequence derived from an expression vector, or a sequence derived from another protein suitable for a host cell.

In one embodiment, examples of the nucleotide sequence of the DNA encoding the amino acid sequence of the DcR3 variant include nucleotide sequences set forth in SEQ ID NO: 25, 27, 29, 31, 33, 35, 37, 39, 41, 43 and 45.

In another embodiment, examples of the nucleotide sequence of the DNA encoding the amino acid sequence of the DcR3 variant include nucleotide sequences set forth in SEQ ID NO: 49, 51, 53, 55, 57, 59, 61, 63, 65, 67, 69, 179, 181, 183, 185, 187, 269, 271, 273, 275, 277, 279, 281, 283 and 285.

In further another embodiment, examples of the nucleotide sequence of the DNA encoding the amino acid sequence of the DcR3 variant include nucleotide sequences set forth in SEQ ID NO: 75, 77, 79, 81, 83, 85, 87, 89, 91, 93, 95, 97, 149, 167, 169, 171, 173, 175, 177, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 287, 289, 291, 293, 295, 297, 299, 301 and 303.

As the expression vector, any expression vector can be used as long as autonomous replication in a host cell used or integration into a chromosome is possible, and an appropriate promoter is contained at a position such that a DNA encoding a polypeptide can be transcribed.

As the host cell, any cell can be used as long as it can express a gene encoding a DcR3 variant, such as a yeast, an insect cell or an animal cell. Preferably a yeast, animal cell or insect cell that is capable of glycosylating a protein is exemplified.

Examples of the yeast include a microorganism belonging to the genus *Saccharomyces,* the genus *Schizosaccharomyces,* the genus *Kluyveromyces,* the genus *Trichosporon,* the genus *Schwanniomyces,* the genus *Pichia,* the genus *Candida* or the like, for example, *Saccharomyces cerevisiae, Schizosaccharomyces pombe, Kluyveromyces lactis, Trichosporon pullulans, Schwanniomyces alluvius,* or *Candida utilis* or the like.

Examples of the insect cell include Sf9 or Sf21, which is an ovary cell of *Spodoptera frugiperda* [Baculovirus Expression Vectors, A Laboratory Manual, W. H. Freeman and Company, New York (1992)], High 5 (manufactured by Invitrogen), which is an ovary cell of *Trichoplusia ni,* or an S2 (Schneider 2) cell (Thermo Scientific) derived from a terminal embryo of *Drosophila melanogaster.*

Examples of the animal cell include a Namalwa cell, which is a human cell, a COS cell, which is a monkey cell, a CHO cell, which is a Chinese hamster cell [Journal of Experimental Medicine, 108, 945 (1958); Proc. Natl. Acad. Sci. USA, 60, 1275 (1968); Genetics, 55, 513 (1968); Chromosoma, 41, 129 (1973); Methods in Cell Science, 18, 115 (1996); Radiation Research, 148, 260 (1997); Proc. Natl. Acad. Sci. USA, 77, 4216 (1980); Proc. Natl. Acad. Sci. USA, 60, 1275 (1968); Cell, 6, 121 (1975); Molecular Cellgenetics, Appendix I, II (pp.883-900)], CHO/DG44, CHO-K1 (ATCC Number :CCL-61), an Freestyle CHO-S cell, an CHO cell deficient in a dihydrofolate reductase gene [Proc. Natl. Acad. Sci. USA, 77, 4216 (1980)], a CHO cell deficient in a 6-fucose transferase gene (WO 2005/035586 A, WO 02/31140 A), a 293 cell, which is a human cell (ATCC Number: CRL-1573), a Freestyle 293F cell, an Expi293 cell (Thermo Scientific), DUkXB11 (ATCC Number: CCL-9096), Pro-5 (ATCC Number: CCL-1781), CHO-S (Life Technologies, Cat#11619), Pro-3, a rat myeloma cell YB2/3HL.P2.G11.16Ag.20 (or also referred to as YB2/0), a mouse myeloma cell NSO, a mouse myeloma cell SP2/0-Ag14, or a Syrian hamster cell BHK, HBT5637 (JP 63-299 A) or the like.

When a yeast is used as a host cell, examples of the expression vector can include YEP13 (ATCC Number: 37115), YEp24 (ATCC37051), YCp50 (ATCC37419), pHS19 or pHS15 or the like.

As the promoter, any promoter may be used as long as it can function in a yeast strain, and examples thereof can include a promoter of a glycolytic gene such as hexosekinase, a PHO5 promoter, a PGK promoter, a GAP promoter, an ADH promoter, a gal 1 promoter, a gal 10 promoter, a heat shock polypeptide promoter, an MFα1 promoter or a CUP 1 promoter or the like.

As the method for introducing a recombinant vector, any method can be used as long as it introduces a DNA into a yeast, and examples thereof can include the electroporation method [Methods Enzymol., 194, 182 (1990)], the spheroplast method [Proc. Natl. Acad. Sci. USA, 75, 1929 (1978)], the lithium acetate method [J. Bacteriology, 153, 163 (1983)], or the method mentioned in Proc. Natl. Acad. Sci. USA, 75, 1929 (1978) or the like.

When an insect cell is used as a host, it is possible to express a peptide by the method mentioned in, for example, Current Protocols in Molecular Biology, Baculovirus Expression Vectors, A Laboratory Manual, W.H. Freeman and Company, New York (1992), Bio/Technology, 6, 47 (1988) or the like.

In other words, a recombinant gene transfer vector and a defective baculovirus genome are co-introduced into an insect cell to obtain a recombinant virus in the insect cell culture supernatant, followed by further infection of an insect cell with the recombinant virus, thereby it is possible to express a peptide.

Examples of the gene transfer vector used in the method can include pVL1392, pVL1393 (manufactured by Becton, Dickinson and Company), or pBlueBac4.5 (manufactured by Invitrogen) or the like.

As the baculovirus, it is possible to use, for example, an *Autographa californica* nuclear polyhedrosis virus, which is a virus which can infect a *Noctuidae* insect or the like.

Examples of the method for co-introducing the abovementioned recombinant gene transfer vector and the abovementioned baculovirus into an insect cell in order to prepare a recombinant virus can include the calcium phosphate method (JP 2-227075 A) or the lipofection method [Proc. Natl. Acad. Sci. USA, 84, 7413 (1987)] or the like.

When an S2 (Schneider 2) cell (Thermo Scientific) corresponding to an insect cell derived from a terminal embryo of *Drosophila melanogaster* is used as a host, it is possible to express a peptide by introducing a gene transfer vector such as pMTBiPV5-HisA (Thermo Scientific) into a host cell by the calcium phosphate method, for example, using the method mentioned in Mol. Biotechnol., 2015, 10: p.914-922 or the like.

When an animal cell is used as a host, examples of the expression vector can include a pCI mammalian expression vector (Promega Corporation), pcDNA3.1(+) (manufactured by Invitrogen), pcDNA I/Amp, pcDNA I, pcDM8 (manufactured by Funakoshi Co., Ltd.), pAGE107 [JP 3-22979 A, Cytotechnology, 3, 133 (1990)], pAS3-3 (JP 2-227075 A), pCDM8 [Nature, 329, 840 (1987)], pREP4 (manufactured by Invitrogen), pAGE103 [J. Biochem., 101, 1307 (1987)], pAGE210, pME18SFL3, or pKANTEX93 (WO 97/10354 A) or the like.

As the promoter, any promoter can be used as long as it functions in an animal cell, and examples thereof can include a promoter of an immediate early (IE) gene of a cytomegalovirus (CMV), an early promoter of SV40, a promoter of a retrovirus, a metallothionein promoter, a heat shock promoter, or an SRa promoter or the like. An enhancer of an IE gene of human CMV may be used together with a promoter.

As the method for introducing a recombinant vector into an animal cell, any method can be used as long as it introduces a DNA into an animal cell, and examples thereof can include the electroporation method [Cytotechnology, 3, 133 (1990)], the calcium phosphate method (JP 2-227075 A), the lipofection method [Proc. Natl. Acad. Sci. USA, 84, 7413 (1987)], or the method mentioned in Virology, 52, 456 (1973) or the like.

By using expression vectors of the DcR3 variant of the present invention obtained by the abovementioned method, or expression vectors obtained by modifying them, it is possible to transiently express the DcR3 variant.

As the host cell into which an expression vector is introduced, any cell can be used as long as it is a host cell that can express the DcR3 variant, and, for example, a COS-7 cell (ATCC Number: CRL1651) is used [Methods in Nucleic Acids Res., CRC Press, 283 (1991)]. For introduction of an expression vector into a COS-7 cell, the DEAE-dextran method [Methods in Nucleic Acids Res., CRC Press, (1991)], or the lipofection method [Proc. Natl. Acad. Sci. USA, 84, 7413 (1987)] or the like is used.

When a CHO-S cell or an Expi293 cell (Thermo Scientific) is used, the lipofection method [Proc. Natl. Acad. Sci. USA, 84, 7413 (1987)] or the like is used for introduction of an expression vector.

By using expression vectors of the DcR3 variant of the present invention obtained by the abovementioned method, or expression vectors obtained by modifying them, it is possible to obtain a transformant stably expressing the DcR3 variant.

After introduction of an expression vector, the transformant stably expressing a gene recombinant antibody is selected by culturing in a medium for culture of animal cells containing a drug such as G418 sulfate (JP 2-257891 A).

When the transformant is a transformant obtained using a eukaryote such as a yeast as a host, as the culture medium of the transformant, any of a natural medium or a synthetic medium can be used as long as the medium contains a carbon source, a nitrogen source and/or inorganic salts that can be assimilated by the transformant, and the medium can efficiently culture the transformant.

As the carbon source, any carbon source may be used as long as the transformant can assimilate it, and it is possible to use, for example, carbohydrates such as glucose, fructose, sucrose, molasses containing these, starch or starch hydrolysates; organic acids such as acetic acid or propionic acid; alcohols such as ethanol or propanol; or the like.

As the nitrogen source, it is possible to use, for example, ammonium salts of inorganic acids or organic acids such as ammonia, ammonium chloride, ammonium sulfate, ammonium acetate and/or ammonium phosphate; other nitrogen-containing compounds, as well as peptone, meat extracts, yeast extracts, corn steep liquor, casein hydrolysates, soybean meal and soybean meal hydrolysates, various fermentative bacterial cells and digests thereof or the like.

As the inorganic salt, it is possible to use, for example, monopotassium phosphate, dipotassium phosphate, magnesium phosphate, magnesium sulfate, sodium chloride, ferrous sulfate, manganese sulfate, copper sulfate and/or calcium carbonate or the like.

Culture is preferably performed under aerobic conditions such as shaking culture or deep aeration stirring culture. The culture temperature is preferably 15 to 40°C, and the culture time is preferably usually 16 hours to 7 days. The pH during culture is preferably kept at 3.0 to 9.0. The pH can be adjusted using an inorganic or organic acid, an alkali solution, urea, calcium carbonate or ammonia or the like.

During culture, if necessary, an antibiotic such as ampicillin or tetracycline may be added to a medium.

As the culture medium of the transformant obtained using an insect cell as a host, it is possible to use a TNM-FH medium (manufactured by Becton, Dickinson and Company), an Sf-900 II SFM medium (manufactured by Invitrogen), Excell400, Excell405(both are manufactured by JRH Biosciences, Inc.), a Grace's insect medium [Nature, 195, 788 (1962)], or Schneider's Medium (Thermo Fisher Scientific, Inc.), which is generally used, or the like.

Regarding the culture of the transformant obtained using an insect cell as a host, usually it is preferable that the pH is 6 to 7 and the temperature is 25 to 30°C, and the culture is preferably performed for 1 to 5 days. During culture, if necessary, an antibiotic such as gentamicin may be added to a medium.

As the medium for animal cell culture, used is an RPMI 1640 medium (manufactured by Invitrogen), a GIT medium (manufactured by NIHON PHARMACEUTICAL CO., LTD.), an EXCELL301 medium (manufactured by JRH Biosciences, Inc.), an EX-CELL325 PF CHO serum-Free medium (Sigma-Aldrich Co., LLC), an IMDM medium (manufactured by Invitrogen), a Hybridoma-SFM medium (manufactured by Invitrogen), an Eagle's minimal essential medium (MEM) [Science, 122, 501 (1952)], a Dulbecco's modified Eagle's medium [Virology, 8, 396 (1959)], a 199 medium [Proceeding of the Society for the Biological Medicine, 73, 1 (1950)] or a medium obtained by adding various additives such as FBS to these media or the like.

Culture of an animal cell is usually preferably performed in the presence of 5% CO₂, and it is preferable that the pH is 6 to 8 and the temperature is 30 to 40°C, and the culture is preferably performed for 1 to 7 days. During culture, if necessary, an antibiotic such as kanamycin or penicillin may be added to a medium.

By culturing the transformant thus obtained in a medium, the DcR3 variant is expressed and accumulated in a culture supernatant. The transformant can increase the expression amount of the DcR3 variant using a DHFR amplification system (JP 2-257891 A) or the like.

In the manner as mentioned above, by generating and accumulating the DcR3 variant according to the present invention in a culture, and collecting from the culture, it is possible to produce the DcR3 variant according to the present invention.

In order to isolate and purify the peptide produced by the transformant, it is possible to use a usual protein isolation and purification method.

For example, when the DcR3 variant of the present invention is extracellularly secreted, the DcR3 variant can be collected from the culture supernatant. In other words, the culture is subjected to a method such as centrifugation to obtain a culture supernatant, and from the culture supernatant, a usual protein isolation and purification method, namely, a method such as a solvent extraction method, a salting-out method with ammonium sulfate or the like, a desalting method, a precipitation method with an organic solvent, an anion exchange chromatography method using a resin such as diethylaminoethyl (DEAE)-Sepharose or DIAION HPA-75 (Mitsubishi Chemical Corporation), a cation exchange chromatography method using a resin such as S-Sepharose FF (GE Healthcare), a hydrophobic chromatography method using a resin such as Butyl Sepharose or Phenyl Sepharose, a gel filtration method using a molecular sieve, an affinity chromatography method, a chromatofocusing method, or an electrophoresis method such as an isoelectric focusing is used alone or in combination, thereby it is possible to obtain a purified product.

For example, when the DcR3 variant of the present invention has Fc of an immunoglobulin that can bind to Protein G or Protein A, a Protein G chromatography method or a Protein A chromatography method in which Protein G or Protein A is bound to a carrier as an affinity ligand can be used as an affinity chromatography method [Monoclonal Antibodies-Principles and practice, Third edition, Academic Press (1996), Antibodies-A Laboratory Manual, Cold Spring Harbor Laboratory (1988)]. It is also possible to combine methods used for protein purification such as gel filtration, ion exchange chromatography and ultrafiltration.

From the DcR3 variant that could be obtained by the abovementioned method, by using a conventional chemical modification method, it is also possible to obtain a DcR3 variant in which a peptide, a glycan or PEG or the like is further chemically modified.

### 6. Method for Evaluating Biological Activity, Physical Property and Kinetics of DcR3 Variant

Examples of the DcR3 variant of the present invention preferably include a DcR3 variant as claimed having neutralizing activity to at least one or more of LIGHT, TL1A and FasL, a DcR3 variant having neutralizing activity to all of LIGHT, TL1A and FasL, a DcR3 variant having no neutralizing activity to FasL and having neutralizing activity to one of LIGHT and TL1A, a DcR3 variant having no neutralizing activity to FasL and having neutralizing activity to both of LIGHT and TL1A, or the like.

It is possible to measure the biological activity such as neutralizing activity, the physical property and the *in vivo* kinetics of the DcR3 variant of the present invention by using the following methods.

### (1) Preparation of Ligand

The origin of LIGHT, TL1A and FasL used in the present invention is not limited, but LIGHT, TL1A and FasL derived from eukaryotes are exemplified. Examples of LIGHT, TL1A and FasL derived from eukaryotes include LIGHT, TL1A and FasL derived from yeasts, insects or mammals. Preferably examples thereof include LIGHT, TL1A and FasL derived from primates including humans, or rodents including mice or the like.

LIGHT, TL1A or FasL or a cell expressing the ligand can be obtained by introducing an expression vector including a cDNA encoding the full length or partial length of LIGHT, TL1A or FasL into an appropriate host cell such as *Escherichia coli, a* yeast, an insect cell or an animal cell or the like. It can also be obtained by purifying the ligand from various human cultured cells or human tissues or the like expressing a large amount of LIGHT, TL1A or FasL. The cultured cell or the tissue can also be used as a ligand as it is. Furthermore, it is also possible to prepare a synthetic peptide having a partial sequence of LIGHT, TL1A or FasL by a chemical synthesis method such as the Fmoc method or the tBoc method.

LIGHT, TL1A and FasL can be produced by using the method mentioned in Molecular Cloning, A Laboratory Manual, Second Edition, Cold Spring Harbor Laboratory Press (1989) or the like and introducing a DNA encoding LIGHT, TL1A or FasL into a host cell to express by, for example, the following method.

Soluble LIGHT, TL1A and FasL are generated by being expressed on the cell membrane as membrane-bound LIGHT, membrane-bound TL1A and membrane-bound FasL, respectively, followed by shedding of the extracellular region by protease. Although a cleavage site has been identified for each ligand, when a recombinant of a soluble ligand is produced, any sequence in the vicinity of the cleavage site can be included. For example, a soluble LIGHT recombinant can be produced from a region at positions 66 to 240, a region at positions 74 to 240, a region at positions 83 to 240 or the like from the N-terminus in the amino acid sequence of membrane-bound LIGHT. A soluble TL1A recombinant can be produced from a region at positions 72 to 251 or the like from the N-terminus in the amino acid sequence of membrane-bound TL1A. A soluble FasL recombinant can be produced from a region at positions 130 to 281, a region at positions 134 to 281 or the like from the N-terminus in the amino acid sequence of membrane-bound FasL.

A recombinant of a soluble ligand can be produced by adding a His tag or a FLAG tag or the like to the N-terminus of the abovementioned region, and obtained by affinity purification. Examples of the amino acid sequences of the soluble recombinants of LIGHT, TL1A and FasL include amino acid sequences set forth in SEQ ID NO: 114, 116, 118, 120, 122, 124, 126, 128, 130 and 306. Examples of the nucleotide sequences of the DNAs encoding the amino acid sequences of the soluble recombinants of LIGHT, TL1A and FasL include nucleotide sequences set forth in SEQ ID NO: 113, 115, 117, 119, 121, 123, 125, 127, 129 and 305.

Functional LIGHT, TL1A or FasL can form a homotrimer. Soluble LIGHT, TL1A or FasL prepared by any of the abovementioned methods is analyzed by SEC-MALS, and the molecular weight can be calculated. SEC-MALS is an analysis method for calculating a molecular weight for each peak detected at a wavelength of 215 nm separated by size exclusion chromatography (SEC)-HPLC, using a maximum value of scattering intensity detected with multi angle light scattering (MALS). Examples of the HPLC system include Prominence manufactured by Shimadzu Corporation; examples of the SEC column include TSKgel manufactured by Tosoh Corporation; and examples of the MALS detector include miniDAWN TREOS manufactured by Wyatt Technology Corporation or the like. Examples of the method for isolating a homotrimer from a crude purified product that forms those other than trimers include SEC purification or the like. Examples of the SEC purification method include a method in which a crude purified product is fractionated according to the molecular weight size using AKTApurifier manufactured by GE Healthcare as an HPLC system and Superdex 200 Increase 10/300 GL manufactured by GE Healthcare as a SEC column, and only a molecular weight fraction of a homotrimer is collected, or the like.

### (2) Evaluation of Binding Activity of DcR3 Variant Including Human Fc Region

Examples of the method for measuring the binding activity of the DcR3 variant to soluble LIGHT, soluble TL1A or soluble FasL include a binding assay by enzyme-linked immunosorbent assay (ELISA), a kinetics analysis by Biacore or the like. As the ligand, a transgenic cell or a recombinant protein obtained by introducing an expression vector including a DNA sequence encoding an extracellular domain of each ligand of LIGHT, TL1A or FasL into *Escherichia coli,* a yeast, an insect cell or an animal cell or the like, and serum, plasma or a culture supernatant or the like including a ligand obtained from a human tissue or a human cell are used.

In the case of ELISA, for example, an anti-human IgG antibody is immobilized to a 96 well plate or the like, and the DcR3 variant is reacted, and then each ligand is dispensed to bind. After washing, an unlabeled anti-ligand antibody or a receptor-Fc fusion of each ligand or the like is reacted, and then an anti-Fc antibody or the like labeled with biotin, an enzyme or a chemiluminescence substance or the like is reacted, or a labeled anti-ligand antibody or a receptor-Fc fusion of each ligand or the like is reacted, followed by detection according to the labeled substance, thereby it is possible to measure the binding of the DcR3 variant to a ligand. In the case of Biacore, for example, using Biacore T100 or Biacore T200, kinetics in the binding between each ligand and the DcR3 variant is measured, and the results are analyzed with analysis software included with the apparatus. Specifically, after an anti-human IgG antibody is immobilized on a sensor chip CM5 by an amine coupling method, the DcR3 variant is injected and an appropriate amount is bound on the sensor chip, and further a plurality of ligands having known concentrations are injected, thereby binding and dissociation are measured. For the data thus obtained, a kinetics analysis is performed with a 1:1 binding model using software included with the apparatus, and various parameters are obtained. Alternatively, after each ligand is immobilized on a sensor chip by, for example, an amine coupling method, a plurality of DcR3 variants having known concentrations are injected, thereby binding and dissociation are measured. For the data thus obtained, a kinetics analysis is performed with a bivalent binding model using software included with the apparatus, and various parameters are obtained. Alternatively, to sensor chip Protein A obtained by immobilizing a MabSelect Sure ligand, which is an IgG binding domain variant protein of Protein A, on a sensor chip, the DcR3 variant is injected and an appropriate amount is bound on the sensor chip, and further a plurality of ligands having known concentrations are injected, thereby binding and dissociation are measured. For the data thus obtained, a kinetics analysis is performed with a 1:1 binding model using software included with the apparatus, and various parameters are obtained.

The binding activity of the DcR3 variant to membrane-bound LIGHT, membrane-bound TL1A or membrane-bound FasL can be measured by flow cytometry, for example, using a transgenic cell obtained by introducing an expression vector including a DNA sequence encoding the full length of each ligand of LIGHT, TL1A and FasL into an animal cell or the like, or a cell such as PBMC or HUVEC stimulated for induction of expression of the membrane-bound ligand by adding appropriate stimulation such as stimulation with a mitogen such as PHA-L, PMA or ionomycin or anti-CD3 antibody and anti-CD28 antibody stimulation, cytokine, IgG or immune complex stimulation. Specifically, measurement can be performed by reacting the DcR3 variant with a cell expressing a membrane-bound ligand, followed by reaction with a fluorescently labeled anti-Fc antibody or the like, or reacting the DcR3 variant labeled with biotin or a fluorescent dye or the like, and after washing, detecting the fluorescence intensity according to the labeled substance using a flow cytometer.

Examples of the DcR3 variant of the present invention measured by the abovementioned method include a DcR3 variant as claimed having binding activity to at least one or more of LIGHT, TL1A and FasL, a DcR3 variant having binding activity to all of LIGHT, TL1A and FasL, a DcR3 variant having no binding activity to FasL and having binding activity to one of LIGHT and TL1A, or a DcR3 variant having no binding activity to FasL and having binding activity to both of LIGHT and TL1A.

### (3) Evaluation of Neutralizing Activity of DcR3 Variant

Examples of the method for measuring the neutralizing activity of the DcR3 variant include a method for measuring the binding of LIGHT, TL1A or FasL with a corresponding receptor in a solution supplemented with the DcR3 variant, or a method for measuring cell functions such as cytokine production or cell proliferation by adding a corresponding ligand to a cell expressing a receptor of LIGHT, TL1A or FasL in a medium supplemented with the DcR3 variant.

The inhibitory activity to the binding of human LIGHT with a corresponding receptor can be measured by the following method using, for example, the method mentioned in US 8,974,787 B2 or the like. Measurement is performed by, in a reaction solution supplemented with the DcR3 variant, reacting HVEM or LTβR labeled with biotin, a fluorescent dye or the like with a cell expressing membrane-bound LIGHT, and after washing, detecting the fluorescence intensity according to the labeled substance using a flow cytometer. Alternatively, measurement is performed by, in a reaction solution supplemented with the DcR3 variant, reacting LIGHT labeled with biotin, a fluorescent dye or the like with a cell expressing HVEM or LTβR, and after washing, detecting the fluorescence intensity according to the labeled substance using a flow cytometer. The inhibitory activity of the DcR3 variant to the binding of LIGHT with a corresponding receptor is confirmed by a decrease in the fluorescence intensity in comparison with the case where no DcR3 variant is added.

Using the same method as mentioned above, it is also possible to measure the inhibitory activity to the binding of TL1A or FasL with a corresponding receptor.

As the DcR3 variant of the present invention, preferred is a DcR3 variant as claimed having inhibitory activity to at least one or more of the bindings of LIGHT, TL1A and FasL with corresponding receptors, a DcR3 variant having inhibitory activity to all of the bindings of LIGHT, TL1A and FasL with corresponding receptors, a DcR3 variant having no inhibitory activity to the binding of FasL with a corresponding receptor and having inhibitory activity to one of the bindings of LIGHT and TL1A with corresponding receptors, or a DcR3 variant having no inhibitory activity to the binding of FasL with a corresponding receptor and having inhibitory activity to the bindings of LIGHT and TL1A with corresponding receptors. In the present invention, the expression of "having no inhibitory activity to the binding of a ligand with a corresponding receptor" is used as a meaning including the case where the inhibitory activity is significantly lowered than that of wild-type DcR3.

The inhibitory activity to a cell function initiated by addition of LIGHT can be measured as follows using, for example, the method mentioned in US 8,974,787 B2 or the like. In a medium supplemented with the DcR3 variant, using a cell such as HT-29 (ATCC Number: HTB-38) which was confirmed to express HVEM or LTβR corresponding to a LIGHT receptor, LIGHT-dependent production of chemokine such as IL-8, CCL5 or CCL20 is measured by the ELISA method, alphaLISA (PerkinElmer, Inc.) or CBA assay (BD Biosciences) or the like using a culture supernatant. Alternatively, using a cell such as an intestinal myofibroblast (Lonza K.K.) stimulated with IFN-γ, soluble or membrane-bound LIGHT-dependent production of a chemokine such as CXCL-10 is also measured using the same method. Alternatively, using, instead of soluble or membrane-bound LIGHT, LIGHT whose expression was induced from PBMC or a T cell stimulated with an anti-CD3 antibody and an anti-CD28 antibody or PMA and ionomycin or the like, measurement can be performed using the same method. The inhibitory activity of the DcR3 variant to a cell function initiated by addition of soluble or membrane-bound LIGHT is confirmed by a decrease in the production amount of the chemokine in comparison with the case where no DcR3 variant is added. Using, for example, the method mentioned in US 8,974,787 B2 or the like, it is possible to evaluate *in vivo* the improvement in survival, body weight, a pathological condition, pathology, engraftment of a human cell or the like by administration of the DcR3 variant in an acute graft versus host disease (GVHD) model in which allogeneic transplantation of human PBMC was performed to an immunodeficient mouse.

The inhibitory activity to a cell function initiated by addition of TL1A can be measured as follows using, for example, the method mentioned in Mucosal Immunology, 2015, 8: p.545-558 or the like. In a medium supplemented with the DcR3 variant, using blood, PBMC, a pan T cell, a CD4-positive T cell or a memory CD4-positive T cell or the like derived from primates such as humans or rodents such as mice, the cell is stimulated with each cytokine cocktail of IL-12, IL-18 and TL1A, or IL-12, IL-18, IL-15 and TL1A, and TL1A-dependent production of a cytokine such as IFN-γ, IL-6, GM-CSF, TNF-a, IL-5, IL-13, IL-17 or IL-22 or the like is measured by the ELISA method, alphaLISA or CBA assay or the like using a culture supernatant. Alternatively, using, for example, the method mentioned in Immunity, 2002, 16: p.479-492, in a medium supplemented with the DcR3 variant, a pan T cell, a CD4-positive T cell or a memory CD4-positive T cell or the like derived from primates such as humans or rodents such as mice is stimulated with an anti-CD3 antibody and an anti-CD28 antibody, and TL1A-dependent production of IFN-γ and IL-2 is measured by the same method as mentioned above. Alternatively, using a cell line forcedly expressing membrane-bound TL1A instead of soluble TL1A, measurement can also be performed by the same method. Alternatively, using TL1A whose expression was induced from PBMC or a monocyte stimulated with IgG or an immune complex or the like, measurement can also be performed by the same method. The inhibitory activity of the DcR3 variant to a cell function initiated by addition of TL1A is confirmed by a decrease in the cytokine or inhibition of cell proliferation in comparison with the case where no DcR3 variant is added. Furthermore, it is possible to evaluate *in vivo* the improvement in survival, body weight, a pathological condition and pathology and the like by administration of the DcR3 variant in, for example, the 2,4,6-trinitrobenzenesulfonic acid (TNBS)-induced colitis model mentioned in Mucosal Immunology, 2011, 4: p.172-185 or the dextran sodium sulfate (DSS)-induced colitis model mentioned in Mucosal Immunology, 2014, 7: p.1492-1503. In addition, it is possible to evaluate the effect on improving a pathological condition in a TL1A-dependent inflammation, allergic disease or autoimmune disease model in rodents such as mice.

The inhibitory activity to a cell function initiated by addition of FasL can be measured as follows using, for example, the method mentioned in J. Rheumatol., 2013, 40: p.1316-1326 or the like. In a medium supplemented with the DcR3 variant, using Jurkat cells (DSMZ Number: ACC 282), apoptosis dependent on soluble FasL or soluble FasL cross-linked with an antibody is measured by Annexin V/Propium Iodide staining or BrdU uptake or ATP amount of a viable cell or the like. Alternatively, using a cell line forcedly expressing membrane-bound FasL instead of soluble FasL, measurement can also be performed by the same method. Alternatively, using FasL induced from PBMC or a T cell stimulated, measurement can also be performed by the same method. The inhibitory activity of the DcR3 variant to a cell function initiated by addition of FasL is confirmed by a decrease in the apoptosis in comparison with the case in which no DcR3 variant is added.

Examples of the DcR3 variant of the present invention measured by the abovementioned method include a DcR3 variant as claimed having inhibitory activity to at least one or more of cell functions initiated by addition of LIGHT, TL1A and FasL, a DcR3 variant having inhibitory activity to all of cell functions initiated by addition of LIGHT, TL1A and FasL, a DcR3 variant having no inhibitory activity to a cell function initiated by addition of FasL and having inhibitory activity to one or more of cell functions initiated by addition of LIGHT and TL1A, or a DcR3 variant having no inhibitory activity to a cell function initiated by addition of FasL and having inhibitory activity to cell functions initiated by addition of LIGHT and TL1A. In the present invention, the expression of "having no inhibitory activity to a cell function initiated by addition of a ligand" is used as a meaning including the case where the inhibitory activity is significantly lowered than that of wild-type DcR3.

### (4) Evaluation of OPG Ligand Reactivity of DcR3 Variant

One characteristic of the DcR3 variant of the present invention is that any of parts of or all of CRD1, CRD4 and CRD3 not involved in ligand binding are sequences derived from OPG.

The fact that the DcR3 variant having no binding activity to RANKL and TRAIL, which are OPG ligands, can be evaluated by, for example, the same method as the method for measuring the binding activity to LIGHT, TL1A and FasL mentioned above or the like.

One characteristic of the DcR3 variant of the present invention is having no neutralizing activity also to RANKL and TRAIL.

The neutralizing activity of the DcR3 variant to RANKL can be evaluated using, for example, tartrate-resistant acid phosphatase (TRAP) activity measurement, which is a differentiation assay of an osteoclast precursor by RANKL stimulation, mentioned in J. Immunol., 2012, 189: p.245-252 or the like.

Regarding the neutralizing activity of the DcR3 variant to TRAIL, for example, apoptosis induction by soluble TRAIL or cross-linked soluble TRAIL in a human cancer cell line expressing DR4 or DR5 can be evaluated by the same method as the method for measuring the neutralizing activity to FasL mentioned above or the like.

### (5) Evaluation of Kinetics of DcR3 Variant

As the, DcR3 variant of the present invention, a DcR3 variant as claimed in which the binding to heparan sulfate included in heparan sulfate proteoglycan (HSPG) on the cell membrane has been lowered or eliminated is preferable, and a DcR3 variant as claimed having no heparan sulfate-binding domain (HBD) is more preferable.

The presence or absence of the binding to heparan sulfate on the cell membrane via HBD can be measured as follows using, for example, the method mentioned in J. Immunol., 2006, 176: p.173-180 or the like. Measurement can be performed by reacting wild-type DcR3 or the DcR3 variant with any cell, for example, a CHO cell or a cell of a human cell line, a vascular endothelial cell, a hepatocyte or a blood cell or the like, followed by reaction with a fluorescently labeled detection antibody or the like, or reacting wild-type DcR3 or the DcR3 variant labeled with biotin or a fluorescent dye or the like, and after washing, detecting the fluorescence intensity according to the labeled substance using a flow cytometer (FCM). Lowering or elimination of the binding to the cell membrane due to deficiency of HBD of DcR3 can be examined by a decrease in the fluorescence intensity by FCM.

Examples of the method for evaluating whether the abovementioned binding on the cell membrane is a binding via HBD of DcR3 or not include a method in which GAG such as heparin, heparan sulfate or the like as an inhibitor is added during a reaction of wild-type DcR3 or the DcR3 variant, or a cell is treated with an enzyme such as heparinase or trypsin or the like in advance, and then the wild-type DcR3 or the DcR3 variant is reacted, using, for example, the method mentioned in J. Immunol., 2006, 176: p.173-180 or the like.

One characteristic of the DcR3 variant of the present invention is that *in vivo* kinetics is improved compared to that of wild-type DcR3 since elimination via heparan sulfate is relieved *in vivo.* In the present invention, *"in vivo* kinetics is improved" means that the half-life in blood is longer compared to that of wild-type DcR3 or the value of the area under the concentration-time curve (AUC) to infinity is higher compared to that of wild-type DcR3.

As a target for comparison, it is also possible to use a molecule including CRD of wild-type DcR3 instead of wild-type DcR3. Specific examples thereof include DcR3 FL-Fc (amino acid sequence: SEQ ID NO: 100, nucleotide sequence of DNA: SEQ ID NO: 99), DcR3 FL-FLAG (amino acid sequence: SEQ ID NO: 104, nucleotide sequence of DNA: SEQ ID NO: 103), S195-Fc obtained by fusing CRD of wild-type DcR3 and Fc to each other (amino acid sequence: SEQ ID NO: 102, nucleotide sequence of DNA: SEQ ID NO: 101), and R128Q-Fc obtained by fusing Fc to the C-terminus of full-length DcR3 having one amino acid mutation in HBD (amino acid sequence: SEQ ID NO: 112, nucleotide sequence of DNA: SEQ ID NO: 111) [US 6,835,814 B1, US 6,965,01 B1] and the like. When a molecule including CRD of these wild-type DcR3 as a target for comparison, it is also referred to as wild-type DcR3 control.

The time course of blood concentration of the DcR3 variant in rodents such as mice or non-human primates such as cynomolgus monkeys can be measured by intravenously or subcutaneously administering an appropriately-selected dose, collecting blood at an appropriately-selected time, and using a detection system of DcR3 or human Fc. Kinetic parameters such as half-life in blood and AUC can be calculated by using a method such as a moment analysis from the time course of blood concentration using, for example, the method mentioned in Xenobiotic Metabolism and Disposition, 1999, 14: p.286-293.

Since it has been known that *in vivo* kinetics is improved if many sialic acids are added to the terminus of an N-linked glycan (J. Pharm. Sci., 2015, 104: p.1866-1884), it is preferable that the DcR3 variant of the present invention having an N-linked glycan also has many sialic acids added. The number of additions of sialic acids per protein molecule can be calculated by, for example, separating sialic acids labeled using a reagent kit for fluorescent labeling of sialic acid (Takara Bio Inc.) or the like by reversed-phase HPLC, and comparing with a standard curve of sialic acid.

### (6) Evaluation of Physical Property of DcR3 Variant

One characteristic of the DcR3 variant of the present invention is that, when expressed in mammalian cells, isolated or purified, the content of aggregates is lower than that of wild-type DcR3.

Whether aggregates are generated during protein expression and/or secretion can be determined by, for example, collecting host cells into which a recombinant vector carrying the DcR3 variant have been introduced and/or a culture supernatant, and examining an approximate molecular weight under non-reducing conditions by immunoblotting using an anti-DcR3 antibody, or, in the case of an Fc fusion or a tag adduct such as His or FLAG, an antibody against it. When proteins are aggregated during protein expression and/or secretion, one or a plurality of band(s) of a larger size than the expected molecular weight is/are detected.

A method for calculating the molecular weight of a protein from the amino acid sequence is exemplified. When a more accurate molecular weight is calculated, an analysis method by SEC-MALS is exemplified. Regarding each peak detected at a wavelength of 215 nm separated by size exclusion chromatography (SEC)-HPLC, the molecular weight can be calculated using a maximum value of scattering intensity detected with multi angle light scattering (MALS). Examples of the HPLC system include Prominence manufactured by Shimadzu Corporation; examples of the SEC column include TSKgel manufactured by Tosoh Corporation; and examples of the MALS detector include miniDAWN TREOS manufactured by Wyatt Technology Corporation or the like.

Whether isolated or purified proteins are aggregated or not can be examined by subjecting the proteins to SDS-PAGE under non-reducing conditions, followed by detection by CBB staining or the like, or by immunoblotting using the same method as mentioned above. Regarding the content of aggregates, the proportion of each peak can be calculated from the area of each peak detected at a wavelength of 215 nm separated by gel filtration chromatography (SEC) using HPLC. Examples of the SEC column include TSKgel G3000SW manufactured by Tosoh Corporation or ACQUITY UPLC Protein BEH SEC manufactured by Waters K.K. or the like. When analysis is performed using the method mentioned above, a preferable content of aggregates is 0 to 60%, a more preferable content of aggregates is 0 to 40%, a still more preferable content of aggregates is 0 to 30%, a yet more preferable content of aggregates is 0 to 20%, and most preferable content of aggregates is 0 to 10%.

The DcR3 variant of the present invention is characterized by having lower hydrophobicity than that of wild-type DcR3. The hydrophobicity of a protein can be measured using a hydrophobic interaction chromatography (HIC) column that interacts with a hydrophobic region existing on the surface of the protein. Examples of the HIC column include TSKgel Butyl-NPR manufactured by Tosoh Corporation or the like.

The DcR3 variant of the present invention is characterized by having more improved thermostability than that of wild-type DcR3. Examples of the method for measuring the thermostability of a protein include calorimetry such as differential scanning calorimetry (DSC); spectroscopy that obtains an intrinsic fluorescence spectrum or a circular dichroism (CD) spectrum during thermal denaturation or chemical denaturation; differential scanning fluorimetry (DSF) that detects exposure of a hydrophobic region existing in the inside of a protein associated with a rise in temperature using a fluorescent dye (Sypro Orange or the like); or the like [J Am Chem Soc, 2009, 131: p.3794-3795].

Regarding DSF that evaluates the thermostability of a protein, it is possible to measure the fluorescence amount at each temperature using, for example, the method mentioned in J. Pharm. Sci., 2013, 102: p.2471-2483 or the like. It is possible to calculate the thermal unfolding transition midpoints (Tm) value by drawing a melting curve using software such as CFX manager manufactured by Bio-Rad Laboratories, Inc. Similarly, regarding DSC that evaluates the thermostability of a protein, it is possible to calculate the heat capacity and the Tm value at each temperature using, for example, the method mentioned in J. Pharm. Sci., 2012, 101: p.955-964 or the like.

### 7. DcR3 Variant Composition

Examples of the DcR3 variant composition of the present invention include a composition including a DcR3 variant molecule(s) as claimed. Examples of the DcR3 variant composition of the present invention include a composition including a plurality of DcR3 variant molecules as claimed that consist of equivalent primary amino acid sequences and can occur by posttranslational modification such as oxidation/reduction reaction, glycosylation reaction or sulfation addition reaction in the amino acid sequence. The DcR3 variant of the present invention may include, for example, a DcR3 variant as claimed having one or more complex N-glycoside-linked glycans, and a DcR3 variant not having a complex N-glycoside-linked glycan. The proportion of the DcR3 variants having one or more complex N-glycoside-linked glycans is, for example, preferably 70 to 100%, more preferably 90 to 99%, and particularly preferably 95 to 98%, relative to the total number of the DcR3 variants of the present invention.

### 8. Pharmaceutical Composition Including DcR3 Variant

One embodiment of the present invention is a composition including an effective dose of the DcR3 variant of the present invention. The composition including the DcR3 variant of the present invention can be used as an active ingredient of a prophylactic or therapeutic agent for an autoimmune disease, an inflammatory disease or an allergic disease, including a mucosal disease. In other words, it is possible to prevent or treat an autoimmune disease, an inflammatory disease or an allergic disease by administering the pharmaceutical composition including the DcR3 variant of the present invention to a patient in need of prevention or treatment of an autoimmune disease, an inflammatory disease or an allergic disease.

Examples of the pathological condition or the disease for which the composition of the present invention is used include, but **are not** limited to, an inflammatory disease, an autoimmune disease or an allergic disease or the like, such as inflammatory bowel disease (IBD), systemic lupus erythematosus, psoriasis, chronic graft versus host disease, acute graft versus host disease, Crohn's disease, ulcerative colitis, inflammatory bowel disease, multiple sclerosis, celiac disease, idiopathic thrombotic thrombocytopenic purpura, myasthenia gravis, Sjogren's syndrome, scleroderma, asthma, uveitis, epidermal hyperplasia, alopecia areata, Behcet's disease, Takayasu's arteritis, cartilaginous inflammation, bone degradation, arthritis, juvenile arthritis, juvenile rheumatoid arthritis, pauciarticular juvenile rheumatoid arthritis, polyarticular juvenile rheumatoid arthritis, systemic onset juvenile rheumatoid arthritis, juvenile ankylosing spondylitis, juvenile enteropathic arthritis, juvenile reactive arthritis, juvenile Reiter's syndrome, seronegative enthesopathy and arthropathy syndrome (SEA syndrome), juvenile dermatomyositis, juvenile psoriatic arthritis, juvenile scleroderma, juvenile systemic lupus erythematosus, juvenile vasculitis, pauciarticular rheumatoid arthritis, polyarticular rheumatoid arthritis, systemic onset rheumatoid arthritis, ankylosing spondylitis, enteropathic arthritis, reactive arthritis, Reiter's syndrome, dermatomyositis, psoriatic arthritis, vasculitis, myositis, polymyositis, dermatomyositis, osteoarthritis, polyarteritis nodosa, Wegener's granulomatosis, arteritis, polymyalgia rheumatica, sarcoidosis, sclerosis, primary biliary cirrhosis, sclerosing cholangitis, dermatitis, atopic dermatitis, atherosclerosis, Still's disease, chronic obstructive pulmonary disease, Guillain-Barre syndrome, type 1 diabetes mellitus, Graves' disease, Addison's disease, Raynaud's phenomenon, autoimmune hepatitis or Wiskott-Aldrich syndrome or the like.

The pharmaceutical composition containing the DcR3 variant of the present invention can contain, as an active ingredient, the DcR3 variant or a mixture of the DcR3 variant with any other active ingredients for treatment. These pharmaceutical formulations are produced by any method well known in the technical field of pharmaceutics by mixing an active ingredient with one or more pharmacologically acceptable carriers.

The content of the DcR3 variant of the present invention in the pharmaceutical composition varies depending on the dosage form, the pharmacologically acceptable dose of the DcR3 variant of the present invention or the like, and the content is, for example, about 0.01 to 100% by weight. The content of the pharmacologically acceptable carrier in the pharmaceutical formulation varies depending on the dosage form, the pharmacologically acceptable dose of the DcR3 variant of the present invention or the like, and the content is, for example, 0 to 99.9% by weight.

Regarding the route of administration, it is desirable to use the most effective one for treatment, and examples thereof include oral administration, or parenteral administration such as intravenous, subcutaneous, intraoral, intratracheal, intrarectal, intramuscular or intraperitoneal administration.

The administration form includes tablets, powders, granules, syrups or injections or the like.

As an appropriate formulation for oral administration, for example, a liquid preparation such as a syrup can be produced using water; saccharides such as sucrose, sorbit or fructose; glycols such as polyethylene glycol or propylene glycol; oils such as sesame oil, olive oil or soybean oil; preservatives such as p-hydroxybenzoic acid esters; flavors such as strawberry flavor or peppermint; or the like. A tablet, a powder and a granule and the like can be produced using excipients such as lactose, glucose, sucrose or mannit; disintegrators such as starch or sodium alginate; lubricants such as magnesium stearate or talc; binders such as polyvinyl alcohol, hydroxypropyl cellulose or gelatin; surfactants such as fatty acid ester; plasticizers such as glycerin; or the like.

An appropriate formulation for parenteral administration preferably consists of a sterilized aqueous agent containing an active compound isotonic with blood of a recipient. For example, in the case of an injection, a solution for injection is prepared using a carrier consisting of a mixture of a salt solution, a glucose solution or a salt water with a glucose solution, or the like.

In these parenteral agents, it is also possible to add one or more auxiliary ingredients selected from the diluents, preservatives, flavors, excipients, disintegrators, lubricants, binders, surfactants and plasticizers and the like exemplified in the oral agents.

A medicament containing the DcR3 variant of the present invention can be safely administered to mammals (e.g., humans, mice, rats, rabbits, dogs, cats, cattle, horses, pigs or monkeys or the like).

The dose and the frequency of administration of the DcR3 variant of the present invention vary depending on administration form, age of a patient, body weight, disease, or nature or seriousness of symptoms to be treated, but usually in the case of oral administration, 0.01 mg to 1 g, and preferably 0.05 to 50 mg per adult are administered once or several times daily. In the case of parenteral administration such as intravenous administration, 0.001 to 100 mg, and preferably 0.01 to 10 mg per adult are administered once or several times daily. However, these dose and frequency of administration vary depending on the various conditions mentioned above.

### EXAMPLES

Examples of the present invention will be described below. However, the present invention is not limited to these Examples.

### [Example 1] Evaluation of Aggregability of Wild-Type DcR3 in Mammalian Cell

A fusion protein (DcR3 FL-Fc) (FIG. 3A, SEQ ID NO: 100) of wild-type DcR3 (also referred to as full-length DcR3) (SEQ ID NO: 4), a linker sequence IEGRMD (SEQ ID NO: 106) and a human IgG1 Fc region (g1S) (SEQ ID NO: 72), a fusion protein (S195-Fc) (FIG. 3B, SEQ ID NO: 102) of human DcR3 lacking an HBD region (SEQ ID NO: 108), a linker sequence IEGRMD (SEQ ID NO: 106) and a human IgG1 Fc region (SEQ ID NO: 72), and a protein (DcR3 FL-FLAG) (SEQ ID NO: 104) obtained by adding a FLAG tag (SEQ ID NO: 110) to full-length DcR3 (SEQ ID NO: 4) were produced as follows.

Regarding DcR3 FL-Fc, a DNA fragment of a signal peptide sequence, a DNA fragment (SEQ ID NO: 3) of human DcR3, a DNA fragment (SEQ ID NO: 105) of a linker sequence IEGRMD, and a DNA fragment (SEQ ID NO: 71) of Fc (g1S) were artificially synthesized (GENEWIZ, Inc. or Sigma-Aldrich Co. LLC), followed by insertion using In-Fusion HD Cloning Kit (Clontech Laboratories, Inc.) into a pCIpuro vector (pCI manufactured by Promega Corporation was partially modified) digested with restriction enzymes NheI and SalI (New England Biolabs, Inc.), and *Escherichia coli* OH5a competent cells (TOYOBO CO., LTD.) were transformed, thus obtaining transformants each including a DcR3 FL-Fc DNA fragment (SEQ ID NO: 99) introduced thereinto. Similarly, regarding S195-Fc, a DNA fragment of a signal peptide sequence, a DNA fragment (SEQ ID NO: 107) of human DcR3 lacking HBD, a DNA fragment (SEQ ID NO: 105) of a linker sequence IEGRMD, and a DNA fragment (SEQ ID NO: 71) of Fc (g1S) were artificially synthesized, thus obtaining transformants each including an S195-Fc DNA fragment (SEQ ID NO: 101) introduced thereinto. Regarding DcR3 FL-FLAG, a DNA fragment of a signal peptide sequence, a DNA fragment (SEQ ID NO: 3) of human DcR3, and a DNA fragment (SEQ ID NO: 109) of a FLAG tag were artificially synthesized, thus obtaining transformants each including a DcR3 FL-FLAG DNA fragment (SEQ ID NO: 103) introduced thereinto.

Each plasmid obtained from each transformant was introduced into host cells selected from Freestyle CHO-S cells, Freestyle 293F cells and Expi293 cells (all of which are manufactured by Thermo Scientific) to transiently express proteins. For plasmid introduction, Freestyle MAX Reagent, 293Fectin Transfection Reagent or ExpiFectamine 293 (all of which are manufactured by Thermo Scientific) was used.

After the transfected cells were cultured for several days, the culture supernatants were collected, and DcR3 FL-Fc and S195-Fc were subjected to affinity purification using MabSelect SuRe (GE Healthcare), and DcR3 FL-FLAG was subjected to affinity purification using Anti-FLAG M2 affinity gel (Sigma-Aldrich Co. LLC). The culture supernatants for DcR3 FL-Fc and S195-Fc were passed through a column filled with a resin, washed with PBS (Nacalai Tesque, Inc.), and eluted with an elution buffer (20 mM citric acid, 50 mM NaCl, pH 3.4), followed by rapid neutralization with a neutralization buffer (1M sodium phosphate, pH 7.0). The culture supernatant for DcR3 FL-FLAG was similarly charged into a resin, washed with PBS, and eluted with an elution buffer (100 mM glycine-HCl, pH 3.5), followed by rapid neutralization with a neutralization buffer (1M Tris-HCl, pH 8.0). The absorbance at 280 nm (A₂₈₀) of each elution fraction was measured, and consecutive fractions having high measurement values were collected. The buffer of the collected fractions was substituted with PBS using a NAP column (GE Healthcare) and passed through a 0.22 µm filter, and the resultant product was regarded as a purified protein. The concentrations of the purified proteins were calculated by using the extinction coefficient of DcR3 FL-Fc, S195-Fc and DcR3 FL-FLAG at 280 nm as 1.03, 1.17 and 0.77, respectively, and after SDS-PAGE under non-reducing conditions and 100 mM DTT reducing conditions, the gel was subjected to Coomassie staining (Nacalai Tesque, Inc.), and the molecular weights were confirmed. The estimated molecular weights of monomers of DcR3 FL-Fc, S195-Fc and DcR3 FL-FLAG, predicted from the amino acid sequences thereof, are about 56.4 kDa, 44.7 kDa and 31.0 kDa, respectively. Under non-reducing conditions, DcR3 FL-Fc and S195-Fc, which are Fc fusions, exist as dimers, and thus the estimated molecular weights are about 112.8 kDa and 89.4 kDa, respectively.

As a result of SDS-PAGE of DcR3 FL-Fc, S195-Fc and DcR3 FL-FLAG that were transiently expressed in mammalian cells under non-reducing conditions, the majorities of the samples of DcR3 FL-Fc, S195-Fc and DcR3 FL-FLAG remained in sample wells, and the electrophoresed parts of the samples resulted in smear-like or ladder-like patterns, revealing that all recombinants were highly aggregated (FIG. 1A).

Furthermore, regarding commercially available full-length DcR3-Fc (Abcam plc.) produced using HEK293 cells as host cells, immunoblotting under non-reducing conditions was performed using a rabbit anti-human IgG Fc polyclonal antibody as a primary antibody and a goat anti-rabbit IgG antibody (Dako) as a secondary antibody, almost all thereof existed as aggregates, like the abovementioned purified products (FIG. 1B).

Meanwhile, commercially available DcR3 FL-Fc (R&D Systems, Inc.) expressed using insect cells Sf21 showed almost no aggregation, and there was a difference in aggregability depending on the types of host cells producing wild-type DcR3 (FIG. 1C). By the following method using insect cells S2 as host cells, DcR3 FL-Fc (SEQ ID NO: 100), R218Q-Fc (US 6835814 B1, US 6965012 B1, SEQ ID NO: 340) obtained by introducing into human DcR3 (SEQ ID NO: 2) an R218Q mutation (i.e., substitution of Arg at position 218 with Gln), and S195-Fc lacking HBD (SEQ ID NO: 102) were produced, and aggregabilities were evaluated. One of a DNA fragment (SEQ ID NO: 3) of human DcR3, a DNA fragment (SEQ ID NO: 111) obtained by introducing into human DcR3 an R218Q mutation, and a DNA fragment (SEQ ID NO: 107) of human DcR3 lacking HBD was artificially synthesized connected to a DNA fragment (SEQ ID NO: 105) of a linker sequence IEGRMD and a DNA fragment (SEQ ID NO: 71) of Fc (g1S), and using pMTBiPV5-HisA (Thermo Scientific) and Drosophila Expression System (Thermo Scientific), an S2 cell line that stably expresses each of DcR3 FL-Fc, R218Q-Fc and S195-Fc was obtained. From the culture supernatants of the cells of the stable expression cell lines, affinity purification by MabSelect SuRe was performed using the same method as mentioned above.

As a result, DcR3 FL-Fc, R218Q-Fc and S195-Fc that were produced using S2 cells showed almost no aggregation (FIG. 1C). Quantitative evaluation of the aggregate content was performed by gel filtration chromatography (SEC) (TSKgel G3000 SWXL 7.8 mm × 300 mm) (Tosoh Corporation) using HPLC (Shimadzu Corporation). S195-Fc derived from each of Expi293 and S2 was analyzed, and the proportions (%) of monomers, aggregates and degradants calculated from the peak areas are shown in Table 1.

**[Table 1]**

| Host cell | Monomer | Aggregate | Degradant |
|---|---|---|---|
| Expi293 | 15.1% | 81.9% | 3.0% |
| S2 | 84.9% | 15.1% | 0.0% |

The above results revealed that, when wild-type DcR3 is expressed using a mammalian cell, the generation amount of aggregates is increased.

### [Example 2] Production of DcR3 Variant That Is Not Aggregated in Mammalian Cell Expression System

There has not been known a human DcR3 variant that is not aggregated in a mammalian cell expression system. Thus, it was attempted to produce a DcR3 variant that results in a decreased amount of aggregates while maintaining the activity of DcR3. DcR3 is a soluble molecule consisting of 300 residues, and has a signal peptide at the N-terminal side, followed by four cysteine rich domains (CRD1, CRD2, CRD3, CRD4), which are characteristic of a TNF receptor superfamily (TNFRSF), and the C-terminal side of DcR3 consists of a heparan sulfate binding domain (HBD), which includes a heparan sulfate-binding motif and is rich in basic amino acids (FIG. 2, 3A). All of LIGHT, TL1A and FasL, which are DcR3 ligands, bind to CRD2 and CRD3 of DcR3. Thus, while maintaining a region including CRD2 and CRD3 of DcR3, CRD1 and/or CRD4 of DcR3 was/were substituted with CRD1 and/or CRD4 of a soluble decoy receptor osteoprotegerin (OPG), which is a related TNFRSF molecule, thus producing a DcR3 variant.

Various DcR3 variants having various structures, i.e.,
chimera A-Fc (FIG. 3C, SEQ ID NO: 80 or 82) obtained by fusion of chimera A (SEQ ID NO: 54) and an Fc sequence, wherein the chimera A was obtained by introducing into human DcR3, substitution of amino acid sequences of CRD1 and CRD4 with amino acid sequences of CRD1 and CRD4 of human OPG, respectively, and removal of an amino acid sequence of HBD, while maintaining amino acid sequences of CRD2 and CRD3 of human DcR3, and wherein the Fc sequence was selected from IEGRMD g1S (SEQ ID NO: 339) and g4PEK (SEQ ID NO: 74) obtained by introducing into an Fc region of heavy chain of human IgG4, substitution of Ser at EU-index position 228 with Pro, substitution of Leu at EU-index position 235 with Glu, and substitution of Arg at EU-index position 409 with Lys;
chimera B-Fc (FIG. 3G, SEQ ID NO: 76) obtained by fusion of chimera B (SEQ ID NO: 50) and an Fc sequence (IEGRMD g1S), wherein the chimera B was obtained by introducing into human DcR3, substitution of an amino acid sequence of CRD1 with an amino acid sequence of CRD1 of human OPG, and removal of an amino acid sequence of HBD, while maintaining amino acid sequences of CRD2, CRD3 and CRD4 of human DcR3;
chimera C-Fc (FIG. 3H, SEQ ID NO: 78) obtained by fusion of chimera C (SEQ ID NO: 52) and an Fc sequence (IEGRMD g1S), wherein the chimera C was obtained by introducing into human DcR3, substitution of an amino acid sequence of CRD4 with an amino acid sequence of CDR4 of human OPG, and removal of an amino acid sequence of HBD, while maintaining amino acid sequences of CRD1, CRD2 and CRD3 of human DcR3; and
103-123OPG-Fc (g4PEK) (FIG. 3D, SEQ ID NO: 84) obtained by fusion of 103-123OPG (SEQ ID NO: 56) and an Fc sequence (g4PEK), wherein the 103-123OPG was obtained by introducing into CRD3 of chimera A, substitution of an amino acid sequence including a part at positions 18 to 36 and two amino acids at the C-terminal side thereof with an amino acid sequence of human OPGwere produced by the following method. In the DcR3 variant including CRD4 derived from DcR3, a TS sequence corresponding to an amino acid sequence at positions 194 to 195 of DcR3 (SEQ ID NO: 2) was added to the C-terminus of the CRD4. In the DcR3 variant including CRD4 derived from OPG, an SGNSESTQK sequence corresponding to an amino acid sequence at positions 186 to 194 of OPG (SEQ ID NO: 14) was added to the C-terminus of the CRD4.

A DNA fragment of a signal peptide sequence and a DNA fragment encoding chimera A, chimera B, chimera C or 103-123OPG lacking an HBD sequence (chimera A: SEQ ID NO: 53, chimera B: SEQ ID NO: 49, chimera C: SEQ ID NO: 51, 103-123OPG: SEQ ID NO: 55) were artificially synthesized, followed by insertion into a pCIpuro vector by the same method as in Example 1 in such a manner that the DNA fragments were linked to a DNA fragment (SEQ ID NO: 338, 73) encoding an Fc sequence (IEGRMD g1S or g4PEK), thus obtaining a plasmid into which DNA fragments (chimera A-Fc: SEQ ID NO: 79 or 81, chimera B-Fc: SEQ ID NO: 75, chimera C-Fc: SEQ ID NO: 77, 103-123OPG-Fc: SEQ ID NO: 83) encoding various DcR3 variants were inserted. In the same manner as in Example 1, the plasmid thus obtained was introduced into host cells selected from Freestyle CHO-S cells, Freestyle 293F cells and Expi293 cells to transiently express a protein, and from the culture supernatant, affinity purification by MabSelect SuRe was performed.

The proportions (%) of monomers, aggregates and degradants of the various DcR3 variants produced were calculated by SEC-HPLC using the same method as in Example 1, or by SEC (Waters K.K.) using ACQUITY UPLC Protein BEH SEC 4.6 mm × 150 mm (Table 2).

**[Table 2]**

| DcR3 variant | Fc | Host cell | Monomer | Aggregate | Degradant |
|---|---|---|---|---|---|
| Chimera A-Fc | IEGRMD g1S | CHO-S | 93.8% | 4.6% | 1.6% |
| Chimera A-Fc | g4PEK | Expi293 | 96.6% | 3.4% | 0.0% |
| Chimera B-Fc | IEGRMD g1S | Expi293 | 83.8% | 15.4% | 0.8% |
| Chimera C-Fc | IEGRMD g1S | Expi293 | 70.0% | 29.5% | 0.5% |
| 103-123OPG-Fc | g4PEK | Expi293 | 98.5% | 1.5% | 0.0% |

As a result, in each of the DcR3 variants, i.e., chimera A-Fc, chimera B-Fc, chimera C-Fc and 103-123OPG-Fc, the proportion of aggregates was decreased compared to that of wild-type DcR3. The effect on decreasing aggregates was highest in chimera A-Fc, followed by chimera B-Fc and chimera C-Fc. Therefore, it was shown that each of substitution with CRD1 derived from OPG and substitution with CRD4 derived **from OPG** has an effect on decreasing aggregation, and that a combination of substitution with CRD1 and substitution with CRD4 has an effect on further decreasing aggregation.

As a result of subjecting chimera A-Fc, chimera B-Fc and chimera C-Fc (each including IEGRMD g1S) to SDS-PAGE under non-reducing conditions and 100 mM DTT reducing conditions, almost no smear or ladder was observed in chimera A-Fc and chimera B-Fc even when the variants were produced using mammalian cells as host cells (FIG. 4).

In order to calculate the absolute molecular weight, an analysis by SEC (TSKgel G3000 SWXL 7.8 mm × 300 mm) (Tosoh Corporation) using HPLC (Prominence, Shimadzu Corporation) was performed for chimera A-Fc (IEGRMD g1S or g4PEK) and 103-123OPG-Fc (g4PEK). After separation at a flow rate of 0.75 mL/min using a 50 mmol/L phosphate buffer (pH 7.0, 500 mmol/L NaCl) as a mobile phase, the scattered light intensity of each peak detected at a wavelength of 215 nm was detected by multi angle light scattering (MALS) (miniDAWN TREOS, Wyatt Technology Corporation), and the absolute molecular weight (% represents uncertainty) was calculated using the scattered light intensity. The results are shown in Table 3.

**[Table 3]**

| DcR3 variant | Fc | Molecular weight (%) | kDa |
|---|---|---|---|
| Chimera A-Fc | IEGRMD q1S | 8.858 x 10⁴ (1%) | 89 |
| Chimera A-Fc | g4PEK | 9.744 x 10⁴ (0.8%) | 97 |
| 103-123OPG-Fc | g4PEK | 1.052 x 10⁵ (0.7%) | 105 |

The molecular weights of dimers predicted from the amino acid sequences of chimera A-Fc (IEGRMD g1S or g4PEK) and 103-123OPG-Fc are 92.0 kDa, 90.1 kDa and 90.5 kDa, respectively, and thus it was confirmed that each of the DcR3 variants exists as dimers.

### [Example 3] Analysis of Proportion of N-Linked Glycosylation of Chimera A-Fc (g4PEK) and Evaluation of Influence on Aggregability

From the amino acid sequence of chimera A-Fc (g4PEK) (SEQ ID NO: 82), it is predicted that the CRD4 derived from OPG includes three potential N-linked glycosylation sites each consisting of Asn (N131, N144, N157), and that the Fc region includes one potential N-linked glycosylation site consisting of Asn (N260). Thus, the presence or absence of N-linked glycosylation of chimera A-Fc (g4PEK) was evaluated by the following method. As the sample preparation, chimera A-Fc (g4PEK) was subjected to reduction and alkylation treatments and the N-linked glycans of chimera A-Fc (g4PEK) were cleaved by PNGaseF treatment, and then the proteins were digested by each protease of trypsin, endoproteinase Asp-N and chymotrypsin. The peptide mixture thus obtained was dissolved in 5%(v/v) acetonitrile/0.1% formic acid, followed by an analysis with a liquid chromatography-electrospray ionization-mass spectrometer (LC-ESI-MS). Using MAGIC2000 HPLC (Michrom Bioresources, Inc.) equipped with a C18 reversed-phase column (0.2 mm × 150 mm) (GL Science Inc.) and LTQ Orbitrap XL ion trap-Orbitrap hybrid mass spectrometer (Thermo Scientific), an analysis was performed by gradient elution of 5-65%(v/v) acetonitrile/0.1% formic acid. The peptide fragments thus obtained were identified by a MASCOT analysis (Matrix Science Inc.) using the amino acid sequence of chimera A-Fc (g4PEK). Using as an index the mass increase by 0.984 Da caused by PNGaseF treatment for converting N-linked glycosylated Asn to Asp, a peptide exhibiting a shift in a peak of an MS spectrum and a glycosylation site included in the peptide were identified. As a result, it was shown that N-linked glycans were added for each of the Asn residues N131, N144, N157 and N260. Of these, regarding N157 and N260, glycans were added in almost all peptide fragments, while regarding N131 and N144, both of glycosylated fragments and non-glycosylated fragments were detected.

Next, in order to evaluate the influence on the aggregability of N-linked glycans added to three sites each consisting of Asn (N131, N144, N157) of CRD4 derived from OPG, an amino acid substitute lacking glycans at two sites of N131 and N144 or at three sites of N131, N144 and N157 was produced. As a variant lacking the two glycans, N131S/N144S-Fc (g4PEK) (SEQ ID NO: 86) obtained by substitution of each of N131 and N144 with Ser or T133A/S146A-Fc (g4PEK) (SEQ ID NO: 88) obtained by substitution of each of T133 and S146 with Ala was produced. As a variant lacking the three glycans, N1315/N144S/N1575-Fc (g4PEK) (SEQ ID NO: 90) obtained by substitution of each of N131, N144 and N157 with Ser or T133A/S146A/T159A-Fc (g4PEK) (SEQ ID NO: 92) obtained by substitution of each of T133, S146 and T159 with Ala was produced. A DNA fragment of a signal peptide sequence and DNA fragments (SEQ ID NO: 85, 87, 89, 91) encoding the respective variants lacking the glycans were artificially synthesized, followed by insertion into a pCIpuro vector by the same method as in Example 1, and each DNA fragment was introduced into Expi293 cells. Each of the variants lacking the glycans was transiently expressed, and then affinity purification from using the culture supernatant by MabSelect SuRe was performed. Each of the produced variants lacking the glycans was analyzed by SEC-UPLC (apparatus: ACQUITY UPLC, column; ACQUITY UPLC Protein BEH SEC 200 Å, 1.7 µm, 4.6 × 150 mm) (Waters K.K.). The proportions (%) of monomers, aggregates and degradants calculated from the peak areas are shown in Table 4.

**[Table 4]**

| Variant lacking glycans | Fc | Monomer | Aggregate | Degradant |
|---|---|---|---|---|
| N131S/N144S-Fc | g4PEK | 92.5% | 7.5% | 0.0% |
| T133A/S146A-Fc | g4PEK | 91.2% | 8.8% | 0.0% |
| N131S/N144S/ N157S-Fc | g4PEK | 64.0% | 36.0% | 0.0% |
| T133A/S146A/ T159A-Fc | g4PEK | 62.3% | 37.7% | 0.0% |

Regarding the aggregate contents of the variants lacking the two glycans, the aggregates were slightly increased **in** all of the amino acid substitutes compared to those of chimera A-Fc (Table 2), and regarding the aggregate contents of the variants lacking the three glycans, further increases in the aggregates were observed in all of the amino acid substitutes. From these results, it was shown that, of the N-linked glycans at three sites (N131, N144, N157) added to CRD4 derived from OPG, particularly a glycan added to N157, contributes to a decrease in the aggregates of chimera A. Meanwhile, even when all of the N-linked glycans at three sites were removed, the aggregates were decreased compared to those of DcR3 FL-Fc and S195-Fc (Table 1). Therefore, it was shown that each of an N-linked glycan and a sequence of OPG has an effect on decreasing the aggregates of wild-type DcR3, and that a combination of both has an effect on further decreasing the aggregates.

### [Example 4] Evaluation of Physical Property of Various DcR3 Variants

In order to analyze the cause of the effect on decreasing aggregation of various DcR3 variants produced in Examples 2 and 3, the following physical and chemical analyses were performed. Hydrophobic interaction chromatography (HIC) is an analysis method in which the higher the hydrophobicity of a protein surface is, the longer the elution time from a column is. Using a hydrophobic interaction chromatography column (TSKgel Butyl-NPR 4.6 mm × 35 mm) (Tosoh Corporation), and using a gradient of buffer A (2 mmol/L ammonium sulfate, 20 mmol/L Tris buffer, pH 7.0) and buffer B (20 mmol/L Tris buffer, pH 7.0) as a mobile phase, 8 µg of a sample was separated at a flow rate of 0.5 mL/min, and the elution time (min) was detected at a wavelength of 215 nm. The elution time (min) is shown in Table 5.

**[Table 5]**

| | | Fc | Host cell | Elution time (min) |
|---|---|---|---|---|
| Wild-type DcR3 control | R218Q-Fc | IEGRMD g1S | S2 | 39.8 |
| | S195-Fc | IEGRMD g1S | S2 | 36.7 |
| DcR3 variant | Chimera A-Fc | IEGRMD g1S | CHO-S | 37.8 |
| | Chimera A-Fc | g4PEK | Expi293 | 38.7 |
| | Chimera C-Fc | IEGRMD g1S | Expi293 | 39.7 |
| | 103-123OPG-Fc | g4PEK | Expi293 | 37.8 |
| | N131S/ N144S-Fc | g4PEK | Expi293 | 39.2 |
| | T133A/ S146A-Fc | g4PEK | Expi293 | 39.3 |
| | N131S/N144S/ N157S-Fc | g4PEK | Expi293 | 40.6 |
| | T133A/S146A/ T159A-Fc | g4PEK | Expi293 | 40.9 |

Each of S195-Fc derived from S2 and the variants derived from mammalian cells, i.e., chimera A-Fc (IEGRMD g1S, g4PEK), 103-123OPG-Fc (g4PEK) and the variants lacking the two glycans, showed a shorter elution time as compared to R218Q-Fc corresponding to a one-amino-acid mutant of full-length DcR3 produced using insect cells S2. It is considered that a decrease in hydrophobicity resulted in improvement in the physical property and a decrease in the amount of aggregates. Regarding chimera C and the variants lacking the three glycans, each of which exhibited the slightly-attenuated effect of decreasing the amount of aggregates, the hydrophobicities were equivalent or superior to that of R218Q-Fc, and it was shown that there is a correlation between the proportion of aggregates and the hydrophobicity of the protein.

Subsequently, the thermostability of the protein was evaluated by the differential scanning fluorimetry (DSF) method. In a 96-well white microplate (Bio-Rad Laboratories, Inc.), 9.5 µg of various DcR3 variants and 1 µL of SYPRO Orange Protein Gel Stain (Invitrogen) diluted with water 50-fold were mixed in 20 µL of a system, and the temperature was raised from 20°C to 95°C by 0.5°C every 10 seconds using a C1000 thermal cycler (Bio-Rad Laboratories, Inc.). Fluorescence at each temperature was detected with a FRET channel, and the melting curve (FIG. 5) and the melting temperature (Tm value) (°C) (Table 6) were calculated using CFX Manager software (Bio-Rad Laboratories, Inc.).

**[Table 6]**

| | | Fc | Host cell | Tm1 | Tm2 |
|---|---|---|---|---|---|
| Wild-type DcR3 control | R218Q-Fc | IEGRMD g1S | S2 | 47.5 | 60.5 |
| | S195-Fc | IEGRMD g1S | S2 | 62.0 | 81.0 |
| DcR3 variant | Chimera A-Fc | IEGRMD g1S | CHO-S | 66.5 | 82.5 |
| | Chimera A-Fc | g4PEK | Expi293 | 61.0 | 82.5 |
| | Chimera C-Fc | IEGRMD g1S | Expi293 | 67.5 | 82.0 |
| | 103-123OPG-Fc | g4PEK | Expi293 | 61.0 | 82.5 |
| | N131S/ N144S-Fc | g4PEK | Expi293 | 60.0 | 82.5 |
| | T133A/ S146A-Fc | g4PEK | Expi293 | 60.0 | 82.5 |
| | N131S/N144S/ N157S-Fc | g4PEK | Expi293 | 61.0 | 83.0 |
| | T133A/S146A/ T159A-Fc | g4PEK | Expi293 | 61.0 | 83.5 |

S195-Fc derived from S2 showed a greatly increased Tm value and improved thermostability as compared to R218Q-Fc corresponding to a one-amino-acid mutant of full-length DcR3 produced using insect cells S2. This suggests that an HBD region contributes to thermolability. Chimera A-Fc (IEGRMD g1S) derived from a mammalian cell showed a further increased Tm value as compared to S195-Fc. This reveals that a DcR3 variant in which a part of CRDs of DcR3 has been substituted with a part of CRDs of OPG shows not only decreased aggregability but also improved thermostability of the protein (Table 6, FIG. 5).

### [Example 5] Evaluation of Reactivity of DcR3 Variant Lacking Heparan Sulfate-Binding Domain (HBD) with Human Normal Cell and CHO Cell

It has been reported that *in vivo* kinetics of wild-type DcR3 and a mutant thereof, FLINT (R218Q mutation), in mice and cynomolgus monkeys are extremely poor (Drug Metabolism and Disposition, 2003, 31: p.502-507). One of the factors thereof is considered as the fact that DcR3 directly binds to heparan sulfate proteoglycan on the cell membrane by a heparan sulfate-binding domain (HBD) existing in wild-type DcR3 (J. Immunol., 2006, 176: 173-180).

Thus, the reactivities of DcR3 variants lacking HBD, i.e., S195-Fc, chimera A-Fc (IEGRMD g1S, g4PEK) and 103-123OPG-Fc (g4PEK) with human primary cells and CHO cells (producing cells) were analyzed by flow cytometry (FCM). As a positive control, DcR3 FL-Fc including an HBD region was used, and as **negative** controls, K194-Fc and an anti-DNP antibody (g4PEK) were used. K194-Fc (amino acid sequence: SEQ ID NO: 152, nucleotide sequence of DNA: SEQ ID NO: 151) is a protein obtained by fusing an IEGRMD linker (SEQ ID NO: 106) and an amino acid sequence (SEQ ID NO: 72) of Fc (g1S) to an amino acid sequence of a part extending from Met at position 1 to Lys at position 194 of OPG (SEQ ID NO: 14), and using the method mentioned in Example 1, K194-Fc was transiently expressed using Expi293 cells, and purified from the culture supernatant using Mabselect SuRe (GE Healthcare). As the anti-DNP antibody (g4PEK), an antibody obtained by inserting the nucleotide sequence of the variable region of the anti-2,4-dinitrophenol (DNP) antibody mentioned in Clin. Cancer Res., 2005, 11(8), p.3126-3135 into a vector encoding an Fc sequence (g4PEK), followed by introduction into CHO cells to express, and performing Protein A purification from the culture supernatant, was used. As the human primary cells, HUVECs (Lonza K.K.) and male human hepatocytes (BioreclamationIVT, LLC) were used. The human primary cells were cultured with a medium designated for each cell in accordance with the package insert using a collagen-coated adherent plate (IWAKI). The CHO cells were suspension-cultured with EX-CELL 325 PF CHO Serum-Free Medium (Sigma Aldrich Co. LLC).

HUVECs and hepatocytes were detached using a 0.02% EDTA solution and a cell scraper, and passed through a cell strainer (40 µm). The detached HUVECs and hepatocytes and CHO cells collected from the suspension culture solution were washed with an FCM buffer (PBS containing 1% BSA, 1 mmol/L EDTA and 0.05% NaN₃), and then suspended with an FCM buffer.

Next, the cells were seeded in a 96-well U-bottom plate (Falcon) so that the concentrations were 1 × 10⁵ cells/well, and each Fc fusion protein produced was added so that the concentration was 10 µg/mL, followed by reaction on ice for 1 hour. The cells were washed with an FCM buffer, and then suspended with LIVE/DEAD Fixable Aqua Dead Cell Stain Kit (Molecular Probes) and 0.1 µg/mL of Goat F(ab')₂ Anti-Human IgG R-phycoerythrin Conjugate (Southern Biotechnology Associates, Inc.), followed by staining on ice for 1 hour. During staining of HUVECs and hepatocytes, Human FcR Blocking Reagent (Miltenyi Biotec K.K.) was added. The cells were washed with an FCM buffer, and then the fluorescence intensity was analyzed by flow cytometer FACS Fortessa (BD Biosciences).

As a result of analysis of the PE staining intensities for viable cell fractions that were negative for LIVE/DEAD Fixable Aqua Dead Cell Stain Kit, DcR3 FL-Fc (IgG1) (R&D Systems, Inc.) showed remarkable binding to all of HUVECs, hepatocytes and CHO cells. Meanwhile, all of the DcR3 variants lacking HBD showed no binding (FIG. 6).

### [Example 6] Evaluation of Mouse In Vivo Kinetics of DcR3 Variant

**[Table 7]**

| | | Fc | Host cell |
|---|---|---|---|
| Wild-type DcR3 control | S195-Fc | IEGRMD g1S | S2 |
| DcR3 variant | Chimera A-Fc | IEGRMD g1S | CHO-S |
| | Chimera A-Fc | g4PEK | CHO-S |
| | Chimera A-Fc | g4PEK | CHO |
| | 103-123OPG-Fc | g4PEK | CHO |
| | T133A/S146A-Fc | g4PEK | CHO-S |

A kinetic study in mouse on S195-Fc and each of the DcR3 variants shown in Table 7 was conducted. Regarding chimera A-Fc (g4PEK) and 103-123OPG-Fc (g4PEK), each of the DcR3 variants was produced by stable expression in CHO cells using the following method. A DNA fragment of a signal peptide sequence and DNA fragments (SEQ ID NO: 81, 83) encoding the respective DcR3 variants were artificially synthesized, followed by insertion into a recombinant vector produced by the method mentioned in WO 2012/081628 using the same method as in Example 1, and the vectors were introduced into CHO cells by electroporation. Culture and drug selection were performed by the general method, and cell lines in which the proportions of viable cells collected to about 98% were regarded as stable expression cell lines. The cells of these stable expression cell lines were cultured in a medium such as EX-CELL 325 PF CHO Serum-Free Medium (Sigma Aldrich Co. LLC) for a certain period of time, followed by collection of the culture supernatants, and each of the DcR3 variants was purified by the method mentioned in Example 1.

Single i.v. administration of 10 mg/kg of S195-Fc or each of the DcR3 variants was performed to 5- to 6-week-old BALB/c mice (female) (n = 2 or 3), followed by blood collection from the tail vein at an appropriate time after administration, and concentrations of S195-Fc and each of the DcR3 variants in blood were measured by the following method. A biotinylated monkey anti-human IgG polyclonal antibody was reacted with a streptavidin-immobilized bead, and S195-Fc and each of the DcR3 variants in serum bound to the biotinylated monkey anti-human IgG polyclonal antibody were detected with an Alexa Fluor 647-labeled monkey anti-human IgG polyclonal antibody. Measurement was performed using Gyrolab xP workstation (Gyros AB), and each kinetic parameter was calculated by the moment analysis method. As the standard substance for preparation of a calibration curve, the same substance as the test substance administered to the animals was used. The time courses of blood concentrations of S195-Fc produced using S2 cells and chimera A-Fc (IEGRMD g1S) produced using CHO-S cells are shown in FIG. 7. Regarding each of S195-Fc and the DcR3 variants shown in Table 7, the half-life in blood (h) during the elimination phase and the area under the concentration-time curve to infinity AUC0-∞ (µg*h/mL) after single administration are shown in Table 8.

**[Table 8]**

| | | Fc | Host cell | Half-life in blood (h) | AUC0-∞ (µg*h/mL) |
|---|---|---|---|---|---|
| Wild-type DcR3 control | S195-Fc | IEGRMD g1S | S2 | 84.1 | 354 |
| DcR3 variant | Chimera A-Fc | IEGRMD g1S | CHO-S | 87.9 | 7140 |
| | Chimera A-Fc | g4PEK | CHO-S | 114 | 5680 |
| | Chimera A-Fc | g4PEK | CHO | 89.5 | 4530 |
| | 103-123OPG-Fc | g4PEK | CHO | 79.3 | 2400 |
| | T133A/S145A-Fc | g4PEK | CHO-S | 114 | 5810 |

The time course of blood concentration of each of the DcR3 variants was greatly improved (FIG. 7), the half-life of each of the DcR3 variants was almost equivalent, and the AUC0-∞ of each of the DcR3 variants was improved 10-fold or more (Table 8), compared to those of S195-Fc corresponding to a wild-type DcR3 control.

It has been reported that, after single i.v. administration of 0.5 mg/kg of wild-type DcR3 or FLINT (R218Q mutation) to CD-1 mice, the half-life in blood was 1.2 hours and 3.1 hours, respectively, and the AUC0-∞ (µg*h/mL) was 0.48 and 0.36, respectively (Drug Metabolism and Disposition, 2003. 31: p.502-507.). When the time courses of concentrations multiplied by a dose ratio assuming linearity were compared, the DcR3 variant showed higher exposure than that of wild-type DcR3 and FLINT.

### [Example 7] Evaluation of Binding Activity to DcR3 Ligand (1) Production of DcR3 Ligand

Soluble recombinants of DcR3 ligands (LIGHT, TL1A, FasL) of a human, a cynomolgus monkey or a mouse were produced (amino acid sequence: SEQ ID NO: 114, 116, 118, 120, 122, 124, 126, 128, 130, nucleotide sequence of DNA: SEQ ID NO: 113, 115, 117, 119, 121, 123, 125, 127, 129).

As a soluble recombinant LIGHT of a human, a cynomolgus monkey or a mouse, a sequence obtained by adding a His tag (His10) and a GS linker (GGGSGGGSGGGSIEGR) to the N-terminus and linking an extracellular region of LIGHT (human LIGHT: Asp74-Val240 (SEQ ID NO: 132), cynomolgus monkey LIGHT: Asp74-Val240 (SEQ ID NO: 134), mouse LIGHT: Asp72-Val239 (SEQ ID NO: 136)) to downstream thereof was used.

As a soluble recombinant TL1A of a human, a cynomolgus monkey or a mouse, a sequence obtained by adding a His tag (His6) and a GS linker (GGGSGGGSGGGS) to the N-terminus and linking an extracellular region of TL1A (human TL1A: Leu72-Leu251 (SEQ ID NO: 138), cynomolgus monkey TL1A: Leu72-Leu251 (SEQ ID NO: 140), mouse TL1A: Ile94-Leu270 (SEQ ID NO: 142)) to downstream thereof was used.

As a soluble recombinant FasL of a human, a cynomolgus monkey or a mouse, a sequence obtained by adding a His tag (His6) to the N-terminus and linking an extracellular region of FasL (human FasL: Pro134-Leu281 (SEQ ID NO: 144), cynomolgus monkey FasL: Pro133-Leu280 (SEQ ID NO: 146), mouse FasL: Pro132-Leu279 (SEQ ID NO: 148)) to downstream thereof was used.

A DNA fragment of a signal peptide sequence and a DNA sequence of a soluble DcR3 ligand including an attached tag were artificially synthesized (GENEWIZ, Inc.), and using In-Fusion HD Cloning Kit (Clontech Laboratories, Inc.), they were inserted into downstream of a CMV promoter of a pCI-Hygro2.01 vector (pCI manufactured by Promega Corporation was partially modified), and *Escherichia coli* DH5a competent cells (TOYOBO CO., LTD.) were transformed.

The plasmids thus obtained were introduced into Expi293 cells (Thermo Scientific) to transiently express the proteins. Using ExpiFectamine 293 (Thermo Scientific), the plasmids were introduced, and after culture for 3 days, the culture supernatants were collected.

Human FasL and cynomolgus monkey FasL were introduced into host cells of Freestyle CHO-S cells (Thermo Scientific) to transiently express proteins. Using Freestyle MAX Reagent (Thermo Scientific), the plasmids were introduced, and after culture for 3 days, the culture supernatants were collected.

Protein purification from the culture supernatants of the Expi293 cells was performed using Ni Sepharose Fast Flow resin and His Buffer Kit (all of which are manufactured by GE Healthcare). The culture supernatants were passed through a column filled with a resin, washed with a washing buffer (60 mmol/L imidazole, 20 mmol/L sodium phosphate, 0.5 mol/L NaCl, pH 7.4), and eluted with an elution buffer (250 mmol/L imidazole, 20 mmol/L sodium phosphate, 0.5 mol/L NaCl, pH 7.4).

Protein purification from the culture supernatants of the Freestyle CHO-S cells was performed using Complete His-Tag Purification Resin (Roche) and His Buffer Kit (GE Healthcare). The culture supernatants were passed through a column filled with a resin, washed with a washing buffer (2 mmol/L imidazole, 20 mmol/L sodium phosphate, 0.5 mol/L NaCl, pH 7.4), and eluted with an elution buffer (250 mmol/L imidazole, 20 mmol/L sodium phosphate, 0.5 mol/L NaCl, pH 7.4).

Each elution buffer of elution fractions was substituted with PBS using a NAP column (GE Healthcare) and passed through a 0.22 µm filter to perform sterilization. Regarding the purified proteins thus obtained, the purities were confirmed by SDS-PAGE. When formations of multimers were analyzed by SEC-UPLC (apparatus: ACQUITY UPLC, column; ACQUITY UPLC Protein BEH SEC 200Å, 1.7 µm, 4.6 × 150 mm) (Waters K.K.), almost all recombinant soluble DcR3 ligands showed a peak of a molecular weight corresponding to a trimer, but in only mouse LIGHT, no peak of a trimer was detected, and a peak of a monomer was detected.

### (2) Measurement of Binding Activity Using BIAcore

Regarding each of the various wild-type DcR3 controls and the DcR3 variants shown in Table 9, the binding activities to DcR3 ligands (LIGHT, TL1A, FasL) were analyzed by the SPR method using BIAcore T-100 (GE Healthcare). As the buffer, HBS-EP+ Buffer was used.

After 10,000 RU of anti-human antibodies were immobilized to Series S Sensor Chip CM5 using Human Antibody Capture Kit (GE Healthcare), various wild-type DcR3 controls and DcR3 variants were injected at the flow rate of 10 µL/min for 30 seconds and captured. Meanwhile, the buffer containing no protein was injected into reference flow cells. Thereafter, as an analyte, DcR3 ligands of a human, a cynomolgus monkey or a mouse diluted to 0.08 to 80 nmol/L were injected at 10 µL/min for 2 minutes to monitor the binding, and subsequently the buffer was injected for 3 minutes to monitor dissociation. Then, 3 mol/L magnesium chloride was injected at 20 µL/min for 1 minute to perform a regeneration reaction. By using BIAcore T-100 evaluation software and 1:1 Binding model, and by regarding each DcR3 ligand as a monomer (human LIGHT monomer: 20.8 kDa, human TL1A monomer: 22.1 kDa, human FasL monomer: 17.7 kDa, cynomolgus monkey LIGHT monomer: 20.8 kDa, cynomolgus monkey TL1A monomer: 22.0 kDa, cynomolgus monkey FasL monomer: 17.7 kDa, mouse LIGHT monomer: 20.9 kDa, mouse TL1A monomer: 21.5 kDa, mouse FasL monomer: 17.7 kDa), each kinetic constant (kₐ, k_{d}, K_{D}) was calculated (Tables 10 to 12). As a result of measurement, except for mouse LIGHT for which a trimer could not be produced, it was confirmed that various wild-type DcR3 controls and DcR3 variants bind to each DcR3 ligand of a human, a cynomolgus monkey or a mouse.

**[Table 9]**

| | | Linker | Fc | Host cell |
|---|---|---|---|---|
| 1 | DcR3 FL-Fc (R&D Systems, Inc.) | IEGRMD | IgG1 | Sf9 |
| 2 | S195-Fc | IEGRMD | g1S | S2 |
| 3 | Chimera A-Fc | IEGRMD | g1S | CHO-S |
| 4 | Chimera A-Fc | None | g4PEK | Expi293 |
| 5 | 103-123OPG-Fc | None | g4PEK | Expi293 |
| 6 | T133A/S146A-Fc | None | g4PEK | Expi293 |
| 7 | N131S/N144S-Fc | None | g4PEK | Expi293 |

**[Table 10]**

| Binding affinity to LIGHT | | | | | | | | | |
|---|---|---|---|---|---|---|---|---|---|
| | Human LIGHT | | | Cynomolgus monkey LIGHT | | | Mouse LIGHT | | |
| | Ka (1/Ms) 1xE+05 | K_{d} (1/s) 1xE-04 | K_{D} (nM) | Kₐ (1/Ms) 1xE+05 | K_{d} (1/s) 1xE-04 | K_{D} (nM) | Kₐ (1/Ms) 1xE+05 | K_{d} (1/s) 1xE-04 | K_{D} (nM) |
| 1 | 3.8 | 4.0 | 1.1 | 3.1 | 3.8 | 1.2 | N.D. | N.D. | N.D. |
| 2 | 2.8 | 6.1 | 2.2 | 3.4 | 5.9 | 1.7 | N.D. | N.D. | N.D. |
| 3 | 3.9 | 2.5 | 0.63 | 5.4 | 2.4 | 0.44 | N.D. | N.D. | N.D. |
| 4 | 5.4 | 3.3 | 0.62 | N.T. | N.T. | N.T. | N.T. | N.T. | N.T. |
| 5 | 4.5 | 3.0 | 0.66 | N.T. | N.T. | N.T. | N.T. | N.T. | N.T. |
| 6 | 3.8 | 3.1 | 0.83 | N.T. | N.T. | N.T. | N.T. | N.T. | N.T. |
| 7 | 3.5 | 3.3 | 0.87 | N.T. | N.T. | N.T. | N.T. | N.T. | N.T. |

| | | | | | | | | | |
|---|---|---|---|---|---|---|---|---|---|
| N.D.: Not detected N.T.: Not tested | | | | | | | | | |

**[Table 11]**

| Binding affinity to TL1A | | | | | | | | | |
|---|---|---|---|---|---|---|---|---|---|
| | Human TL1A | | | Cynomolgus monkey TL1A | | | Mouse TL1A | | |
| | Kₐ (1/Ms) 1xE+05 | K_{d} (1/s) 1xE-04 | K_{D} (nM) | Kₐ (1/Ms) 1xE+05 | K_{cl} (1/s) 1xE-04 | K_{D} (nM) | Kₐ (1/Ms) 1xE+05 | K_{cl} (1/s) 1xE-04 | K_{D} (nM) |
| 1 | 3.9 | 3.5 | 0.89 | 3.8 | 3.5 | 0.92 | 8.4 | 4.9 | 0.58 |
| 2 | 8.4 | 9.1 | 1.1 | 7.9 | 8.8 | 1.1 | 17 | 20 | 1.2 |
| 3 | 8.3 | 3.0 | 0.36 | 7.5 | 2.9 | 0.38 | 31 | 23 | 0.73 |
| 4 | 8.7 | 3.6 | 0.42 | N.T. | N.T. | N.T. | N.T. | N.T. | N.T. |
| 5 | 7.1 | 3.3 | 0.47 | N.T. | N.T. | N.T. | N.T. | N.T. | N.T. |
| 6 | 6.3 | 3.3 | 0.53 | N.T. | N.T. | N.T. | N.T. | N.T. | N.T. |
| 7 | 6.1 | 3.1 | 0.51 | N.T. | N.T. | N.T. | N.T. | N.T. | N.T. |

| | | | | | | | | | |
|---|---|---|---|---|---|---|---|---|---|
| N.T.: Not tested | | | | | | | | | |

**[Table 12]**

| Cynomolgus monkey FasL | | | | | | | | | |
|---|---|---|---|---|---|---|---|---|---|
| | Human FasL | | | Cynomolgus monkey FasL | | | Mouse FasL | | |
| | Ka (1/Ms) 1xE+05 | K_{d} (1/s) 1xE-04 | K_{D} (nM) | Ka (1/Ms) 1xE+05 | K_{d} (1/s) 1xE-04 | K_{D} (nM) | Kₐ (1/Ms) 1xE+05 | K_{d} (1/s) 1xE-04 | K_{D} (nM) |
| 1 | 3.1 | 4.0 | 1.3 | 3.0 | 3.3 | 1.1 | 1.5 | 97 | 65 |
| 2 | 8.6 | 15 | 1.7 | 7.5 | 8.4 | 1.1 | 5.8 | 56 | 96 |
| 3 | 10 | 7.8 | 0.76 | 8.4 | 4.0 | 0.47 | 12 | 587 | 47 |
| 4 | 10 | 10 | 1.0 | N.T. | N.T. | N.T. | N.T. | N.T. | N.T. |
| 5 | 8.5 | 12 | 1.4 | N.T. | N.T. | N.T. | N.T. | N.T. | N.T. |
| 6 | 5.6 | 13 | 2.3 | N.T. | N.T. | N.T. | N.T. | N.T. | N.T. |
| 7 | 5.7 | 15 | 2.5 | N.T. | N.T. | N.T. | N.T. | N.T. | N.T. |

| | | | | | | | | | |
|---|---|---|---|---|---|---|---|---|---|
| N.T.: Not tested | | | | | | | | | |

### [Example 8] Evaluation of Binding Activity to OPG Ligands (RANKL, TRAIL)

It has been known that RANKL binds to RANK and is involved in bone resorption, and that TRAIL binds to a TRAIL receptor and is involved in cell death. Using various human DcR3 recombinants produced, the binding activities to RANKL and TRAIL were analyzed by ELISA. As positive controls that bind to the OPG ligands, K194-Fc corresponding to a variant lacking a C-terminus region of OPG, produced in Example 5, RANK-Fc (Enzo Life Sciences, Inc.) and TRAIL R1-Fc (R&D systems, Inc.) were used. As a negative control, an anti-DNP antibody (IgG1) corresponding to an antibody obtained by inserting the nucleotide sequence of the variable region of the anti-2,4-dinitrophenol (DNP) antibody mentioned in Clin. Cancer Res., 2005, 11(8), p.3126-3135 into a vector encoding Fc of IgG1, followed by introduction into CHO cells to express, and performing Protein A purification, was used.

Into 96-well plates (MAXISORP NUNC-IMMUNO PLATE, Thermo Scientific), anti-human IgG1 (American Qualex International, Inc.) prepared to 10 µg/mL with PBS was dispensed at 50 µL/well, followed by allowing to stand at 4°C overnight for adsorption. After removal of the immobilizing solution, 1% Block Ace produced by dissolving 1 g of Block Ace powder (DS Pharma Biomedical Co., Ltd.) in 100 mL of water was dispensed at 100 µL/well, followed by allowing to stand at room temperature for 1 hour to perform blocking, and the plates were washed 3 times with PBS containing 0.1% Tween (hereinafter referred to as PBST). Then, into plate No. 1, various human DcR3 recombinants or various DcR3 variants and RANK-Fc diluted to the concentration of 1 µg/mL with 1% BSA-PBS were dispensed at 50 µL/well, and into plate No. 2, various wild-type DcR3 controls or various DcR3 variants and TRAIL R1-Fc diluted to the concentration of 1 µg/mL with 1% BSA-PBS were dispensed at 50 µL/well, followed by allowing to stand at room temperature for 1 hour.

After each plate was washed 3 times with PBST, into plate No. 1, RANKL (Peprotech, Inc.) diluted to 0.64 to 50,000 pg/mL with 1% BSA-PBS was dispensed at 50 µL/well, and into plate No. 2, TRAIL (Peprotech, Inc.) to 0.64 to 50,000 pg/mL diluted with 1% BSA-PBS was dispensed at 50 µL/well, followed by allowing to stand at room temperature for 1 hour.

After each plate was washed 3 times with PBST, into plate No. 1, a biotinylated anti-RANKL antibody (Peprotech, Inc.) diluted to 0.4 µg/mL with 1% BSA-PBS was dispensed at 50 µL/well, and into plate No. 2, a biotinylated anti-TRAIL antibody (R&D Systems, Inc.) diluted to 0.4 µg/mL with 1% BSA-PBS was dispensed at 50 µL/well, followed by allowing to stand at room temperature for 1 hour.

After each plate was washed 3 times with PBST, streptavidin-HRP (PIERCE) diluted 10,000-fold with 0.1% Block Ace was dispensed thereinto at 50 µL/well, followed by allowing to stand at room temperature for 1 hour.

After each plate was washed 3 times with PBST, TMB+ Substrate Chromogen (Dako) was dispensed at 50 µL/well, followed by allowing to stand at room temperature for 1 minute. 0.5 mol/L sulfuric acid solution was dispensed at 50 µL/well to stop the chromogenic reaction, and the absorbances at a sample wavelength of 450 nm and a reference wavelength of 570 nm were measured using a plate reader.

As a result, K194-Fc showed binding to RANKL and TRAIL, which are OPG ligands, and RANK-Fc and TRAIL R1-Fc showed binding to RANKL and TRAIL, respectively, but DcR3 FL-Fc (R&D Systems, Inc.), S195-Fc, chimera A-Fc (IEGRMD g1S) and chimera B-Fc (IEGRMD g1S) did not show binding to any of the ligands (FIG. 8). In conjunction with the results of Example 7, it was confirmed that DcR3 variants produced by substituting a part of CRDs of DcR3 with a part of CRDs of OPG show binding activities to DcR3 ligands equivalent to that of wild-type DcR3, but do not bind to OPG ligands.

### [Example 9] Measurement of Neutralizing Activity to DcR3 Ligand

By the following method, the neutralizing activities of various wild-type DcR3 controls and DcR3 variants to LIGHT, TL1A and FasL were measured. A DNP antibody was produced by the methods mentioned in Examples 5 and 8.

### (1) Measurement of Neutralizing Activity to LIGHT

Using a human colon cancer cell HT-29 cell line (ATCC Number: HTB-38), the neutralizing activities of various wild-type DcR3 controls and various DcR3 variants were measured using IL-8 production by addition of LIGHT as an index. For cell culture and evaluation of the neutralizing activity, a McCoy's 5A medium (Gibco) supplemented with 10% FBS (Gibco) and penicillin/streptomycin (Nacalai Tesque, Inc.) was used.

The cells of HT-29 cell line were seeded at 2 x 10⁴ cells/well in a 96-well adherent culture plate (Sumitomo Bakelite Co., Ltd.), and then various wild-type DcR3 controls or various DcR3 variants were added at the final concentration of 0.1, 1 or 10 µg/mL. Thereafter, human LIGHT (Gly66-Val240) including a FLAG tag (DYKDDDDK) added to the N-terminus (mentioned in JP 2013153749 A) was added at the final concentration of 0.1 µg/mL so that the total amount of the culture solution was adjusted at 200 µL/well. After culture in a 5% CO₂ incubator at 37°C for 3 days, the culture supernatant was collected, and the IL-8 concentration in the culture supernatant was measured using AlphaLISA IL-8 Immunoassay Research Kit (PerkinElmer, Inc.).

As a result of the measurement, it was confirmed that, in each of the various human DcR3 variants, the production amount of IL-8 is concentration-dependently decreased, and the neutralizing activity to LIGHT is exhibited (FIG. 9).

### (2) Measurement of Neutralizing Activity to TL1A

Using a human T cell, the neutralizing activities of various wild-type DcR3 controls and various DcR3 variants were measured using IFN-γ production by addition of TL1A as an index. For cell culture and evaluation of the neutralizing activity, an X-VIVO15 medium (Lonza K.K.) was used.

Frozen healthy individual PBMCs (AllCells, LLC) were thawed in a water bath at 37°C, and suspended in a medium containing DNaseI (STEMCELL Technologies Inc.) warmed to 37°C. After shaking at a low speed at 37°C for 2 hours, T cells were isolated using EasySep Human T cell Enrichment Kit (STEMCELL Technologies Inc.). The isolated T cells were seeded at 1 x 10⁵ cells/well in a 96-well suspension culture plate (Sumitomo Bakelite Co., Ltd.), and then various wild-type DcR3 controls or various DcR3 variants were added at the final concentration of 0.1, 1 or 10 µg/mL. Thereafter, the recombinant His10 human TL1A produced in Example 6 was added at the final concentration of 0.1 µg/mL. Human IL-12 **(Miltenyi** Biotec K.K.) at a final concentration of 2 ng/mL and Recombinant Human IL-18 (MEDICAL & BIOLOGICAL LABORATORIES CO., LTD.) at a final concentration of 50 ng/mL were added so that the total amount of the culture solution was adjusted to 200 µL/well. After culture in a 5% CO₂ incubator at 37°C for 3 days, the culture supernatant was collected, and the IFN-γ concentration in the culture supernatant was measured using AlphaLISA IFN-γ Immunoassay Research Kit (PerkinElmer, Inc.).

As a result of the measurement, it was confirmed that, in each of the various DcR3 variants, the production amount of IFN-γ is concentration-dependently decreased, and the neutralizing activity to TL1A is exhibited (FIG. 10).

### (3) Measurement of Neutralizing Activity to FasL

Using a T-cell leukemia Jurkat cell line (DSMZ Number: ACC 282), the neutralizing activities of various wild-type DcR3 controls and various DcR3 variants were measured using apoptosis by addition of FasL as an index. For cell culture and evaluation of the neutralizing activity, an RPMI1640 medium (Nacalai Tesque, Inc.) supplemented with 10% FBS and penicillin/streptomycin was used.

The cells of Jurkat cell line were seeded at 5 x 10⁴ cells/well in a 96-well suspension culture plate, and then various wild-type DcR3 controls or DcR3 variants were added at the final concentration of 0.01, 0.1 or 1 µg/mL. Thereafter, recombinant human His6 Fas Ligand (Cell Signaling Technology Japan, K.K.) was added at the final concentration of 0.1 µg/mL so that the total amount of the culture solution was adjusted to 100 µL/well. After culture in a 5% CO₂ incubator at 37°C overnight, CellTiter-Glo (Promega Corporation) was added at 100 µL/well in a Jurkat culture plate, and using a luminometer (Veritas, Promega Corporation), the viable cell count using the production amount of ATP as an index was measured.

As a result of the measurement, it was confirmed that, in each of the various DcR3 variants, the production amount of ATP derived from viable cells is concentration-dependently increased, and the neutralizing activity to FasL is exhibited (FIG. 11).

### [Example 10] Neutralizing Activity and Binding Activity of Variant with lowered FasL binding activity to Ligand

Based on chimera A-Fc (g4PEK), one-amino-acid substitutes that have binding and neutralizing activities to TL1A and LIGHT of DcR3 ligands, but have low binding and neutralizing activities to FasL of DcR3 ligands were produced and evaluated. Variants were produced by substituting an appropriately-selected amino acid of CRD2 or CRD3 with Ala or an amino acid other than Ala as shown in Table 13, and the neutralizing activities to DcR3 ligands were measured by the same method as in Example 9. As a result, the neutralizing activities to FasL were selectively lowered in one-amino-acid substitutes, i.e., chimera A-E57K-Fc (g4PEK) (amino acid sequence: SEQ ID NO: 94, nucleotide sequence of DNA: SEQ ID NO: 93) and chimera A-E57L-Fc (g4PEK) (amino acid sequence: SEQ ID NO: 96, nucleotide sequence of DNA: SEQ ID NO: 95), which were obtained by substituting Glu at position 57 from the N-terminus of chimera A-Fc (g4PEK) with Lys and Leu, respectively, and chimera A-R60K-Fc (g4PEK) (amino acid sequence: SEQ ID NO: 98, nucleotide sequence of DNA: SEQ ID NO: 97), which was obtained by substituting Arg at position 60 with Lys (Table 13).

Regarding each of the variants exhibiting the selectively-lowered neutralizing activities to FasL, i.e., chimera A-E57K-Fc (g4PEK) (SEQ ID NO: 94), chimera A-E57L-Fc (g4PEK) (SEQ ID NO: 96) and chimera A-R60K-Fc (g4PEK) (SEQ ID NO: 98), the binding activities to DcR3 ligands were measured by the same method as in Example 7. As a result, in each of the variants of chimera A-E57K-Fc (g4PEK), chimera A-E57L-Fc (g4PEK) and chimera A-R60K-Fc (g4PEK), only the binding activity to FasL was remarkably lowered, and the binding activities to TL1A and LIGHT were maintained (FIG. 12A, B, C).

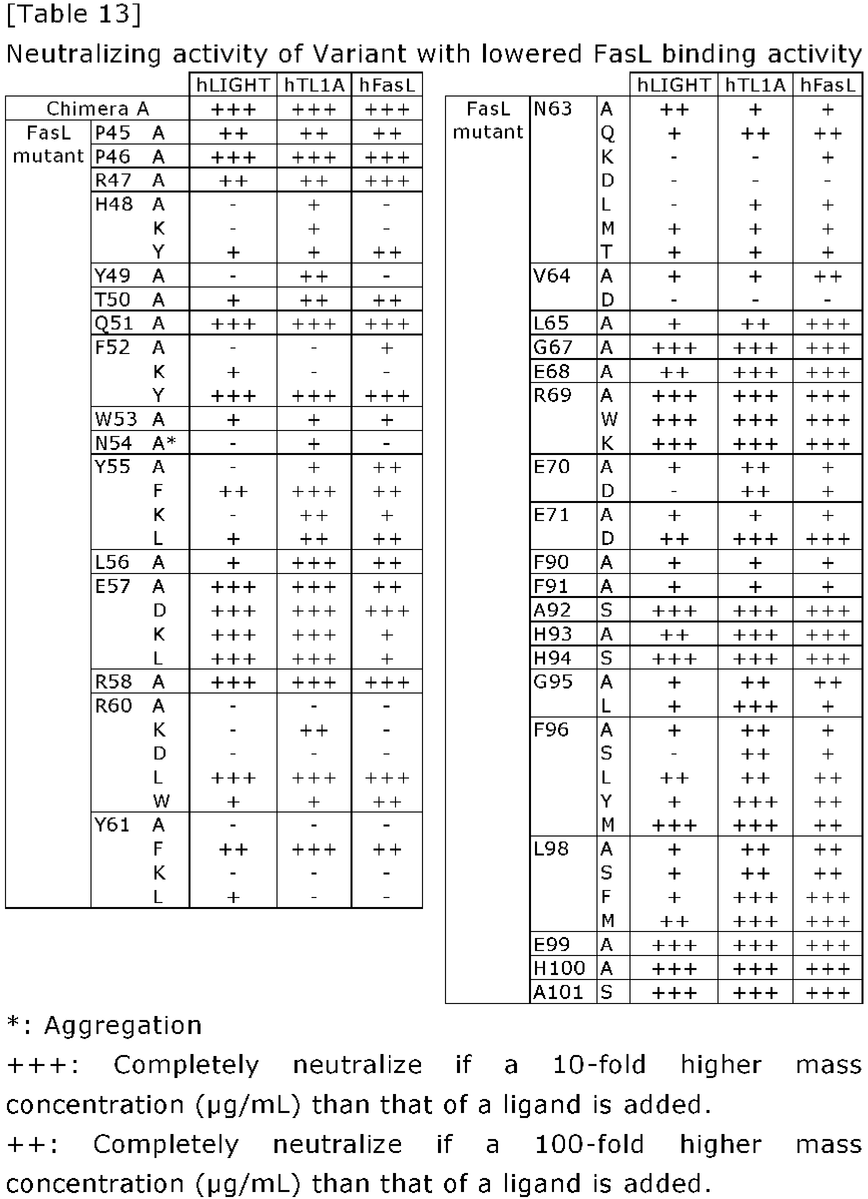

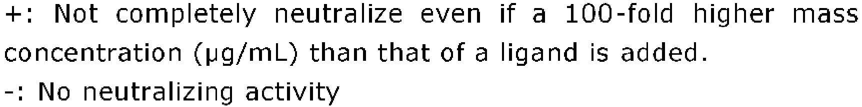

### [Example 11] Evaluation of Aggregability of Commercially Available Wild-Type DcR3 in Mammalian Cell

By the same method as in Example 1, commercially available full-length DcR3-Fc (Abcam plc.) produced using HEK293 cells as host cells, commercially available full-length DcR3-Fc (AdipoGen Life Sciences, Inc.) produced using CHO cells as host cells, and a commercially available Fc fusion (Enzo Life Sciences, Inc.) of a DcR3 molecule lacking about half of the C-terminal side of HBD produced using HEK293 cells as host cells were electrophoresed under reducing conditions and non-reducing conditions. The molecular weights of monomers expected from the mobilities under reducing conditions were about 50 kDa to 60 kDa, while the mobilities under non-reducing conditions were greatly higher than the molecular weights of dimers expected, and all of the commercially available products mostly existed as aggregates (FIG. 13).

### [Example 12] Production of Variant with lowered FasL binding activity

In order to select variants that bind to TL1A and LIGHT among DcR3 ligands and have the selectively-lowered binding activity to FasL, one-amino-acid substitutes (E57X; X is any amino acid other than Glu) were produced by introducing substitution of Glu at position 57 (E57) from the N-terminus with an amino acid other than Glu into chimera A-Fc (g4PEK) (SEQ ID NO: 82),. Regarding E57K, E57L, E57R and E57V (which correspond to substitution of E57 with Lys, Leu, Arg and Val, respectively), Fc (g4PEK) fusions each including a two-amino-acid substitute (E57X_W53Z, E57X_N54Z, E57X_Y55Z, E57X_L56Z, E57X_R58Z; a variant number shown in FIG. 14A is imparted to each two-amino-acid substitute) were produced by introducing in addition to one of E57K, E57L, E57R and E57V, one of further substitution of Trp at position 53 (W53) from the N-terminus, Asn at position 54 (N54) from the N-terminus, Tyr at position 55 (Y55) from the N-terminus, Leu at position 56 (L56) from the N-terminus, and Arg at position 58 (R58) from the N-terminus, which are amino acids in the vicinity of E57K, E57L, E57R and E57V, with a certain amino acid Z (Z is selected from Asp, Glu, Asn, Gln, Pro, Thr and Gly) into chimera A-Fc (g4PEK) (SEQ ID NO: 82).

Of these, regarding chimera A-E57K (amino acid sequence: SEQ ID NO: 66, nucleotide sequence of DNA: SEQ ID NO: 65), chimera A-E57R (amino acid sequence: SEQ ID NO: 180, nucleotide sequence of DNA: SEQ ID NO: 179), chimera A-E57V (amino acid sequence: SEQ ID NO: 182, nucleotide sequence of DNA: SEQ ID NO: 181), chimera A-E57K_R58D (variant number: 45-10, amino acid sequence: SEQ ID NO: 184, nucleotide sequence of DNA: SEQ ID NO: 183), chimera A-E57K_R58E (variant number: 45-18, amino acid sequence: SEQ ID NO: 186, nucleotide sequence of DNA: SEQ ID NO: 185) and chimera A, fusions with some of various mutation-introduced Fc sequences shown in Table 14 were produced (nucleotide sequence: SEQ ID NO: 213, 217, 219, 221, 223, 227, 231, 233, 235, 237, 255, 259, 261, 263, 265, 149, 167, 169, 171, 173, 175, 177, amino acid sequence: SEQ ID NO: 214, 218, 220, 222, 224, 228, 232, 234, 236, 238, 256, 260, 262, 264, 266, 317, 319, 320, 321, 322, 324, 326, 327, 328, 329, 331, 333, 334, 335, 336, 150, 168, 170, 172, 174, 176, 178). In Table 14, E216 represents Glu at EU-index position 216 of a human IgG1 heavy chain. As mutation-introduced sites, C220S, M252Y, S254T, T256E, N434A, L234A, L235A and G237A represent substitution of Cys at EU-index position 220 of a human IgG1 heavy chain with Ser, substitution of Met at EU-index position 252 of the human IgG1 heavy chain with Tyr, substitution of Ser at EU-index position 254 of the human IgG1 heavy chain with Thr, substitution of Thr at EU-index position 256 of the human IgG1 heavy chain with Glu, substitution of Asn at EU-index position 434 of the human IgG1 heavy chain with Ala, substitution of Leu at EU-index position 234 of the human IgG1 heavy chain with Ala, substitution of Leu at EU-index position 235 of the human IgG1 heavy chain with Ala, and substitution of Gly at EU-index position 237 of the human IgG1 heavy chain with Ala, respectively.

**[Table 14]**

| Fc name | E216 | Mutation introduced site | SEQ ID NO |
|---|---|---|---|
| Eg1S | Include | C220S | 156 |
| Eg1S YTE | Include | C220S, M252Y, S254T, T256E | 158 |
| Eg1S N434A | Include | C220S, N434A | 160 |
| Eg1S LALAGANA | Include | C220S, L234A, L235A, G237A, N434A | 166 |
| Eg1S LALAGA | Include | C220S, L234A, L235A, G237A | 164 |
| g1S | Not include | C220S | 72 |
| g1 YTE | Not include | C220S, M252Y, S254T, T256E | 311 |
| g1S N434A | Not include | C220S, N434A | 312 |
| g1S LALAGANA | Not include | C220S, L234A, L235A, G237A, N434A | 313 |
| g1S LALAGA | Not include | C220S, L234A, L235A, G237A | 162 |

Regarding chimera A-Fc (g1S) (nucleotide sequence: SEQ ID NO: 149, amino acid sequence: SEQ ID NO: 150), a variant obtained by deleting the IEGRMD sequence from chimera A-Fc (IEGRMD g1S) (SEQ ID NO: 80) including a linker sequence IEGRMD (SEQ ID NO: 106), produced in Example 2, was produced by stable expression in CHO cells using the method mentioned in Example 6. Regarding chimera A-Fc (Eg1S) (nucleotide sequence: SEQ ID NO: 167, amino acid sequence: SEQ ID NO: 168), a sequence obtained by linking chimera A (SEQ ID NO: 54) and Eg1S to each other was produced by stable expression in CHO cells by the method mentioned in Example 6.

Regarding each of the chimera A-Fc variants (Eg1S YTE, Eg1S N434A, Eg1S LALAGA, Eg1S LALAGANA) (nucleotide sequence: SEQ ID NO: 169, 171, 175, 177, amino acid sequence: SEQ ID NO: 170, 172, 176, 178), a sequence obtained by fusing each Fc to the C-terminus of chimera A (SEQ ID NO: 54) was obtained by transient expression in CHO-S cells.

Regarding each of the chimera A-Fc variants (g1S YTE, g1S N434A, g1S LALAGA, g1S LALAGANA) (amino acid sequence: SEQ ID NO: 314, 315, 174, 316), a sequence obtained by fusing each Fc to the C-terminus of chimera A (SEQ ID NO: 54) was obtained by transient expression in Expi293 cells.

A plasmid expressing a fusion of each amino acid substitute and Fc (g4PEK) was produced as follows: using a DNA sequence of either chimera A-Fc (g4PEK) or E57X-Fc (g4PEK; X is K, R or V) as a template, and using a PCR primer designed to include a mutation site, two regions of a region from an NheI site to a site into which an amino acid substitution was introduced and a region from the site into which an amino acid substitution was introduced to an SaiI site each were subjected to PCR amplification, and the resultant products were inserted under a CMV promoter of a pCIpuro vector by the same method as in Example 1.

Regarding a plasmid expressing a fusion of each amino acid substitute and each Fc of Eg1S YTE, Eg1S N434A or Eg1S LALAGANA, using a DNA sequence encoding one of chimera A-Fc variants including the respective mutated Fc sequences as a **template,** and using a PCR primer designed to include a mutation site, each of two regions, i.e., a region extending from an EcoRI site to a site into which an amino acid substitution was introduced and a region extending from the site into which an amino acid substitution was introduced to a Bsu36I site was subjected to PCR amplification, and each of the resultant products was inserted into the EcoRI and Bsu36I sites of the chimera A-Fc vector including the mutated Fc sequence used as a template to produce the plasmid.

Regarding a plasmid expressing a fusion of each amino acid substitute and each Fc of g1S YTE, g1S N434A or g1S LALAGANA, using a DNA sequence encoding one of the abovementioned amino acid substitute-Fc variants as a template, and using a PCR primer designed to exclude E216, each of two regions, i.e., a region extending from an EcoRI site to a site of Glu to be deleted and a region extending from the site of Glu to be deleted to a Bsu36I site was subjected to PCR amplification, and each of the resultant products was inserted into the EcoRI and Bsu36I sites of the amino acid substitute-Fc vector used as a template to produce the plasmid.

Each plasmid was introduced into Expi293 cells to transiently express, and affinity purification from the culture supernatant was performed by MabSelect SuRe. Each content of monomers, aggregates and degradants of the chimera A-Fc variants, the one-amino-acid substitute-Fc variants and the two-amino-acid substitute-Fc variants produced was calculated from the peak areas analyzed by SEC-UPLC (ACQUITY UPLC Protein BEH SEC 4.6 mm × 150 mm) (Waters K.K.) or SEC-HPLC (TSKgel SuperSW3000 4.0 µm, 4.6 mm x 300 mm) (Tosoh Corporation) using the same method as in Example 2.

As a result, many of the chimera A-Fc variants, the one-amino-acid substitute-Fc variants and the two-amino-acid substitute-Fc variants produced, regardless of mutated Fc **sequences,** maintained lower aggregate contents than that of S195-Fc (FIG. 14A, B, C).

### [Example 13] Evaluation of Binding Activity to DcR3 Soluble Trimer Ligand

Regarding each of DcR3-Fc, S195-Fc, the various chimera A-Fc variants including different Fc sequences and the various variants with lowered FasL binding activities produced in Example 12, the binding activities to a soluble human LIGHT trimer, a soluble human TL1A trimer and a soluble human FasL trimer were evaluated by partially modifying the method mentioned in Example 7.

### (1) Production of DcR3 Soluble Trimer Ligand

As a human soluble recombinant LIGHT, a sequence (FLAG-LIGHT) obtained by adding a FLAG tag (DYKDDDDK) to the N-terminus and linking an extracellular region (Asp74-Val240) (SEQ ID NO: 132) of LIGHT to downstream thereof was used (nucleotide sequence: SEQ ID NO: 305, amino acid sequence: SEQ ID NO: 306). As a human soluble recombinant TL1A, a sequence (His6-TL1A) obtained by adding a His tag (His6) and a GS linker (GGGSGGGSGGGS) to the N-terminus and linking an extracellular region (Leu72-Leu251) (SEQ ID NO: 138) of TL1A to downstream thereof was used (nucleotide sequence: SEQ ID NO: 115, amino acid sequence: SEQ ID NO: 116). Each plasmid was produced by the same method as in Example 7, and transient expression by Expi293 cells was performed.

FLAG-LIGHT was purified using ANTI-FLAG M2 Affinity Gel (Sigma-Aldrich Co. LLC). The culture supernatant was passed through a column filled with a resin, washed with a washing buffer (50 mM Tris HCl, 150 mM NaCl, pH 7.4), and eluted with an eluent (0.1M glycine hydrochloride, pH 3.5).

His6-TL1A was purified by the following method. The culture supernatant was passed through a column filled with Complete His-Tag Purification Resin (Roche), washed with a washing buffer (50 mM NaH₂PO₄ pH 8.0, 300 mM NaCl), and eluted with an eluent (50 mM NaH₂PO₄ pH 8.0, 300 mM NaCl, 250 mM imidazole). The eluent substituted with PBS using a NAP column (GE Healthcare), passed through a column filled with Ni Sepharose Fast Flow resin (GE Healthcare), washed with a washing buffer (60 mM imidazole, 20 mM sodium phosphate, 0.5 M NaCl, pH 7.4) prepared using His Buffer Kit (GE Healthcare), and eluted with an elution buffer (250 mM imidazole, 20 mM sodium phosphate, 0.5 M NaCl, pH 7.4).

Each elution buffer of elution fractions of FLAG-LIGHT and His6-TL1A was substituted with PBS using a NAP column (GE Healthcare) and passed through a 0.22 µm filter to perform sterilization. The purified proteins thus obtained were subjected to gel filtration chromatography (SEC) (TSKgel G3000 SWXL 7.8 mm x 300 mm) (Tosoh Corporation) using HPLC (Shimadzu Corporation) to collect trimer fractions.

As a human soluble recombinant FasL, Human His6 Fas Ligand/TNFSF6 (Cell Signaling Technology, Inc.) obtained by adding a His tag (His6) to the N-terminus and linking an extracellular region (Pro134-Leu281) (SEQ ID NO: 144) of FasL to downstream thereof was used. SEC-MALS was performed by the same method as in Example 2 to confirm it was a trimer.

### (2) Measurement of Binding Activity Using BIAcore

Regarding both of DcR3-Fc (R&D Systems, Inc.) and S195-Fc, those mentioned in Example 7 were evaluated. Chimera A-g4PEK (SEQ ID NO: 82) was produced in Example 6. Chimera A-Fc (g1S, Eg1S) was produced in Example 12. As various variants with lowered FasL binding activities, the Fc (g4PEK) fusions produced in Example 9 or Example 12 were evaluated.

The binding activities to human DcR3 trimer ligands were analyzed by the SPR method. BIAcore T-100 (GE Healthcare) was used for measurement of the binding activities to human LIGHT and human TL1A, and BIAcore T-100 (GE Healthcare) or BIAcore T-200 (GE Healthcare) was used for measurement of the binding activity to human FasL. As the buffer, HBS-EP+ Buffer was used.

After 10,000 RU of anti-human antibodies were immobilized to Series S Sensor Chip CM5 using Human Antibody Capture Kit (both of which are manufactured by GE Healthcare), various wild-type DcR3 controls and DcR3 variants were injected at the flow rate of 10 µL/min for 30 seconds and captured. Meanwhile, the buffer containing no protein was injected into a reference flow cell. Thereafter, as an analyte, each human DcR3 trimer ligand diluted to 0.02 to 80 nmol/L was injected at 30 µL/min for 2 minutes to monitor the binding, and subsequently the buffer was injected for 3 minutes to monitor dissociation. Then, 3 mol/L magnesium chloride was injected at 30 µL/min for 1 minute to perform a regeneration reaction. For measurement of the binding activities to human LIGHT and human TL1A, BIAcore T-100 evaluation software and 1:1 Binding model were used, and by regarding each ligand as a trimer (human LIGHT trimer: 62.4 kDa, human TL1A trimer: 66.2 kDa), each kinetic constant (kₐ, k_{d}, K_{D}) was calculated. For measurement of the binding activity to human FasL, BIAcore T-100 evaluation software and 1:1 Binding model or BIAcore T-200 evaluation software and 1:1 Binding model were used, and by regarding the ligand as a monomer (human FasL monomer: 19.8 kDa), each kinetic constant (kₐ, k_{d}, K_{D}) was calculated.

As a result, it was confirmed that each of chimera A-Fc (g1S), chimera A-Fc (Eg1S) and chimera A-Fc (g4PEK) binds to each DcR3 trimer ligand (FIG. 15A).

Of the variants (g4PEK) with lowered FasL binding activities produced in Example 12, in each of the one-amino-acid substitutes, i.e., chimera A-E57K-Fc (g4PEK) (amino acid sequence: SEQ ID NO: 94, nucleotide sequence of DNA: SEQ ID NO: 93) (hereinafter sometimes referred to as "E57K-Fc"), chimera A-E57L-Fc (g4PEK) (amino acid sequence: SEQ ID NO: 96, nucleotide sequence of DNA: SEQ ID NO: 95) (hereinafter sometimes referred to as "E57L-Fc"), chimera A-E57R-Fc (g4PEK) (amino acid sequence: SEQ ID NO: 190, nucleotide sequence of DNA: SEQ ID NO: 189) (hereinafter sometimes referred to as "E57R-Fc"), chimera A-E57V-Fc (g4PEK) (amino acid sequence: SEQ ID NO: 192, nucleotide sequence of DNA : SEQ ID NO: 191) (hereinafter sometimes referred to as "E57V-Fc"), chimera A-E57A-Fc (g4PEK) (amino acid sequence: SEQ ID NO: 288, nucleotide sequence of DNA: SEQ ID NO: 287) (hereinafter sometimes referred to as "E57A-Fc"), chimera A-E57F-Fc (g4PEK) (amino acid sequence: SEQ ID NO: 290, nucleotide sequence of DNA: SEQ ID NO: 289) (hereinafter sometimes referred to as "E57F-Fc"), chimera A-E57H-Fc (g4PEK) (amino acid sequence: SEQ ID NO: 292, nucleotide sequence of DNA: SEQ ID NO: 291) (hereinafter sometimes referred to as "E57H-Fc"), chimera A-E57I-Fc (g4PEK) (amino acid sequence: SEQ ID NO: 294, nucleotide sequence of DNA: SEQ ID NO: 293) (hereinafter sometimes referred to as "E57I-Fc"), and chimera A-E57M-Fc (g4PEK) (amino acid sequence: SEQ ID NO: 296, nucleotide sequence of DNA: SEQ ID NO: 295) (hereinafter sometimes referred to as "E57M-Fc"), and in each of the two-amino-acid substitutes, i.e., chimera A-E57K_R58D-Fc (g4PEK) (variant number: 45-10, amino acid sequence: SEQ ID NO: 194, nucleotide sequence of DNA: SEQ ID NO: 193) (hereinafter sometimes referred to as "45-10-Fc"), chimera A-E57K_R58T-Fc (g4PEK) (variant number: 45-11, amino acid sequence: SEQ ID NO: 298, nucleotide sequence of DNA: SEQ ID NO: 297) (hereinafter sometimes referred to as "45-11-Fc"), chimera A-E57K_R58E-Fc (g4PEK) (variant number: 45-18, amino acid sequence: SEQ ID NO: 196, nucleotide sequence of DNA: SEQ ID NO: 195) (hereinafter sometimes referred to as "45-18-Fc"), chimera A-E57L_R58E-Fc (g4PEK) (variant number: 46-4, amino acid sequence: SEQ ID NO: 300, nucleotide sequence of DNA: SEQ ID NO: 299) (hereinafter sometimes referred to as "46-4-Fc"), chimera A-E57R_R58D-Fc (g4PEK) (variant number: 82-5, amino acid sequence: SEQ ID NO: 198, nucleotide sequence of DNA: SEQ ID NO: 197) (hereinafter sometimes referred to as "82-5-Fc"), chimera A-E57V_R58T-Fc (g4PEK) (variant number: 85-6, amino acid sequence: SEQ ID NO: 302, nucleotide sequence of DNA: SEQ ID NO: 301) (hereinafter sometimes referred to as "85-6-Fc"), and chimera A-E57V_R58E-Fc (g4PEK) (variant number: 85-8, amino acid sequence: SEQ ID NO: 304, nucleotide sequence of DNA: SEQ ID NO: 303) (hereinafter sometimes referred to as "85-8-Fc"), it was confirmed that the K_{D} values to LIGHT trimer and TL1A trimer were less than 3-fold and the K_{D} value to FasL trimer was 3-fold or more or the Rmax value was decreased to less than 5, compared to those of chimera A-Fc (g4PEK), and that the binding activity to FasL was selectively lowered (FIG. 16A, B).

Of these, E57K-Fc, E57L-Fc, E57R-Fc, E57V-Fc, 45-10-Fc, 45-18-Fc and 82-5-Fc (each having g4PEK as an Fc) were purified to 95% or more of monomers by SEC-HPLC (column; TSKgel G3000 SWXL 7.8 mm x 300 mm, Tosoh Corporation, HPLC; Shimadzu Corporation) and subjected to BIAcore measurement, and each kinetic constant is shown in FIG. 15B.

### [Example 14] Evaluation of Neutralizing Activity to DcR3 Soluble Ligand

The neutralizing activities of various DcR3 variants to soluble human LIGHT, soluble human TL1A and soluble human FasL were evaluated by partially modifying the method mentioned in Example 9.

### (1) Production of Various Chimera A-Fc variants including Different Fc Sequences

The chimera A-Fc variants (g1S, Eg1S) produced in Example 12 were used. The chimera A-Fc variants produced in Example 12, each including chimera A and an Fc fused to the C-terminal side of chimera A selected from Eg1S YTE, Eg1S N434A, Eg1S LALAGA and Eg1S LALAGANA shown in Table 14, were produced by transient expression in CHO-S cells, and purified to 95% or more of monomers by SEC (Superdex 200 Increase 10/300 GL) (GE Healthcare) using AKTApurifier (GE Healthcare) for use.

### (2) Neutralizing Activity to Soluble Human LIGHT

Regarding each of the chimera A-Fc variants (g1S, Eg1S, Eg1S YTE, Eg1S N434A, Eg1S LALAGA, Eg1S LALAGANA, g4PEK), the inhibitory activity to LIGHT-dependent CXCL10 production from an IFN-γ-stimulated intestinal myofibroblast (Lonza K.K.) was evaluated. The intestinal myofibroblast was cultured in a collagen I-coated flask (Becton, Dickinson and Company) using an SmGM-2 Bullet Kit (Lonza K.K.) medium. After the cells were seeded at 1 x 10⁴ cells/well in a collagen I-coated 96-well plate (Becton, Dickinson and Company), IFN-γ at the final concentration of 10 ng/mL, the trimer FLAG-LIGHT produced in Example 13 at the final concentration of 20 ng/mL, and various DcR3 variants at each final concentration of 19.5, 78.1, 313, 1,250, 5,000, 20,000 ng/mL, or 4.88, 19.5, 78.1, 313, 1,250, 5,000 ng/mL were added, followed by culture for 3 days. The culture supernatants were collected, and the CXCL10 concentrations in the culture supernatants were measured using CXCL10/IP-10 (human) AlphaLisa Detection Kit (PerkinElmer, Inc.). As a result, it was confirmed that each of the chimera A-Fc variants, regardless of an Fc sequence, concentration-dependently inhibits the CXCL10 production and has neutralizing activity to soluble LIGHT (FIG. 17A, B).

Regarding each of the variants with lowered FasL binding activities, i.e., E57K-Fc, E57L-Fc, E57R-Fc and E57V-Fc (each including g4PEK as an Fc), the inhibitory activity to LIGHT-dependent IL-8 production from HT-29 cells was evaluated by the same method as in Example 9. As a result, it was confirmed that each of the variants concentration-dependently inhibits the IL-8 production and has neutralizing activity to soluble LIGHT (FIG. 17C).

Regarding each of the variants with lowered FasL binding activities, i.e., 45-10-Fc, 45-18-Fc and 82-5-Fc (each including g4PEK as an Fc), similarly to chimera A-Fc, the inhibitory activity to LIGHT-dependent CXCL10 production from an IFN-γ-stimulated intestinal myofibroblast was evaluated under the conditions of 2 x 10⁴ of intestinal myofibroblast per well, and final concentration of 19.5, 78.1, 313, 1,250, 5,000 or 20,000 ng/mL of each test substance. As a result, it was confirmed that each of the variants concentration-dependently inhibits the CXCL10 production and has neutralizing activity to soluble LIGHT (FIG. 17D).

### (3) Neutralizing Activity to Soluble Human TL1A

Regarding each of the DcR3 variants, the inhibitory activity to TL1A-dependent IFN-γ production from IL-12- and IL-18-stimulated human T cells was evaluated by the same method as in Example 9. As a result, it was confirmed that, each of the chimera A-Fc variants, regardless of an Fc sequence, concentration-dependently inhibits the production amount of IFN-γ and has neutralizing activity to soluble TL1A (FIG. 18A, B). Furthermore, it was also confirmed that each of the variants with lowered FasL binding activities, i.e., E57K-Fc, E57L-Fc, E57R-Fc, E57V-Fc, 45-10-Fc, 45-18-Fc and 82-5-Fc (each including g4PEK as an Fc) similarly has neutralizing activity to soluble TL1A (FIG. 18C, D).

### (4) Neutralizing Activity to Soluble Human FasL

Regarding each of the DcR3 variants, the inhibitory activity to FasL-dependent cell death of Jurkat cells or A3 cells corresponding to a Jurkat subclone was evaluated by the same method as in Example 9. As a result, it was confirmed that each of the chimera A-Fc variants, regardless of an Fc sequence, concentration-dependently inhibits cell death of A3 cells and has neutralizing activity to soluble FasL (FIG. 19A, B). Meanwhile, regarding each of the variants with lowered FasL binding activities, i.e., E57K-Fc, E57L-Fc, E57R-Fc, E57V-Fc, 45-10-Fc, 45-18-Fc and 82-5-Fc (each including g4PEK as an Fc), it was confirmed that, in each of the variants, the inhibitory activity to Jurkat cell death was remarkably decreased and the neutralizing activity to soluble FasL was selectively lowered (FIG. 19C, D).

### [Example 15] Evaluation of Binding Activity to Membrane-bound DcR3 Ligand

The binding activities of various DcR3 variants to membrane-bound human LIGHT, membrane-bound human TL1A and membrane-bound human FasL were evaluated by flow cytometry using membrane-bound ligand forced expression cell lines. As the membrane-bound human LIGHT forced expression HEK293 cells, those mentioned in US 8974787 were used. Regarding each of membrane-bound TL1A and membrane-bound FasL, using a commercially available ORF clone (Origene Technologies, Inc.) thereof as a template, a sequence obtained by adding a Met residue and a FLAG tag (DYKDDDDK) to the N-terminus was subjected to PCR amplification (nucleotide sequence: SEQ ID NO: 307, 309, amino acid sequence: SEQ ID NO: 308, 310), and the resultant product was inserted into downstream of a CMV promoter of a pCIpuro vector using InFusion HD Cloning Kit (Clontech Laboratories, Inc.), and *Escherichia coli* DH5α competent cells (TOYOBO CO., LTD.) were transformed.

The plasmids thus obtained were introduced into CHO-K1 cells (ECACC) using Nucleofector and Cell Line Nucleofector Kit T (both of which are manufactured by Lonza K.K.), and subjected to drug selection by 10 µg/mL of Puromycin (Thermo Fisher Scientific, Inc.). The drug-resistant cells thus obtained were stained with a DyLight488-labeled anti-human TL1A antibody (Novus Biologicals, LLC) or an APC-labeled anti-human FasL antibody (BD Pharmingen), and high expression fractions were sorted using a cell sorter (Sony Corporation). After expansion culture, the cells were stained with a PE-labeled anti-human TL1A antibody (Novus Biologicals, LLC) or a PE-labeled anti-human FasL antibody (BioLegend, Inc.), followed by sorting again, thus obtaining a membrane-bound human TL1A or membrane-bound human FasL high expression cell line.

The binding activities of various DcR3 variants to membrane-bound ligand forced expression cell lines and host cells were evaluated using the same method as in Example 5 by changing the following conditions. Regarding HEK293 and a membrane-bound LIGHT forced expression cell line, 1 µg/mL of each protein and 10 ng/mL of a secondary antibody Goat F(ab')₂ Anti-Human IgG R-phycoerythrin Conjugate (Southern Biotechnology Associates, Inc.) were reacted. Regarding membrane-bound TL1A and a membrane-bound FasL forced expression cell line, 1 or 10 µg/mL of each protein and 0.1 or 1 µg/mL of the secondary antibody each were reacted.

As a result, each of the chimera A-Fc variants (g1S, Eg1S, g4PEK) did not react with 293 or CHO-K1 cells used as host cells, but reacted specifically with each cell of membrane-bound ligand forced expression cell line. Therefore, it was confirmed that each of the various chimera A variants including different Fc sequences has binding activities to membrane-bound DcR3 ligands (FIG. 20A, B). Furthermore, regarding each of the variants with lowered FasL binding activities, i.e., E57K-Fc, E57L-Fc, E57R-Fc, E57V-Fc, 45-10-Fc, 45-18-Fc and 82-5-Fc (each including g4PEK as an Fc), the binding activities to ligands were similarly evaluated, and as a result, it was confirmed that the binding activities to membrane-bound human LIGHT and membrane-bound human TL1A were maintained, while the binding activity to membrane-bound human FasL was remarkably decreased except for E57L (FIG. 21A, B, C).

### [Example 16] Evaluation of Binding Activity to DcR3 Primary Ligand

The binding activities of various DcR3 variants to each DcR3 ligand derived from a primary cell were evaluated by the following method.

### (1) Binding Activity to Primary LIGHT

It has been known that expression of membrane-bound LIGHT is induced in activated human T cells (The Journal of Immunology, 2004, 173: p.502-507.). Frozen healthy individual PBMCs (AllCells, LLC) were thawed, and stimulated with PMA (Sigma-Aldrich Co. LLC) at a final concentration of 50 ng/mL and ionomycin (Sigma-Aldrich Co. LLC) at 1 µg/mL overnight, and then the binding of chimera A-Fc (Eg1S) to membrane-bound LIGHT, expression of which was induced in CD3-positive T cells, was evaluated by the following method. As a negative control, the anti-DNP antibody (IgG1) mentioned in Example 8 was used. The stimulated PBMCs were collected and reacted using Human FcR Blocking Reagent (Miltenyi Biotec B.V. & Co. KG), and then a BV421-labeled CD3 antibody (BD Pharmingen), 7-AAD Staining Solution (BD Pharmingen) and chimera A-Fc or anti-DNP antibody at a final concentration of 0.4 µg/mL labeled with Alexa Fluor488 Antibody Labeling Kit (Thermo Scientific) each were added. After reaction, washing was performed, and the fluorescence intensities of Alexa Fluor488 in CD3-positive T cells of viable cell fractions were analyzed with a flow cytometer. For confirmation of expression of membrane-bound LIGHT in the PBMCs stimulated for induction, the PBMCs stimulated for induction were reacted using Human FcR Blocking Reagent (Miltenyi Biotec B.V. & Co. KG), and then a BV510-labeled CD3 antibody (BioLegend, Inc.), 7-AAD Staining Solution (BD Pharmingen) and a PE-labeled LIGHT antibody (LifeSpan BioSciences, Inc.) or a PE-labeled mouse IgG1κ isotype control antibody (BioLegend, Inc.) each were added. After reaction, washing was performed, and the fluorescence intensities of PE in CD3-positive T cells of viable cell fractions were analyzed with a flow cytometer.

As a result, it was confirmed that expression of LIGHT is induced on CD3-positive human T cells in PBMCs stimulated for induction, and that Alexa Fluor488-labeled chimera A-Fc binds to membrane-bound LIGHT on activated CD3-positive human T cells (FIG. 22A, B).

### (2) Binding Activity to Primary TL1A

HUVECs (Lonza K.K.) cultured by the same method as in Example 5 were treated with Recombinant Human IL-1 alpha (R&D Systems, Inc.) at a final concentration of 10 ng/mL and a TACE inhibitor TAPI-1 (Calbiochem) at a final concentration of 20 µM for 24 hours, and the binding of chimera A-Fc (IEGRMD g1S) to membrane-bound TL1A, expression of which was induced, was evaluated by a competition experiment with a TL1A antibody 1D1 1.31 (US2015/0132311). As the TL1A antibody 1D1 1.31, an antibody obtained by linking the amino acid sequences of VL and VH mentioned in US 2015/0132311 to a constant region of human IgG1, followed by transient expression using Expi293 cells and purification using Mabselect SuRe (GE Healthcare) from the culture supernatant by the method mentioned in Example 1, was used. As a negative control, the anti-DNP antibody (IgG1) mentioned in Example 8 was used. The collected cells were reacted with Human FcR Blocking Reagent (Miltenyi Biotec B.V. & Co. KG), and then either anti-DNP antibody, chimera A-Fc or TL1A antibody labeled with Zenon Alexa Fluor 647 Human IgG Labeling Kit (Molecular Probes) was added at the final concentration of 8.3 µg/mL. After reaction, washing was performed, and the fluorescence intensities of Alexa Fluor 647 were analyzed with a flow cytometer. Under the competition conditions, either unlabeled DNP antibody, TL1A antibody or chimera A-Fc at a final concentration of 50 µg/mL was reacted in advance.

As a result, the binding of the labeled chimera A-Fc competed with the unlabeled TL1A antibody and the binding of the labeled TL1A antibody competed with the unlabeled chimera A-Fc, and thus it was confirmed that chimera A binds to membrane-bound TL1A on the stimulated HUVEC (FIG. 23).

### (3) Binding Activity to Primary FasL

The binding of chimera A-Fc (IEGRMD g1S) to primary soluble FasL produced from human T cells stimulated for activation-induced cell death (AICD) was evaluated by the following method. The primary soluble FasL was prepared by the following method. In other words, human T cells isolated from frozen healthy individual PBMCs by the same method as in Example 9 were seeded at 2 x 10⁴ cells/well in a 96-well U-bottom plate (Becton, Dickinson and Company), and cultured with PHA-L (eBioscience, Inc.) at a final concentration of 1 µg/mL for 24 hours, and then IL-2 (Peprotech, Inc.) at a final concentration of 1 µg/mL was added to culture for 5 days. After 5 days, the cells were collected and seeded in a 96-well U-bottom plate to which 5 µg/mL of anti-CD3 antibody OKT3 (BioLegend, Inc.) was immobilized. AICD was induced and culture was performed overnight, and then the culture supernatant was collected, and using Amicon Ultra-15 having a molecular weight cutoff of 10 kDa (Merck Millipore) prerinsed with sterile water, the solution volume was concentrated to one-tenth.

A 96-well immunoplate (Thermo Scientific), to which 10 µg/mL of anti-human IgG antibodies (American Qualex International, Inc.) were immobilized, was blocked with 1% Block Ace (DS Pharma Biomedical Co., Ltd.), and then 20 µg/mL of chimera A-Fc (IEGRMD g1S) or Fas-Fc (R&D Systems, Inc.) was captured. After washing, recombinant FasL (Abcam plc) used as a standard or a 10-fold concentrated AICD culture supernatant was reacted. After washing, a biotinylated anti-FasL antibody (Abcam plc) was reacted. After washing, streptavidin-HRP (PIERCE) was reacted. After washing again, a TMB solution (Abcam plc) was added to develop a color. Then, a 2 N sulfuric acid solution was used to stop the chromogenic reaction, and the absorbance at 450 nm was measured.

The results for the plate on which chimera A-Fc (IEGRMD g1S) was captured are shown in FIG. 24A, and the results for the plate on which Fas-Fc was captured are shown in FIG. 24B. "CD3" for 10 × AICD supernatant refers to use of the culture supernatant of the T cells stimulated for AICD under the anti-CD3 antibody OKT3 stimulation conditions, and "none" for 10 × AICD supernatant refers to use of the culture supernatant of the T cells not stimulated for AICD. Also regarding the plate on which any of chimera A-Fc or Fas-Fc was captured, FasL was detected only in the culture supernatant of the T cells stimulated for AICD. The results confirmed that chimera A-Fc binds to soluble FasL produced from the T cells stimulated for AICD (FIG. 24A, B).

### [Example 17] Evaluation of Physical Property of DcR3 Variant

Regarding various chimera A-Fc variants (g1S, Eg1S, Eg1S YTE, Eg1S N434A, Eg1S LALAGANA, Eg1S LALAGA, g4PEK) and variants with lowered FasL binding activities, i.e., E57K-Fc, E57L-Fc, E57R-Fc, E57V-Fc, 45-10-Fc, 45-18-Fc and 82-5-Fc (each having g4PEK as an Fc), using the same method as in Example 4, the elution times (min) were calculated by hydrophobic interaction chromatography (HIC) and the Tm values (°C) were calculated by the differential scanning fluorimetry (DSF) method.

As a result, it was confirmed that introduction of multiple amino acid mutations into an IgG1 Fc sequence does not remarkably affect the hydrophobicity. Since the elution time of any of variants with lowered FasL binding activities did not remarkably differ from that of chimera A-g4PEK, it was confirmed that one-amino-acid or two-amino-acid substitution does not affect the hydrophobicity (FIG. 25).

Regarding the Tm value by DSF, of amino acid substitution or insertion in an IgG1 Fc sequence, introduction of a YTE mutation affected the thermostability. Since the Tm value for any of variants with lowered FasL binding activities did not remarkably differ from that of chimera A-g4PEK, it was confirmed that introduction of one-amino-acid or two-amino-acid substitution into a part of CRDs of DcR3 does not affect the thermostability (FIG. 26).

### [Example 18] Evaluation of Mouse In Vivo Kinetics of DcR3 Variant

Regarding chimera A-Fc (Eg1S) and the variants with lowered FasL binding activities, i.e., E57K-Fc, E57L-Fc, E57R-Fc, E57V-Fc, 45-10-Fc, 45-18-Fc and 82-5-Fc (each having g4PEK as an Fc), *in vivo* kinetics in mice were evaluated by the same method as the method mentioned in Example 6. Chimera A-Fc (Eg1S) was produced by stable expression in CHO-K1 cells and each of the variants with lowered FasL binding activities was produced by transient expression in CHO-S cells, and purified to 95% or more of monomers by SEC (Superdex 200 Increase 10/300 GL) (GE Healthcare) using AKTApurifier (GE Healthcare) for use.

When single i.v. administration of 10 mg/kg of each DcR3 variant was performed to 5- to 6-week-old BALB/c mice (female) (n = 2 or 3), the half-life in blood (h) during the elimination phase and the area under the concentration-time curve to infinity AUC0-∞ (µg*h·mL) after single administration were calculated (FIG. 27). As a standard substance in measurement of the concentration of each DcR3 variant in serum of a mouse to which E57K-Fc, E57R-Fc or E57V-Fc was administered, each DcR3 variant produced by transient expression in Expi293 cells was used.

As a result, each of the DcR3 variants showed greater improvement in AUC as compared to S195-Fc (IEGRMD g1S) corresponding wild-type DcR3 mentioned in Example 6.

### [Example 19] Evaluation of In Vivo Drug Efficacy of DcR3 Variant

*In vivo* drug efficacy of chimera A-Fc (g4PEK) was evaluated in a mouse acute xenogeneic graft versus host disease (GVHD) model.

### (1) Production of Mouse Acute Xenogeneic GVHD Model

A mouse acute xenogeneic GVHD model was produced by the same method as mentioned in JP 5209625 B2. Using 6-week-old severe combined immunodeficient (SCID) female mice, on days -2 and -5, 100 µg of rat anti-mouse IL2 receptor-β (IL2Rβ) chain antibody TMβI (Bio X Cell, Inc.) was intraperitoneally injected to deplete endogenous mouse natural killer cells. On day -1, using a CellRad X-ray irradiator (Faxitron), irradiation treatment with a sublethal dose of 1.7 Gy was performed to the mice. On day 0, 3 x 10⁶ human PBMCs (AllCells, LLC) were intraperitoneally transferred, and subsequently 300 µg of chimera A-Fc or a DNP antibody (both are g4PEK) prepared in 100 µL of PBS was intraperitoneally injected. Regarding the chimera A-Fc administration group, 300 µg of chimera A-Fc was additionally administered on days 4 and 8. After 12 days, gross pathological scores by GVHD reaction were determined, and in addition, spleen was collected from sacrificed mice, and the human cell count in the spleen was evaluated.

### (2) Evaluation of GVHD Pathological Score

The gross pathological conditions observed on day 12 were scored based on four indices of fur, erythema in the intestinal tract, activity and weight loss (FIG. 28A). Regarding indices, none, mild and severe were scored as 0, 1 and 2, respectively, and by summing the scores of all indices, the GVHD pathological scores were determined.

As a result, an increase in the GVHD pathological scores by transfer of human PBMCs and a decrease in the pathological scores by administration of chimera A-Fc as compared to the DNP antibody administration group were confirmed, and drug efficacy by chimera A-Fc was observed (FIG. 28B).

### (3) Measurement of Human Cell Count in Spleen

Mouse spleen was collected, and using gentleMACS Dissociators (Miltenyi Biotec B.V. & Co. KG), the spleen was homogenized to prepare a splenic cell suspension. After a hemolysis treatment with Lysing buffer (BD Biosciences), staining was performed with a PE/Cy7-labeled anti-human CD45 antibody, an FITC-labeled anti-human CD3 antibody, an APC-labeled anti-human CD4 antibody and a PE-labeled anti-human CD8 antibody (all of which are manufactured by BioLegend, Inc.), and each human cell subset was analyzed by a flow cytometry analysis. For measurement of the cell count, CountBright Absolute Counting Beads, for flow cytometry (Thermo Fisher Scientific, Inc.) was used, and the abundance ratio of the fluorescent bead count and each cell subset count detected by flow cytometry was corrected by a known amount of beads added, thus calculating the total cell count in the spleen.

As a result, by transfer of human PBMCs, human cells were detected in mouse spleen, and it was confirmed that the human cell count in mouse spleen is decreased by administration of chimera A-Fc as compared to the DNP antibody administration group (FIG. 29).

### [Example 20] Evaluation of Binding Activity to Soluble DcR3 Ligand Trimer

Regarding each of the chimera A-Fc variants and the variants with lowered FasL binding activities including various mutated Fc sequences produced in Example 12, the binding activities to a soluble human LIGHT trimer, a soluble cynomolgus monkey LIGHT trimer, a soluble human TL1A trimer, a soluble cynomolgus monkey TL1A trimer, a soluble human FasL trimer or a soluble cynomolgus monkey FasL trimer were evaluated as follows by partially modifying the methods mentioned in Examples 7 and 13.

### (1) Production of Soluble DcR3 Ligand Trimer

As human soluble DcR3 ligand trimers, those produced in Example 13 were used. As a cynomolgus monkey soluble recombinant LIGHT, a sequence (FLAG-cynoLIGHT) obtained by adding a FLAG tag (DYKDDDDK) to the N-terminus and linking an extracellular region (Asp74-Val240) (SEQ ID NO: 134) of cynomolgus monkey LIGHT to downstream thereof was used (nucleotide sequence: SEQ ID NO: 341, amino acid sequence: SEQ ID NO: 342). As a cynomolgus monkey soluble recombinant TL1A (His6-cynoTL1A) and a cynomolgus monkey soluble FasL (His6-cynoFasL) (amino acid sequence is SEQ ID NO: 122 and 124, respectively), the same sequences as in Example 7 were used. For LIGHT and TL1A, transient expression by Expi293 was performed, and for FasL, transient expression by CHO-S was performed.

FLAG-cynoLIGHT and His6-cynoTL1A were purified by the method mentioned in Example 13. Regarding His6-cynoFasL, the culture supernatant was passed through a column filled with Ni Sepharose Fast Flow resin (GE Healthcare), washed with a washing buffer (60 mM imidazole, 20 mM sodium phosphate, 0.5 M NaCl, pH 7.4) prepared using His Buffer Kit (GE Healthcare), and eluted with an elution buffer (250 mM imidazole, 20 mM sodium phosphate, 0.5 M NaCl, pH 7.4), and the buffer was substituted with PBS.

Purified FLAG-cynoLIGHT and His6-cynoTL1A were subjected to gel filtration chromatography (SEC) (Superdex 200 Increase 10/300 GL) (GE Healthcare) using AKTApurifier (GE Healthcare) to collect trimer fractions. Purified His6-cynoFasL was analyzed by SEC-UHPLC (apparatus: Nexera X2 (Shimadzu Corporation), column: ACQUITY UPLC Protein BEH SEC 200Å, 1.7 µm, 4.6 × 150 mm (Waters K.K.)) to confirm that 85% or more of purified His6-cynoFasL was trimers.

### (2) Measurement of Binding Activity Using BIAcore

As chimera A-Fc variants and the variants with lowered FasL binding activities including various mutated Fc sequences, the variants mentioned in Example 12 were purified to 95% or more of monomers by SEC (Superdex 200 Increase 10/300 GL) (GE Healthcare) using AKTApurifier (GE Healthcare) or SEC (HiLoad 26/600 Superdex 200 pg) (GE Healthcare) using AKTA pure 25 (GE Healthcare) for use.

The binding activity to human and cynomolgus monkey DcR3 ligand trimers were analyzed by the SPR method. BIAcore T-100 (GE Healthcare) was used for measurement of the binding activities to human LIGHT, cynomolgus monkey LIGHT, human TL1A and cynomolgus monkey TL1A, and BIAcore T-200 (GE Healthcare) was used for measurement of the binding activities to human FasL and cynomolgus monkey FasL. As the buffer, HBS-EP+ Buffer was used.

Various DcR3 variants were injected into Series S Sensor Chip Protein A (GE Healthcare) at 10 µL/min for 30 seconds and captured. Meanwhile, the buffer containing no protein was injected into a reference flow cell. Thereafter, as an analyte, each human DcR3 ligand trimer or each cynomolgus monkey DcR3 ligand trimer diluted to 0.08 to 20 nmol/L was injected. A human LIGHT trimer, a human TL1A trimer or each cynomolgus monkey DcR3 ligand trimer was injected at the flow rate of 30 µL/min for 2 minutes to monitor the binding, and subsequently the buffer was injected for 5 minutes to monitor dissociation. A human FasL trimer was injected at 30 µL/min for 1 minute to monitor the binding, and subsequently the buffer was injected for 3 minutes to monitor dissociation. Then, 3 mol/L magnesium chloride was injected at 30 µL/min for 1 minute to perform a regeneration reaction. For measurement of the binding activities to human LIGHT, cynomolgus monkey LIGHT, human TL1A and cynomolgus monkey TL1A, BIAcore T-100 evaluation software and 1:1 Binding model were used, and for measurement of the binding activities to human FasL and cynomolgus monkey FasL, BIAcore T-200 evaluation software and 1:1 Binding model were used, and by regarding each DcR3 ligand as a trimer (human LIGHT trimer: 62.4 kDa, cynomolgus monkey LIGHT trimer: 57.9 kDa, human TL1A trimer: 66.2 kDa, cynomolgus monkey TL1A trimer: 66.1 kDa, human FasL trimer: 53.1 kDa, cynomolgus monkey FasL trimer: 53.1 kDa), each kinetic constant (kₐ, k_{d}, K_{D}) was calculated.

As a result, it was confirmed that each of chimera A-Fc variants including various mutated Fc sequences (Eg1S, Eg1S YTE, Eg1S N434A, Eg1S LALAGA, Eg1S LALAGANA) (each amino acid sequence: SEQ ID NO: 168, 170, 172, 176, 178) binds to each DcR3 trimer ligand of a human and a cynomolgus monkey (FIG. 30A, B).

Each of the variants with lowered FasL binding activities including various mutated Fc sequences, i.e., E57K-Fc, E57R-Fc, E57V-Fc, 45-10-Fc and 45-18-Fc (each including Eg1S YTE, Eg1S N434A or Eg1S LALAGANA as an Fc) showed no remarkable difference in the K_{D} values to a human LIGHT trimer and a human TL1A trimer compared to those of chimera A-Eg1S. However, in each of these variants with lowered FasL binding activities, it was confirmed that the K_{D} value to a human FasL trimer is greatly increased compared to that of chimera A-Eg1S, and that the binding activity to human FasL is selectively lowered (FIG. 30A).

In each of the variants with lowered FasL binding activities including various mutated Fc sequences, the K_{D} value to a cynomolgus monkey LIGHT trimer or a cynomolgus monkey TL1A trimer was similar to that of chimera A-Eg1S, but in E57R-Fc (Eg1S YTE, Eg1S N434A), the binding activity to a cynomolgus monkey TL1A trimer tended to be attenuated. Meanwhile, in each of the variants with lowered FasL binding activities, it was confirmed that the K_{D} value to a cynomolgus monkey FasL trimer is greatly increased compared to that of chimera A-Eg1S, and that the binding activity to FasL is selectively lowered (FIG. 30B).

### [Example 21] Evaluation of Neutralizing Activity to DcR3 Soluble Ligand

Regarding each of the variants with lowered FasL binding activities including various mutated Fc sequences, evaluation of the neutralizing activities to soluble human LIGHT, soluble human TL1A and soluble human FasL was performed by the same method as in Example 9 or 14. As various DcR3 variants, chimera A-Fc variants produced in Example 12 or the variants with lowered FasL binding activities produced by transient expression in CHO-S cells, i.e., E57K-Fc variants and 45-18-Fc variants (each including Eg1S YTE or Eg1S LALAGANA as an Fc) were purified to 95% or more of monomers by the method mentioned in Example 14(1) or SEC (HiLoad 16/600 Superdex 200 pg) (GE Healthcare) using AKTApurifier (GE Healthcare) for use.

### (1) Neutralizing Activity to Soluble Human LIGHT

Regarding each of various DcR3 variants, the inhibitory activity to LIGHT-dependent CXCL10 production from an IFN-γ-stimulated intestinal myofibroblast was evaluated by the method mentioned in Example 14. As a result, it was confirmed that each of chimera A-Fc variants, i.e., E57K-Fc variants and 45-18-Fc variants (each including Eg1S YTE or Eg1S LALAGANA as an Fc) concentration-dependently inhibits the CXCL10 production and has neutralizing activity to soluble LIGHT. The results are shown in FIG. 31.

### (2) Neutralizing Activity to Soluble Human TL1A

Regarding each of various DcR3 variants, the inhibitory activity to TL1A-dependent IFN-γ production from IL-12- and IL-18-stimulated human T cells was evaluated by the method mentioned in Example 9. As a result, it was confirmed that each of chimera A-Fc variants, E57K-Fc variants and 45-18-Fc variants (each including Eg1S YTE or Eg1S LALAGANA as an Fc) concentration-dependently inhibits the IFN-γ production and has neutralizing activity to soluble TL1A. The results are shown in FIG. 32.

### (3) Neutralizing Activity to Soluble Human FasL

Regarding each of various DcR3 variants, the inhibitory activity to FasL-dependent cell death of Jurkat cells was evaluated by the method mentioned in Example 9. As a result, it was confirmed that each of chimera A-Fc variants (Eg1S YTE, Eg1S LALAGANA) concentration-dependently inhibits cell death of Jurkat cells and has neutralizing activity to soluble FasL. Meanwhile, regarding E57K-Fc variants and 45-18-Fc variants (each including Eg1S YTE or Eg1S LALAGANA as an Fc), it was confirmed that, in each of the variants, the inhibitory activity to Jurkat cell death is remarkably decreased and the neutralizing activity to soluble FasL is selectively lowered. The results are shown in FIG. 33.

In FIGS. 31 to 33, the solid line and X (-×-) represents an anti-DNP antibody as a negative control; the solid line and solid circle **(-●-)** represents chimera A-Fc (Eg1S YTE); the solid line and open triangle (-△-) represents E57K-Fc (Eg1S YTE): the solid line and open circle (-∘-) represents 45-18-Fc (Eg1S YTE); the dotted line and solid circle (--●--) represents chimera A-Fc (Eg1S LALAGANA); the dotted line and open triangle (--△--) represents E57K-Fc (Eg1S LALAGANA); and the dotted line and open circle (--∘--) represents 45-18-Fc (Eg1S LALAGANA).

### [Example 22] Evaluation of Binding Activity to Membrane-bound DcR3 Ligand

Regarding each of the chimera A variants and the variants with lowered FasL binding activities including various mutated Fc sequences, the binding activities to membrane-bound human LIGHT, membrane-bound human TL1A and membrane-bound human FasL were evaluated by the same method as in Example 15.

Chimera A-Fc (Eg1S) was produced by cells of a stable expression cell line from CHO cells, and purified to 95% or more of monomers by SEC for use. All of chimera A-Fc variants (Eg1S YTE, Eg1S N434A, Eg1S LALAGA, Eg1S LALAGANA) were produced by transient expression in CHO-S cells, and purified to 95% or more of monomers by SEC for use. All of the variants with lowered FasL binding activities, i.e., E57K-Fc, E57R-Fc, E57V-Fc, 45-10-Fc and 45-18-Fc (each including Eg1S YTE or Eg1S LALAGANA as an Fc) were produced by transient expression in Expi293F cells, and purified to 95% or more of monomers by SEC for use. In all cases, SEC was performed by the same method as in Example 21.

With the cell lines each forcedly expressing a membrane molecule of human LIGHT, human TL1A and human FasL (also referred to as LIGHT/HEK293, TL1A/CHO and FasL/CHO, respectively) mentioned in Example 15, and HEK293 and CHO-K1 cells, which were used host cells thereof, the abovementioned samples were reacted at a final concentration of 10 µg/mL, and a secondary antibody Goat F(ab')2 Anti-Human IgG R-phycoerythrin Conjugate (Southern Biotechnology Associates, Inc.) was reacted at a final concentration of 100 ng/mL, and the fluorescence intensity of PE was analyzed with a flow cytometer. The results are shown in FIGS. 34A, 34B and 34C. In FIG. 34A, each of the solid bars represents reactivity to LIGHT/HEK293, and each of the open bars represents reactivity to an HEK293 cell. In FIG. 34B, each of the solid bars represents reactivity to TL1A/CHO, and each of the open bars represents reactivity to a CHO-K1 cell. In FIG. 34C, each of the solid bars represents reactivity to FasL/CHO, and each of the open bars represents reactivity to a CHO-K1 cell.

As a result, as shown in FIGs. 34A and 34B, it was confirmed that each of chimera A-Fc variants (Eg1S, Eg1S YTE, Eg1S N434A, Eg1S LALAGA, Eg1S LALAGANA), E57K-Fc, E57R-Fc, E57V-Fc, 45-10-Fc and 45-18-Fc (each including Eg1S YTE or Eg1S LALAGANA as an Fc) binds to membrane-bound human LIGHT and membrane-bound TL1A. Meanwhile, as shown in FIG. 34C, it was confirmed that each of the variants with lowered FasL binding activities shows remarkably decreased binding activity to membrane-bound FasL as compared to chimera A variants including various mutated Fc sequences.

### [Example 23] Evaluation of Neutralizing Activity to Membrane-bound DcR3 Ligand

Regarding each of chimera A variants and the variants with lowered FasL binding activities including various mutated Fc sequences, the neutralizing activities to membrane-bound human LIGHT, membrane-bound human TL1A and membrane-bound human FasL were evaluated by the method shown below. All of chimera A-Fc, E57K-Fc and 45-18-Fc (each including Eg1S YTE or Eg1S LALAGANA as an Fc) were produced by transient expression in CHO-S, and, similarly to Example 21, purified to 95% or more of monomers by SEC for use.

### (1) Neutralizing Activity to Membrane-bound Human LIGHT

Regarding each of chimera A-Fc, E57K-Fc and 45-18-Fc (each including Eg1S YTE or Eg1S LALAGANA as an Fc), the neutralizing activity to membrane-bound human LIGHT was evaluated using a human intestinal myofibroblast (Lonza K.K.) and using CXCL10 production by co-culture with the cells of the membrane-bound LIGHT forced expression HEK293 cell line (LIGHT/HEK293) used in Example 22 as an index.

Human intestinal myofibroblasts were seeded at 1 × 10⁴ cells/well in a Collagen I coat 96 well plate (Corning Incorporated), and cultured at 37°C overnight. After fixation with Fixation Buffer (BD Biosciences), 1 × 10⁴ cells/well of LIGHT/HEK293s washed with PBS and a medium, recombinant human IFN-γ (FUJIFILM Wako Pure Chemical Corporation) at the final concentration of 10 ng/mL, chimera A-Fc, E57K-Fc and 45-18-Fc (in all of which Fc is Eg1S YTE or Eg1S LALAGANA) at the final concentration of 4.88, 19.5, 78.1, 313, 1,250 or 5,000 ng/mL were added so that the total amount of the culture solution was adjusted to 200 µL/well. The culture supernatants after culture for 4 days were collected, and the CXCL10 concentrations in the culture supernatants were measured using CXCL10/IP-10 (human) AlphaLISA Detection Kit (PerkinElmer, Inc.). The results are shown in FIG. 35. In FIG. 35, the solid line and X (-×-) represents an anti-DNP antibody as a negative control; the solid line and solid circle (-●-) represents chimera A-Fc (Eg1S YTE); the solid line and open triangle (-△-) represents E57K-Fc (Eg1S YTE); the solid line and open circle (-∘-) represents 45-18-Fc (Eg1S YTE); the dotted line and solid circle (--●--) represents chimera A-Fc (Eg1S LALAGANA); the dotted line and open triangle (--△--) represents E57K-Fc (Eg1S LALAGANA); and the dotted line and open circle (--∘--) represents 45-18-Fc (Eg1S LALAGANA).

As shown in FIG. 35, it was confirmed that each of chimera A-Fc, E57K-Fc and 45-18-Fc (each including Eg1S YTE or Eg1S LALAGANA as an Fc) concentration-dependently inhibits the CXCL10 production by membrane-bound LIGHT, and that each of chimera A and the variants with lowered FasL binding activities, i.e., E57K-Fc and 45-18-Fc, has neutralizing activity to membrane-bound LIGHT.

### (2) Neutralizing Activity to Membrane-bound Human TL1A

Regarding each of chimera A-Fc, E57K-Fc and 45-18-Fc (each including Eg1S YTE or Eg1S LALAGANA as an Fc), the neutralizing activity to membrane-bound human TL1A was evaluated using human CD4-positive T cells and using IFN-γ production by co-culture with the cells of the membrane-bound TL1A forced expression CHO-K1 cell line (TL1A/CHO) used in Example 22 as an index.

From frozen healthy individual PBMCs (AllCells, LLC), CD4-positive T cells were isolated using EasySep Human CD4+ T cell Isolation Kit (STEMCELL Technologies Inc.). The isolated CD4-positive T cells were seeded at 2 × 10⁵ cells/well in a 96-well suspension culture plate, and then Human IL-12 (Miltenyi Biotec K.K.) at the final concentration of 2 ng/mL, Recombinant Human IL-18 (MEDICAL & BIOLOGICAL LABORATORIES CO., LTD.) at the final concentration of 50 ng/mL, and Human IL-15 (Miltenyi Biotec K.K.) at the final concentration of 25 ng/mL were added, followed by fixation with Fixation Buffer (BD Biosciences). Thereafter, membrane-bound TL1A expression CHO-K1 cells washed with PBS and a medium at the concentration of 3 × 10⁴ cells/well, and chimera A-Fc, E57K-Fc and 45-18-Fc (each including Eg1S YTE or Eg1S LALAGANA as an Fc) at the final concentration of 20, 50, 100, 250, 500 or 1,000 ng/mL were added so that the total amounts of the culture solution were adjusted to 200 µL/well. The culture supernatants after culture for 2 days were collected, and the IFN-γ concentrations in the culture supernatants were measured using AlphaLISA IFN-γ Immunoassay Research Kit (PerkinElmer, Inc.). The results are shown in FIG. 36. In FIG. 36, the solid line and X (-×-) represents an anti-DNP antibody as a negative control; the solid line and solid circle **(-●-)** represents chimera A-Fc (Eg1S YTE); the solid line and open triangle (-△-) represents E57K-Fc (Eg1S YTE); the solid line and open circle (-o-) represents 45-18-Fc (Eg1S YTE); the dotted line and solid circle (--●--) represents chimera A-Fc (Eg1S LALAGANA); the dotted line and open triangle (--△--) represents E57K-Fc (Eg1S LALAGANA); and the dotted line and open circle (--o--) represents 45-18-Fc (Eg1S LALAGANA).

As shown in FIG. 36, it was confirmed that each of chimera A-Fc, E57K-Fc and 45-18-Fc (each including Eg1S YTE or Eg1S LALAGANA as an Fc) concentration-dependently inhibits the IFN-γ production by membrane-bound TL1A, and that each of the variants with lowered FasL binding activities, i.e., E57K-Fc and 45-18-Fc, maintains neutralizing activity to membrane-bound TL1A similar to that of chimera A-Fc.

### (3) Neutralizing Activity to Membrane-bound Human FasL

Regarding each of chimera A-Fc, E57K-Fc and 45-18-Fc (each including Eg1S YTE or Eg1S LALAGANA as an Fc), the neutralizing activity to membrane-bound human FasL was measured using as an index apoptosis of Jurkat cells caused by co-culture with a cell line forcedly expressing membrane-bound FasL modified so as not to be cleaved to a soluble form.

A plasmid expressing a membrane-bound human FasL variant sequence (nucleotide sequence: SEQ ID NO: 343, amino acid sequence: SEQ ID NO: 344) obtained by adding a FLAG tag (DYKDDDDK) to the N-terminus and removing a FasL cleavage site (Glu128-Ile131) (Nat. Med., 1998, 4(1), p.31-36) and a proline-rich region of an intracellular region (Pro8-Pro69) (Nat. Med., 1996, 2(3), p.317-322) was produced by the following method. Using the membrane-bound FasL expression plasmid mentioned in Example 15 as a template and a primer designed to delete a target sequence, each of three regions, i.e., a region extending from a PstI site to Tyr7, a region extending from Pro70 to Leu127 and a region extending from Gly132 to a NotI site each was subjected to PCR amplification, and each of the resultant products was inserted under a CMV promoter of a pCIpuro vector by the same method as in Example 1. The plasmid thus obtained was introduced into CHO-K1 cells by the same method as in Example 15, and a membrane-bound human FasL variant high expression CHO-K1 cell line (FasLdel/CHO) was obtained by cell sorting of PE-labeled anti-human FasL antibody (BioLegend, Inc.)-positive fractions.

The FasLdel/CHO was seeded at 5 × 10³ cells/well in a 96-well adherent culture plate (Sumitomo Bakelite Co., Ltd.), and cultured in a 5% CO₂ incubator at 37°C overnight, followed by washing the cells once. Jurkat cells were seeded at 5 x 10⁴ cells/well therein, and chimera A-Fc, E57K-Fc and 45-18-Fc (in all of which Fc is Eg1S YTE or Eg1S LALAGANA) were added at the final concentration of 24.4, 97.7, 391, 1,563, 6,250 or 25,000 ng/mL so that the total amount of the culture solution was adjusted to 100 µL/well. After culture in a 5% CO₂ incubator at 37°C for 4 hours, only Jurkat cells were collected. The collected Jurkat cells were seeded in a 96-well U-bottom plate (Falcon), and stained using PE annexin V apoptosis detection kit (BD Biosciences) at room temperature for 15 minutes. After the cells were washed with an FCM buffer, the fluorescence intensity was analyzed by flow cytometer FACS Fortessa (BD Biosciences), and Annexin V-positive cells were detected as a dead cell subset. The results are shown in FIG. 37. In FIG. 37, the solid line and X (-×-) represents an anti-DNP antibody as a negative control; the solid line and solid circle (-●-) represents chimera A-Fc (Eg1S YTE); the solid line and open triangle (-△-) represents E57K-Fc (Eg1S YTE); the solid line and open circle (-∘-) represents 45-18-Fc (Eg1S YTE); the dotted line and solid circle (--●--) represents chimera A-Fc (Eg1S LALAGANA); the dotted line and open triangle (--△- -) represents E57K-Fc (Eg1S LALAGANA); and the dotted line and open circle (--∘--) represents 45-18-Fc (Eg1S LALAGANA).

As shown in FIG. 37, it was confirmed that chimera A-Fc (Eg1S YTE, Eg1S LALAGANA) concentration-dependently inhibits cell death of Jurkat cells and has neutralizing activity to membrane-bound FasL. Meanwhile, regarding each of E57K-Fc and 45-18-Fc (each including Eg1S YTE or Eg1S LALAGANA as an Fc), it was confirmed that, in each of the variants, the inhibitory activity to Jurkat cell death is hardly exhibited even in the 25,000 ng/mL high concentration group and the neutralizing activity to membrane-bound FasL is remarkably decreased.

### [Example 24] Evaluation of Binding Activity to Soluble DcR3 Ligand Derived from Human Primary Cell

Regarding each of chimera A-Fc, E57K-Fc and 45-18-Fc (each including Eg1S YTE or Eg1S LALAGANA as an Fc), the binding activities to various soluble DcR ligands were evaluated using soluble DcR3 ligands derived from human primary cells. All of the test substances were produced by transient expression in CHO-S, and, similarly to Example 21, purified to 95% or more of monomers by SEC for use.

### (1) Binding Activity to Primary LIGHT

Regarding each of chimera A-Fc, E57K-Fc and 45-18-Fc (each including Eg1S YTE or Eg1S LALAGANA as an Fc), the binding activity to primary soluble LIGHT produced from activated human T cells was evaluated by the following method. The primary soluble LIGHT was prepared by the following method. In a 6-well adherent culture plate (Sumitomo Bakelite Co., Ltd.) to which 10 µg/mL of anti-CD3 antibody OKT3 (BioLegend, Inc.) was immobilized, human T cells isolated from thawed frozen healthy individual PBMCs (AllCells, LLC) were seeded at 7 × 10⁶ cells/well, and anti-CD28 antibodies (BioLegend, Inc.) at a final concentration of 1 µg/mL were added. After culture in a 5% CO₂ incubator for 3 days, the culture supernatant was collected. The concentration of primary soluble LIGHT in the collected culture supernatant was measured using Human LIGHT/TNFSF14 Quantikine ELISA Kit (R&D Systems, Inc.), and it was confirmed that soluble LIGHT existed at 14 pg/mL in the culture supernatant of unstimulated human T cells and at 5.6 ng/mL in the culture supernatant of human T cells stimulated with an anti-CD3 antibody and an anti-CD28 antibody.

Then, 10 µg/mL of anti-human IgG antibodies (American Qualex International, Inc.) were immobilized to a 96-well immunoplate (Thermo Scientific), and blocking was performed with 2% Block Ace (DS Pharma Biomedical Co., Ltd.), and then 2 µg/mL of chimera A-Fc, E57K-Fc and 45-18-Fc (in all of which Fc is Eg1S YTE or Eg1S LALAGANA) were captured. The culture supernatant of human T cells stimulated for LIGHT production was concentrated 6-fold using Amicon Ultra-15 having a molecular weight cutoff of 3 kDa (Merck Millipore) prerinsed with sterile water. After the plate was washed, the 6-fold concentrated culture supernatant was reacted. As a standard of LIGHT, the soluble recombinant FLAG-LIGHT trimer produced in Example 13 was used. After the plate was washed, a biotinylated anti-LIGHT antibody (R&D Systems, Inc.) was reacted. After the plate was washed, streptavidin-HRP (PIERCE) was reacted. After washing again, a TMB solution (Dako) was added to develop a color. Then, a 2 N sulfuric acid solution was used to stop the chromogenic reaction, and the absorbance at 450 nm (reference wavelength of 570 nm) was measured.

The results for the soluble recombinant FLAG-LIGHT trimer are shown in FIG. 38A, and the results for the culture supernatant of the human T cells stimulated for LIGHT expression are shown in FIG. 38B. In FIG. 38A, the solid line with X (-×-) represents an anti-DNP antibody as a negative control; the solid line with solid circle (-●-) represents chimera A-Fc (Eg1S YTE); the solid line with open square (-o-) represents chimera A-Fc(Eg1S); the solid line with open triangle (-△-) represents E57K-Fc (Eg1S YTE); the solid line with open circle (-∘-) represents 45-18-Fc (Eg1S YTE); the dotted line with solid circle (--●--) represents chimera A-Fc (Eg1S LALAGANA); the dotted line with open triangle (--△--) represents E57K-Fc (Eg1S LALAGANA); and the dotted line with open circle (--∘--) represents 45-18-Fc (Eg1S LALAGANA). In FIG. 38B, "6×sup. CD3/28" represented by the solid bar represents use of the 6-fold concentrated culture supernatant of human T cells stimulated for LIGHT expression under anti-CD3 antibody OKT3 and anti-CD28 antibody stimulation conditions, and "6×sup. none" represented by the open bar represents use of the 6-fold concentrated culture supernatant of unstimulated human T cells.

As shown in FIG. 38B, in a well in which each of chimera A-Fc, E57K-Fc and 45-18-Fc (each including Eg1S, Eg1S YTE or Eg1S LALAGANA as an Fc) was captured, LIGHT was detected only in the culture supernatant of human T cells stimulated for LIGHT expression. The results confirmed that each of chimera A-Fc, E57K-Fc and 45-18-Fc (each including Eg1S, Eg1S YTE or Eg1S LALAGANA as an Fc) binds to soluble LIGHT produced from human T cells stimulated with an anti-CD3 antibody and an anti-CD28 antibody.

### (2) Binding Activity to Primary TL1A

Regarding each of chimera A-Fc, E57K-Fc and 45-18-Fc (each including Eg1S YTE or Eg1S LALAGANA as an Fc), the binding activity to primary soluble TL1A produced from an activated human PBMC was evaluated. The primary soluble TL1A was prepared by the following method. To a 6-well adherent culture plate (Sumitomo Bakelite Co., Ltd.), 250 µg/mL of human IgG antibodies (Jackson ImmunoResearch Laboratories, Inc.) were immobilized, and further anti-human κ light chain antibodies (Bethyl Laboratories, Inc.) and anti-human A light chain antibodies (Bethyl Laboratories, Inc.) were added at each final concentration of 100 µg/mL to treat for 1 hour, thus producing the immune complex. In the plate, thawed frozen healthy individual PBMCs (AllCells, LLC) were seeded at 6 × 10⁶ cells/well, followed by culture in a 5% CO₂ incubator at 37°C. After 3 days, the culture supernatant was collected, and concentrated 4-fold using Amicon Ultra-15 having a molecular weight cutoff of 3 kDa (Merck Millipore) prerinsed with sterile water.

Sandwich ELISA was performed by the same method as in (1). On a 96-well immunoplate, chimera A-Fc, E57K-Fc and 45-18-Fc (in all of which Fc is Eg1S YTE or Eg1S LALAGANA) or the anti-human TL1A antibody produced in Example 16 was captured. Then, with the plate, a 4-fold concentrated culture supernatant of PBMCs stimulated for TL1A production or as a standard, the soluble recombinant His6-TL1A trimer produced in Example 13 was reacted, followed by detection with a biotinylated anti-TL1A antibody (R&D Systems, Inc.).

The results for the soluble recombinant His6-TL1A trimer are shown in FIG. 38C, and the results for the culture supernatant of PBMCs stimulated for TL1A production are shown in FIG. 38D. In FIG. 38C, the solid line with X (-×-) represents an anti-DNP antibody as a negative control; the solid line with solid circle (-●-) represents chimera A-Fc (Eg1S YTE); the solid line with open square (-□-) represents chimera A-Fc (Eg1S); the solid line with open triangle (-△-) represents E57K-Fc (Eg1S YTE); the solid line with open circle (-o-) represents 45-18-Fc (Eg1S YTE); the dotted line with solid circle (--●--) represents chimera A-Fc (Eg1S LALAGANA); the dotted line with open triangle (--△--) represents E57K-Fc (Eg1S LALAGANA); the dotted line with open circle (--∘--) represents 45-18-Fc (Eg1S LALAGANA); and the solid line with solid triangle (-▲-) represents an anti-TL1A antibody. In FIG. 38D, "4×sup. I.C." represented by the solid bar represents use of the culture supernatant of PBMCs stimulated for TL1A expression under immune complex stimulation conditions, and "4×sup. none" represented by the open bar represents use of the culture supernatant of unstimulated PBMCs. As shown in FIG. 38D, in a well in which each of chimera A-Fc, E57K-Fc and 45-18-Fc (each including Eg1S, Eg1S YTE or Eg1S LALAGANA as an Fc) was captured, TL1A was detected only in the culture supernatant of PBMCs stimulated for TL1A expression. The results confirmed that chimera A-Fc, E57K-Fc and 45-18-Fc (each including Eg1S, Eg1S YTE or Eg1S LALAGANA as an Fc) binds to soluble TL1A produced from PBMCs stimulated with the immune complex.

### (3) Binding Activity to Primary FasL

Regarding each of chimera A-Fc, E57K-Fc and 45-18-Fc (each including Eg1S YTE or Eg1S LALAGANA as an Fc), the binding activity to primary soluble human FasL was evaluated. As the primary soluble FasL, soluble FasL produced from human T cells stimulated for activation-induced cell death (AICD) was prepared by the same method as in Example 16 for use.

Sandwich ELISA was performed by the same method as in (1). On a 96-well immunoplate, chimera A-Fc, E57K-Fc and 45-18-Fc (each including Eg1S YTE or Eg1S LALAGANA as an Fc) or Fas-Fc (R&D Systems, Inc.) was captured, and then a 6-fold concentrated culture supernatant of human T cells stimulated for AICD or as a standard, Human His6 Fas Ligand/TNFSF6 (Cell Signaling Technology, Inc.) was reacted, followed by detection with a biotinylated anti-FasL antibody included with Human Fas Ligand ELISA Kit (Abcam plc).

The results for the soluble recombinant His6-FasL are shown in FIG. 38E, and the results for the culture supernatant of human T cells stimulated for AICD are shown in FIG. 38F. In FIG. 38E, the solid line with X (-×-) represents an anti-DNP antibody as a negative control; the solid line with solid circle (-●-) represents chimera A-Fc (Eg1S YTE); the solid line with open square (-□-) represents chimera A-Fc(Eg1S); the solid line with open triangle (-△-) represents E57K-Fc (Eg1S YTE); the solid line with open circle (-∘-) represents 45-18-Fc (Eg1S YTE); the dotted line with solid circle (--●--) represents chimera A-Fc (Eg1S LALAGANA); the dotted line with open triangle (--△- -) represents E57K-Fc (Eg1S LALAGANA); the dotted line with open circle (--∘--) represents 45-18-Fc (Eg1S LALAGANA); and the solid line with solid triangle (-▲-) represents Fas-Fc. In FIG. 38F, "6×sup. CD3" represented by the solid bar represents use of the culture supernatant of human T cells stimulated for AICD under anti-CD3 antibody OKT3 stimulation conditions, and "6×sup. none" represented by the open bar represents use of the culture supernatant of human T cells not stimulated for AICD. As shown in FIG. 38F, in a well in which chimera A-Fc (Eg1S, Eg1S YTE, Eg1S LALAGANA) was captured, FasL was detected only in the culture supernatant of human T cells stimulated for AICD. Meanwhile, in a well in which E57K-Fc or 45-18-Fc (each including Eg1S YTE or Eg1S LALAGANA as an Fc) was captured, it was confirmed that FasL detected in the culture supernatant of human T cells stimulated for AICD is remarkably decreased, and that, in each of these variants, the binding activity to soluble FasL derived from human T cells is attenuated.

### [Example 25] Evaluation of Physical Property of DcR3 Variant

Regarding E57K-Fc and 45-18-Fc (each including Eg1S YTE or Eg1S LALAGANA as an Fc) that were produced by transient expression in CHO-S and purified to 95% or more of monomers by SEC similarly to Example 21, using the same method as in Example 4, the elution times (min) were calculated by hydrophobic interaction chromatography (HIC) and the Tm values (°C) were calculated by the differential scanning fluorimetry (DSF) method.

As a result, since all values showed values equivalent to those in Example 17, it was confirmed that a combination of the abovementioned amino acid mutation introduction into an IgG1 Fc sequence and the abovementioned amino acid substitution into the chimera A sequence does not affect the hydrophobicity and the thermostability.

### [Example 26] Evaluation of Mouse In Vivo Kinetics of DcR3 Variant

Regarding chimera A-Fc, E57K-Fc and 45-18-Fc (each including Eg1S YTE or Eg1S LALAGANA as an Fc) that were produced by transient expression in CHO-S and purified to 95% or more of monomers by SEC similarly to Example 21, *in vivo* kinetics in mice were evaluated by the same method as the method mentioned in Example 6. The results are shown in FIG. 39.

As shown in FIG. 39, compared to the AUC value of 354 for S195-Fc (IEGRMD g1S) corresponding to wild-type DcR3 mentioned in Example 6, each of the DcR3 variants showed greater improvement in AUC.

### [Example 27] Evaluation of Aggregability of DcR3 Variant without Fc Fusion

A protein obtained by adding a His6 tag to the C-terminus of human wild-type DcR3 (S195) lacking an HBD region (S195-His6) (nucleotide sequence: SEQ ID NO: 345, amino acid sequence: SEQ ID NO: 346), a protein obtained by adding a His6 tag to the C-terminus of chimera A (chimera A-His6) (nucleotide sequence: SEQ ID NO: 347, amino acid sequence: SEQ ID NO: 348), a protein obtained by adding a His6 tag to the C-terminus of chimera A-E57K (chimera A-E57K-His6) (nucleotide sequence: SEQ ID NO: 349, amino acid sequence: SEQ ID NO: 350), and a protein obtained by adding a His6 tag to the C-terminus of 45-18 (45-18-His6) (nucleotide sequence: SEQ ID NO: 351, amino acid sequence: SEQ ID NO: 352) were produced as follows.

Regarding S195-His6, a sequence obtained by adding a signal peptide sequence to the N-terminus of human DcR3 lacking HBD (DNA fragment: SEQ ID NO: 107) and adding a His6 tag sequence to the C-terminus thereof was artificially synthesized (Fasmac Co., Ltd.). Regarding chimera A-His6, chimera A-E57K-His6 and 45-18-His6, sequences obtained by adding a signal peptide sequence to the N-terminus of chimera A (DNA fragment: SEQ ID NO: 353), chimera A-E57K (DNA fragment: SEQ ID NO: 354) or 45-18 (DNA fragment: SEQ ID NO: 355), and adding a His6 tag sequence to the C-terminus thereof were artificially synthesized. Each artificial synthesis fragment was introduced into a pCIpuro vector by the method mentioned in Example 1, and transient expression by Expi293 cells was performed. Using Amicon Ultra-0.5 having a molecular weight cutoff of 3 kDa (Merck Millipore) prerinsed with sterile water, the culture supernatant of the cells expressing S195-His6 was concentrated 30-fold. Each culture supernatant of the cells expressing chimera A-His6, chimera A-E57K-His6 or 45-18-His6 was diluted 6-fold.

Using these culture supernatants, SDS-PAGE was performed under non-reducing conditions or 100 mM DTT reducing conditions, and then immunoblotting was performed using a mouse anti-6-His tag monoclonal antibody (Thermo Scientific) as a primary antibody and a goat anti-mouse IgG antibody (Southern Biotechnology Associates, Inc.) as a secondary antibody.

The results are shown in FIG. 40. In the culture supernatant of the cells transiently expressing S195-His6, under reducing conditions in lane 1, a band was detected at near 20.9 kDa corresponding to an expected molecular weight, while under non-reducing conditions in lane 5, no bands of monomers were detected, and the whole was smear. The results confirmed that all of S195-His6 existed as aggregates in the culture supernatant. Meanwhile, in all of the culture supernatants of the cells transiently expressing chimera A-His6, chimera A-E57K-His6 and 45-18-His6, under non-reducing conditions in lanes 2 to 4, bands of monomers were detected at near 22.2 kDa corresponding to an expected molecular weight, like reducing conditions in lanes 6 to 8. The results showed that, in the DcR3 variant of the present invention, the aggregate content is greatly decreased compared to that of wild-type DcR3. In other words, it was shown that substitution of CRD1 and CRD4 of DcR3 with CRD1 and CRD4 derived from OPG, respectively, has an effect to decrease aggregation that is observed in wild-type DcR3, and that this effect is exerted regardless of whether the binding activity to FasL is lowered.

### Sequence Listing Free Text

SEQ ID NO: 1: nucleotide sequence of human DcR3 cDNA (Accession Number: NM_003823.3)
SEQ ID NO: 2: amino acid sequence of human DcR3 (Accession Number: NP_003814.1)
SEQ ID NO: 3: nucleotide sequence of human DcR3 (without signal peptide)
SEQ ID NO: 4: amino acid sequence of human DcR3 (without signal peptide)
SEQ ID NO: 5: nucleotide sequence of CRD1 of human DcR3
SEQ ID NO: 6: amino acid sequence of CRD1 of human DcR3
SEQ ID NO: 7: nucleotide sequence of CRD2 of human DcR3
SEQ ID NO: 8: amino acid sequence of CRD2 of human DcR3
SEQ ID NO: 9: nucleotide sequence of CRD3 of human DcR3
SEQ ID NO: 10: amino acid sequence of CRD3 of human DcR3
SEQ ID NO: 11: nucleotide sequence of CRD4 of human DcR3
SEQ ID NO: 12: amino acid sequence of CRD4 of human DcR3
SEQ ID NO: 13: nucleotide sequence of human OPG cDNA (Accession Number: NM_002546.3)
SEQ ID NO: 14: amino acid sequence of human OPG (Accession Number: NP_002537.3)
SEQ ID NO: 15: nucleotide sequence of human OPG (without signal peptide)
SEQ ID NO: 16: amino acid sequence of human OPG (without signal peptide)
SEQ ID NO: 17: nucleotide sequence of CRD1 of human OPG
SEQ ID NO: 18: amino acid sequence of CRD1 of human OPG
SEQ ID NO: 19: nucleotide sequence of CRD2 of human OPG
SEQ ID NO: 20: amino acid sequence of CRD2 of human OPG
SEQ ID NO: 21: nucleotide sequence of CRD3 of human OPG
SEQ ID NO: 22: amino acid sequence of CRD3 of human OPG
SEQ ID NO: 23: nucleotide sequence of CRD4 of human OPG
SEQ ID NO: 24: amino acid sequence of CRD4 of human OPG
SEQ ID NO: 25: nucleotide sequence of chimera B-HBD
SEQ ID NO: 26: amino acid sequence of chimera B-HBD
SEQ ID NO: 27: nucleotide sequence of chimera C-HBD
SEQ ID NO: 28: amino acid sequence of chimera C-HBD
SEQ ID NO: 29: nucleotide sequence of chimera A-HBD
SEQ ID NO: 30: amino acid sequence of chimera A-HBD
SEQ ID NO: 31: nucleotide sequence of 103-123OPG-HBD
SEQ ID NO: 32: amino acid sequence of 103-123OPG-HBD
SEQ ID NO: 33: nucleotide sequence of N131S/N144S-HBD
SEQ ID NO: 34: amino acid sequence of N131S/N144S-HBD
SEQ ID NO: 35: nucleotide sequence of T133A/S146A-HBD
SEQ ID NO: 36: amino acid sequence of T133A/S146A-HBD
SEQ ID NO: 37: nucleotide sequence of N131S/N144S/N157S-HBD
SEQ ID NO: 38: amino acid sequence of N131S/N144S/N157S-HBD
SEQ ID NO: 39: nucleotide sequence of T133A/S146A/T159A-HBD
SEQ ID NO: 40: amino acid sequence of T133A/S146A/T159A-HBD
SEQ ID NO: 41: nucleotide sequence of chimera A-E57K-HBD
SEQ ID NO: 42: amino acid sequence of chimera A-E57K-HBD
SEQ ID NO: 43: nucleotide sequence of chimera A-E57L-HBD
SEQ ID NO: 44: amino acid sequence of chimera A-E57L-HBD
SEQ ID NO: 45: nucleotide sequence of chimera A-R60K-HBD
SEQ ID NO: 46: amino acid sequence of chimera A-R60K-HBD
SEQ ID NO: 47: nucleotide sequence of HBD of wild-type DcR3
SEQ ID NO: 48: amino acid sequence of HBD of wild-type DcR3
SEQ ID NO: 49: nucleotide sequence of chimera B
SEQ ID NO: 50: amino acid sequence of chimera B
SEQ ID NO: 51: nucleotide sequence of chimera C
SEQ ID NO: 52: amino acid sequence of chimera C
SEQ ID NO: 53: nucleotide sequence of chimera A
SEQ ID NO: 54: amino acid sequence of chimera A
SEQ ID NO: 55: nucleotide sequence of 103-123OPG
SEQ ID NO: 56: amino acid sequence of 103-123OPG
SEQ ID NO: 57: nucleotide sequence of N131S/N144S
SEQ ID NO: 58: amino acid sequence of N131S/N144S
SEQ ID NO: 59: nucleotide sequence of T133A/S146A
SEQ ID NO: 60: amino acid sequence of T133A/S146A
SEQ ID NO: 61: nucleotide sequence of N131S/N144S/N157S
SEQ ID NO: 62: amino acid sequence of N131S/N144S/N157S
SEQ ID NO: 63: nucleotide sequence of T133A/S146A/T159A
SEQ ID NO: 64: amino acid sequence of T133A/S146A/T159A
SEQ ID NO: 65: nucleotide sequence of chimera A-E57K
SEQ ID NO: 66: amino acid sequence of chimera A-E57K
SEQ ID NO: 67: nucleotide sequence of chimera A-E57L
SEQ ID NO: 68: amino acid sequence of chimera A-E57L
SEQ ID NO: 69: nucleotide sequence of chimera A-R60K
SEQ ID NO: 70: amino acid sequence of chimera A-R60K
SEQ ID NO: 71: nucleotide sequence of Fc (g1S)
SEQ ID NO: 72: amino acid sequence of Fc (g1S)
SEQ ID NO: 73: nucleotide sequence of Fc (g4PEK)
SEQ ID NO: 74: amino acid sequence of Fc (g4PEK)
SEQ ID NO: 75: nucleotide sequence of chimera B-Fc (IEGRMD g1S)
SEQ ID NO: 76: amino acid sequence of chimera B-Fc (IEGRMD g1S)
SEQ ID NO: 77: nucleotide sequence of chimera C-Fc (IEGRMD g1S)
SEQ ID NO: 78: amino acid sequence of chimera C-Fc (IEGRMD g1S)
SEQ ID NO: 79: nucleotide sequence of chimera A-Fc (IEGRMD g1S)
SEQ ID NO: 80: amino acid sequence of chimera A-Fc (IEGRMD g1S)
SEQ ID NO: 81: nucleotide sequence of chimera A-Fc (g4PEK)
SEQ ID NO: 82: amino acid sequence of chimera A-Fc (g4PEK)
SEQ ID NO: 83: nucleotide sequence of 103-123OPG-Fc (g4PEK)
SEQ ID NO: 84: amino acid sequence of 103-123OPG-Fc (g4PEK)
SEQ ID NO: 85: nucleotide sequence of N131S/N144S-Fc (g4PEK)
SEQ ID NO: 86: amino acid sequence of N1315/N1445-Fc (g4PEK)
SEQ ID NO: 87: nucleotide sequence of T133A/S146A-Fc (g4PEK)
SEQ ID NO: 88: amino acid sequence of T133A/S146A-Fc (g4PEK)
SEQ ID NO: 89: nucleotide sequence of N131S/N144S/N157S-Fc (g4PEK)
SEQ ID NO: 90: amino acid sequence of N131S/N144S/N157S-Fc (g4PEK)
SEQ ID NO: 91: nucleotide sequence of T133A/S146A/T159A-Fc (g4PEK)
SEQ ID NO: 92: amino acid sequence of T133A/S146A/T159A-Fc (g4PEK)
SEQ ID NO: 93: nucleotide sequence of chimera A-E57K-Fc (g4PEK)
SEQ ID NO: 94: amino acid sequence of chimera A-E57K-Fc (g4PEK)
SEQ ID NO: 95: nucleotide sequence of chimera A-E57L-Fc (g4PEK)
SEQ ID NO: 96: amino acid sequence of chimera A-E57L-Fc (g4PEK)
SEQ ID NO: 97: nucleotide sequence of chimera A-R60K-Fc (g4PEK)
SEQ ID NO: 98: amino acid sequence of chimera A-R60K-Fc (g4PEK)
SEQ ID NO: 99: nucleotide sequence of DcR3 FL-Fc
SEQ ID NO: 100: amino acid sequence of DcR3 FL-Fc
SEQ ID NO: 101: nucleotide sequence of S195-Fc
SEQ ID NO: 102: amino acid sequence of S195-Fc
SEQ ID NO: 103: nucleotide sequence of DcR3 FL-FLAG
SEQ ID NO: 104: amino acid sequence of DcR3 FL-FLAG
SEQ ID NO: 105: nucleotide sequence of linker IEGRMD
SEQ ID NO: 106: amino acid sequence of linker IEGRMD
SEQ ID NO: 107: nucleotide sequence of DcR3 (without signal peptide) with HBD deleted
SEQ ID NO: 108: amino acid sequence of DcR3 (without signal peptide) with HBD deleted
SEQ ID NO: 109: nucleotide sequence of FLAG tag
SEQ ID NO: 110: amino acid sequence of FLAG tag
SEQ ID NO: 111: nucleotide sequence of R218Q DcR3
SEQ ID NO: 112: amino acid sequence of R218Q DcR3
SEQ ID NO: 113: nucleotide sequence of soluble recombinant of human LIGHT
SEQ ID NO: 114: amino acid sequence of soluble recombinant of human LIGHT
SEQ ID NO: 115: nucleotide sequence of soluble recombinant of human TL1A
SEQ ID NO: 116: amino acid sequence of soluble recombinant of human TL1A
SEQ ID NO: 117: nucleotide sequence of soluble recombinant of human FasL
SEQ ID NO: 118: amino acid sequence of soluble recombinant of human FasL
SEQ ID NO: 119: nucleotide sequence of soluble recombinant of cynomolgus monkey LIGHT
SEQ ID NO: 120: amino acid sequence of soluble recombinant of cynomolgus monkey LIGHT
SEQ ID NO: 121: nucleotide sequence of soluble recombinant of cynomolgus monkey TL1A
SEQ ID NO: 122: amino acid sequence of soluble recombinant of cynomolgus monkey TL1A
SEQ ID NO: 123: nucleotide sequence of soluble recombinant of cynomolgus monkey FasL
SEQ ID NO: 124: amino acid sequence of soluble recombinant of cynomolgus monkey FasL
SEQ ID NO: 125: nucleotide sequence of soluble recombinant of mouse LIGHT
SEQ ID NO: 126: amino acid sequence of soluble recombinant of mouse LIGHT
SEQ ID NO: 127: nucleotide sequence of soluble recombinant of mouse TL1A
SEQ ID NO: 128: amino acid sequence of soluble recombinant of mouse TL1A
SEQ ID NO: 129: nucleotide sequence of soluble recombinant of mouse FasL
SEQ ID NO: 130: amino acid sequence of soluble recombinant of mouse FasL
SEQ ID NO: 131: nucleotide sequence of extracellular region (Asp74-Val240) of human LIGHT
SEQ ID NO: 132: amino acid sequence of extracellular region (Asp74-Val240) of human LIGHT
SEQ ID NO: 133: nucleotide sequence of extracellular region (Asp74-Val240) of cynomolgus monkey LIGHT
SEQ ID NO: 134: amino acid sequence of extracellular region (Asp74-Val240) of cynomolgus monkey LIGHT
SEQ ID NO: 135: nucleotide sequence of extracellular region (Asp72-Val239) of mouse LIGHT
SEQ ID NO: 136: amino acid sequence of extracellular region (Asp72-Val239) of mouse LIGHT
SEQ ID NO: 137: nucleotide sequence of extracellular region (Leu72-Leu251) of human TL1A
SEQ ID NO: 138: amino acid sequence of extracellular region (Leu72-Leu251) of human TL1A
SEQ ID NO: 139: nucleotide sequence of extracellular region (Leu72-Leu251) of cynomolgus monkey TL1A
SEQ ID NO: 140: amino acid sequence of extracellular region (Leu72-Leu251) of cynomolgus monkey TL1A
SEQ ID NO: 141: nucleotide sequence of extracellular region (Ile94-Leu270) of mouse TL1A
SEQ ID NO: 142: amino acid sequence of extracellular region (Ile94-Leu270) of mouse TL1A
SEQ ID NO: 143: nucleotide sequence of extracellular region (Pro134-Leu281) of human FasL
SEQ ID NO: 144: amino acid sequence of extracellular region (Pro134-Leu281) of human FasL
SEQ ID NO: 145: nucleotide sequence of extracellular region (Pro133-Leu280) of cynomolgus monkey FasL
SEQ ID NO: 146: amino acid sequence of extracellular region (Pro133-Leu280) of cynomolgus monkey FasL
SEQ ID NO: 147: nucleotide sequence of extracellular region (Pro132-Leu279) of mouse FasL
SEQ ID NO: 148: amino acid sequence of extracellular region (Pro132-Leu279) of mouse FasL
SEQ ID NO: 149: nucleotide sequence of chimera A-Fc (g1S)
SEQ ID NO: 150: amino acid sequence of chimera A-Fc (g1S)
SEQ ID NO: 151: nucleotide sequence of OPG K194-Fc
SEQ ID NO: 152: amino acid sequence of OPG K194-Fc
SEQ ID NO: 153: amino acid sequence of constant region of human IgG1 heavy chain
SEQ ID NO: 154: amino acid sequence of constant region of human IgG4 heavy chain
SEQ ID NO: 155: nucleotide sequence of Fc (Eg1S)
SEQ ID NO: 156: amino acid sequence of Fc (Eg1S)
SEQ ID NO: 157: nucleotide sequence of Fc (Eg1S-YTE)
SEQ ID NO: 158: amino acid sequence of Fc (Eg1S-YTE)
SEQ ID NO: 159: nucleotide sequence of Fc (Eg1S-N434A)
SEQ ID NO: 160: amino acid sequence of Fc (Eg15-N434A)
SEQ ID NO: 161: nucleotide sequence of Fc (g1S-LALAGA)
SEQ ID NO: 162: amino acid sequence of Fc (g1S-LALAGA)
SEQ ID NO: 163: nucleotide sequence of Fc (Eg1S-LALAGA)
SEQ ID NO: 164: amino acid sequence of Fc (Eg1S-LALAGA)
SEQ ID NO: 165: nucleotide sequence of Fc (Eg1S-LALAGANA)
SEQ ID NO: 166: amino acid sequence of Fc (Eg1S-LALAGANA)
SEQ ID NO: 167: nucleotide sequence of chimera A-Fc (Eg1S)
SEQ ID NO: 168: amino acid sequence of chimera A-Fc (Eg1S)
SEQ ID NO: 169: nucleotide sequence of chimera A-Fc (Eg1S-YTE)
SEQ ID NO: 170: amino acid sequence of chimera A-Fc (Eg1S-YTE)
SEQ ID NO: 171: nucleotide sequence of chimera A-Fc (Eg1S-N434A)
SEQ ID NO: 172: amino acid sequence of chimera A-Fc (Eg1S-N434A)
SEQ ID NO: 173: nucleotide sequence of chimera A-Fc (g1S-LALAGA)
SEQ ID NO: 174: amino acid sequence of chimera A-Fc (g15-LALAGA)
SEQ ID NO: 175: nucleotide sequence of chimera A-Fc (Eg1S-LALAGA)
SEQ ID NO: 176: amino acid sequence of chimera A-Fc (Eg1S-LALAGA)
SEQ ID NO: 177: nucleotide sequence of chimera A-Fc (Eg1S-LALAGANA)
SEQ ID NO: 178: amino acid sequence of chimera A-Fc (Eg1S-LALAGANA)
SEQ ID NO: 179: nucleotide sequence of chimera A-E57R
SEQ ID NO: 180: amino acid sequence of chimera A-E57R
SEQ ID NO: 181: nucleotide sequence of chimera A-E57V
SEQ ID NO: 182: amino acid sequence of chimera A-E57V
SEQ ID NO: 183: nucleotide sequence of chimera A-E57K_R58D
SEQ ID NO: 184: amino acid sequence of chimera A-E57K_R58D
SEQ ID NO: 185: nucleotide sequence of chimera A-E57K_R58E
SEQ ID NO: 186: amino acid sequence of chimera A-E57K_R58E
SEQ ID NO: 187: nucleotide sequence of chimera A-E57R_R58D
SEQ ID NO: 188: amino acid sequence of chimera A-E57R_R58D
SEQ ID NO: 189: nucleotide sequence of chimera A-E57R-Fc (g4PEK)
SEQ ID NO: 190: amino acid sequence of chimera A-E57R-Fc (g4PEK)
SEQ ID NO: 191: nucleotide sequence of chimera A-E57V-Fc (g4PEK)
SEQ ID NO: 192: amino acid sequence of chimera A-E57V-Fc (g4PEK)
SEQ ID NO: 193: nucleotide sequence of chimera A-E57K_R58D-Fc (g4PEK)
SEQ ID NO: 194: amino acid sequence of chimera A-E57K_R58D-Fc (g4PEK)
SEQ ID NO: 195: nucleotide sequence of chimera A-E57K_R58E-Fc (g4PEK)
SEQ ID NO: 196: amino acid sequence of chimera A-E57K_R58E-Fc (g4PEK)
SEQ ID NO: 197: nucleotide sequence of chimera A-E57R_R58D-Fc (g4PEK)
SEQ ID NO: 198: amino acid sequence of chimera A-E57R_R58D-Fc (g4PEK)
SEQ ID NO: 199: nucleotide sequence of chimera A-E57K-Fc (Eg1S)
SEQ ID NO: 200: amino acid sequence of chimera A-E57K-Fc (Eg1S)
SEQ ID NO: 201: nucleotide sequence of chimera A-E57L-Fc (Eg1S)
SEQ ID NO: 202: amino acid sequence of chimera A-E57L-Fc (Eg1S)
SEQ ID NO: 203: nucleotide sequence of chimera A-E57R-Fc (Eg1S)
SEQ ID NO: 204: amino acid sequence of chimera A-E57R-Fc (Eg1S)
SEQ ID NO: 205: nucleotide sequence of chimera A-E57V-Fc (Eg1S)
SEQ ID NO: 206: amino acid sequence of chimera A-E57V-Fc (Eg1S)
SEQ ID NO: 207: nucleotide sequence of chimera A-E57K_R58D-Fc (Eg1S)
SEQ ID NO: 208: amino acid sequence of chimera A-E57K_R58D-Fc (Eg1S)
SEQ ID NO: 209: nucleotide sequence of chimera A-E57K_R58E-Fc (Eg1S)
SEQ ID NO: 210: amino acid sequence of chimera A-E57K_R58E-Fc (Eg1S)
SEQ ID NO: 211: nucleotide sequence of chimera A-E57R_R58D-Fc (Eg1S)
SEQ ID NO: 212: amino acid sequence of chimera A-E57R_R58D-Fc (Eg1S)
SEQ ID NO: 213: nucleotide sequence of chimera A-E57K-Fc (Eg1S YTE)
SEQ ID NO: 214: amino acid sequence of chimera A-E57K-Fc (Eg1S YTE)
SEQ ID NO: 215: nucleotide sequence of chimera A-E57L-Fc (Eg1S YTE)
SEQ ID NO: 216: amino acid sequence of chimera A-E57L-Fc (Eg1S YTE)
SEQ ID NO: 217: nucleotide sequence of chimera A-E57R-Fc (Eg1S YTE)
SEQ ID NO: 218: amino acid sequence of chimera A-E57R-Fc (Eg1S YTE)
SEQ ID NO: 219: nucleotide sequence of chimera A-E57V-Fc (Eg1S YTE)
SEQ ID NO: 220: amino acid sequence of chimera A-E57V-Fc (Eg1S YTE)
SEQ ID NO: 221: nucleotide sequence of chimera A-E57K_R58D-Fc (Eg1S YTE)
SEQ ID NO: 222: amino acid sequence of chimera A-E57K_R58D-Fc (Eg1S YTE)
SEQ ID NO: 223: nucleotide sequence of chimera A-E57K_R58E-Fc (Eg1S YTE)
SEQ ID NO: 224: amino acid sequence of chimera A-E57K_R58E-Fc (Eg1S YTE)
SEQ ID NO: 225: nucleotide sequence of chimera A-E57R_R58D-Fc (Eg1S YTE)
SEQ ID NO: 226: amino acid sequence of chimera A-E57R_R58D-Fc (Eg1S YTE)
SEQ ID NO: 227: nucleotide sequence of chimera A-E57K-Fc (Eg1S N434A)
SEQ ID NO: 228: amino acid sequence of chimera A-E57K-Fc (Eg1S N434A)
SEQ ID NO: 229: nucleotide sequence of chimera A-E57L-Fc (Eg1S N434A)
SEQ ID NO: 230: amino acid sequence of chimera A-E57L-Fc (Eg1S N434A)
SEQ ID NO: 231: nucleotide sequence of chimera A-E57R-Fc (Eg1S N434A)
SEQ ID NO: 232: amino acid sequence of chimera A-E57R-Fc (Eg1S N434A)
SEQ ID NO: 233: nucleotide sequence of chimera A-E57V-Fc (Eg1S N434A)
SEQ ID NO: 234: amino acid sequence of chimera A-E57V-Fc (Eg1S N434A)
SEQ ID NO: 235: nucleotide sequence of chimera A-E57K_R58D-Fc (Eg1S N434A)
SEQ ID NO: 236: amino acid sequence of chimera A-E57K_R58D-Fc (Eg1S N434A)
SEQ ID NO: 237: nucleotide sequence of chimera A-E57K_R58E-Fc (Eg1S N434A)
SEQ ID NO: 238: amino acid sequence of chimera A-E57K_R58E-Fc (Eg1S N434A)
SEQ ID NO: 239: nucleotide sequence of chimera A-E57R_R58D-Fc (Eg1S N434A)
SEQ ID NO: 240: amino acid sequence of chimera A-E57R_R58D-Fc (Eg1S N434A)
SEQ ID NO: 241: nucleotide sequence of chimera A-E57K-Fc (Eg1S LALAGA)
SEQ ID NO: 242: amino acid sequence of chimera A-E57K-Fc (Eg1S LALAGA)
SEQ ID NO: 243: nucleotide sequence of chimera A-E57L-Fc (Eg1S LALAGA)
SEQ ID NO: 244: amino acid sequence of chimera A-E57L-Fc (Eg1S LALAGA)
SEQ ID NO: 245: nucleotide sequence of chimera A-E57R-Fc (Eg1S LALAGA)
SEQ ID NO: 246: amino acid sequence of chimera A-E57R-Fc (Eg1S LALAGA)
SEQ ID NO: 247: nucleotide sequence of chimera A-E57V-Fc (Eg1S LALAGA)
SEQ ID NO: 248: amino acid sequence of chimera A-E57V-Fc (Eg1S LALAGA)
SEQ ID NO: 249: nucleotide sequence of chimera A-E57K_R58D-Fc (Eg1S LALAGA)
SEQ ID NO: 250: amino acid sequence of chimera A-E57K_R58D-Fc (Eg1S LALAGA)
SEQ ID NO: 251: nucleotide sequence of chimera A-E57K_R58E-Fc (Eg1S LALAGA)
SEQ ID NO: 252: amino acid sequence of chimera A-E57K_R58E-Fc (Eg1S LALAGA)
SEQ ID NO: 253: nucleotide sequence of chimera A-E57R_R58D-Fc (Eg1S LALAGA)
SEQ ID NO: 254: amino acid sequence of chimera A-E57R_R58D-Fc (Eg1S LALAGA)
SEQ ID NO: 255: nucleotide sequence of chimera A-E57K-Fc (Eg1S LALAGANA)
SEQ ID NO: 256: amino acid sequence of chimera A-E57K-Fc (Eg1S LALAGANA)
SEQ ID NO: 257: nucleotide sequence of chimera A-E57L-Fc (Eg1S LALAGANA)
SEQ ID NO: 258: amino acid sequence of chimera A-E57L-Fc (Eg1S LALAGANA)
SEQ ID NO: 259: nucleotide sequence of chimera A-E57R-Fc (Eg1S LALAGANA)
SEQ ID NO: 260: amino acid sequence of chimera A-E57R-Fc (Eg1S LALAGANA)
SEQ ID NO: 261: nucleotide sequence of chimera A-E57V-Fc (Eg1S LALAGANA)
SEQ ID NO: 262: amino acid sequence of chimera A-E57V-Fc (Eg1S LALAGANA)
SEQ ID NO: 263: nucleotide sequence of chimera A-E57K_R58D-Fc (Eg1S LALAGANA)
SEQ ID NO: 264: amino acid sequence of chimera A-E57K_R58D-Fc (Eg1S LALAGANA)
SEQ ID NO: 265: nucleotide sequence of chimera A-E57K_R58E-Fc (Eg1S LALAGANA)
SEQ ID NO: 266: amino acid sequence of chimera A-E57K_R58E-Fc (Eg1S LALAGANA)
SEQ ID NO: 267: nucleotide sequence of chimera A-E57R_R58D-Fc (Eg1S LALAGANA)
SEQ ID NO: 268: amino acid sequence of chimera A-E57R_R58D-Fc (Eg1S LALAGANA)
SEQ ID NO: 269: nucleotide sequence of chimera A-E57A
SEQ ID NO: 270: amino acid sequence of chimera A-E57A
SEQ ID NO: 271: nucleotide sequence of chimera A-E57F
SEQ ID NO: 272: amino acid sequence of chimera A-E57F
SEQ ID NO: 273: nucleotide sequence of chimera A-E57H
SEQ ID NO: 274: amino acid sequence of chimera A-E57H
SEQ ID NO: 275: nucleotide sequence of chimera A-E571
SEQ ID NO: 276: amino acid sequence of chimera A-E57I
SEQ ID NO: 277: nucleotide sequence of chimera A-E57M
SEQ ID NO: 278: amino acid sequence of chimera A-E57M
SEQ ID NO: 279: nucleotide sequence of chimera A-E57K_R58T
SEQ ID NO: 280: amino acid sequence of chimera A-E57K_R58T
SEQ ID NO: 281: nucleotide sequence of chimera A-E57L_R58E
SEQ ID NO: 282: amino acid sequence of chimera A-E57L_R58E
SEQ ID NO: 283: nucleotide sequence of chimera A-E57V_R58T
SEQ ID NO: 284: amino acid sequence of chimera A-E57V_R58T
SEQ ID NO: 285: nucleotide sequence of chimera A-E57V_R58E
SEQ ID NO: 286: amino acid sequence of chimera A-E57V_R58E
SEQ ID NO: 287: nucleotide sequence of chimera A-E57A-Fc (g4PEK)
SEQ ID NO: 288: amino acid sequence of chimera A-E57A-Fc (g4PEK)
SEQ ID NO: 289: nucleotide sequence of chimera A-E57F-Fc (g4PEK)
SEQ ID NO: 290: amino acid sequence of chimera A-E57F-Fc (g4PEK)
SEQ ID NO: 291: nucleotide sequence of chimera A-E57H-Fc (g4PEK)
SEQ ID NO: 292: amino acid sequence of chimera A-E57H-Fc (g4PEK)
SEQ ID NO: 293: nucleotide sequence of chimera A-E57I-Fc (g4PEK)
SEQ ID NO: 294: amino acid sequence of chimera A-E57I-Fc (g4PEK)
SEQ ID NO: 295: nucleotide sequence of chimera A-E57M-Fc (g4PEK)
SEQ ID NO: 296: amino acid sequence of chimera A-E57M-Fc (g4PEK)
SEQ ID NO: 297: nucleotide sequence of chimera A-E57K_R58T-Fc (g4PEK)
SEQ ID NO: 298: amino acid sequence of chimera A-E57K_R58T-Fc (g4PEK)
SEQ ID NO: 299: nucleotide sequence of chimera A-E57L_R58E-Fc (g4PEK)
SEQ ID NO: 300: amino acid sequence of chimera A-E57L_R58E-Fc (g4PEK)
SEQ ID NO: 301: nucleotide sequence of chimera A-E57V_R58T-Fc (g4PEK)
SEQ ID NO: 302: amino acid sequence of chimera A-E57V_R58T-Fc (g4PEK)
SEQ ID NO: 303: nucleotide sequence of chimera A-E57V_R58E-Fc (g4PEK)
SEQ ID NO: 304: amino acid sequence of chimera A-E57V_R58E-Fc (g4PEK)
SEQ ID NO: 305: nucleotide sequence of soluble recombinant FLAG tagged LIGHT
SEQ ID NO: 306: amino acid sequence of soluble recombinant FLAG tagged LIGHT
SEQ ID NO: 307: nucleotide sequence of TL1A variant in which FLAG tag has been inserted at N-terminus of TL1A
SEQ ID NO: 308: amino acid sequence of TL1A variant in which FLAG tag is inserted at N-terminus of TL1A
SEQ ID NO: 309: nucleotide sequence of FasL variant in which FLAG tag is inserted at N-terminus of FasL
SEQ ID NO: 310: amino acid sequence of FasL variant in which FLAG tag is inserted at N-terminus of FasL
SEQ ID NO: 311: amino acid sequence of Fc (g1S YTE)
SEQ ID NO: 312: amino acid sequence of Fc (g1S N434A)
SEQ ID NO: 313: amino acid sequence of Fc (g1S LALAGANA)
SEQ ID NO: 314: amino acid sequence of chimera A-Fc (g1S YTE)
SEQ ID NO: 315: amino acid sequence of chimera A-Fc (g1S N434A)
SEQ ID NO: 316: amino acid sequence of chimera A-Fc (g1S LALAGANA)
SEQ ID NO: 317: amino acid sequence of chimera A-E57K-Fc (g1S YTE)
SEQ ID NO: 318: amino acid sequence of chimera A-E57L-Fc (g1S YTE)
SEQ ID NO: 319: amino acid sequence of chimera A-E57R-Fc (g1S YTE)
SEQ ID NO: 320: amino acid sequence of chimera A-E57V-Fc (g1S YTE)
SEQ ID NO: 321: amino acid sequence of chimera A-E57K_R58D-Fc (g1S YTE)
SEQ ID NO: 322: amino acid sequence of chimera A-E57K_R58E-Fc (g1S YTE)
SEQ ID NO: 323: amino acid sequence of chimera A-E57R_R58D-Fc (g1S YTE)
SEQ ID NO: 324: amino acid sequence of chimera A-E57K-Fc (g1S N434A)
SEQ ID NO: 325: amino acid sequence of chimera A-E57L-Fc (g1S N434A)
SEQ ID NO: 326: amino acid sequence of chimera A-E57R-Fc (g1S N434A)
SEQ ID NO: 327: amino acid sequence of chimera A-E57V-Fc (g1S N434A)
SEQ ID NO: 328: amino acid sequence of chimera A-E57K_R58D-Fc (g1S N434A)
SEQ ID NO: 329: amino acid sequence of chimera A-E57K_R58E-Fc (g1S N434A)
SEQ ID NO: 330: amino acid sequence of chimera A-E57R_R58D-Fc (g15 N434A)
SEQ ID NO: 331: amino acid sequence of chimera A-E57K-Fc (g1S LALAGANA)
SEQ ID NO: 332: amino acid sequence of chimera A-E57L-Fc (g1S LALAGANA)
SEQ ID NO: 333: amino acid sequence of chimera A-E57R-Fc (g1S LALAGANA)
SEQ ID NO: 334: amino acid sequence of chimera A-E57V-Fc (g1S LALAGANA)
SEQ ID NO: 335: amino acid sequence of chimera A-E57K_R58D-Fc (g1S LALAGANA)
SEQ ID NO: 336: amino acid sequence of chimera A-E57K_R58E-Fc (g1S LALAGANA)
SEQ ID NO: 337: amino acid sequence of chimera A-E57R_R58D-Fc (g1S LALAGANA)
SEQ ID NO: 338: nucleotide sequence of Fc (IEGRMD g1S)
SEQ ID NO: 339: amino acid sequence of Fc (IEGRMD g15)
SEQ ID NO: 340: amino acid sequence of R218Q-Fc
SEQ ID NO: 341: nucleotide sequence of FLAG-cynoLIGHT
SEQ ID NO: 342: amino acid sequence of FLAG-cynoLIGHT
SEQ ID NO: 343: nucleotide sequence of FasL variant in which cleavage site and intracellular proline-rich region are deleted from FasL as human DcR3 ligand and a FLAG tag is inserted at N-terminus
SEQ ID NO: 344: amino acid sequence of FasL variant in which cleavage site and intracellular proline-rich region are deleted from FasL as human DcR3 ligand and a FLAG tag is inserted at N-terminus
SEQ ID NO: 345: nucleotide sequence of S195-His6
SEQ ID NO: 346: amino acid sequence of S195-His6
SEQ ID NO: 347: nucleotide sequence of chimera A-His6
SEQ ID NO: 348: amino acid sequence of chimera A-His6
SEQ ID NO: 349: nucleotide sequence of chimera A-E57K-His6
SEQ ID NO: 350: amino acid sequence of chimera A-E57K-His6
SEQ ID NO: 351: nucleotide sequence of chimera A-E57K_R58E-His6
SEQ ID NO: 352: amino acid sequence of chimera A-E57K_R58E-His6
SEQ ID NO: 353: optimized nucleotide sequence of chimera A
SEQ ID NO: 354: optimized nucleotide sequence of chimera A-E57K
SEQ ID NO: 355: optimized nucleotide sequence of chimera A-E57K_R58E

## Claims

1. A DcR3 variant comprising a first chimeric cysteine-rich region or a second chimeric cysteine-rich region, wherein:
the first chimeric cysteine-rich region consists of any one of the following amino acid sequences:
(a) an amino acid sequence consisting of amino acids at positions 1 to 164 from the N-terminus of the amino acid sequence set forth in SEQ ID NO: 26 or 50;
(b) an amino acid sequence consisting of amino acids at positions 1 to 164 from the N-terminus of the amino acid sequence set forth in SEQ ID NO: 28 or 52;
(c) an amino acid sequence consisting of amino acids at positions 1 to 164 from the N-terminus of the amino acid sequence set forth in SEQ ID NO: 30 or 54; and
(d) an amino acid sequence consisting of amino acids at positions 1 to 164 from the N-terminus of the amino acid sequence set forth in SEQ ID NO: 32 or 56; and
the second chimeric cysteine-rich region consists of an amino acid sequence (e) obtained by introducing into the amino acid sequence of the first chimeric cysteine-rich region, deletion, substitution, insertion or addition of 1 to 5 amino acids.

2. The DcR3 variant according to claim 1, wherein the DcR3 variant comprises one or more complex N-glycoside-linked glycans.

3. The DcR3 variant according to claim 1 or 2, wherein the DcR3 variant has neutralizing activity to at least one or more of LIGHT, TL1A and FasL.

4. The DcR3 variant according to any one of claims 1 to 3, wherein the DcR3 variant has neutralizing activity to all of LIGHT, TL1A and FasL.

5. The DcR3 variant according to any one of claims 1 to 3, wherein the DcR3 variant has no neutralizing activity to FasL and has neutralizing activity to one or more of LIGHT and TL1A.

6. The DcR3 variant according to any one of claims 1 to 3 and 5, wherein the DcR3 variant has no neutralizing activity to FasL and has neutralizing activity to both of LIGHT and TL1A.

7. The DcR3 variant according to any one of claims 1 to 6, wherein the first cysteine rich region consists of an amino acid sequence consisting of amino acids at positions 1 to 164 from the N-terminus of the amino acid sequence set forth in SEQ ID NO: 54.

8. The DcR3 variant according to any one of claims 1 to 7, wherein the amino acid sequence (e) comprises one or two or more substitution selected from the group consisting of:
substitution of Glu at position 57 from the N-terminus of the amino acid sequence (a), (b), (c) or (d) with another amino acid;
substitution of Arg at position 58 from the N-terminus of the amino acid sequence (a), (b), (c) or (d) with another amino acid; and
substitution of Arg at position 60 from the N-terminus of the amino acid sequence (a), (b), (c) or (d) with another amino acid.

9. The DcR3 variant according to any one of claims 1 to 8, wherein the amino acid sequence (e) comprises substitution of Glu at position 57 and Arg at position 58 from the N-terminus of the amino acid sequence (a), (b), (c) or (d) with other amino acids.

10. The DcR3 variant according to any one of claims 1 to 9, wherein the amino acid sequence (e) comprises one or two or more substitution selected from the group consisting of:
substitution of Glu at position 57 from the N-terminus of the amino acid sequence (a), (b), (c) or (d) with Lys, Leu, Arg, Val, Ala, Phe, His, Ile or Met;
substitution of Arg at position 58 from the N-terminus of the amino acid sequence (a), (b), (c) or (d) with Asp, Glu or Thr; and
substitution of Arg at position 60 from the N-terminus of the amino acid sequence (a), (b), (c) or (d) with Lys.

11. The DcR3 variant according to any one of claims 1 to 10, wherein the amino acid sequence (e) comprises substitution of Glu at position 57 from the N-terminus of the amino acid sequence (a), (b), (c) or (d) with Lys, Leu, Arg, Val, Ala, Phe, His, Ile or Met, and substitution of Arg at position 58 from the N-terminus of the amino acid sequence (a), (b), (c) or (d) with Asp, Glu or Thr.

12. The DcR3 variant according to any one of claims 1 to 11, wherein the amino acid sequence (e) comprises substitution selected from the following (f) to (i):
(f) substitution of Asn at positions 131 and 144 from the N-terminus of the amino acid sequence (b), (c) or (d) with other amino acids;
(g) substitution of Asn at positions 131, 144 and 157 from the N-terminus of the amino acid sequence (b), (c) or (d) with other amino acids;
(h) substitution of Thr at position 133 and Ser at position 146 from the N-terminus of the amino acid sequence (b), (c) or (d) with other amino acids; and
(i) substitution of Thr at position 133, Ser at position 146 and Thr at position 159 from the N-terminus of the amino acid sequence (b), (c) or (d) with other amino acids.

13. The DcR3 variant according to any one of claims 1 to 12, wherein the amino acid sequence (e) comprises substitution selected from the following (f') to (i'):
(f') substitution of Asn at positions 131 and 144 from the N-terminus of the amino acid sequence (b), (c) or (d) with Ser;
(g') substitution of Asn at positions 131, 144 and 157 from the N-terminus of the amino acid sequence (b), (c) or (d) with Ser;
(h') substitution of Thr at position 133 and Ser at position 146 from the N-terminus of the amino acid sequence (b), (c) or (d) with Ala; and
(i') substitution of Thr at position 133, Ser at position 146 and Thr at position 159 from the N-terminus of the amino acid sequence (b), (c) or (d) with Ala.

14. The DcR3 variant according to any one of claims 1 to 13, wherein the amino acid sequence (e) is an amino acid sequence consisting of amino acids at positions 1 to 164 from the N-terminus of an amino acid sequence set forth in SEQ ID NO: 58, 60, 62, 64, 66, 68, 70, 180, 182, 184, 186, 188, 270, 272, 274, 276, 278, 280, 282, 284 or 286.

15. The DcR3 variant according to any one of claims 1 to 14, wherein the DcR3 variant is a DcR3 variant comprising the first or second chimeric cysteine-rich region, and a part or a whole of a heparan sulfate-binding domain of the wild-type DcR3 bound to the C-terminal side of the first or second chimeric cysteine-rich region.

16. The DcR3 variant according to any one of claims 1 to 14, wherein the DcR3 variant is a DcR3 variant comprising the first or second chimeric cysteine-rich region and not comprising a heparan sulfate-binding domain of the wild-type DcR3.

17. The DcR3 variant according to claim 15 or 16, wherein the DcR3 variant comprises one of amino acid sequences selected from:
(I) an amino acid sequence set forth in SEQ ID NO: 26, 28, 30, 32, 34, 36, 38, 40, 42, 44 or 46, or an amino acid sequence having 99% or more identity to the abovementioned amino acid sequence; and
(II) an amino acid sequence set forth in SEQ ID NO: 50, 52, 54, 56, 58, 60, 62, 64, 66, 68, 70, 180, 182, 184, 186, 188, 270, 272, 274, 276, 278, 280, 282, 284 or 286, or an amino acid sequence having 99% or more identity to the abovementioned amino acid sequence.

18. The DcR3 variant according to any one of claims 1 to 17, wherein the DcR3 variant comprises an amino acid sequence set forth in SEQ ID NO: 66 or 186.

19. The DcR3 variant according to any one of claims 1 to 18, wherein the DcR3 variant comprises an Fc region derived from a human IgG1, IgG2 or IgG4 antibody, or a mutated Fc region consisting of an amino acid sequence obtained by introducing into an amino acid sequence of the abovementioned Fc region, deletion, substitution, insertion or addition of one or several amino acids.

20. The DcR3 variant according to claim 19, wherein the Fc region or the mutated Fc region is bound to the C-terminal side of the first or second chimeric cysteine-rich region via another region or a linker.

21. The DcR3 variant according to claim 19 or 20, wherein the mutated Fc region comprises substitution of Cys with Ser at EU-index position 220 of an amino acid sequence of a heavy chain of human IgG1.

22. The DcR3 variant according to claim 21, wherein the mutated Fc region comprises substitution of Leu with Ala at EU-index position 234, substitution of Leu with Ala at EU-index position 235, and substitution of Gly with Ala at EU-index position 237 of an amino acid sequence of a heavy chain of human IgG1.

23. The DcR3 variant according to claim 21 or 22, wherein the mutated Fc region comprises substitution of Asn with Ala at EU-index position 434 of an amino acid sequence of a heavy chain of human IgG1.

24. The DcR3 variant according to claim 21, wherein the mutated Fc region comprises substitution of Met with Tyr at EU-index position 252, substitution of Ser with Thr at EU-index position 254, and substitution of Thr with Glu at EU-index position 256 of an amino acid sequence of a heavy chain of human IgG1.

25. The DcR3 variant according to claim 19 or 20, wherein the mutated Fc region comprises substitution of Ser with Pro at EU-index position 228, substitution of Leu with Glu at EU-index position 235, and substitution of Arg with Lys at EU-index position 409 of an amino acid sequence of a heavy chain of human IgG4.

26. The DcR3 variant according to claim 19 or 20, wherein the DcR3 variant comprises a mutated Fc region consisting of an amino acid sequence set forth in SEQ ID NO: 72, 74, 156, 158, 160, 162, 164, 166, 311, 312 or 313, or an amino acid sequence having 90% or more identity to an amino acid sequence set forth in SEQ ID NO: 72, 74, 156, 158, 160, 162, 164, 166, 311, 312 or 313.

27. The DcR3 variant according to claim 19, wherein the DcR3 variant comprises or consists of an amino acid sequence set forth in SEQ ID NO: 76, 78, 80, 82, 84, 86, 88, 90, 92, 94, 96, 98, 150, 168, 170, 172, 174, 176, 178, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 288, 290, 292, 294, 296, 298, 300, 302, 304, 314, 315, 316, 317, 318, 319, 320, 321, 322, 323, 324, 325, 326, 327, 328, 329, 330, 331, 332, 333, 334, 335, 336 or 337, or an amino acid sequence having 99% or more identity to the abovementioned amino acid sequences.

28. The DcR3 variant according to claim 19, wherein the DcR3 variant comprises or consists of an amino acid sequence obtained by introducing substitution, deletion, insertion or addition of 1 amino acid into an amino acid sequence set forth in SEQ ID NO: 76, 78, 80, 82, 84, 86, 88, 90, 92, 94, 96, 98, 150, 168, 170, 172, 174, 176, 178, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 288, 290, 292, 294, 296, 298, 300, 302, 304, 314, 315, 316, 317, 318, 319, 320, 321, 322, 323, 324, 325, 326, 327, 328, 329, 330, 331, 332, 333, 334, 335, 336 or 337.

29. The DcR3 variant according to claim 27, wherein the DcR3 variant comprises an amino acid sequence set forth in SEQ ID NO: 214, or an amino acid sequence having 99% or more identity to SEQ ID NO: 214.

30. The DcR3 variant according to claim 27, wherein the DcR3 variant comprises an amino acid sequence set forth in SEQ ID NO: 224, or an amino acid sequence having 99% or more identity to SEQ ID NO: 224.

31. The DcR3 variant according to claim 27, wherein the DcR3 variant comprises an amino acid sequence set forth in SEQ ID NO: 256, or an amino acid sequence having 99% or more identity to SEQ ID NO: 256.

32. The DcR3 variant according to claim 27, wherein the DcR3 variant comprises an amino acid sequence set forth in SEQ ID NO: 266, or an amino acid sequence having 99% or more identity to SEQ ID NO: 266.

33. The DcR3 variant according to claim 27, wherein the DcR3 variant consists of an amino acid sequence set forth in SEQ ID NO: 214, or an amino acid sequence having 99% or more identity to SEQ ID NO: 214.

34. The DcR3 variant according to claim 27, wherein the DcR3 variant consists of an amino acid sequence set forth in SEQ ID NO: 224, or an amino acid sequence having 99% or more identity to SEQ ID NO: 224.

35. The DcR3 variant according to claim 27, wherein the DcR3 variant consists of an amino acid sequence set forth in SEQ ID NO: 256, or an amino acid sequence having 99% or more identity to SEQ ID NO: 256.

36. The DcR3 variant according to claim 27, wherein the DcR3 variant consists of an amino acid sequence set forth in SEQ ID NO: 266, or an amino acid sequence having 99% or more identity to SEQ ID NO: 266.

37. A DNA encoding the DcR3 variant according to any one of the preceding claims.

38. A vector comprising the DNA according to claim 37.

39. A transformant obtained by introducing the vector according to claim 38 into a host cell.

40. The transformant according to claim 39, wherein the host cell is a cell derived from a mammal.

41. The transformant according to claim 40, wherein the cell derived from a mammal is a CHO cell.

42. A method of producing a DcR3 variant, wherein the method comprises culturing the transformant according to any one of claims 39 to 41 in a medium to generate and accumulate the DcR3 variant, and purifying the DcR3 variant from the obtained culture solution.

43. A DcR3 variant produced using the production method according to claim 42.

44. A pharmaceutical composition comprising the DcR3 variant according to any one of claims 1 to 36 and 43 as an active ingredient.

45. The pharmaceutical composition according to claim 44, wherein the pharmaceutical composition is a prophylactic or therapeutic agent for an autoimmune disease, an inflammatory disease or an allergic disease.

46. The pharmaceutical composition according to claim 44 for use in a method of preventing or treating an autoimmune disease, an inflammatory disease or an allergic disease.

47. The pharmaceutical composition according to claim 44 for use in the treatment of a condition or disease selected from the group consisting of inflammatory bowel disease (IBD), systemic lupus erythematosus, psoriasis, chronic graft versus host disease, acute graft versus host disease, Crohn's disease, ulcerative colitis, multiple sclerosis, celiac disease, idiopathic thrombotic thrombocytopenic purpura, myasthenia gravis, Sjogren's syndrome, scleroderma, asthma, uveitis, epidermal hyperplasia, alopecia areata, Behcet's disease, Takayasu's arteritis, cartilaginous inflammation, bone degradation, arthritis, juvenile arthritis, juvenile rheumatoid arthritis, pauciarticular juvenile rheumatoid arthritis, polyarticular juvenile rheumatoid arthritis, systemic onset juvenile rheumatoid arthritis, juvenile ankylosing spondylitis, juvenile enteropathic arthritis, juvenile reactive arthritis, juvenile Reiter's syndrome, seronegative enthesopathy and arthropathy syndrome (SEA syndrome), juvenile dermatomyositis, juvenile psoriatic arthritis, juvenile scleroderma, juvenile systemic lupus erythematosus, juvenile vasculitis, pauciarticular rheumatoid arthritis, polyarticular rheumatoid arthritis, systemic onset rheumatoid arthritis, ankylosing spondylitis, enteropathic arthritis, reactive arthritis, Reiter's syndrome, dermatomyositis, psoriatic arthritis, vasculitis, myositis, polymyositis, dermatomyositis, osteoarthritis, polyarteritis nodosa, Wegener's granulomatosis, arteritis, polymyalgia rheumatica, sarcoidosis, sclerosis, primary biliary cirrhosis, sclerosing cholangitis, dermatitis, atopic dermatitis, atherosclerosis, Still's disease, chronic obstructive pulmonary disease, Guillain-Barre syndrome, type 1 diabetes mellitus, Graves' disease, Addison's disease, Raynaud's phenomenon, autoimmune hepatitis and Wiskott-Aldrich syndrome.

48. The pharmaceutical composition according to claim 47 for use in the treatment of inflammatory bowel disease, Crohn's disease or ulcerative colitis.

49. The pharmaceutical composition according to claim 47 for use in the treatment of sclerosis, multiple sclerosis, atherosclerosis or scleroderma.

50. The pharmaceutical composition according to claim 47 for use in the treatment of psoriasis or lupus erythematosus.

51. The pharmaceutical composition according to claim 44 for use as a medicament.

## Patentansprüche

1. DcR3-Variante, umfassend eine erste chimäre cysteinreiche Region oder eine zweite chimäre cysteinreiche Region, wobei:
die erste chimäre cysteinreiche Region aus irgendeiner der folgenden Aminosäuresequenzen besteht:
(a) einer Aminosäuresequenz, bestehend aus Aminosäuren an den Positionen 1 bis 164 vom N-Terminus der in SEQ ID NO: 26 oder 50 ausgeführten Aminosäuresequenz;
(b) einer Aminosäuresequenz, bestehend aus Aminosäuren an den Positionen 1 bis 164 vom N-Terminus der in SEQ ID NO: 28 oder 52 ausgeführten Aminosäuresequenz;
(c) einer Aminosäuresequenz, bestehend aus Aminosäuren an den Positionen 1 bis 164 vom N-Terminus der in SEQ ID NO: 30 oder 54 ausgeführten Aminosäuresequenz; und
(d) einer Aminosäuresequenz, bestehend aus Aminosäuren an den Positionen 1 bis 164 vom N-Terminus der in SEQ ID NO: 32 oder 56 ausgeführten Aminosäuresequenz; und
die zweite chimäre cysteinreiche Region aus einer Aminosäuresequenz (e) besteht, erhalten durch Einbringen von Deletion, Substitution, Insertion oder Addition von 1 bis 5 Aminosäuren in die Aminosäuresequenz der ersten chimären cysteinreichen Region.

2. DcR3-Variante gemäß Anspruch 1, wobei die DcR3-Variante ein oder mehrere komplexe N-Glycosid-verknüpfte Glycane umfasst.

3. DcR3-Variante gemäß Anspruch 1 oder 2, wobei die DcR3-Variante neutralisierende Aktivität auf wenigstens eines oder mehrere von LIGHT, TL1A und FasL aufweist.

4. DcR3-Variante gemäß irgendeinem der Ansprüche 1 bis 3, wobei die DcR3-Variante neutralisierende Aktivität auf alle von LIGHT, TL1A und FasL aufweist.

5. DcR3-Variante gemäß irgendeinem der Ansprüche 1 bis 3, wobei die DcR3-Variante keine neutralisierende Aktivität auf FasL aufweist und neutralisierende Aktivität auf eines oder mehrere von LIGHT und TL1A aufweist.

6. DcR3-Variante gemäß irgendeinem der Ansprüche 1 bis 3 und 5, wobei die DcR3-Variante keine neutralisierende Aktivität auf FasL aufweist und neutralisierende Aktivität auf beide von LIGHT und TL1A aufweist.

7. DcR3-Variante gemäß irgendeinem der Ansprüche 1 bis 6, wobei die erste cysteinreiche Region aus einer Aminosäuresequenz, bestehend aus Aminosäuren an den Positionen 1 bis 164 vom N-Terminus der in SEQ ID NO: 54 ausgeführten Aminosäuresequenz, besteht.

8. DcR3-Variante gemäß irgendeinem der Ansprüche 1 bis 7, wobei die Aminosäuresequenz (e) eine oder zwei oder mehr Substitutionen umfasst, ausgewählt aus der Gruppe, bestehend aus:
Substitution von Glu an Position 57 vom N-Terminus der Aminosäuresequenz (a), (b), (c) oder (d) mit einer anderen Aminosäure;
Substitution von Arg an Position 58 vom N-Terminus der Aminosäuresequenz (a), (b), (c) oder (d) mit einer anderen Aminosäure; und
Substitution von Arg an Position 60 vom N-Terminus der Aminosäuresequenz (a), (b), (c) oder (d) mit einer anderen Aminosäure.

9. DcR3-Variante gemäß irgendeinem der Ansprüche 1 bis 8, wobei die Aminosäuresequenz (e) Substitution von Glu an Position 57 und Arg an Position 58 vom N-Terminus der Aminosäuresequenz (a), (b), (c) oder (d) mit anderen Aminosäuren umfasst.

10. DcR3-Variante gemäß irgendeinem der Ansprüche 1 bis 9, wobei die Aminosäuresequenz (e) eine oder zwei oder mehr Substitutionen umfasst, ausgewählt aus der Gruppe, bestehend aus:
Substitution von Glu an Position 57 vom N-Terminus der Aminosäuresequenz (a), (b), (c) oder (d) mit Lys, Leu, Arg, Val, Ala, Phe, His, Ile oder Met;
Substitution von Arg an Position 58 vom N-Terminus der Aminosäuresequenz (a), (b), (c) oder (d) mit Asp, Glu oder Thr; und
Substitution von Arg an Position 60 vom N-Terminus der Aminosäuresequenz (a), (b), (c) oder (d) mit Lys.

11. DcR3-Variante gemäß irgendeinem der Ansprüche 1 bis 10, wobei die Aminosäuresequenz (e) Substitution von Glu an Position 57 vom N-Terminus der Aminosäuresequenz (a), (b), (c) oder (d) mit Lys, Leu, Arg, Val, Ala, Phe, His, Ile oder Met und Substitution von Arg an Position 58 vom N-Terminus der Aminosäuresequenz (a), (b), (c) oder (d) mit Asp, Glu oder Thr umfasst.

12. DcR3-Variante gemäß irgendeinem der Ansprüche 1 bis 11, wobei die Aminosäuresequenz (e) Substitution umfasst, ausgewählt aus den folgenden (f) bis (i):
(f) Substitution von Asn an den Positionen 131 und 144 vom N-Terminus der Aminosäuresequenz (b), (c) oder (d) mit anderen Aminosäuren;
(g) Substitution von Asn an den Positionen 131, 144 und 157 vom N-Terminus der Aminosäuresequenz (b), (c) oder (d) mit anderen Aminosäuren;
(h) Substitution von Thr an Position 133 und Ser an Position 146 vom N-Terminus der Aminosäuresequenz (b), (c) oder (d) mit anderen Aminosäuren; und
(i) Substitution von Thr an Position 133, Ser an Position 146 und Thr an Position 159 vom N-Terminus der Aminosäuresequenz (b), (c) oder (d) mit anderen Aminosäuren.

13. DcR3-Variante gemäß irgendeinem der Ansprüche 1 bis 12, wobei die Aminosäuresequenz (e) Substitution umfasst, ausgewählt aus den folgenden (f') bis (i'):
(f') Substitution von Asn an den Positionen 131 und 144 vom N-Terminus der Aminosäuresequenz (b), (c) oder (d) mit Ser;
(g') Substitution von Asn an den Positionen 131, 144 und 157 vom N-Terminus der Aminosäuresequenz (b), (c) oder (d) mit Ser;
(h') Substitution von Thr an Position 133 und Ser an Position 146 vom N-Terminus der Aminosäuresequenz (b), (c) oder (d) mit Ala; und
(i') Substitution von Thr an Position 133, Ser an Position 146 und Thr an Position 159 vom N-Terminus der Aminosäuresequenz (b), (c) oder (d) mit Ala.

14. DcR3-Variante gemäß irgendeinem der Ansprüche 1 bis 13, wobei die Aminosäuresequenz (e) eine Aminosäuresequenz ist, bestehend aus Aminosäuren an den Positionen 1 bis 164 vom N-Terminus einer in SEQ ID NO: 58, 60, 62, 64, 66, 68, 70, 180, 182, 184, 186, 188, 270, 272, 274, 276, 278, 280, 282, 284 oder 286 ausgeführten Aminosäuresequenz.

15. DcR3-Variante gemäß irgendeinem der Ansprüche 1 bis 14, wobei die DcR3-Variante eine DcR3-Variante ist, umfassend die erste oder zweite chimäre cysteinreiche Region und einen Teil einer oder eine ganze Heparansulfat-Bindungsdomäne des Wildtyp-DcR3, gebunden an die C-terminale Seite der ersten oder zweiten chimären cysteinreichen Region.

16. DcR3-Variante gemäß irgendeinem der Ansprüche 1 bis 14, wobei die DcR3-Variante eine DcR3-Variante ist, welche die erste oder zweite chimäre cysteinreiche Region umfasst und keine Heparansulfat-Bindungsdomäne des Wildtyp-DcR3 umfasst.

17. DcR3-Variante gemäß Anspruch 15 oder 16, wobei die DcR3-Variante eine der Aminosäuresequenzen umfasst, ausgewählt aus:
(I) einer in SEQ ID NO: 26, 28, 30, 32, 34, 36, 38, 40, 42, 44 oder 46 ausgeführten Aminosäuresequenz oder einer Aminosäuresequenz mit 99% oder mehr Identität zu der vorgenannten Aminosäuresequenz; und
(II) einer in SEQ ID NO: 50, 52, 54, 56, 58, 60, 62, 64, 66, 68, 70, 180, 182, 184, 186, 188, 270, 272, 274, 276, 278, 280, 282, 284 oder 286 ausgeführten Aminosäuresequenz oder einer Aminosäuresequenz mit 99% oder mehr Identität zu der vorgenannten Aminosäuresequenz.

18. DcR3-Variante gemäß irgendeinem der Ansprüche 1 bis 17, wobei die DcR3-Variante eine in SEQ ID NO: 66 oder 186 ausgeführte Aminosäuresequenz umfasst.

19. DcR3-Variante gemäß irgendeinem der Ansprüche 1 bis 18, wobei die DcR3-Variante eine Fc-Region, abgeleitet von einem humanen IgG1-, IgG2- oder IgG4-Antikörper, oder eine mutierte Fc-Region, bestehend aus einer Aminosäuresequenz, erhalten durch Einbringen von Deletion, Substitution, Insertion oder Addition einer oder mehrerer Aminosäuren in eine Aminosäuresequenz der vorgenannten Fc-Region, umfasst.

20. DcR3-Variante gemäß Anspruch 19, wobei die Fc-Region oder die mutierte Fc-Region über eine andere Region oder einen Linker an die C-terminale Seite der ersten oder zweiten chimären cysteinreichen Region gebunden ist.

21. DcR3-Variante gemäß Anspruch 19 oder 20, wobei die mutierte Fc-Region Substitution von Cys mit Ser an EU-Index-Position 220 einer Aminosäuresequenz einer schweren Kette von humanem IgG1 umfasst.

22. DcR3-Variante gemäß Anspruch 21, wobei die mutierte Fc-Region Substitution von Leu mit Ala an EU-Index-Position 234, Substitution von Leu mit Ala an EU-Index-Position 235 und Substitution von Gly mit Ala an EU-Index-Position 237 einer Aminosäuresequenz einer schweren Kette von humanem IgG1 umfasst.

23. DcR3-Variante gemäß Anspruch 21 oder 22, wobei die mutierte Fc-Region Substitution von Asn mit Ala an EU-Index-Position 434 einer Aminosäuresequenz einer schweren Kette von humanem IgG1 umfasst.

24. DcR3-Variante gemäß Anspruch 21, wobei die mutierte Fc-Region Substitution von Met mit Tyr an EU-Index-Position 252, Substitution von Ser mit Thr an EU-Index-Position 254 und Substitution von Thr mit Glu an EU-Index-Position 256 einer Aminosäuresequenz einer schweren Kette von humanem IgG1 umfasst.

25. DcR3-Variante gemäß Anspruch 19 oder 20, wobei die mutierte Fc-Region Substitution von Ser mit Pro an EU-Index-Position 228, Substitution von Leu mit Glu an EU-Index-Position 235 und Substitution von Arg mit Lys an EU-Index-Position 409 einer Aminosäuresequenz einer schweren Kette von humanem IgG4 umfasst.

26. DcR3-Variante gemäß Anspruch 19 oder 20, wobei die DcR3-Variante eine mutierte Fc-Region, bestehend aus einer in SEQ ID NO: 72, 74, 156, 158, 160, 162, 164, 166, 311, 312 oder 313 ausgeführten Aminosäuresequenz oder einer Aminosäuresequenz mit 90% oder mehr Identität zu einer in SEQ ID NO: 72, 74, 156, 158, 160, 162, 164, 166, 311, 312 oder 313 ausgeführten Aminosäuresequenz umfasst.

27. DcR3-Variante gemäß Anspruch 19, wobei die DcR3-Variante eine in SEQ ID NO: 76, 78, 80, 82, 84, 86, 88, 90, 92, 94, 96, 98, 150, 168, 170, 172, 174, 176, 178, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 288, 290, 292, 294, 296, 298, 300, 302, 304, 314, 315, 316, 317, 318, 319, 320, 321, 322, 323, 324, 325, 326, 327, 328, 329, 330, 331, 332, 333, 334, 335, 336 oder 337 ausgeführte Aminosäuresequenz oder eine Aminosäuresequenz mit 99% oder mehr Identität zu den vorgenannten Aminosäuresequenzen umfasst oder daraus besteht.

28. DcR3-Variante gemäß Anspruch 19, wobei die DcR3-Variante eine Aminosäuresequenz, erhalten durch Einbringen von Substitution, Deletion, Insertion oder Addition von 1 Aminosäure in eine in SEQ ID NO: 76, 78, 80, 82, 84, 86, 88, 90, 92, 94, 96, 98, 150, 168, 170, 172, 174, 176, 178, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 288, 290, 292, 294, 296, 298, 300, 302, 304, 314, 315, 316, 317, 318, 319, 320, 321, 322, 323, 324, 325, 326, 327, 328, 329, 330, 331, 332, 333, 334, 335, 336 oder 337 ausgeführte Aminosäuresequenz, umfasst oder daraus besteht.

29. DcR3-Variante gemäß Anspruch 27, wobei die DcR3-Variante eine in SEQ ID NO: 214 ausgeführte Aminosäuresequenz oder eine Aminosäuresequenz mit 99% oder mehr Identität zu SEQ ID NO: 214 umfasst.

30. DcR3-Variante gemäß Anspruch 27, wobei die DcR3-Variante eine in SEQ ID NO: 224 ausgeführte Aminosäuresequenz oder eine Aminosäuresequenz mit 99% oder mehr Identität zu SEQ ID NO: 224 umfasst.

31. DcR3-Variante gemäß Anspruch 27, wobei die DcR3-Variante eine in SEQ ID NO: 256 ausgeführte Aminosäuresequenz oder eine Aminosäuresequenz mit 99% oder mehr Identität zu SEQ ID NO: 256 umfasst.

32. DcR3-Variante gemäß Anspruch 27, wobei die DcR3-Variante eine in SEQ ID NO: 266 ausgeführte Aminosäuresequenz oder eine Aminosäuresequenz mit 99% oder mehr Identität zu SEQ ID NO: 266 umfasst.

33. DcR3-Variante gemäß Anspruch 27, wobei die DcR3-Variante aus einer in SEQ ID NO: 214 ausgeführten Aminosäuresequenz oder einer Aminosäuresequenz mit 99% oder mehr Identität zu SEQ ID NO: 214 besteht.

34. DcR3-Variante gemäß Anspruch 27, wobei die DcR3-Variante aus einer in SEQ ID NO: 224 ausgeführten Aminosäuresequenz oder einer Aminosäuresequenz mit 99% oder mehr Identität zu SEQ ID NO: 224 besteht.

35. DcR3-Variante gemäß Anspruch 27, wobei die DcR3-Variante aus einer in SEQ ID NO: 256 ausgeführten Aminosäuresequenz oder einer Aminosäuresequenz mit 99% oder mehr Identität zu SEQ ID NO: 256 besteht.

36. DcR3-Variante gemäß Anspruch 27, wobei die DcR3-Variante aus einer in SEQ ID NO: 266 ausgeführten Aminosäuresequenz oder einer Aminosäuresequenz mit 99% oder mehr Identität zu SEQ ID NO: 266 besteht.

37. DNA, welche die DcR3-Variante gemäß irgendeinem der vorstehenden Ansprüche codiert.

38. Vektor, welcher die DNA gemäß Anspruch 37 umfasst.

39. Transformante, erhalten durch Einbringen des Vektors gemäß Anspruch 38 in eine Wirtszelle.

40. Transformante gemäß Anspruch 39, wobei die Wirtszelle eine von einem Säugetier abgeleitete Zelle ist.

41. Transformante gemäß Anspruch 40, wobei die von einem Säugetier abgeleitete Zelle eine CHO-Zelle ist.

42. Verfahren zum Produzieren einer DcR3-Variante, wobei das Verfahren das Kultivieren der Transformante gemäß irgendeinem der Ansprüche 39 bis 41 in einem Medium, um die DcR3-Variante zu erzeugen und zu akkumulieren, und das Aufreinigen der DcR3-Variante aus der erhaltenen Kulturlösung umfasst.

43. DcR3-Variante, produziert unter Verwendung des Produktionsverfahrens gemäß Anspruch 42.

44. Pharmazeutische Zusammensetzung, umfassend die DcR3-Variante gemäß irgendeinem der Ansprüche 1 bis 36 und 43 als aktiven Inhaltsstoff.

45. Pharmazeutische Zusammensetzung gemäß Anspruch 44, wobei die pharmazeutische Zusammensetzung ein prophylaktisches oder therapeutisches Mittel für eine Autoimmunerkrankung, eine Entzündungserkrankung oder eine allergische Erkrankung ist.

46. Pharmazeutische Zusammensetzung gemäß Anspruch 44 zur Verwendung in einem Verfahren zur Prävention oder Behandlung einer Autoimmunerkrankung, einer Entzündungserkrankung oder einer allergischen Erkrankung.

47. Pharmazeutische Zusammensetzung gemäß Anspruch 44 zur Verwendung bei der Behandlung eines Zustands oder einer Erkrankung, ausgewählt aus der Gruppe, bestehend aus entzündlicher Darmerkrankung (inflammatory bowel disease; IBD), systemischem Lupus erythematodes, Psoriasis, chronischer Graft-versus-Host-Erkrankung, akuter Graft-versus-Host-Erkrankung, Morbus Crohn, ulzerativer Colitis, Multipler Sklerose, Zöliakie, idiopathischer thrombotischer thrombozytopenischer Purpura, Myasthenia gravis, Sjögren-Syndrom, Sklerodermie, Asthma, Uveitis, epidermaler Hyperplasie, Alopecia areata, Beh 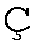 et-Krankheit, Takayasu-Arteriitis, Knorpelentzündung, Knochenabbau, Arthritis, juveniler Arthritis, juveniler rheumatoider Arthritis, pauciartikulärer juveniler rheumatoider Arthritis, polyartikulärer juveniler rheumatoider Arthritis, systemisch beginnender juveniler rheumatoider Arthritis (systemic onset juvenile rheumatoid arthritis), juveniler ankylosierender Spondylitis, juveniler enteropathischer Arthritis, juveniler reaktiver Arthritis, juvenilem Reiter-Syndrom, seronegative-Enthesiopathie-und-Arthropathie-Syndrom (SEA-Syndrom), juveniler Dermatomyositis, juveniler psoriatischer Arthritis, juveniler Sklerodermie, juvenilem systemischem Lupus erythematodes, juveniler Vaskulitis, pauciartikulärer rheumatoider Arthritis, polyartikulärer rheumatoider Arthritis, systemisch beginnender rheumatoider Arthritis, ankylosierender Spondylitis, enteropathischer Arthritis, reaktiver Arthritis, Reiter-Syndrom, Dermatomyositis, psoriatischer Arthritis, Vaskulitis, Myositis, Polymyositis, Dermatomyositis, Osteoarthritis, Polyarteriitis nodosa, Wegener-Granulomatose, Arteriitis, Polymyalgia rheumatica, Sarkoidose, Sklerose, primärer biliärer Zirrhose, sklerosierender Cholangitis, Dermatitis, atopischer Dermatitis, Atherosklerose, Still-Krankheit, chronischobstruktiver Lungenerkrankung, Guillain-Barré-Syndrom, Typ 1-Diabetes mellitus, Graves-Krankheit, Addison-Krankheit, Raynaud-Phänomen, autoimmuner Hepatitis und Wiskott-Aldrich-Syndrom.

48. Pharmazeutische Zusammensetzung gemäß Anspruch 47 zur Verwendung bei der Behandlung von entzündlicher Darmerkrankung, Morbus Crohn oder Colitis ulcerosa.

49. Pharmazeutische Zusammensetzung gemäß Anspruch 47 zur Verwendung bei der Behandlung von Sklerose, Multipler Sklerose, Atherosklerose oder Sklerodermie.

50. Pharmazeutische Zusammensetzung gemäß Anspruch 47 zur Verwendung bei der Behandlung von Psoriasis oder Lupus erythematodes.

51. Pharmazeutische Zusammensetzung gemäß Anspruch 44 zur Verwendung als Medikament.

## Revendications

1. Variant de DcR3 comprenant une première région riche en cystéine chimérique ou une deuxième région riche en cystéine chimérique, dans lequel :
la première région riche en cystéine chimérique consiste en l'une quelconque des séquences d'acides aminés suivantes :
(a) une séquence d'acides aminés consistant en des acides aminés aux positions 1 à 164 à partir de l'extrémité N-terminale de la séquence d'acides aminés exposée dans SEQ ID NO: 26 ou 50 ;
(b) une séquence d'acides aminés consistant en des acides aminés aux positions 1 à 164 à partir de l'extrémité N-terminale de la séquence d'acides aminés exposée dans SEQ ID NO: 28 ou 52 ;
(c) une séquence d'acides aminés consistant en des acides aminés aux positions 1 à 164 à partir de l'extrémité N-terminale de la séquence d'acides aminés exposée dans SEQ ID NO: 30 ou 54 ; et
(d) une séquence d'acides aminés consistant en des acides aminés aux positions 1 à 164 à partir de l'extrémité N-terminale de la séquence d'acides aminés exposée dans SEQ ID NO: 32 ou 56 ; et
la deuxième région riche en cystéine chimérique consiste en une séquence d'acides aminés (e) obtenue en introduisant, dans la séquence d'acides aminés de la première région riche en cystéine chimérique, une délétion, une substitution, une insertion ou une addition de 1 à 5 acides aminés.

2. Variant de DcR3 selon la revendication 1, dans lequel le variant de DcR3 comprend un ou plusieurs glycanes liés à un N-glycoside complexes.

3. Variant de DcR3 selon la revendication 1 ou la revendication 2, dans lequel le variant de DcR3 présente une activité neutralisante envers au moins un ou plusieurs de LIGHT, TL1A et FasL.

4. Variant de DcR3 selon l'une quelconque des revendications 1 à 3, dans lequel le variant de DcR3 présente une activité neutralisante envers la totalité de LIGHT, TL1A et FasL.

5. Variant de DcR3 selon l'une quelconque des revendications 1 à 3, dans lequel le variant de DcR3 ne présente pas d'activité neutralisante envers FasL et présente une activité neutralisante envers un ou plusieurs de LIGHT et TL1A.

6. Variant de DcR3 selon l'une quelconque des revendications 1 à 3 et de la revendication 5, dans lequel le variant de DcR3 ne présente pas d'activité neutralisante envers FasL et présente une activité neutralisante envers à la fois LIGHT et TL1A.

7. Variant de DcR3 selon l'une quelconque des revendications 1 à 6, dans lequel la première région riche en cystéine consiste en une séquence d'acides aminés consistant en des acides aminés aux positions 1 à 164 à partir de l'extrémité N-terminale de la séquence d'acides aminés exposée dans SEQ ID NO: 54.

8. Variant de DcR3 selon l'une quelconque des revendications 1 à 7, dans lequel la séquence d'acides aminés (e) comprend une ou deux substitutions ou plus sélectionnées dans le groupe consistant en :
substitution de Glu en position 57 à partir de l'extrémité N-terminale de la séquence d'acides aminés (a), (b), (c) ou (d) par un autre acide aminé ;
substitution de Arg en position 58 à partir de l'extrémité N-terminale de la séquence d'acides aminés (a), (b), (c) ou (d) par un autre acide aminé ; et
substitution de Arg en position 60 à partir de l'extrémité N-terminale de la séquence d'acides aminés (a), (b), (c) ou (d) par un autre acide aminé.

9. Variant de DcR3 selon l'une quelconque des revendications 1 à 8, dans lequel la séquence d'acides aminés (e) comprend la substitution de Glu en position 57 et de Arg en position 58 à partir de l'extrémité N-terminale de la séquence d'acides aminés (a), (b), (c) ou (d) par d'autres acides aminés.

10. Variant de DcR3 selon l'une quelconque des revendications 1 à 9, dans lequel la séquence d'acides aminés (e) comprend une ou deux substitutions ou plus sélectionnées dans le groupe consistant en :
substitution de Glu en position 57 à partir de l'extrémité N-terminale de la séquence d'acides aminés (a), (b), (c) ou (d) par Lys, Leu, Arg, Val, Ala, Phe, His, Ile ou Met ;
substitution de Arg en position 58 à partir de l'extrémité N-terminale de la séquence d'acides aminés (a), (b), (c) ou (d) par Asp, Glu ou Thr ; et
substitution de Arg en position 60 à partir de l'extrémité N-terminale de la séquence d'acides aminés (a), (b), (c) ou (d) par Lys.

11. Variant de DcR3 selon l'une quelconque des revendications 1 à 10, dans lequel la séquence d'acides aminés (e) comprend la substitution de Glu en position 57 à partir de l'extrémité N-terminale de la séquence d'acides aminés (a), (b), (c) ou (d) par Lys, Leu, Arg, Val, Ala, Phe, His, Ile ou Met, et la substitution de Arg en position 58 à partir de l'extrémité N-terminale de la séquence d'acides aminés (a), (b), (c) ou (d) par Asp, Glu ou Thr.

12. Variant de DcR3 selon l'une quelconque des revendications 1 à 11, dans lequel la séquence d'acides aminés (e) comprend une substitution sélectionnée parmi les (f) à (i) suivantes :
(f) substitution de Asn aux positions 131 et 144 à partir de l'extrémité N-terminale de la séquence d'acides aminés (b), (c) ou (d) par d'autres acides aminés ;
(g) substitution de Asn aux positions 131, 144 et 157 à partir de l'extrémité N-terminale de la séquence d'acides aminés (b), (c) ou (d) par d'autres acides aminés ;
(h) substitution de Thr en position 133 et de Ser en position 146 à partir de l'extrémité N-terminale de la séquence d'acides aminés (b), (c) ou (d) par d'autres acides aminés ; et
(i) substitution de Thr en position 133, de Ser en position 146 et de Thr en position 159 à partir de l'extrémité N-terminale de la séquence d'acides aminés (b), (c) ou (d) par d'autres acides aminés.

13. Variant de DcR3 selon l'une quelconque des revendications 1 à 12, dans lequel la séquence d'acides aminés (e) comprend une substitution sélectionnée parmi les (f') à (i') suivantes :
(f) substitution de Asn aux positions 131 et 144 à partir de l'extrémité N-terminale de la séquence d'acides aminés (b), (c) ou (d) par Ser ;
(g') substitution de Asn aux positions 131, 144 et 157 à partir de l'extrémité N-terminale de la séquence d'acides aminés (b), (c) ou (d) par Ser ;
(h') substitution de Thr en position 133 et de Ser en position 146 à partir de l'extrémité N-terminale de la séquence d'acides aminés (b), (c) ou (d) par Ala ; et
(i') substitution de Thr en position 133, de Ser en position 146 et de Thr en position 159 à partir de l'extrémité N-terminale de la séquence d'acides aminés (b), (c) ou (d) par Ala.

14. Variant de DcR3 selon l'une quelconque des revendications 1 à 13, dans lequel la séquence d'acides aminés (e) est une séquence d'acides aminés consistant en des acides aminés aux positions 1 à 164 à partir de l'extrémité N-terminale d'une séquence d'acides aminés exposée dans SEQ ID NO: 58, 60, 62, 64, 66, 68, 70, 180, 182, 184, 186, 188, 270, 272, 274, 276, 278, 280, 282, 284 ou 286.

15. Variant de DcR3 selon l'une quelconque des revendications 1 à 14, dans lequel le variant de DcR3 est un variant de DcR3 comprenant la première ou deuxième région riche en cystéine chimérique, et une partie ou une totalité d'un domaine de liaison au sulfate d'héparane du DcR3 de type sauvage liée au côté C-terminal de la première ou deuxième région riche en cystéine chimérique.

16. Variant de DcR3 selon l'une quelconque des revendications 1 à 14, dans lequel le variant de DcR3 est un variant de DcR3 comprenant la première ou deuxième région riche en cystéine chimérique et ne comprenant pas un domaine de liaison au sulfate d'héparane du DcR3 de type sauvage.

17. Variant de DcR3 selon la revendication 15 ou la revendication 16, dans lequel le variant de DcR3 comprend l'une des séquences d'acides aminés sélectionnées parmi :
(I) une séquence d'acides aminés exposée dans SEQ ID NO: 26, 28, 30, 32, 34, 36, 38, 40, 42, 44 ou 46, ou une séquence d'acides aminés présentant une identité de 99 % ou plus avec la séquence d'acides aminés susmentionnée ; et
(II) une séquence d'acides aminés exposée dans SEQ ID NO: 50, 52, 54, 56, 58, 60, 62, 64, 66, 68, 70, 180, 182, 184, 186, 188, 270, 272, 274, 276, 278, 280, 282, 284 ou 286, ou une séquence d'acides aminés présentant une identité de 99 % ou plus avec la séquence d'acides aminés susmentionnée.

18. Variant de DcR3 selon l'une quelconque des revendications 1 à 17, dans lequel le variant de DcR3 comprend une séquence d'acides aminés exposée dans SEQ ID NO: 66 ou 186.

19. Variant de DcR3 selon l'une quelconque des revendications 1 à 18, dans lequel le variant de DcR3 comprend une région Fc dérivée d'un anticorps IgG1, IgG2 ou IgG4 humain, ou une région Fc mutée consistant en une séquence d'acides aminés obtenue en introduisant, dans une séquence d'acides aminés de la région Fc susmentionnée, une délétion, une substitution, une insertion ou une addition d'un ou de plusieurs acides aminés.

20. Variant de DcR3 selon la revendication 19, dans lequel la région Fc ou la région Fc mutée est liée au côté C-terminal de la première ou deuxième région riche en cystéine chimérique par l'intermédiaire d'une autre région ou d'un lieur.

21. Variant de DcR3 selon la revendication 19 ou la revendication 20, dans lequel la région Fc mutée comprend la substitution de Cys par Ser à la position 220 d'indice EU d'une séquence d'acides aminés d'une chaîne lourde d'IgG1 humaine.

22. Variant de DcR3 selon la revendication 21, dans lequel la région Fc mutée comprend la substitution de Leu par Ala à la position 234 d'indice EU, la substitution de Leu par Ala à la position 235 d'indice EU, et la substitution de Gly par Ala à la position 237 d'indice EU d'une séquence d'acides aminés d'une chaîne lourde d'IgG1 humaine.

23. Variant de DcR3 selon la revendication 21 ou la revendication 22, dans lequel la région Fc mutée comprend la substitution de Asn par Ala à la position 434 d'indice EU d'une séquence d'acides aminés d'une chaîne lourde d'IgG1 humaine.

24. Variant de DcR3 selon la revendication 21, dans lequel la région Fc mutée comprend la substitution de Met par Tyr à la position 252 d'indice EU, la substitution de Ser par Thr à la position 254 d'indice EU, et la substitution de Thr par Glu à la position 256 d'indice EU d'une séquence d'acides aminés d'une chaîne lourde d'IgG1 humaine.

25. Variant de DcR3 selon la revendication 19 ou la revendication 20, dans lequel la région Fc mutée comprend la substitution de Ser par Pro à la position 228 d'indice EU, la substitution de Leu par Glu à la position 235 d'indice EU, et la substitution de Arg par Lys à la position 409 d'indice EU d'une séquence d'acides aminés d'une chaîne lourde d'IgG4 humaine.

26. Variant de DcR3 selon la revendication 19 ou la revendication 20, dans lequel le variant de DcR3 comprend une région Fc mutée consistant en une séquence d'acides aminés exposée dans SEQ ID NO: 72, 74, 156, 158, 160, 162, 164, 166, 311, 312 ou 313, ou une séquence d'acides aminés présentant une identité de 90 % ou plus avec une séquence d'acides aminés exposée dans SEQ ID NO: 72, 74, 156, 158, 160, 162, 164, 166, 311, 312 ou 313.

27. Variant de DcR3 selon la revendication 19, dans lequel le variant de DcR3 comprend ou consiste en une séquence d'acides aminés exposée dans SEQ ID NO: 76, 78, 80, 82, 84, 86, 88, 90, 92, 94, 96, 98, 150, 168, 170, 172, 174, 176, 178, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 288, 290, 292, 294, 296, 298, 300, 302, 304, 314, 315, 316, 317, 318, 319, 320, 321, 322, 323, 324, 325, 326, 327, 328, 329, 330, 331, 332, 333, 334, 335, 336 ou 337, ou une séquence d'acides aminés présentant une identité de 99 % ou plus avec la séquence d'acides aminés susmentionnée.

28. Variant de DcR3 selon la revendication 19, dans lequel le variant de DcR3 comprend ou consiste en une séquence d'acides aminés obtenue en introduisant une substitution, une délétion, une insertion ou une addition de 1 acide aminé dans une séquence d'acides aminés exposée dans SEQ ID NO: 76, 78, 80, 82, 84, 86, 88, 90, 92, 94, 96, 98, 150, 168, 170, 172, 174, 176, 178, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 288, 290, 292, 294, 296, 298, 300, 302, 304, 314, 315, 316, 317, 318, 319, 320, 321, 322, 323, 324, 325, 326, 327, 328, 329, 330, 331, 332, 333, 334, 335, 336 ou 337.

29. Variant de DcR3 selon la revendication 27, dans lequel le variant de DcR3 comprend une séquence d'acides aminés exposée dans SEQ ID NO: 214, ou une séquence d'acides aminés présentant une identité de 99 % ou plus avec SEQ ID NO: 214.

30. Variant de DcR3 selon la revendication 27, dans lequel le variant de DcR3 comprend une séquence d'acides aminés exposée dans SEQ ID NO: 224, ou une séquence d'acides aminés présentant une identité de 99 % ou plus avec SEQ ID NO: 224.

31. Variant de DcR3 selon la revendication 27, dans lequel le variant de DcR3 comprend une séquence d'acides aminés exposée dans SEQ ID NO: 256, ou une séquence d'acides aminés présentant une identité de 99 % ou plus avec SEQ ID NO: 256.

32. Variant de DcR3 selon la revendication 27, dans lequel le variant de DcR3 comprend une séquence d'acides aminés exposée dans SEQ ID NO: 266, ou une séquence d'acides aminés présentant une identité de 99 % ou plus avec SEQ ID NO: 266.

33. Variant de DcR3 selon la revendication 27, dans lequel le variant de DcR3 consiste en une séquence d'acides aminés exposée dans SEQ ID NO: 214, ou en une séquence d'acides aminés présentant une identité de 99 % ou plus avec SEQ ID NO: 214.

34. Variant de DcR3 selon la revendication 27, dans lequel le variant de DcR3 consiste en une séquence d'acides aminés exposée dans SEQ ID NO: 224, ou en une séquence d'acides aminés présentant une identité de 99 % ou plus avec SEQ ID NO: 224.

35. Variant de DcR3 selon la revendication 27, dans lequel le variant de DcR3 consiste en une séquence d'acides aminés exposée dans SEQ ID NO: 256, ou en une séquence d'acides aminés présentant une identité de 99 % ou plus avec SEQ ID NO: 256.

36. Variant de DcR3 selon la revendication 27, dans lequel le variant de DcR3 consiste en une séquence d'acides aminés exposée dans SEQ ID NO: 266, ou en une séquence d'acides aminés présentant une identité de 99 % ou plus avec SEQ ID NO: 266.

37. ADN codant pour le variant de DcR3 selon l'une quelconque des revendications précédentes.

38. Vecteur comprenant l'ADN selon la revendication 37.

39. Transformant obtenu en introduisant le vecteur selon la revendication 38 dans une cellule hôte.

40. Transformant selon la revendication 39, dans lequel la cellule hôte est une cellule dérivée d'un mammifère.

41. Transformant selon la revendication 40, dans lequel la cellule dérivée d'un mammifère est une cellule CHO.

42. Procédé de production d'un variant de DcR3, dans lequel le procédé comprend la culture du transformant selon l'une quelconque des revendications 39 à 41 dans un milieu pour générer et accumuler le variant de DcR3, et la purification du variant de DcR3 à partir de la solution de culture obtenue.

43. Variant de DcR3 produit en utilisant le procédé de production selon la revendication 42.

44. Composition pharmaceutique comprenant le variant de DcR3 selon l'une quelconque des revendications 1 à 36 et de la revendication 43 en tant que principe actif.

45. Composition pharmaceutique selon la revendication 44, dans laquelle la composition pharmaceutique est un agent prophylactique ou thérapeutique pour une maladie auto-immune, une maladie inflammatoire ou une maladie allergique.

46. Composition pharmaceutique selon la revendication 44 pour une utilisation dans un procédé de prévention ou de traitement d'une maladie auto-immune, d'une maladie inflammatoire ou d'une maladie allergique.

47. Composition pharmaceutique selon la revendication 44 pour une utilisation dans le traitement d'une affection ou d'une maladie sélectionnée dans le groupe consistant en une maladie inflammatoire de l'intestin (IBD), un lupus érythémateux systémique, un psoriasis, une maladie chronique du greffon contre l'hôte, une maladie aiguë du greffon contre l'hôte, une maladie de Crohn, une colite ulcéreuse, une sclérose en plaques, une maladie cœliaque, un purpura thrombopénique thrombotique idiopathique, une myasthénie grave, un syndrome de Sjögren, une sclérodermie, un asthme, une uvéite, une hyperplasie épidermique, une alopécie areata, une maladie de Behçet, une artérite de Takayasu, une inflammation cartilagineuse, une dégradation osseuse, une arthrite, une arthrite juvénile, une polyarthrite rhumatoïde juvénile, une polyarthrite rhumatoïde juvénile pauciarticulaire, une polyarthrite rhumatoïde juvénile polyarticulaire, une polyarthrite rhumatoïde juvénile à début systémique, une spondylarthrite ankylosante juvénile, une arthrite entéropathique juvénile, une arthrite réactionnelle juvénile, un syndrome de Reiter juvénile, un syndrome d'enthésopathie et d'arthropathie séronégatives (syndrome SEA), une dermatomyosite juvénile, une arthrite psoriasique juvénile, une sclérodermie juvénile, un lupus érythémateux systémique juvénile, une vascularite juvénile, une polyarthrite rhumatoïde pauciarticulaire, une polyarthrite rhumatoïde polyarticulaire, une polyarthrite rhumatoïde à début systémique, une spondylarthrite ankylosante, une arthrite entéropathique, une arthrite réactionnelle, un syndrome de Reiter, une dermatomyosite, une arthrite psoriasique, une vascularite, une myosite, une polymyosite, une dermatomyosite, une ostéoarthrite, une périartérite noueuse, une granulomatose de Wegener, une artérite, une polymyalgie rhumatismale, une sarcoïdose, une sclérose, une cirrhose biliaire primitive, une cholangite sclérosante, une dermatite, une dermatite atopique, une athérosclérose, une maladie de Still, une bronchopneumopathie chronique obstructive, un syndrome de Guillain-Barré, un diabète sucré de type 1, une maladie de Basedow, une maladie d'Addison, un phénomène de Raynaud, une hépatite auto-immune et un syndrome de Wiskott-Aldrich.

48. Composition pharmaceutique selon la revendication 47 pour une utilisation dans le traitement d'une maladie inflammatoire de l'intestin, d'une maladie de Crohn ou d'une colite ulcéreuse.

49. Composition pharmaceutique selon la revendication 47 pour une utilisation dans le traitement d'une sclérose, d'une sclérose en plaques, d'une athérosclérose ou d'une sclérodermie.

50. Composition pharmaceutique selon la revendication 47 pour une utilisation dans le traitement d'un psoriasis ou d'un lupus érythémateux.

51. Composition pharmaceutique selon la revendication 44 pour une utilisation en tant que médicament.
